# EUROPEAN PATENT SPECIFICATION

(11) **EP 1 274 850 B1**
(45) Date of publication and mention of the grant of the patent: **20.06.2007**
(21) Application number: 01927055.2
(22) Date of filing: 13.04.2001
(51) Int. Cl.: C12N 15/60, C12N 15/82, A01H 5/00, A23K 1/00, C11B 1/00, C12P 17/06

(54) **NUCLEIC ACID SEQUENCES TO PROTEINS INVOLVED IN TOCOPHEROL SYNTHESIS**
NUKELINSÄURESEQUENZEN FÜR PROTEINE, DIE AN DER TOCOPHEROLBIOSYNTHESE BETEILIGT SIND
SEQUENCES NUCLEOTIDIQUES DE PROTEINES INTERVENANT DANS LA SYNTHESE DU TOCOPHEROL

(30) Priority: 14.04.2000 US 549848; 14.10.2000 US 688069
(43) Date of publication of application: 15.01.2003
(73) Proprietor: Calgene LLC, Davis, CA 95626 (US)
(72) Inventor: SUBRAMANIAM, Sai, S., Sharon, MA 02067 (US); SLATER, Steven, C., Acton, MA 01720 (US); KARBERG, Katherine, St.Louis, Missouri 63131 (US); CHEN, Ridong, Schaumburg, Illinois 60193 (US); VALENTIN, Henry, E., Chesterfield, Missouri 63017 (US); WONG, Yun-Hua, Huang, Chesterfield, MO 63017 (US)
(74) Representative: UEXKÜLL & STOLBERG
(86) International application number: PCT/US2001/012334
(87) International publication number: WO 2001/079472

(56) References cited:
- EP-A- 0 531 639
- WO-A-00/10380
- WO-A-00/63391
- WO-A-99/04622
- GB-A- 560 529
- DATABASE TREMBL DATABASE [Online] 1 August 1998 (1998-08-01) BEVAN, M., ET AL. : "EU Arabidopsis sequencing project - Arabidopsis thaliana DNA chromosome 4, BAC clone F4D11 (ESSAII project); " XP002193029
- DATABASE EMBL SEQUENCE DATABASE [Online] 31 October 1996 (1996-10-31) KANEKO, T., ET AL. : "Sequence analysis of the genome of the unicellular cyanobacterium Synechocystis sp. strain PCC6803. I. Sequence features in the 1 Mb region from map positions 64% to 92% of the genome; " XP002193030
- DATABASE TREMBL DATABASE [Online] 1 February 1997 (1997-02-01) KANEKO, T., ET AL. : "Sequence analysis of the genome of the unicellular cyanobacterium Synechocystis sp. strain PCC6803. II. Sequence determination of theentire genome and assignment of potential protein-coding regions - hypothetical protein slr1737 - Synechocystis sp. (strain PCC 6803)" XP002193031
- DATABASE EMBL SEQUENCE DATABASE [Online] 9 September 1999 (1999-09-09) CHEN, J., ET AL. : "Arabidopsis thaliana Gene Expression MicroArray - 701673779 A. thaliana, Columbia Col-0, inflorescence-1 Arabidopsis thaliana cDNA clone 701673779, mRNA sequence" XP002193032
- STOCKER A ET AL: "Identification of the Tocopherol-cyclase in the blue-green algae Anabaena variabilis Kützing (Cyanobacteria)" HELVETICA CHIMICA ACTA, VERLAG HELVETICA CHIMICA ACTA. BASEL, CH, vol. 76, 1993, pages 1729-1738, XP002134836 ISSN: 0018-019X
- ARANGO YOLANDA ET AL: "Tocopherol synthesis from homogentisate in Capsicum anuum L. (yellow pepper) chromoplast membranes: Evidence for tocopherol cyclase." BIOCHEMICAL JOURNAL, vol. 336, no. 3, 15 December 1998 (1998-12-15), pages 531-533, XP002193027 ISSN: 0264-6021
- STOCKER, A. , ET AL.: "the substrate specificity of tocopherol cyclase" BIOORGANIC & MEDICINAL CHEMISTRY, vol. 4, no. 7, 1996, pages 1129-1134, XP002193028

### Annex

### SEQUENCE LISTING

<110> Subramaniam, S.; Slater, S.; Karberg, K.; Chen, R.; Valentin, H.; Wong, Y.
<120> Nucleic Acid Sequences to Proteins Involved in Tocopherol Synthesis
<130> 16515.051
<140> PCT/US01/12334
   <141> 2001-04-13
<150> US 09/549,848
   <151> 2000-04-14
<150> US 09/688,069
   <151> 2000-10-14
<160> 114
<210> 1
   <211> 1182
   <212> DNA
   <213> Arabidopsis sp.
<400> 1
   atggagtctc tgctctctag ttcttctctt gtttccgctg ctggtgggtt ttgttggaag   60
   aagcagaatc taaagctcca ctctttatca gaaatccgag ttctgcgttg tgattcgagt  120
   aaagttgtcg caaaaccgaa gtttaggaac aatcttgtta ggcctgatgg tcaaggatct  180
   tcattgttgt tgtatccaaa acataagtcg agatttcggg ttaatgccac tgcgggtcag  240
   cctgaggctt tcgactcgaa tagcaaacag aagtctttta gagactcgtt agatgcgttt  300
   tacaggtttt ctaggcctca tacagttatt ggcacagtgc ttagcatttt atctgtatct  360
   ttcttagcag tagagaaggt ttctgatata tctcctttac ttttcactgg catcttggag  420
   gctgttgttg cagctctcat gatgaacatt tacatagttg ggctaaatca gttgtctgat  480
   gttgaaatag ataaggttaa caagccctat cttccattgg catcaggaga atattctgtt  540
   aacaccggca ttgcaatagt agcttccttc tccatcatga gtttctggct tgggtggatt  600
   gttggttcat ggccattgtt ctgggctctt tttgtgagtt tcatgctcgg tactgcatac  660
   tctatcaatt tgccactttt acggtggaaa agatttgcat tggttgcagc aatgtgtatc  720
   ctcgctgtcc gagctattat tgttcaaatc gccttttatc tacatattca gacacatgtg  780
   tttggaagac caatcttgtt cactaggcct cttattttcg ccactgcgtt tatgagcttt  840
   ttctctgtcg ttattgcatt gtttaaggat atacctgata tcgaagggga taagatattc  900
   ggaatccgat cattctctgt aactctgggt cagaaacggg tgttttggac atgtgttaca  960
   ctacttcaaa tggcttacgc tgttgcaatt ctagttggag ccacatctcc attcatatgg 1020
   agcaaagtca tctcggttgt gggtcatgtt atactcgcaa caactttgtg ggctcgagct 1080
   aagtccgttg atctgagtag caaaaccgaa ataacttcat gttatatgtt catatggaag 1140
   ctcttttatg cagagtactt gctgttacct tttttgaagt ga                     1182
<210> 2
   <211> 393
   <212> PRT
   <213> Arabidopsis sp.
<400> 2
   Met Glu Ser Leu Leu Ser Ser Ser Ser Leu Val Ser Ala Ala Gly Gly
    1               5                  10                  15
   Phe Cys Trp Lys Lys Gln Asn Leu Lys Leu His Ser Leu Ser Glu Ile
               20                  25                  30
   Arg Val Leu Arg Cys Asp Ser Ser Lys Val Val Ala Lys Pro Lys Phe
           35                  40                  45
   Arg Asn Asn Leu Val Arg Pro Asp Gly Gln Gly Ser Ser Leu Leu Leu
       50                  55                  60
   Tyr Pro Lys His Lys Ser Arg Phe Arg Val Asn Ala Thr Ala Gly Gln
   65 70 75 80
   Pro Glu Ala Phe Asp Ser Asn Ser Lys Gln Lys Ser Phe Arg Asp Ser
   85 90 95
   Leu Asp Ala Phe Tyr Arg Phe Ser Arg Pro His Thr Val Ile Gly Thr
   100 105 110
   Val Leu Ser Ile Leu Ser Val Ser Phe Leu Ala Val Glu Lys Val Ser
   115 120 125
   Asp Ile Ser Pro Leu Leu Phe Thr Gly Ile Leu Glu Ala Val Val Ala
   130 135 140
   Ala Leu Met Met Asn Ile Tyr Ile Val Gly Leu Asn Gln Leu Ser Asp
   145 150 155 160
   Val Glu Ile Asp Lys Val Asn Lys Pro Tyr Leu Pro Leu Ala Ser Gly
   165 170 175
   Glu Tyr Ser Val Asn Thr Gly Ile Ala Ile Val Ala Ser Phe Ser Ile
   180 185 190
   Met Ser Phe Trp Leu Gly Trp Ile Val Gly Ser Trp Pro Leu Phe Trp
   195 200 205
   Ala Leu Phe Val Ser Phe Met Leu Gly Thr Ala Tyr Ser Ile Asn Leu
   210 215 220
   Pro Leu Leu Arg Trp Lys Arg Phe Ala Leu Val Ala Ala Met Cys Ile
   225 230 235 240
   Leu Ala Val Arg Ala Ile Ile Val Gln Ile Ala Phe Tyr Leu His Ile
   245 250 255
   Gln Thr His Val Phe Gly Arg Pro Ile Leu Phe Thr Arg Pro Leu Ile
   260 265 270
   Phe Ala Thr Ala Phe Met Ser Phe Phe Ser Val Val Ile Ala Leu Phe
   275 280 285
   Lys Asp Ile Pro Asp Ile Glu Gly Asp Lys Ile Phe Gly Ile Arg Ser
   290 295 300
   Phe Ser Val Thr Leu Gly Gln Lys Arg Val Phe Trp Thr Cys Val Thr
   305 310 315 320
   Leu Leu Gln Met Ala Tyr Ala Val Ala Ile Leu Val Gly Ala Thr Ser
   325 330 335
   Pro Phe Ile Trp Ser Lys Val Ile Ser Val Val Gly His Val Ile Leu
   340 345 350
   Ala Thr Thr Leu Trp Ala Arg Ala Lys Ser Val Asp Leu Ser Ser Lys
   355 360 365
   Thr Glu Ile Thr Ser Cys Tyr Met Phe Ile Trp Lys Leu Phe Tyr Ala
   370 375 380
   Glu Tyr Leu Leu Leu Pro Phe Leu Lys
   385 390
<210> 3
   <211> 1224
   <212> DNA
   <213> Arabidopsis sp.
<400> 3
   atggcgtttt ttgggctctc ccgtgtttca agacggttgt tgaaatcttc cgtctccgta   60
   actccatctt cttcctctgc tcttttgcaa tcacaacata aatccttgtc caatcctgtg  120
   actacccatt acacaaatcc tttcactaag tgttatcctt catggaatga taattaccaa  180
   gtatggagta aaggaagaga attgcatcag gagaagtttt ttggtgttgg ttggaattac  240
   agattaattt gtggaatgtc gtcgtcttct tcggttttgg agggaaagcc gaagaaagat  300
   gataaggaga agagtgatgg tgttgttgtt aagaaagctt cttggataga tttgtattta  360
   ccagaagaag ttagaggtta tgctaagctt gctcgattgg ataaacccat tggaacttgg  420
   ttgcttgcgt ggccttgtat gtggtcgatt gcgttggctg ctgatcctgg aagccttcca  480
   agttttaaat atatggcttt atttggttgc ggagcattac ttcttagagg tgctggttgt  540
   actataaatg atctgcttga tcaggacata gatacaaagg ttgatcgtac aaaactaaga  600
   cctatcgcca gtggtctttt gacaccattt caagggattg gatttctcgg gctgcagttg  660
   cttttaggct tagggattct tctccaactt aacaattaca gccgtgtttt aggggcttca  720
   tctttgttac ttgtcttttc ctacccactt atgaagaggt ttacattttg gcctcaagcc  780
   tttttaggtt tgaccataaa ctggggagca ttgttaggat ggactgcagt taaaggaagc  840
   atagcaccat ctattgtact ccctctctat ctctccggag tctgctggac ccttgtttat  900
   gatactattt atgcacatca ggacaaagaa gatgatgtaa aagttggtgt taagtcaaca  960
   gcccttagat tcggtgataa tacaaagctt tggttaactg gatttggcac agcatccata 1020
   ggttttcttg cactttctgg attcagtgca gatctcgggt ggcaatatta cgcatcactg 1080
   gccgctgcat caggacagtt aggatggcaa atagggacag ctgacttatc atctggtgct 1140
   gactgcagta gaaaatttgt gtcgaacaag tggtttggtg ctattatatt tagtggagtt 1200
   gtacttggaa gaagttttca ataa                                        1224
<210> 4
   <211> 407
   <212> PRT
   <213> Arabidopsis sp.
<400> 4
   Met Ala Phe Phe Gly Leu Ser Arg Val Ser Arg Arg Leu Leu Lys Ser
   1               5                   10                  15
   Ser Val Ser Val Thr Pro Ser Ser Ser Ser Ala Leu Leu Gln Ser Gln
               20                  25                  30
   His Lys Ser Leu Ser Asn Pro Val Thr Thr His Tyr Thr Asn Pro Phe
           35                  40                  45
   Thr Lys Cys Tyr Pro Ser Trp Asn Asp Asn Tyr Gln Val Trp Ser Lys
       50                  55                  60
   Gly Arg Glu Leu His Gln Glu Lys Phe Phe Gly Val Gly Trp Asn Tyr
   65                  70                  75                  80
   Arg Leu Ile Cys Gly Met Ser Ser Ser Ser Ser Val Leu Glu Gly Lys
                   85                  90                  95
   Pro Lys Lys Asp Asp Lys Glu Lys Ser Asp Gly Val Val Val Lys Lys
               100                 105                 110
   Ala Ser Trp Ile Asp Leu Tyr Leu Pro Glu Glu Val Arg Gly Tyr Ala
           115                 120                 125
   Lys Leu Ala Arg Leu Asp Lys Pro Ile Gly Thr Trp Leu Leu Ala Trp
       130                 135                 140
   Pro Cys Met Trp Ser Ile Ala Leu Ala Ala Asp Pro Gly Ser Leu Pro
   145                 150                 155                 160
   Ser Phe Lys Tyr Met Ala Leu Phe Gly Cys Gly Ala Leu Leu Leu Arg
                   165                 170                 175
   Gly Ala Gly Cys Thr Ile Asn Asp Leu Leu Asp Gln Asp Ile Asp Thr
               180                 185                 190
   Lys Val Asp Arg Thr Lys Leu Arg Pro Ile Ala Ser Gly Leu Leu Thr
           195                 200                 205
   Pro Phe Gln Gly Ile Gly Phe Leu Gly Leu Gln Leu Leu Leu Gly Leu
       210                 215                 220
   Gly Ile Leu Leu Gln Leu Asn Asn Tyr Ser Arg Val Leu Gly Ala Ser
   225                 230                 235                 240
   Ser Leu Leu Leu Val Phe Ser Tyr Pro Leu Met Lys Arg Phe Thr Phe
                   245                 250                 255
   Trp Pro Gln Ala Phe Leu Gly Leu Thr Ile Asn Trp Gly Ala Leu Leu
               260                 265                 270
   Gly Trp Thr Ala Val Lys Gly Ser Ile Ala Pro Ser Ile Val Leu Pro
           275                 280                 285
   Leu Tyr Leu Ser Gly Val Cys Trp Thr Leu Val Tyr Asp Thr Ile Tyr
       290                 295                 300
   Ala His Gln Asp Lys Glu Asp Asp Val Lys Val Gly Val Lys Ser Thr
   305                 310                 315                 320
   Ala Leu Arg Phe Gly Asp Asn Thr Lys Leu Trp Leu Thr Gly Phe Gly
                   325                 330                 335
   Thr Ala Ser Ile Gly Phe Leu Ala Leu Ser Gly Phe Ser Ala Asp Leu
               340                 345                 350
   Gly Trp Gln Tyr Tyr Ala Ser Leu Ala Ala Ala Ser Gly Gln Leu Gly
           355                 360                 365
   Trp Gln Ile Gly Thr Ala Asp Leu Ser Ser Gly Ala Asp Cys Ser Arg
       370                 375                 380
   Lys Phe Val Ser Asn Lys Trp Phe Gly Ala Ile Ile Phe Ser Gly Val
   385                 390                 395                 400
   Val Leu Gly Arg Ser Phe Gln
                   405
<210> 5
   <211> 1296
   <212> DNA
   <213> Arabidopsis sp.
<400> 5
   atgtggcgaa gatctgttgt ttctcgttta tcttcaagaa tctctgtttc ttcttcgtta   60
   ccaaacccta gactgattcc ttggtcccgc gaattatgtg ccgttaatag cttctcccag  120
   cctccggtct cgacggaatc aactgctaag ttagggatca ctggtgttag atctgatgcc  180
   aatcgagttt ttgccactgc tactgccgcc gctacagcta cagctaccac cggtgagatt  240
   tcgtctagag ttgcggcttt ggctggatta gggcatcact acgctcgttg ttattgggag  300
   ctttctaaag ctaaacttag tatgcttgtg gttgcaactt ctggaactgg gtatattctg  360
   ggtacgggaa atgctgcaat tagcttcccg gggctttgtt acacatgtgc aggaaccatg  420
   atgattgctg catctgctaa ttccttgaat cagatttttg agataagcaa tgattctaag  480
   atgaaaagaa cgatgctaag gccattgcct tcaggacgta ttagtgttcc acacgctgtt  540
   gcatgggcta ctattgctgg tgcttctggt gcttgtttgt tggccagcaa gactaatatg  600
   ttggctgctg gacttgcatc tgccaatctt gtactttatg cgtttgttta tactccgttg  660
   aagcaacttc accctatcaa tacatgggtt ggcgctgttg ttggtgctat cccacccttg  720
   cttgggtggg cggcagcgtc tggtcagatt tcatacaatt cgatgattct tccagctgct  780
   ctttactttt ggcagatacc tcattttatg gcccttgcac atctctgccg caatgattat  840
   gcagctggag gttacaagat gttgtcactc tttgatccgt cagggaagag aatagcagca  900
   gtggctctaa ggaactgctt ttacatgatc cctctcggtt tcatcgccta tgactggggg  960
   ttaacctcaa gttggttttg cctcgaatca acacttctca cactagcaat cgctgcaaca 1020
   gcattttcat tctaccgaga ccggaccatg cataaagcaa ggaaaatgtt ccatgccagt 1080
   cttctcttcc ttcctgtttt catgtctggt cttcttctac accgtgtctc taatgataat 1140
   cagcaacaac tcgtagaaga agccggatta acaaattctg tatctggtga agtcaaaact 1200
   cagaggcgaa agaaacgtgt ggctcaacct ccggtggctt atgcctctgc tgcaccgttt 1260
   cctttcctcc cagctccttc cttctactct ccatga                          1296
<210> 6
   <211> 431
   <212> PRT
   <213> Arabidopsis sp.
<400> 6
   Met Trp Arg Arg Ser Val Val Tyr Arg Phe Ser Ser Arg Ile Ser Val
   1 5 10 15
   Ser Ser Ser Leu Pro Asn Pro Arg Leu Ile Pro Trp Ser Arg Glu Leu
   20 25 30
   Cys Ala Val Asn Ser Phe Ser Gln Pro Pro Val Ser Thr Glu Ser Thr
   35 40 45
   Ala Lys Leu Gly Ile Thr Gly Val Arg Ser Asp Ala Asn Arg Val Phe
   50 55 60
   Ala Thr Ala Thr Ala Ala Ala Thr Ala Thr Ala Thr Thr Gly Glu Ile
   65 70 75 80
   Ser Ser Arg Val Ala Ala Leu Ala Gly Leu Gly His His Tyr Ala Arg
   85 90 95
   Cys Tyr Trp Glu Leu Ser Lys Ala Lys Leu Ser Met Leu Val Val Ala
   100 105 110
   Thr Ser Gly Thr Gly Tyr Ile Leu Gly Thr Gly Asn Ala Ala Ile Ser
   115 120 125
   Phe Pro Gly Leu Cys Tyr Thr Cys Ala Gly Thr Met Met Ile Ala Ala
   130 135 140
   Ser Ala Asn Ser Leu Asn Gln Ile Phe Glu Ile Ser Asn Asp Ser Lys
   145 150 155 160
   Met Lys Arg Thr Met Leu Arg Pro Leu Pro Ser Gly Arg Ile Ser Val
   165 170 175
   Pro His Ala Val Ala Trp Ala Thr Ile Ala Gly Ala Ser Gly Ala Cys
   180 185 190
   Leu Leu Ala Ser Lys Thr Asn Met Leu Ala Ala Gly Leu Ala Ser Ala
   195 200 205
   Asn Leu Val Leu Tyr Ala Phe Val Tyr Thr Pro Leu Lys Gln Leu His
   210 215 220
   Pro Ile Asn Thr Trp Val Gly Ala Val Val Gly Ala Ile Pro Pro Leu
   225 230 235 240
   Leu Gly Trp Ala Ala Ala Ser Gly Gln Ile Ser Tyr Asn Ser Met Ile
   245 250 255
   Leu Pro Ala Ala Leu Tyr Phe Trp Gln Ile Pro His Phe Met Ala Leu
   260 265 270
   Ala His Leu Cys Arg Asn Asp Tyr Ala Ala Gly Gly Tyr Lys Met Leu
   275 280 285
   Ser Leu Phe Asp Pro Ser Gly Lys Arg Ile Ala Ala Val Ala Leu Arg
       290                 295                 300
   Asn Cys Phe Tyr Met Ile Pro Leu Gly Phe Ile Ala Tyr Asp Trp Gly
   305                 310                 315                 320
   Leu Thr Ser Ser Trp Phe Cys Leu Glu Ser Thr Leu Leu Thr Leu Ala
                   325                 330                 335
   Ile Ala Ala Thr Ala Phe Ser Phe Tyr Arg Asp Arg Thr Met His Lys
               340                 345                 350
   Ala Arg Lys Met Phe His Ala Ser Leu Leu Phe Leu Pro Val Phe Met
           355                 360                 365
   Ser Gly Leu Leu Leu His Arg Val Ser Asn Asp Asn Gln Gln Gln Leu
       370                 375                 380
   Val Glu Glu Ala Gly Leu Thr Asn Ser Val Ser Gly Glu Val Lys Thr
   385                 390                 395                 400
   Gln Arg Arg Lys Lys Arg Val Ala Gln Pro Pro Val Ala Tyr Ala Ser
                   405                 410                 415
   Ala Ala Pro Phe Pro Phe Leu Pro Ala Pro Ser Phe Tyr Ser Pro
               420                 425                 430
<210> 7
   <211> 479
   <212> DNA
   <213> Arabidopsis sp.
<400> 7
   ggaaactccc ggagcacctg tttgcaggta ccgctaacct taatcgataa tttatttctc    60
   ttgtcaggaa ttatgtaagt ctggtggaag gctcgcatac catttttgca ttgcctttcg    120
   ctatgatcgg gtttactttg ggtgtgatga gaccaggcgt ggctttatgg tatggcgaaa   180
   acccattttt atccaatgct gcattccctc ccgatgattc gttctttcat tcctatacag   240
   gtatcatgct gataaaactg ttactggtac tggtttgtat ggtatcagca agaagcgcgg   300
   cgatggcgtt taaccggtat ctcgacaggc attttgacgc gaagaacccg cgtactgcca   360
   tccgtgaaat acctgcgggc gtcatatctg ccaacagtgc gctggtgttt acgataggct   420
   gctgcgtggt attctgggtg gcctgttatt tcattaacac gatctgtttt tacctggcg    479
<210> 8
   <211> 551
   <212> DNA
   <213> Arabidopsis sp.
<220>
   <221> misc_feature
   <222> (1) ... (551)
   <223> n = A,T,C or G
<400> 8
<210> 9
   <211> 297
   <212> PRT
   <213> Arabidopsis sp.
<400> 9
   Met Val Leu Ala Glu Val Pro Lys Leu Ala Ser Ala Ala Glu Tyr Phe
   1               5                   10                  15
   Phe Lys Arg Gly Val Gln Gly Lys Gln Phe Arg Ser Thr Ile Leu Leu
               20                  25                  30
   Leu Met Ala Thr Ala Leu Asn Val Arg Val Pro Glu Ala Leu Ile Gly
           35                  40                  45
   Glu Ser Thr Asp Ile Val Thr Ser Glu Leu Arg Val Arg Gln Arg Gly
       50                  55                  60
   Ile Ala Glu Ile Thr Glu Met Ile His Val Ala Ser Leu Leu His Asp
   65                  70                  75                  80
   Asp Val Leu Asp Asp Ala Asp Thr Arg Arg Gly Val Gly Ser Leu Asn
                   85                  90                  95
   Val Val Met Gly Asn Lys Val Val Ala Leu Leu Ala Thr Ala Val Glu
               100                 105                 110
   His Leu Val Thr Gly Glu Thr Met Glu Ile Thr Ser Ser Thr Glu Gln
           115                 120                 125
   Arg Tyr Ser Met Asp Tyr Tyr Met Gln Lys Thr Tyr Tyr Lys Thr Ala
       130                 135                 140
   Ser Leu Ile Ser Asn Ser Cys Lys Ala Val Ala Val Leu Thr Gly Gln
   145                 150                 155                 160
   Thr Ala Glu Val Ala Val Leu Ala Phe Glu Tyr Gly Arg Asn Leu Gly
                   165                 170                 175
   Leu Ala Phe Gln Leu Ile Asp Asp Ile Leu Asp Phe Thr Gly Thr Ser
               180                 185                 190
   Ala Ser Leu Gly Lys Gly Ser Leu Ser Asp Ile Arg His Gly Val Ile
           195                 200                 205
   Thr Ala Pro Ile Leu Phe Ala Met Glu Glu Phe Pro Gln Leu Arg Glu
       210                 215                 220
   Val Val Asp Gln Val Glu Lys Asp Pro Arg Asn Val Asp Ile Ala Leu
   225                 230                 235                 240
   Glu Tyr Leu Gly Lys Ser Lys Gly Ile Gln Arg Ala Arg Glu Leu Ala
                   245                 250                 255
   Met Glu His Ala Asn Leu Ala Ala Ala Ala Ile Gly Ser Leu Pro Glu
               260                 265                 270
   Thr Asp Asn Glu Asp Val Lys Arg Ser Arg Arg Ala Leu Ile Asp Leu
           275                 280                 285
   Thr His Arg Val Ile Thr Arg Asn Lys
       290                 295
<210> 10
   <211> 561
   <212> DNA
   <213> Arabidopsis sp.
<400> 10
   aagcgcatcc gtcctcttct acgattgccg ccagccgcat gtatggctgc ataaccgacc   60
   gcccctatcc gctcgcggcc gcggtcgaat tcattcacac cgcgacgctg ctgcatgacg  120
   acgtcgtcga tgaaagcgat ttgcgccgcg gccgcgaaag cgcgcataag gttttcggca  180
   atcaggcgag cgtgctcgtc ggcgatttcc ttttctcccg cgccttccag ctgatggtgg  240
   aagacggctc gctcgacgcg ctgcgcattc tctcggatgc ctccgccgtg atcgcgcagg  300
   gcgaagtgat gcagctcggc accgcgcgca atcttgaaac caatatgagc cagtatctcg  360
   atgtgatcag cgcgaagacc gccgcgctct ttgccgccgc ctgcgaaatc ggcccggtga  420
   tggcgaacgc gaaggcggaa gatgctgccg cgatgtgcga atacggcatg aatctcggta  480
   tcgccttcca gatcatcgac gaccttctcg attacggcac cggcggccac gccgagcttg  540
   gcaagaacac gggcgacgat t                                              561
<210> 11
   <211> 966
   <212> DNA
   <213> Arabidopsis sp.
<400> 11
   atggtacttg ccgaggttcc aaagcttgcc tctgctgctg agtacttctt caaaaggggt   60
   gtgcaaggaa aacagtttcg ttcaactatt ttgctgctga tggcgacagc tctgaatgta  120
   cgcgttccag aagcattgat tggggaatca acagatatag tcacatcaga attacgcgta  180
   aggcaacggg gtattgctga aatcactgaa atgatacacg tcgcaagtct actgcacgat  240
   gatgtcttgg atgatgccga tacaaggcgt ggtgttggtt ccttaaatgt tgtaatgggt  300
   aacaagatgt cggtattagc aggagacttc ttgctctccc gggcttgtgg ggctctcgct  360
   gctttaaaga acacagaggt tgtagcatta cttgcaactg ctgtagaaca tcttgttacc  420
   ggtgaaacca tggaaataac tagttcaacc gagcagcgtt atagtatgga ctactacatg  480
   cagaagacat attataagac agcatcgcta atctctaaca gctgcaaagc tgttgccgtt  540
   ctcactggac aaacagcaga agttgccgtg ttagcttttg agtatgggag gaatctgggt  600
   ttagcattcc aattaataga cgacattctt gatttcacgg gcacatctgc ctctctcgga  660
   aagggatcgt tgtcagatat tcgccatgga gtcataacag ccccaatcct ctttgccatg  720
   gaagagtttc ctcaactacg cgaagttgtt gatcaagttg aaaaagatcc taggaatgtt  780
   gacattgctt tagagtatct tgggaagagc aagggaatac agagggcaag agaattagcc  840
   atggaacatg cgaatctagc agcagctgca atcgggtctc tacctgaaac agacaatgaa  900
   gatgtcaaaa gatcgaggcg ggcacttatt gacttgaccc atagagtcat caccagaaac  960
   aagtga                                                               966
<210> 12
   <211> 321
   <212> PRT
   <213> Arabidopsis sp.
<400> 12
   Met Val Leu Ala Glu Val Pro Lys Leu Ala Ser Ala Ala Glu Tyr Phe
   1               5                   10                  15
   Phe Lys Arg Gly Val Gln Gly Lys Gln Phe Arg Ser Thr Ile Leu Leu
               20                  25                  30
   Leu Met Ala Thr Ala Leu Asn Val Arg Val Pro Glu Ala Leu Ile Gly
           35                  40                  45
   Glu Ser Thr Asp Ile Val Thr Ser Glu Leu Arg Val Arg Gln Arg Gly
       50                  55                  60
   Ile Ala Glu Ile Thr Glu Met Ile His Val Ala Ser Leu Leu His Asp
   65                  70                  75                  80
   Asp Val Leu Asp Asp Ala Asp Thr Arg Arg Gly Val Gly Ser Leu Asn
                   85                  90                  95
   Val Val Met Gly Asn Lys Met Ser Val Leu Ala Gly Asp Phe Leu Leu
   100 105 110
   Ser Arg Ala Cys Gly Ala Leu Ala Ala Leu Lys Asn Thr Glu Val Val
   115 120 125
   Ala Leu Leu Ala Thr Ala Val Glu His Leu Val Thr Gly Glu Thr Met
   130 135 140
   Glu Ile Thr Ser Ser Thr Glu Gln Arg Tyr Ser Met Asp Tyr Tyr Met
   145 150 155 160
   Gln Lys Thr Tyr Tyr Lys Thr Ala Ser Leu Ile Ser Asn Ser Cys Lys
   165 170 175
   Ala Val Ala Val Leu Thr Gly Gln Thr Ala Glu Val Ala Val Leu Ala
   180 185 190
   Phe Glu Tyr Gly Arg Asn Leu Gly Leu Ala Phe Gln Leu Ile Asp Asp
   195 200 205
   Ile Leu Asp Phe Thr Gly Thr Ser Ala Ser Leu Gly Lys Gly Ser Leu
   210 215 220
   Ser Asp Ile Arg His Gly Val Ile Thr Ala Pro Ile Leu Phe Ala Met
   225 230 235 240
   Glu Glu Phe Pro Gln Leu Arg Glu Val Val Asp Gln Val Glu Lys Asp
   245 250 255
   Pro Arg Asn Val Asp Ile Ala Leu Glu Tyr Leu Gly Lys Ser Lys Gly
   260 265 270
   Ile Gln Arg Ala Arg Glu Leu Ala Met Glu His Ala Asn Leu Ala Ala
   275 280 285
   Ala Ala Ile Gly Ser Leu Pro Glu Thr Asp Asn Glu Asp Val Lys Arg
   290 295 300
   Ser Arg Arg Ala Leu Ile Asp Leu Thr His Arg Val Ile Thr Arg Asn
   305 310 315 320
   Lys
<210> 13
   <211> 621
   <212> DNA
   <213> Arabidopsis sp.
<400> 13
   gctttctcct ttgctaattc ttgagctttc ttgatcccac cgcgatttct aactatttca    60
   atcgcttctt caagcgatcc aggctcacaa aactcagact caatgatctc tcttagcctt   120
   ggctcattct ctagcgcgaa gatcactggc gccgttatgt tacctttggc taagtcatta   180
   gctgcaggct tacctaactg ctctgtggac tgagtgaagt ccagaatgtc atcaactact   240
   tgaaaagata aaccgagatt cttcccgaac tgatacattt gctctgcgac cttgctttcg   300
   actttactga aaattgctgc tcctttggtg cttgcagcta ctaatgaagc tgtcttgtag   360
   taactcttta gcatgtagtc atcaagcttg acatcacaat cgaataaact cgatgcttgc   420
   tttatctcac cgcttgcaaa atctttgatc acctgcaaaa agataaatca agattcagac   480
   caaatgttct ttgtattgag tagcttcatc taatctcaga aaggaatatt acctgactta   540
   tgagcttaat gacttcaagg ttttcgagat ttgtaagtac catgatgctt gagcaacatg   600
   aaatccccag ctaatacagc t                                             621
<210> 14
   <211> 741
   <212> DNA
   <213> Arabidopsis sp.
<400> 14
   ggtgagtttt gttaatagtt atgagattca tctatttttg tcataaaatt gtttggtttg    60
   gtttaaactc tgtgtataat tgcaggaaag gaaacagttc atgagctttt cggcacaaga   120
   gtagcggtgc tagctggaga tttcatgttt gctcaagcgt catggtactt agcaaatctc   180
   gagaatcttg aagttattaa gctcatcagt caggtactta gttactctta cattgttttt   240
   ctatgaggtt gagctatgaa tctcatttcg ttgaataatg ctgtgcctca aacttttttt   300
   catgttttca ggtgatcaaa gactttgcaa gcggagagat aaagcaggcg tccagcttat   360
   ttgactgcga caccaagctc gacgagtact tactcaaaag tttctacaag acagcctctt   420
   tagtggctgc gagcaccaaa ggagctgcca ttttcagcag agttgagcct gatgtgacag   480
   aacaaatgta cgagtttggg aagaatctcg gtctctcttt ccagatagtt gatgatattt   540
   tggatttcac tcagtcgaca gagcagctcg ggaagccagc agggagtgat ttggctaaag   600
   gtaacttaac agcacctgtg attttcgctc tggagaggga gccaaggcta agagagatca   660
   ttgagtcaaa gttctgtgag gcgggttctc tggaagaagc gattgaagcg gtgacaaaag   720
   gtggggggat taagagagca c                                              741
<210> 15
   <211> 10B7
   <212> DNA
   <213> Arabidopsis sp.
<400> 15
<210> 16
   <211> 1164
   <212> DNA
   <213> Arabidopsis sp.
<400> 16
   atgacttcga ttctcaacac tgtctccacc atccactctt ccagagttac ctccgtcgat   60
   cgagtcggag tcctctctct tcggaattcg gattccgttg agttcactcg ccggcgttct  120
   ggtttctcga cgttgatcta cgaatcaccc gggcggagat ttgttgtgcg tgcggcggag  180
   actgatactg ataaagttaa atctcagaca cctgacaagg caccagccgg tggttcaagc  240
   attaaccagc ttctcggtat caaaggagca tctcaagaaa ctaataaatg gaagattcgt  300
   cttcagctta caaaaccagt cacttggcct ccactggttt ggggagtcgt ctgtggtgct  360
   gctgcttcag ggaactttca ttggacccca gaggatgttg ctaagtcgat tctttgcatg  420
   atgatgtctg gtccttgtct tactggctat acacagacaa tcaacgactg gtatgataga  480
   gatatcgacg caattaatga gccatatcgt ccaattccat ctggagcaat atcagagcca  540
   gaggttatta cacaagtctg ggtgctatta ttgggaggtc ttggtattgc tggaatatta  600
   gatgtgtggg cagggcatac cactcccact gtcttctatc ttgctttggg aggatcattg  660
   ctatcttata tatactctgc tccacctctt aagctaaaac aaaatggatg ggttggaaat  720
   tttgcacttg gagcaagcta tattagtttg ccatggtggg ctggccaagc attgtttggc  780
   actcttacgc cagatgttgt tgttctaaca ctcttgtaca gcatagctgg gttaggaata  840
   gccattgtta acgacttcaa aagtgttgaa ggagatagag cattaggact tcagtctctc  900
   ccagtagctt ttggcaccga aactgcaaaa tggatatgcg ttggtgctat agacattact  960
   cagctttctg ttgccggata tctattagca tctgggaaac cttattatgc gttggcgttg 1020
   gttgctttga tcattcctca gattgtgttc cagtttaaat actttctcaa ggaccctgtc 1080
   aaatacgacg tcaagtacca ggcaagcgcg cagccattct tggtgctcgg aatatttgta 1140
   acggcattag catcgcaaca ctga                                        1164
<210> 17
   <211> 387
   <212> PRT
   <213> Arabidopsis sp.
<400> 17
   Met Thr Ser Ile Leu Asn Thr Val Ser Thr Ile His Ser Ser Arg Val
    1               5                   10                  15
   Thr Ser Val Asp Arg Val Gly Val Leu Ser Leu Arg Asn Ser Asp Ser
               20                  25                  30
   Val Glu Phe Thr Arg Arg Arg Ser Gly Phe Ser Thr Leu Ile Tyr Glu
           35                  40                  45
   Ser Pro Gly Arg Arg Phe Val Val Arg Ala Ala Glu Thr Asp Thr Asp
       50                  55                  60
   Lys Val Lys Ser Gln Thr Pro Asp Lys Ala Pro Ala Gly Gly Ser Ser
   65                  70                  75                  80
   Ile Asn Gln Leu Leu Gly Ile Lys Gly Ala Ser Gln Glu Thr Asn Lys
                   85                  90                  95
   Trp Lys Ile Arg Leu Gln Leu Thr Lys Pro Val Thr Trp Pro Pro Leu
               100                 105                 110
   Val Trp Gly Val Val Cys Gly Ala Ala Ala Ser Gly Asn Phe His Trp
           115                 120                 125
   Thr Pro Glu Asp Val Ala Lys Ser Ile Leu Cys Met Met Met Ser Gly
       130                 135                 140
   Pro Cys Leu Thr Gly Tyr Thr Gln Thr Ile Asn Asp Trp Tyr Asp Arg
   145                 150                 155                 160
   Asp Ile Asp Ala Ile Asn Glu Pro Tyr Arg Pro Ile Pro Ser Gly Ala
                   165                 170                 175
   Ile Ser Glu Pro Glu Val Ile Thr Gln Val Trp Val Leu Leu Leu Gly
               180                 185                 190
   Gly Leu Gly Ile Ala Gly Ile Leu Asp Val Trp Ala Gly His Thr Thr
           195                 200                 205
   Pro Thr Val Phe Tyr Leu Ala Leu Gly Gly Ser Leu Leu Ser Tyr Ile
       210                 215                 220
   Tyr Ser Ala Pro Pro Leu Lys Leu Lys Gln Asn Gly Trp Val Gly Asn
   225                 230                 235                 240
   Phe Ala Leu Gly Ala Ser Tyr Ile Ser Leu Pro Trp Trp Ala Gly Gln
                   245                 250                 255
   Ala Leu Phe Gly Thr Leu Thr Pro Asp Val Val Val Leu Thr Leu Leu
               260                 265                 270
   Tyr Ser Ile Ala Gly Leu Gly Ile Ala Ile Val Asn Asp Phe Lys Ser
           275                 280                 285
   Val Glu Gly Asp Arg Ala Leu Gly Leu Gln Ser Leu Pro Val Ala Phe
   290 295 300
   Gly Thr Glu Thr Ala Lys Trp Ile Cys Val Gly Ala Ile Asp Ile Thr
   305 310 315 320
   Gln Leu Ser Val Ala Gly Tyr Leu Leu Ala Ser Gly Lys Pro Tyr Tyr
   325 330 335
   Ala Leu Ala Leu Val Ala Leu Ile Ile Pro Gln Ile Val Phe Gln Phe
   340 345 350
   Lys Tyr Phe Leu Lys Asp Pro Val Lys Tyr Asp Val Lys Tyr Gln Ala
   355 360 365
   Ser Ala Gln Pro Phe Leu Val Leu Gly Ile Phe Val Thr Ala Leu Ala
   370 375 380
   Ser Gln His
   385
<210> 18
   <211> 981
   <212> DNA
   <213> Arabidopsis sp.
<400> 18
   atgttgttta gtggttcagc gatcccatta agcagcttct gctctcttcc ggagaaaccc   60
   cacactcttc ctatgaaact ctctcccgct gcaatccgat cttcatcctc atctgccccg  120
   gggtcgttga acttcgatct gaggacgtat tggacgactc tgatcaccga gatcaaccag  180
   aagctggatg aggccatacc ggtcaagcac cctgcgggga tctacgaggc tatgagatac  240
   tctgtactcg cacaaggcgc caagcgtgcc cctcctgtga tgtgtgtggc ggcctgcgag  300
   ctcttcggtg gcgatcgcct cgccgctttc cccaccgcct gtgccctaga aatggtgcac  360
   gcggcttcgt tgatacacga cgacctcccc tgtatggacg acgatcctgt gcgcagagga  420
   aagccatcta accacactgt ctacggctct ggcatggcca ttctcgccgg tgacgccctc  480
   ttcccactcg ccttccagca cattgtctcc cacacgcctc ctgaccttgt tccccgagcc  540
   accatcctca gactcatcac tgagattgcc cgcactgtcg gctccactgg tatggctgca  600
   ggccagtacg tcgaccttga aggaggtccc tttcctcttt cctttgttca ggagaagaaa  660
   ttcggagcca tgggtgaatg ctctgccgtg tgcggtggcc tattgggcgg tgccactgag  720
   gatgagctcc agagtctccg aaggtacggg agagccgtcg ggatgctgta tcaggtggtc  780
   gatgacatca ccgaggacaa gaagaagagc tatgatggtg gagcagagaa gggaatgatg  840
   gaaatggcgg aagagctcaa ggagaaggcg aagaaggagc ttcaagtgtt tgacaacaag  900
   tatggaggag gagacacact tgttcctctc tacaccttcg ttgactacgc tgctcatcga  960
   cattttcttc ttcccctctg a                                            981
<210> 19
   <211> 245
   <212> DNA
   <213> Glycine sp.
<400> 19
   gcaacatctg ggactgggtt tgtcttgggg agtggtagtg ctgttgatct ttcggcactt   60
   tcttgcactt gcttgggtac catgatggtt gctgcatctg ctaactcttt gaatcaggtg  120
   tttgagatca ataatgatgc taaaatgaag agaacaagtc gcaggccact accctcagga  180
   cgcatcacaa tacctcatgc agttggctgg gcatcctctg ttggattagc tggtacggct  240
   ctact                                                              245
<210> 20
   <211> 253
   <212> DNA
   <213> Glycine sp.
<400> 20
   attggctttc caagatcatt gggttttctt gttgcattca tgaccttcta ctccttgggt    60
   ttggcattgt ccaaggatat acctgacgtt gaaggagata aagagcacgg cattgattct   120
   tttgcagtac gtctaggtca gaaacgggca ttttggattt gcgtttcctt ttttgaaatg   180
   gctttcggag ttggtatcct ggccggagca tcatgctcac acttttggac taaaattttc   240
   acgggtatgg gaa                                                      253
<210> 21
   <211> 275
   <212> DNA
   <213> Glycine sp.
<400> 21
   tgatcttcta ctctctgggt atggcattgt ccaaggatat atctgacgtt aaaggagata    60
   aagcatacgg catcgatact ttagcgatac gtttgggtca aaaatgggta ttttggattt   120
   gcattatcct ttttgaaatg gcttttggag ttgccctctt ggcaggagca acatcttctt   180
   acctttggat taaaattgtc acgggtctgg gacatgctat tcttgcttca attctcttgt   240
   accaagccaa atctatatac ttgagcaaca aagtt                              275
<210> 22
   <211> 299
   <212> DNA
   <213> Glycine sp.
<220>
   <221> misc_feature
   <222> (1)...(299)
   <223> n = A,T,C or G
<400> 22
   ccanaatang tncatcttng aaagacaatt ggcctcttca acacacaagt ctgcatgtga    60
   agaagaggcc aattgtcttt ccaagatcac ttatngtggc tattgtaatc atgaacttct   120
   tctttgtggg tatggcattg gcaaaggata tacctanctg ttgaaggaga taaaatatat   180
   ggcattgata cttttgcaat acgtataggt caaaaacaag tattttggat ttgtattttc   240
   ctttttgaaa ggctttcgga gtttccctag tggcaggagc aacatcttct agccttggt    299
<210> 23
   <211> 767
   <212> DNA
   <213> Glycine sp.
<400> 23
   gtggaggctg tggttgctgc cctgtttatg aatatttata ttgttggttt gaatcaattg    60
   tctgatgttg aaatagacaa gataaacaag ccgtatcttc cattagcatc tggggaatat   120
   tcctttgaaa ctggtgtcac tattgttgca tctttttcaa ttctgagttt ttggcttggc   180
   tgggttgtag gttcatggcc attattttgg gccctttttg taagctttgt gctaggaact   240
   gcttattcaa tcaatgtgcc tctgttgaga tggaagaggt ttgcagtgct tgcagcgatg   300
   tgcattctag ctgttcgggc agtaatagtt caacttgcat ttttccttca catgcagact    360
   catgtgtaca agaggccacc tgtcttttca agaccattga tttttgctac tgcattcatg   420
   agcttcttct ctgtagttat agcactgttt aaggatatac ctgacattga aggagataaa   480
   gtatttggca tccaatcttt ttcagtgtgt ttaggtcaga agccggtgtt ctggacttgt   540
   gttacccttc ttgaaatagc ttatggagtc gccctcctgg tgggagctgc atctccttgt   600
   ctttggagca aaattttcac gggtctggga cacgctgtgc tggcttcaat tctctggttt   660
   catgccaaat ctgtagattt gaaaagcaaa gcttcgataa catccttcta tatgtttatt   720
   tggaagctat tttatgcaga atacttactc attccttttg ttagatg                 767
<210> 24
   <211> 255
   <212> PRT
   <213> Glycine sp.
<400> 24
   Val Glu Ala Val Val Ala Ala Leu Phe Met Asn Ile Tyr Ile Val Gly
   1               5                   10                  15
   Leu Asn Gln Leu Ser Asp Val Glu Ile Asp Lys Ile Asn Lys Pro Tyr
               20                  25                  30
   Leu Pro Leu Ala Ser Gly Glu Tyr Ser Phe Glu Thr Gly Val Thr Ile
           35                  40                  45
   Val Ala Ser Phe Ser Ile Leu Ser Phe Trp Leu Gly Trp Val Val Gly
       50                  55                  60
   Ser Trp Pro Leu Phe Trp Ala Leu Phe Val Ser Phe Val Leu Gly Thr
   65                  70                  75                  80
   Ala Tyr Ser Ile Asn Val Pro Leu Leu Arg Trp Lys Arg Phe Ala Val
                   85                  90                  95
   Leu Ala Ala Met Cys Ile Leu Ala Val Arg Ala Val Ile Val Gln Leu
               100                 105                 110
   Ala Phe Phe Leu His Met Gln Thr His Val Tyr Lys Arg Pro Pro Val
           115                 120                 125
   Phe Ser Arg Pro Leu Ile Phe Ala Thr Ala Phe Met Ser Phe Phe Ser
       130                 135                 140
   Val Val Ile Ala Leu Phe Lys Asp Ile Pro Asp Ile Glu Gly Asp Lys
   145                 150                 155                 160
   Val Phe Gly Ile Gln Ser Phe Ser Val Cys Leu Gly Gln Lys Pro Val
                   165                 170                 175
   Phe Trp Thr Cys Val Thr Leu Leu Glu Ile Ala Tyr Gly Val Ala Leu
               180                 185                 190
   Leu Val Gly Ala Ala Ser Pro Cys Leu Trp Ser Lys Ile Phe Thr Gly
           195                 200                 205
   Leu Gly His Ala Val Leu Ala Ser Ile Leu Trp Phe His Ala Lys Ser
       210                 215                 220
   Val Asp Leu Lys Ser Lys Ala Ser Ile Thr Ser Phe Tyr Met Phe Ile
   225                 230                 235                 240
   Trp Lys Leu Phe Tyr Ala Glu Tyr Leu Leu Ile Pro Phe Val Arg
                   245                 250                 255
<210> 25
   <211> 360
   <212> DNA
   <213> Zea sp.
<220>
   <221> misc_feature
   <222> (1)...(360)
   <223> n = A,T,C or G
<400> 25
   ggcgtcttca cttgttctgg tcttctcgta tcccctgatg aagaggttca cattttggcc    60
   tcaggcttat cttggcctga cattcaactg gggagcttta ctagggtggg ctgctattaa   120
   ggaaagcata gaccctgcaa atcatccttc cattgtatac agctggtatt tgttggacgc   180
   tggtgtatga tactatatat gcgcatcagg tgtttcgcta tccctacttt catattaatc   240
   cttgatgaag tggccatttc atgttgtcgc ggtggtctta tacttgcata tctccatgca   300
   tctcaggaca aagangatga cctgaaagta ggagtccaag tccacagctt aagatttggg   360
<210> 26
   <211> 299
   <212> DNA
   <213> Zea sp.
<220>
   <221> misc_feature
   <222> (1)..(299)
   <223> n = A,T,C or G
<400> 26
   gatggttgca gcatctgcaa ataccctcaa ccaggtgttt gngataaaaa atgatgctaa    60
   aatgaaaagg acaatgcgtg ccccctgcca tctggtcgca ttagtcctgc acatgctgcg   120
   atgtgggcta caagtgttgg agttgcagga acagctttgt tggcctggaa ggctaatggc   180
   ttggcagctg ggcttgcagc ttctaatctt gttctgtatg catttgtgta tacgccgttg   240
   aagcaaatac accctgttaa tacatgggtt ggggcagtcg ttggtgccat cccaccact    299
<210> 27
   <211> 255
   <212> DNA
   <213> Zea sp.
<220>
   <221> misc_feature
   <222> (1)...(255)
   <223> n = A,T,C or G
<400> 27
   anacttgcat atctccatgc ntctcaggac aaagangatg acctgaaagt aggtgtcaag    60
   tccacagcat taagatttgg agatttgacc nnatactgna tcagtggctt tggcgcggca   120
   tgcttcggca gcttagcact cagtggttac aatgctgacc ttggttggtg tttagtgtga   180
   tgcttgagcg aagaatggta tngtttttac ttgatattga ctccagacct gaaatcatgt   240
   tggacagggt ggccc                                                    255
<210> 28
   <211> 257
   <212> DNA
   <213> Zea sp.
<400> 28
   attgaagggg ataggactct ggggcttcag tcacttcctg ttgcttttgg gatggaaact    60
   gcaaaatgga tttgtgttgg agcaattgat atcactcaat tatctgttgc aggttaccta   120
   ttgagcaccg gtaagctgta ttatgccctg gtgttgcttg ggctaacaat tcctcaggtg   180
   ttctttcagt tccagtactt cctgaaggac cctgtgaagt atgatgtcaa atatcaggca   240
   agcgcacaac cattctt                                                  257
<210> 29
   <211> 368
   <212> DNA
   <213> Zea sp.
<400> 29
   atccagttgc aaataataat ggcgttcttc tctgttgtaa tagcactatt caaggatata   60
   cctgacatcg aaggggaccg catattcggg atccgatcct tcagcgtccg gttagggcaa  120
   aagaaggtct tttggatctg cgttggcttg cttgagatgg cctacagcgt tgcgatactg  180
   atgggagcta cctcttcctg tttgtggagc aaaacagcaa ccatcgctgg ccattccata  240
   cttgccgcga tcctatggag ctgcgcgcga tcggtggact tgacgagcaa agccgcaata  300
   acgtccttct acatgttcat ctggaagctg ttctacgcgg agtacctgct catccctctg  360
   gtgcggtg                                                           368
<210> 30
   <211> 122
   <212> PRT
   <213> Zea sp.
<400> 30
   Ile Gln Leu Gln Ile Ile Met Ala Phe Phe Ser Val Val Ile Ala Leu
   1               5                   10                  15
   Phe Lys Asp Ile Pro Asp Ile Glu Gly Asp Arg Ile Phe Gly Ile Arg
               20                  25                  30
   Ser Phe Ser Val Arg Leu Gly Gln Lys Lys Val Phe Trp Ile Cys Val
           35                  40                  45
   Gly Leu Leu Glu Met Ala Tyr Ser Val Ala Ile Leu Met Gly Ala Thr
       50                  55                  60
   Ser Ser Cys Leu Trp Ser Lys Thr Ala Thr Ile Ala Gly His Ser Ile
   65                  70                  75                  80
   Leu Ala Ala Ile Leu Trp Ser Cys Ala Arg Ser Val Asp Leu Thr Ser
                   85                  90                  95
   Lys Ala Ala Ile Thr Ser Phe Tyr Met Phe Ile Trp Lys Leu Phe Tyr
               100                 105                 110
   Ala Glu Tyr Leu Leu Ile Pro Leu Val Arg
           115                 120
<210> 31
   <211> 278
   <212> DNA
   <213> Zea sp.
<400> 31
   tattcagcac cacctctcaa gctcaagcag aatggatgga ttgggaactt cgctctgggt   60
   gcgagttaca tcagcttgcc ctggtgggct ggccaggcgt tatttggaac tcttacacca  120
   gatatcattg tcttgactac tttgtacagc atagctgggc tagggattgc tattgtaaat  180
   gatttcaaga gtattgaagg ggataggact ctggggcttc agtcacttcc tgttgctttt  240
   gggatggaaa ctgcaaaatg gatttgtgtt ggagcaat                         278
<210> 32
   <211> 292
   <212> PRT
   <213> Synechocystis sp.
<400> 32
   Met Val Ala Gln Thr Pro Ser Ser Pro Pro Leu Trp Leu Thr Ile Ile
   1               5                   10                  15
   Tyr Leu Leu Arg Trp His Lys Pro Ala Gly Arg Leu Ile Leu Met Ile
               20                  25                  30
   Pro Ala Leu Trp Ala Val Cys Leu Ala Ala Gln Gly Leu Pro Pro Leu
           35                  40                  45
   Pro Leu Leu Gly Thr Ile Ala Leu Gly Thr Leu Ala Thr Ser Gly Leu
       50                  55                  60
   Gly Cys Val Val Asn Asp Leu Trp Asp Arg Asp Ile Asp Pro Gln Val
   65                  70                  75                  80
   Glu Arg Thr Lys Gln Arg Pro Leu Ala Ala Arg Ala Leu Ser Val Gln
                   85                  90                  95
   Val Gly Ile Gly Val Ala Leu Val Ala Leu Leu Cys Ala Ala Gly Leu
               100                 105                 110
   Ala Phe Tyr Leu Thr Pro Leu Ser Phe Trp Leu Cys Val Ala Ala Val
           115                 120                 125
   Pro Val Ile Val Ala Tyr Pro Gly Ala Lys Arg Val Phe Pro Val Pro
       130                 135                 140
   Gln Leu Val Leu Ser Ile Ala Trp Gly Phe Ala Val Leu Ile Ser Trp
   145                 150                 155                 160
   Ser Ala Val Thr Gly Asp Leu Thr Asp Ala Thr Trp Val Leu Trp Gly
                   165                 170                 175
   Ala Thr Val Phe Trp Thr Leu Gly Phe Asp Thr Val Tyr Ala Met Ala
               180                 185                 190
   Asp Arg Glu Asp Asp Arg Arg Ile Gly Val Asn Ser Ser Ala Leu Phe
           195                 200                 205
   Phe Gly Gln Tyr Val Gly Glu Ala Val Gly Ile Phe Phe Ala Leu Thr
       210                 215                 220
   Ile Gly Cys Leu Phe Tyr Leu Gly Met Ile Leu Met Leu Asn Pro Leu
   225                 230                 235                 240
   Tyr Trp Leu Ser Leu Ala Ile Ala Ile Val Gly Trp Val Ile Gln Tyr
                   245                 250                 255
   Ile Gln Leu Ser Ala Pro Thr Pro Glu Pro Lys Leu Tyr Gly Gln Ile
               260                 265                 270
   Phe Gly Gln Asn Val Ile Ile Gly Phe Val Leu Leu Ala Gly Met Leu
           275                 280                 285
   Leu Gly Trp Leu
       290
<210> 33
   <211> 316
   <212> PRT
   <213> Synechocystis sp.
<400> 33
   Met Val Thr Ser Thr Lys Ile His Arg Gln His Asp Ser Met Gly Ala
   1               5                   10                  15
   Val Cys Lys Ser Tyr Tyr Gln Leu Thr Lys Pro Arg Ile Ile Pro Leu
               20                  25                  30
   Leu Leu Ile Thr Thr Ala Ala Ser Met Trp Ile Ala Ser Glu Gly Arg
           35                  40                  45
   Val Asp Leu Pro Lys Leu Leu Ile Thr Leu Leu Gly Gly Thr Leu Ala
       50                  55                  60
   Ala Ala Ser Ala Gln Thr Leu Asn Cys Ile Tyr Asp Gln Asp Ile Asp
   65                  70                  75                  80
   Tyr Glu Met Leu Arg Thr Arg Ala Arg Pro Ile Pro Ala Gly Lys Val
                   85                  90                  95
   Gln Pro Arg His Ala Leu Ile Phe Ala Leu Ala Leu Gly Val Leu Ser
               100                 105                 110
   Phe Ala Leu Leu Ala Thr Phe Val Asn Val Leu Ser Gly Cys Leu Ala
           115                 120                 125
   Leu Ser Gly Ile Val Phe Tyr Met Leu Val Tyr Thr His Trp Leu Lys
       130                 135                 140
   Arg His Thr Ala Gln Asn Ile Val Ile Gly Gly Ala Ala Gly Ser Ile
   145                 150                 155                 160
   Pro Pro Leu Val Gly Trp Ala Ala Val Thr Gly Asp Leu Ser Trp Thr
                   165                 170                 175
   Pro Trp Val Leu Phe Ala Leu Ile Phe Leu Trp Thr Pro Pro His Phe
               180                 185                 190
   Trp Ala Leu Ala Leu Met Ile Lys Asp Asp Tyr Ala Gln Val Asn Val
           195                 200                 205
   Pro Met Leu Pro Val Ile Ala Gly Glu Glu Lys Thr Val Ser Gln Ile
       210                 215                 220
   Trp Tyr Tyr Ser Leu Leu Val Val Pro Phe Ser Leu Leu Leu Val Tyr
   225                 230                 235                 240
   Pro Leu His Gln Leu Gly Ile Leu Tyr Leu Ala Ile Ala Ile Ile Leu
                   245                 250                 255
   Gly Gly Gln Phe Leu Val Lys Ala Trp Gln Leu Lys Gln Ala Pro Gly
               260                 265                 270
   Asp Arg Asp Leu Ala Arg Gly Leu Phe Lys Phe Ser Ile Phe Tyr Leu
           275                 280                 285
   Met Leu Leu Cys Leu Ala Met Val Ile Asp Ser Leu Pro Val Thr His
       290                 295                 300
   Gln Leu Val Ala Gln Met Gly Thr Leu Leu Leu Gly
   305                 310                 315
<210> 34
   <211> 324
   <212> PRT
   <213> Synechocystis sp.
<400> 34
   Met Ser Asp Thr Gln Asn Thr Gly Gln Asn Gln Ala Lys Ala Arg Gln
   1               5                   10                  15
   Leu Leu Gly Met Lys Gly Ala Ala Pro Gly Glu Ser Ser Ile Trp Lys
               20                  25                  30
   Ile Arg Leu Gln Leu Met Lys Pro Ile Thr Trp Ile Pro Leu Ile Trp
           35                  40                  45
   Gly Val Val Cys Gly Ala Ala Ser Ser Gly Gly Tyr Ile Trp Ser Val
       50                  55                  60
   Glu Asp Phe Leu Lys Ala Leu Thr Cys Met Leu Leu Ser Gly Pro Leu
   65                  70                  75                  80
   Met Thr Gly Tyr Thr Gln Thr Leu Asn Asp Phe Tyr Asp Arg Asp Ile
                   85                  90                  95
   Asp Ala Ile Asn Glu Pro Tyr Arg Pro Ile Pro Ser Gly Ala Ile Ser
               100                 105                 110
   Val Pro Gln Val Val Thr Gln Ile Leu Ile Leu Leu Val Ala Gly Ile
           115                 120                 125
   Gly Val Ala Tyr Gly Leu Asp Val Trp Ala Gln His Asp Phe Pro Ile
       130                 135                 140
   Met Met Val Leu Thr Leu Gly Gly Ala Phe Val Ala Tyr Ile Tyr Ser
   145                 150                 155                 160
   Ala Pro Pro Leu Lys Leu Lys Gln Asn Gly Trp Leu Gly Asn Tyr Ala
                   165                 170                 175
   Leu Gly Ala Ser Tyr Ile Ala Leu Pro Trp Trp Ala Gly His Ala Leu
               180                 185                 190
   Phe Gly Thr Leu Asn Pro Thr Ile Met Val Leu Thr Leu Ile Tyr Ser
           195                 200                 205
   Leu Ala Gly Leu Gly Ile Ala Val Val Asn Asp Phe Lys Ser Val Glu
       210                 215                 220
   Gly Asp Arg Gln Leu Gly Leu Lys Ser Leu Pro Val Met Phe Gly Ile
   225                 230                 235                 240
   Gly Thr Ala Ala Trp Ile Cys Val Ile Met Ile Asp Val Phe Gln Ala
                   245                 250                 255
   Gly Ile Ala Gly Tyr Leu Ile Tyr Val His Gln Gln Leu Tyr Ala Thr
               260                 265                 270
   Ile Val Leu Leu Leu Leu Ile Pro Gln Ile Thr Phe Gln Asp Met Tyr
           275                 280                 285
   Phe Leu Arg Asn Pro Leu Glu Asn Asp Val Lys Tyr Gln Ala Ser Ala
       290                 295                 300
   Gln Pro Phe Leu Val Phe Gly Met Leu Ala Thr Gly Leu Ala Leu Gly
   305                 310                 315                 320
   His Ala Gly Ile
<210> 35
   <211> 307
   <212> PRT
   <213> Synechocystis sp.
<400> 35
   Met Thr Glu Ser Ser Pro Leu Ala Pro Ser Thr Ala Pro Ala Thr Arg
   1               5                   10                  15
   Lys Leu Trp Leu Ala Ala Ile Lys Pro Pro Met Tyr Thr Val Ala Val
               20                  25                  30
   Val Pro Ile Thr Val Gly Ser Ala Val Ala Tyr Gly Leu Thr Gly Gln
           35                  40                  45
   Trp His Gly Asp Val Phe Thr Ile Phe Leu Leu Ser Ala Ile Ala Ile
       50                  55                  60
   Ile Ala Trp Ile Asn Leu Ser Asn Asp Val Phe Asp Ser Asp Thr Gly
   65                  70                  75                  80
   Ile Asp Val Arg Lys Ala His Ser Val Val Asn Leu Thr Gly Asn Arg
                   85                  90                  95
   Asn Leu Val Phe Leu Ile Ser Asn Phe Phe Leu Leu Ala Gly Val Leu
               100                 105                 110
   Gly Leu Met Ser Met Ser Trp Arg Ala Gln Asp Trp Thr Val Leu Glu
           115                 120                 125
   Leu Ile Gly Val Ala Ile Phe Leu Gly Tyr Thr Tyr Gln Gly Pro Pro
       130                 135                 140
   Phe Arg Leu Gly Tyr Leu Gly Leu Gly Glu Leu Ile Cys Leu Ile Thr
   145                 150                 155                 160
   Phe Gly Pro Leu Ala Ile Ala Ala Ala Tyr Tyr Ser Gln Ser Gln Ser
                   165                 170                 175
   Phe Ser Trp Asn Leu Leu Thr Pro Ser Val Phe Val Gly Ile Ser Thr
               180                 185                 190
   Ala Ile Ile Leu Phe Cys Ser His Phe His Gln Val Glu Asp Asp Leu
           195                 200                 205
   Ala Ala Gly Lys Lys Ser Pro Ile Val Arg Leu Gly Thr Lys Leu Gly
       210                 215                 220
   Ser Gln Val Leu Thr Leu Ser Val Val Ser Leu Tyr Leu Ile Thr Ala
   225                 230                 235                 240
   Ile Gly Val Leu Cys His Gln Ala Pro Trp Gln Thr Leu Leu Ile Ile
                   245                 250                 255
   Ala Ser Leu Pro Trp Ala Val Gln Leu Ile Arg His Val Gly Gln Tyr
               260                 265                 270
   His Asp Gln Pro Glu Gln Val Ser Asn Cys Lys Phe Ile Ala Val Asn
           275                 280                 285
   Leu His Phe Phe Ser Gly Met Leu Met Ala Ala Gly Tyr Gly Trp Ala
       290                 295                 300
   Gly Leu Gly
   305
<210> 36
   <211> 927
   <212> DNA
   <213> Synechocystis sp.
<400> 36
   atggcaacta tccaagcttt ttggcgcttc tcccgccccc ataccatcat tggtacaact    60
   ctgagcgtct gggctgtgta tctgttaact attctcgggg atggaaactc agttaactcc   120
   cctgcttccc tggatttagt gttcggcgct tggctggcct gcctgttggg taatgtgtac   180
   attgtcggcc tcaaccaatt gtgggatgtg gacattgacc gcatcaataa gccgaatttg   240
   cccctagcta acggagattt ttctatcgcc cagggccgtt ggattgtggg actttgtggc   300
   gttgcttcct tggcgatcgc ctggggatta gggctatggc tggggctaac ggtgggcatt   360
   agtttgatta ttggcacggc ctattcggtg ccgccagtga ggttaaagcg cttttccctg   420
   ctggcggccc tgtgtattct gacggtgcgg ggaattgtgg ttaacttggg cttattttta    480
   ttttttagaa ttggtttagg ttatcccccc actttaataa cccccatctg ggttttgact    540
   ttatttatct tagttttcac cgtggcgatc gccattttta aagatgtgcc agatatggaa   600
   ggcgatcggc aatttaagat tcaaacttta actttgcaaa tcggcaaaca aaacgttttt   660
   cggggaacct taattttact cactggttgt tatttagcca tggcaatctg gggcttatgg    720
   gcggctatgc ctttaaatac tgctttcttg attgtttccc atttgtgctt attagcctta   780
   ctctggtggc ggagtcgaga tgtacactta gaaagcaaaa ccgaaattgc tagtttttat   840
   cagtttattt ggaagctatt tttcttagag tacttgctgt atcccttggc tctgtggtta   900
   cctaattttt ctaatactat tttttag                                       927
<210> 37
   <211> 308
   <212> PRT
   <213> Synechocystis sp.
<400> 37
   Met Ala Thr Ile Gln Ala Phe Trp Arg Phe Ser Arg Pro His Thr Ile
   1 5 10 15
   Ile Gly Thr Thr Leu Ser Val Trp Ala Val Tyr Leu Leu Thr Ile Leu
   20 25 30
   Gly Asp Gly Asn Ser Val Asn Ser Pro Ala Ser Leu Asp Leu Val Phe
   35 40 45
   Gly Ala Trp Leu Ala Cys Leu Leu Gly Asn Val Tyr Ile Val Gly Leu
   50 55 60
   Asn Gln Leu Trp Asp Val Asp Ile Asp Arg Ile Asn Lys Pro Asn Leu
   65 70 75 80
   Pro Leu Ala Asn Gly Asp Phe Ser Ile Ala Gln Gly Arg Trp Ile Val
   85 90 95
   Gly Leu Cys Gly Val Ala Ser Leu Ala Ile Ala Trp Gly Leu Gly Leu
   100 105 110
   Trp Leu Gly Leu Thr Val Gly Ile Ser Leu Ile Ile Gly Thr Ala Tyr
   115 120 125
   Ser Val Pro Pro Val Arg Leu Lys Arg Phe Ser Leu Leu Ala Ala Leu
   130 135 140
   Cys Ile Leu Thr Val Arg Gly Ile Val Val Asn Leu Gly Leu Phe Leu
   145 150 155 160
   Phe Phe Arg Ile Gly Leu Gly Tyr Pro Pro Thr Leu Ile Thr Pro Ile
   165 170 175
   Trp Val Leu Thr Leu Phe Ile Leu Val Phe Thr Val Ala Ile Ala Ile
   180 185 190
   Phe Lys Asp Val Pro Asp Met Glu Gly Asp Arg Gln Phe Lys Ile Gln
   195 200 205
   Thr Leu Thr Leu Gln Ile Gly Lys Gln Asn Val Phe Arg Gly Thr Leu
   210 215 220
   Ile Leu Leu Thr Gly Cys Tyr Leu Ala Met Ala Ile Trp Gly Leu Trp
   225 230 235 240
   Ala Ala Met Pro Leu Asn Thr Ala Phe Leu Ile Val Ser His Leu Cys
   245 250 255
   Leu Leu Ala Leu Leu Trp Trp Arg Ser Arg Asp Val His Leu Glu Ser
   260 265 270
   Lys Thr Glu Ile Ala Ser Phe Tyr Gln Phe Ile Trp Lys Leu Phe Phe
   275 280 285
   Leu Glu Tyr Leu Leu Tyr Pro Leu Ala Leu Trp Leu Pro Asn Phe Ser
   290 295 300
   Asn Thr Ile Phe
   305
<210> 38
   <211> 1092
   <212> DNA
   <213> Synechocystis sp.
<400> 38
   atgaaatttc cgccccacag tggttaccat tggcaaggtc aatcaccttt ctttgaaggt   60
   tggtacgtgc gcctgctttt gccccaatcc ggggaaagtt ttgcttttat gtactccatc  120
   gaaaatcctg ctagcgatca tcattacggc ggcggtgctg tgcaaatttt agggccggct  180
   acgaaaaaac aagaaaatca ggaagaccaa cttgtttggc ggacatttcc ctcggtaaaa  240
   aaattttggg ccagtcctcg ccagtttgcc ctagggcatt ggggaaaatg tagggataac  300
   aggcaggcga aacccctact ctccgaagaa ttttttgcca cggtcaagga aggttatcaa  360
   atccatcaaa atcagcacca aggacaaatc attcatggcg atcgccattg tcgttggcag  420
   ttcaccgtag aaccggaagt aacttggggg agtcctaacc gatttcctcg ggctacagcg  480
   ggttggcttt cctttttacc cttgtttgat cccggttggc aaattctttt agcccaaggt  540
   agagcgcacg gctggctgaa atggcagagg gaacagtatg aatttgacca cgccctagtt  600
   tatgccgaaa aaaattgggg tcactccttt ccctcccgct ggttttggct ccaagcaaat  660
   tattttcctg accatccagg actgagcgtc actgccgctg gcggggaacg gattgttctt  720
   ggtcgccccg aagaggtagc tttaattggc ttacatcacc aaggtaattt ttacgaattt  780
   ggcccgggcc atggcacagt cacttggcaa gtagctccct ggggccgttg gcaattaaaa  840
   gccagcaatg ataggtattg ggtcaagttg tccggaaaaa cagataaaaa aggcagttta  900
   gtccacactc ccaccgccca gggcttacaa ctcaactgcc gagataccac taggggctat  960
   ttgtatttgc aattgggatc tgtgggtcac ggcctgatag tgcaagggga aacggacacc 1020
   gcggggctag aagttggagg tgattggggt ttaacagagg aaaatttgag caaaaaaaca 1080
   gtgccattct ga                                                     1092
<210> 39
   <211> 363
   <212> PRT
   <213> Synechocystis sp.
<400> 39
   Met Lys Phe Pro Pro His Ser Gly Tyr His Trp Gln Gly Gln Ser Pro
   1               5                   10                  15
   Phe Phe Glu Gly Trp Tyr Val Arg Leu Leu Leu Pro Gln Ser Gly Glu
               20                  25                  30
   Ser Phe Ala Phe Met Tyr Ser Ile Glu Asn Pro Ala Ser Asp His His
           35                  40                  45
   Tyr Gly Gly Gly Ala Val Gln Ile Leu Gly Pro Ala Thr Lys Lys Gln
       50                  55                  60
   Glu Asn Gln Glu Asp Gln Leu Val Trp Arg Thr Phe Pro Ser Val Lys
   65                  70                  75                  80
   Lys Phe Trp Ala Ser Pro Arg Gln Phe Ala Leu Gly His Trp Gly Lys
                   85                  90                  95
   Cys Arg Asp Asn Arg Gln Ala Lys Pro Leu Leu Ser Glu Glu Phe Phe
               100                 105                 110
   Ala Thr Val Lys Glu Gly Tyr Gln Ile His Gln Asn Gln His Gln Gly
           115                 120                 125
   Gln Ile Ile His Gly Asp Arg His Cys Arg Trp Gln Phe Thr Val Glu
       130                 135                 140
   Pro Glu Val Thr Trp Gly Ser Pro Asn Arg Phe Pro Arg Ala Thr Ala
   145                 150                 155                 160
   Gly Trp Leu Ser Phe Leu Pro Leu Phe Asp Pro Gly Trp Gln Ile Leu
   165 170 175
   Leu Ala Gln Gly Arg Ala His Gly Trp Leu Lys Trp Gln Arg Glu Gln
   180 185 190
   Tyr Glu Phe Asp His Ala Leu Val Tyr Ala Glu Lys Asn Trp Gly His
   195 200 205
   Ser Phe Pro Ser Arg Trp Phe Trp Leu Gln Ala Asn Tyr Phe Pro Asp
   210 215 220
   His Pro Gly Leu Ser Val Thr Ala Ala Gly Gly Glu Arg Ile Val Leu
   225 230 235 240
   Gly Arg Pro Glu Glu Val Ala Leu Ile Gly Leu His His Gln Gly Asn
   245 250 255
   Phe Tyr Glu Phe Gly Pro Gly His Gly Thr Val Thr Trp Gln Val Ala
   260 265 270
   Pro Trp Gly Arg Trp Gln Leu Lys Ala Ser Asn Asp Arg Tyr Trp Val
   275 280 285
   Lys Leu Ser Gly Lys Thr Asp Lys Lys Gly Ser Leu Val His Thr Pro
   290 295 300
   Thr Ala Gln Gly Leu Gln Leu Asn Cys Arg Asp Thr Thr Arg Gly Tyr
   305 310 315 320
   Leu Tyr Leu Gln Leu Gly Ser Val Gly His Gly Leu Ile Val Gln Gly
   325 330 335
   Glu Thr Asp Thr Ala Gly Leu Glu Val Gly Gly Asp Trp Gly Leu Thr
   340 345 350
   Glu Glu Asn Leu Ser Lys Lys Thr Val Pro Phe
   355 360
<210> 40
   <211> 56
   <212> DNA
   <213> Artificial Sequence
<220>
   <223> oligonucleotide adapter
<400> 40
   cgcgatttaa atggcgcgcc ctgcaggcgg ccgcctgcag ggcgcgccat ttaaat 56
<210> 41
   <211> 32
   <212> DNA
   <213> Artificial Sequence
<220>
   <223> oligonucleotide
<400> 41
   tcgaggatcc gcggccgcaa gcttcctgca gg 32
<210> 42
   <211> 32
   <212> DNA
   <213> Artificial Sequence
<220>
   <223> oligonucleotide
<400> 42
   tcgacctgca ggaagcttgc ggccgcggat cc 32
<210> 43
   <211> 32
   <212> DNA
   <213> Artificial Sequence
<220>
   <223> oligonucleotide
<400> 43
   tcgacctgca ggaagcttgc ggccgcggat cc 32
<210> 44
   <211> 32
   <212> DNA
   <213> Artificial Sequence
<220>
   <223> oligonucleotide
<400> 44
   tcgaggatcc gcggccgcaa gcttcctgca gg 32
<210> 45
   <211> 36
   <212> DNA
   <213> Artificial Sequence
<220>
   <223> oligonucleotide
<400> 45
   tcgaggatcc gcggccgcaa gcttcctgca ggagct 36
<210> 46
   <211> 28
   <212> DNA
   <213> Artificial Sequence
<220>
   <223> oligonucleotide
<400> 46
   cctgcaggaa gcttgcggcc gcggatcc 28
<210> 47
   <211> 36
   <212> DNA
   <213> Artificial Sequence
<220>
   <223> oligonucleotide
<400> 47
   tcgacctgca ggaagcttgc ggccgcggat ccagct 36
<210> 48
   <211> 28
   <212> DNA
   <213> Artificial Sequence
<220>
   <223> oligonucleotide
<400> 48
   ggatccgcgg ccgcaagctt cctgcagg 28
<210> 49
   <211> 39
   <212> DNA
   <213> Artificial Sequence
<220>
   <223> oligonucleotide
<400> 49
   gatcacctgc aggaagcttg cggccgcgga tccaatgca 39
<210> 50
   <211> 31
   <212> DNA
   <213> Artificial Sequence
<220>
   <223> oligonucleotide
<400> 50
   ttggatccgc ggccgcaagc ttcctgcagg t 31
<210> 51
   <211> 41
   <212> DNA
   <213> Artificial Sequence
<220>
   <223> Synthetic Oligonucleotides
<400> 51
   ggatccgcgg ccgcacaatg gagtctctgc tctctagttc t 41
<210> 52
   <211> 38
   <212> DNA
   <213> Artificial Sequence
<220>
   <223> Synthetic oligonucleotides
<400> 52
   ggatcctgca ggtcacttca aaaaaggtaa cagcaagt 38
<210> 53
   <211> 45
   <212> DNA
   <213> Artificial Sequence
<220>
   <223> Synthetic Oligonucleotides
<400> 53
   ggatccgcgg ccgcacaatg gcgttttttg ggctctcccg tgttt 45
<210> 54
   <211> 40
   <212> DNA
   <213> Artificial Sequence
<220>
   <223> Synthetic Oligonucleotides
<400> 54
   ggatcctgca ggttattgaa aacttcttcc aagtacaact 40
<210> 55
   <211> 38
   <212> DNA
   <213> Artificial Sequence
<220>
   <223> Synthetic Oligonucleotides
<400> 55
   ggatccgcgg ccgcacaatg tggcgaagat ctgttgtt 38
<210> 56
   <211> 37
   <212> DNA
   <213> Artificial Sequence
<220>
   <223> Synthetic Oligonucleotides
<400> 56
   ggatcctgca ggtcatggag agtagaagga aggagct 37
<210> 57
   <211> 50
   <212> DNA
   <213> Artificial Sequence
<220>
   <223> Synthetic Oligonucleotides
<400> 57
   ggatccgcgg ccgcacaatg gtacttgccg aggttccaaa gcttgcctct 50
<210> 58
   <211> 38
   <212> DNA
   <213> Artificial Sequence
<220>
   <223> Synthetic Oligonucleotides
<400> 58
   ggatcctgca ggtcacttgt ttctggtgat gactctat 38
<210> 59
   <211> 38
   <212> DNA
   <213> Artificial Sequence
<220>
   <223> Synthetic Oligonucleotides
<400> 59
   ggatccgcgg ccgcacaatg acttcgattc tcaacact 38
<210> 60
   <211> 36
   <212> DNA
   <213> Artificial Sequence
<220>
   <223> Synthetic Oligonucleotides
<400> 60
   ggatcctgca ggtcagtgtt gcgatgctaa tgccgt 36
<210> 61
   <211> 22
   <212> DNA
   <213> Artificial Sequence
<220>
   <223> Region of slr1736 open reading frame
<400> 61
   taatgtgtac attgtcggcc tc 22
<210> 62
   <211> 60
   <212> DNA
   <213> Artificial Sequence
<220>
   <223> Region of slr1736 open reading frame
<400> 62
   gcaatgtaac atcagagatt ttgagacaca acgtggcttt ccacaattcc ccgcaccgtc 60
<210> 63
   <211> 22
   <212> DNA
   <213> Artificial Sequence
<220>
   <223> Region of s1r1736 open reading frame
<400> 63
   aggctaataa gcacaaatgg ga 22
<210> 64
   <211> 63
   <212> DNA
   <213> Artificial Sequence
<220>
   <223> Region of slr1736 open reading frame
<400> 64
   ggtatgagtc agcaacacct tcttcacgag gcagacctca gcggaattgg tttaggttat 60
   ccc 63
<210> 65
   <211> 26
   <212> DNA
   <213> Artificial Sequence
<220>
   <223> Primer
<400> 65
   ggatccatgg ttgcccaaac cccatc 26
<210> 66
   <211> 61
   <212> DNA
   <213> Artificial Sequence
<220>
   <223> Primer
<400> 66
   gcaatgtaac atcagagatt ttgagacaca acgtggcttt gggtaagcaa caatgaccgg 60
   c 61
<210> 67
   <211> 25
   <212> DNA
   <213> Artificial Sequence
<220>
   <223> Oligonucleotide primer
<400> 67
   gaattctcaa agccagccca gtaac 25
<210> 68
   <211> 63
   <212> DNA
   <213> Artificial Sequence
<220>
   <223> Oligonucleotide primer
<400> 68
   ggtatgagtc agcaacacct tcttcacgag gcagacctca gcgggtgcga aaagggtttt 60
   ccc 63
<210> 69
   <211> 23
   <212> DNA
   <213> Artificial Sequence
<220>
   <223> End of open reading frame fragment
<400> 69
   ccagtggttt aggctgtgtg gtc 23
<210> 70
   <211> 21
   <212> DNA
   <213> Artificial Sequence
<220>
   <223> End of open reading frame fragment
<400> 70
   ctgagttgga tgtattggat c 21
<210> 71
   <211> 28
   <212> DNA
   <213> Artificial Sequence
<220>
   <223> Primer
<400> 71
   ggatccatgg ttacttcgac aaaaatcc 28
<220> 72
   <211> 60
   <212> DNA
   <213> Artificial Sequence
<220>
   <223> Primer
<400> 72
   gcaatgtaac atcagagatt ttgagacaca acgtggcttt gctaggcaac cgcttagtac 60
<210> 73
   <211> 28
   <212> DNA
   <213> Artificial Sequence
<220>
   <223> Oligonucleotide primer
<400> 73
   gaattcttaa cccaacagta aagttccc 28
<210> 74
   <211> 63
   <212> DNA
   <213> Artificial Sequence
<220>
   <223> Oligonucleotide primer
<400> 74
   ggtatgagtc agcaacacct tcttcacgag gcagacctca gcgccggcat tgtcttttac 60
   atg 63
<210> 75
   <211> 20
   <212> DNA
   <213> Artificial Sequence
<220>
   <223> Open reading frame fragment
<400> 75
   ggaacccttg cagccgcttc 20
<210> 76
   <211> 22
   <212> DNA
   <213> Artificial Sequence
<220>
   <223> Open reading frame fragment
<400> 76
   gtatgcccaa ctggtgcaga gg 22
<210> 77
   <211> 28
   <212> DNA
   <213> Artificial Sequence
<220>
   <223> Primer
<400> 77
   ggatccatgt ctgacacaca aaataccg 28
<210> 78
   <211> 62
   <212> DNA
   <213> Artificial Sequence
<220>
   <223> Primer
<400> 78
   gcaatgtaac atcagagatt ttgagacaca acgtggcttt cgccaatacc agccaccaac 60
   ag 62
<210> 79
   <211> 27
   <212> DNA
   <213> Artificial Sequence
<220>
   <223> Synthetic oligonucleotide primer
<400> 79
   gaattctcaa atccccgcat ggcctag 27
<210> 80
   <211> 65
   <212> DNA
   <213> Artificial Sequence
<220>
   <223> Synthetic oligonucleotide primer
<400> 80
   ggtatgagtc agcaacacct tcttcacgag gcagacctca gcggcctacg gcttggacgt 60
   gtggg 65
<210> 81
   <211> 21
   <212> DNA
   <213> Artificial Sequence
<220>
   <223> Open reading frame fragment
<400> 81
   cacttggatt cccctgatct g 21
<210> 82
   <211> 21
   <212> DNA
   <213> Artificial Sequence
<220>
   <223> Open reading frame fragment
<400> 82
   gcaatacccg cttggaaaac g 21
<210> 83
   <211> 29
   <212> DNA
   <213> Artificial Sequence
<220>
   <223> Primer
<400> 83
   ggatccatga ccgaatcttc gcccctagc 29
<210> 84
   <211> 61
   <212> DNA
   <213> Artificial Sequence
<220>
   <223> Primer
<400> 84
   gcaatgtaac atcagagatt ttgagacaca acgtggcttt caatcctagg tagccgaggc 60
   g 61
<210> 85
   <211> 27
   <212> DNA
   <213> Artificial Sequence
<220>
   <223> Synthetic oligonucleotide primer
<400> 85
   gaattcttag cccaggccag cccagcc 27
<210> 86
   <211> 66
   <212> DNA
   <213> Artificial Sequence
<220>
   <223> Synthetic oligonucleotide primer
<400> 86
   ggtatgagtc agcaacacct tcttcacgag gcagacctca gcggggaatt gatttgttta 60
   attacc 66
<210> 87
   <211> 21
   <212> DNA
   <213> Artificial Sequence
<220>
   <223> Open reading frame fragment
<400> 87
   gcgatcgcca ttatcgcttg g 21
<210> 88
   <211> 24
   <212> DNA
   <213> Artificial Sequence
<220>
   <223> Open reading frame fragment
<400> 88
   gcagactggc aattatcagt aacg 24
<210> 89
   <211> 25
   <212> DNA
   <213> Artificial Sequence
<220>
   <223> Primer
<400> 89
   ccatggattc gagtaaagtt gtcgc 25
<210> 90
   <211> 25
   <212> DNA
   <213> Artificial Sequence
<220>
   <223> Primer
<400> 90
   gaattcactt caaaaaaggt aacag
   25
<210> 91
   <211> 4550
   <212> DNA
   <213> Arabidopsis sp.
<400> 91
   attttacacc aatttgatca cttaactaaa ttaattaaat tagatgatta tcccaccata   60
   tttttgagca ttaaaccata aaaccatagt tataagtaac tgttttaatc gaatatgact  120
   cgattaagat taggaaaaat ttataaccgg taattaagaa aacattaacc gtagtaaccg  180
   taaatgccga ttcctccctt gtctaaaaga cagaaaacat atattttatt ttgccccata  240
   tgtttcactc tatttaattt caggcacaat acttttggtt ggtaacaaaa ctaaaaagga  300
   caacacgtga tacttttcct cgtccgtcag tcagattttt tttaaactag aaacaagtgg  360
   caaatctaca ccacattttt tgcttaatct attaacttgt aagttttaaa ttcctaaaaa  420
   agtctaacta attcttctaa tataagtaca ttccctaaat ttcccaaaaa gtcaaattaa  480
   taattttcaa aatctaatct aaatatctaa taattcaaaa tcattaaaaa gacacgcaac  540
   aatgacacca attaatcatc ctcgacccac acaattctac agttctcatg ctaaaccata  600
   ttttttgctc tctgttcctt caaaatcatt tctttctctt ctttgattcc caaagatcac  660
   ttctttgtct ttgatttttg attttttttc tctctggcgt gaaggaagaa gctttatttc  720
   atggagtctc tgctctctag ttcttctctt gtttccgctg gtaaatctcg tccttttctg  780
   gtttcaggtt ttatttgttg tttaggtttc gtttttgtga ttcagaacca tacaaaaagt  840
   ttgaactttt ctgaatataa aataaggaaa aagtttcgat ttttataatg aattgtttac  900
   tagatcgaag taggtgacaa aggttattgt gtggagaagc ataatttctg ggcttgactt  960
   tgaattttgt ttctcatgca tgcaacttat caatcagctg gtgggttttg ttggaagaag 1020
   cagaatctaa agctccactc tttatcaggt tcgttagggt tttatgggtt tttgaaatta 1080
   aatactcaat catcttagtc tcattattct attggttgaa tcacattttc taatttggaa 1140
   tttatgagac aatgtatgtt ggacttagtt gaagttcttc tctttggtta tagttgaagt 1200
   gttactgatg ttgtttagct ctttacacca atatatacac ccaattttgc agaaatccga 1260
   gttctgcgtt gtgattcgag taaagttgtc gcaaaaccga agtttaggaa caatcttgtt 1320
   aggcctgatg gtcaaggatc ttcattgttg ttgtatccaa aacataagtc gagatttcgg 1380
   gttaatgcca ctgcgggtca gcctgaggct ttcgactcga atagcaaaca gaagtctttt 1440
   agagactcgt tagatgcgtt ttacaggttt tctaggcctc atacagttat tggcacagtt 1500
   aagtttctct ttaaaaatgt aactctttta aaacgcaatc tttcagggtt ttcaaggaga 1560
   taacattagc tctgtgattg gatttgcagg tgcttagcat tttatctgta tctttcttag 1620
   cagtagagaa ggtttctgat atatctcctt tacttttcac tggcatcttg gaggtaatga 1680
   atatataaca cataatgacc gatgaagaag atacattttt ttcgtctctc tgtttaaaca 1740
   attgggtttt gttttcaggc tgttgttgca gctctcatga tgaacattta catagttggg 1800
   ctaaatcagt tgtctgatgt tgaaatagat aaggtaacat gcaaattttc ttcatatgag  1860
   ttcgagagac tgatgagatt aatagcagct agtgcctaga tcatctctat gtgggttttt 1920
   gcaggttaac aagccctatc ttccattggc atcaggagaa tattctgtta acaccggcat 1980
   tgcaatagta gcttccttct ccatcatggt atggtgccat tttcacaaaa tttcaacttt 2040
   tagaattcta taagttactg aaatagtttg ttataaatcg ttatagagtt tctggcttgg 2100
   gtggattgtt ggttcatggc cattgttctg ggctcttttt gtgagtttca tgctcggtac 2160
   tgcatactct atcaatgtaa gtaagtttct caatactaga atttggctca aatcaaaatc 2220
   tgcagtttct agttttaggt taatgaggtt ttaataactt acttctacta caaacagttg 2280
   ccacttttac ggtggaaaag atttgcattg gttgcagcaa tgtgtatcct cgctgtccga 2340
   gctattattg ttcaaatcgc cttttatcta catattcagg tactaaacca ttttccttat 2400
   gttttgtagt tgttttcatc aaaatcactt ttatattact aaagctgtga aactttgttg 2460
   cagacacatg tgtttggaag accaatcttg ttcactaggc ctcttatttt cgccactgcg 2520
   tttatgagct ttttctctgt cgttattgca ttgtttaagg taaacaaaga tggaaaaaga 2580
   ttaaatctat gtatacttaa agtaaagcat tctactgtta ttgatgagaa gttttctttt 2640
   ttggttggat gcaggatata cctgatatcg aaggggataa gatattcgga atccgatcat 2700
   tctctgtaac tctgggtcag aaacgggtac gatatctaaa ctaaagaaat tgttttgact 2760
   caagtgttgg attaagatta cagaagaaag aaaactgttt ttgtttcttg caaaattcag 2820
   gtgttttgga catgtgttac actacttcaa atggcttacg ctgttgcaat tctagttgga 2880
   gccacatctc cattcatatg gagcaaagtc atctcggtaa caatctttct ttacccatcg 2940
   aaaactcgct aattcatcgt ttgagtggta ctggtttcat tttgttccgt tctgttgatt 3000
   ttttttcagg ttgtgggtca tgttatactc gcaacaactt tgtgggctcg agctaagtcc 3060
   gttgatctga gtagcaaaac cgaaataact tcatgttata tgttcatatg gaaggttaga 3120
   ttcgtttata aatagagtct ttactgcctt tttatgcgct ccaatttgga attaaaatag 3180
   cctttcagtt tcatcgaatc accattatac tgataaattc tcatttctgc atcagctctt 3240
   ttatgcagag tacttgctgt tacctttttt gaagtgactg acattagaag agaagaagat 3300
   ggagataaaa gaataagtca tcactatgct tctgttttta ttacaagttc atgaaattag 3360
   gtagtgaact agtgaattag agttttattc tgaaacatgg cagactgcaa aaatatgtca 3420
   aagatatgaa tttctgttgg gtaaagaagt ctctgcttgg gcaaaatctt aaggttcggt 3480
   gtgttgatat aatgctaagc gaagaaatcg attctatgta gaaatttccg aaactatgtg 3540
   taaacatgtc agaacatctc cattctatat cttcttctgc aagaaagctc tgtttttatc 3600
   acctaaactc tttatctctg tgtagttaag atatgtatat gtacgtgact acattttttt 3660
   gttgatgtaa tttgcagaac gtatggattt ttgttagaaa gcatgagttc gaaagtatat 3720
   gtttatatat atggataatt cagacctaac gtcgaagctc acaagcataa attcactact 3780
   atagtttgct ctgtaataga tagttccatt gatgtcttga aactgtacgt aactgcctgg 3840
   gcgttttgtg gttgatactg actactgagt gttctttgtg agtgttgtaa gtatacaaga 3900
   agaagaatat aggctcacgg gaacgactgt ggtggaagat gaaatggaga tcatcacgta 3960
   gcggctttgc caaagaccga gtcacgatcg agtctatgaa gtctttacag ctgctgatta 4020
   tgattgacca ttgcttagag acgcattgga atcttactag ggacttgcct gggagtttct 4080
   tcaagtacgt gtcagatcat acgatgtagg agatttcacg gctttgatgt gtttgtttgg 4140
   agtcacaatg cttaatgggc ttattggccc aataatagct agctcttttg ctttagccgt 4200
   ttcgtttgtc ccctggtggt gagtattatt agggtatggt gtgaccaaag tcaccagacc 4260
   tagagtgaat ctagtagagt cctagaccat ggtccatggc ttttatttgt aatttgaaaa 4320
   atgaacaatt ctttttgtaa ggaaaacttt tatatagtag acgtttacta tatagaaact 4380
   agttgaacta acttcgtgca attgcataat aatggtgtga aatagagggt gcaaaactca 4440
   ataaacattt cgacgtacca agagttcgaa acaataagca aaatagattt ttttgcttca 4500
   gactaatttg tacaatgaat ggttaataaa ccattgaagc ttttattaat            4550
<210> 92
   <211> 4450
   <212> DNA
   <213> Arabidopsis sp.
<400> 92
   tttaggttac aaaatcaatg atattgcgta tgtcaactat aaaagccaaa agtaaagcct   60
   cttgtttgac cagaaggtca tgatcattgt atacatacag ccaaactacc tcctggaaga  120
   aaagacatgg atcccaaaca acaacaatag cttcttttac aagaaccagt agtaactagt  180
   cactaatcta aaagagttaa gtttcagctt ttctggcaat ggctccttga tcatttcaat  240
   cctgaaggag acccactttg tagcaagacc atgtcctctg tttcacttac agtgtgtctc  300
   aaaagtctac ttcaattctt catatatagg ttcctcacac tacagcttca tcctcattcg  360
   ttgacagaga gagagtcttt attgaaaact tcttccaagt acaactccac taaatataat  420
   agcaccaaac cacttgttcg acacaaatct gtacagatat aaaaacacta ttaggttttc  480
   caaggcaaat cacataattg gattgtgaaa gagtacaaaa gataaaccca aattttcata  540
   ctttctactg cagtcagcac cagatgataa gtcagctgtc cctatttgcc atcctaactg  600
   tcctgatgca gcggccagtg atgcgtaata ttgccaccct taatcattag agcgagaaac  660
   aaaaagaatc aaaagacagt aaatggaatt aggaatcaca aatgagtcct tgtaaagttt  720
   attgagtacc gagatctgca ctgaatccag aaagtgcaag aaaacctatg gatgctgtgc  780
   caaatccagt taaccaaagc tttgtattat caccgaatct aagggctgtt gacttaacac  840
   caacttttac atcatcttct ttgtcctgga gacacaatat attagacatt agtccatgga 900
   aaaaaaatga tttaacctag aatatctcaa aattacttgc ataaaaactg aacttgagct 960
   gaaattttgg gttcgtagct tgtggcatat actatttcat tttcaatggg ccacaaaggt 1020
   aactttcttt tctcacttct gttgcaaacg ggaagacttt tatggggcta actcttcact 1080
   taaagtatag aaatcagatg gaaaaggtgg gagatcaggg taattttctt ctttatgatt 1140
   gacaaaagtc gaacatcgaa atggatgcat ttgcatgaga catgaaacaa aagctgaaaa 1200
   agaaatctgt ggtggtgaag ctagaaaaag aaaacaaagc aagcaatatg cacacattga 1260
   gattaactac tttgctactg gtcataatca aatagatttt gaagctaaaa aataaaaagt 1320
   gaatatacct gatgtgcata aatagtatca taaacaaggg tccagcagac tccggagaga 1380
   tagagaggga gtacaataga tggtgctatg cttcctttaa ctgcagtcca tcctaacaat 1440
   gctccccagt ttatggtcaa acctaaaaag gcttgaggct gcaattataa aaacgaatca 1500
   atcataagaa aatcagaaaa tatataatgt ctaactttga gaagccagaa tagatttaaa 1560
   ttacccaaaa tgtaaacctc ttcataagtg ggtaggaaaa gacaagtaac aaagatgaag 1620
   cccctaaaac acggctgcag aatatacata ctgaaatgag ctcaagtaga aaagaatttg 1680
   atcacaaaac taaagacaag acctgagaac atatcttcag aatttgggcc aactacataa 1740
   gggtgaacca tatgtgtatg tgaattttta aacaaacact tgcaaatacg cgactttagg 1800
   gcaagtaaaa aatccaaaca aacctgtaat tgttaagttg gagaagaatc cctaagccta 1860
   aaagcaactg cagcccgaga aatccaatcc cttgaaatgg tgtcaaaaga ccactggcga 1920
   taggtcttag ttttgtacga tcaacctgga tataaaagaa atttgtaaga caacataatc 1980
   taaaacaaaa caaccataca aaatcttgag ctttacatac aagcaaccca tctttgttta 2040
   tggaagaatg aatccagtta catgaatgct gtgtatctac cctaactact aaacacatat 2100
   ttcaatcgaa aaacatattc caccttcacc atatctaaca cctgaagtct ttcacttttt 2160
   gaacgaagtc atcagaacat gcagataagc tattacccaa aacagagata tgactggaaa 2220
   tgttgtcgta aattgatcca acatagaaaa atcaagacca gttccagatg tcaaagcaat 2280
   aacactttcc caccatggtt acagaaacca tagttacaca aaacatgttt cctaaaccaa 2340
   catactaaag ggatatataa atttgacatc actttatcac cataccataa gatagcttaa 2400
   aaacaaactg acctttgtat ctatgtcctg atcaagcaga tcatttatag tacaaccagc 2460
   acctctaaga agtaatgctc cgcaaccaaa taaagccata tatttaaaac ttggaaggct 2520
   tccaggatca gcagccaacg caatcgacct atacaacaat gatggagatt cagagtatcg 2580
   atctatttac atagctctgg aactagatcc atgacgaaac atggaacatc gttataatat 2640
   ctaaagactt ccaaacagat tcctgagtaa gaaacccagt ggaactatag tactgtaaca 2700
   tatataaaat caaagaaaac tcaggtttat agcattatcc aatcctgatt tctgccaatc 2760
   cttaaccact ctcccatgct atcaaaaacc tcagctcaag atcatactac ctaattgcct 2820
   atgagctctt gggaagatca ttatggattt gataactgaa aaaagtaaca gagaaatagc 2880
   agactgcaag aactactcca aacttctcca ctgatatgta tgtagtctaa caataataaa 2940
   cagacataaa ttcttttatc aagcttcaag agcaagttag tcagaaaaca tcacagccaa 3000
   accaaccagg aaaacacata actttatcac ataaaactaa atttaatgta atctgactta 3060
   acataaacca tcctttggga cgaaaggaaa ctatataaac atgcagtctt tctttccctc 3120
   agctattctt tcggatggat tataatgaat ctcaaaagtg aaatgtcttg attctcagct 3180
   acattactca aaggcgaaga taaacttacc acatacaagg ccacgcaagc aaccaagttc 3240
   caatgggttt atccaatcga gcaagcttag cataacctct aacttcttct ggtaaataca 3300
   aatctatcca agaagcttcc ttaacaacaa caccatcact cttctcctta tcatctttct 3360
   tcggctttcc ctccaaaacc gaagaagacg acgacattcc acaaattaat ctgtaattcc 3420
   aaccaacacc aaaaaacttc tcctgatgca attctcttcc tttactccat acttggtaat 3480
   tatcattcca tgaaggataa cacttagtga aaggatttgt gtaatgggta gtcacaggat 3540
   tggacaagga tttatgttgt gattgcaaaa gagcagagga agaagatgga gttacggaga 3600
   cggaagattt caacaaccgt cttgaaacac gggagagccc aaaaaacgcc atctttgaga 3660
   gaaattgttg cctggaagaa acaaagactt gagatttcaa acgtaagtga attcttacga 3720
   acgaaagcta acttctcaag agaatcagat tagtgattcc tcaaaaacaa acaaaactat 3780
   ctaatttcag tttcgagtga tgaagcctta agaatctaga acctccatgg cgtttctaat 3840
   ctctcagaga taatcgaatt ccttaaacaa tcaaagctta gaaagagaag aacaacaaca 3900
   acaacaaaaa aaatcagatt aacaaccgac cagagagcaa cgacgacgcc ggcgagaaag 3960
   agcacgtcgt ctcggagcaa gacttcttct ccagtaaccc ggatggatcg ttaatgggcc 4020
   tgtagattat tatatttggg ccgaaacaat tgggtcagca aaaacttggg ggataatgaa 4080
   gaaacacgta cagtatgcat ttaggctcca aattaattgg ccatataatt cgaatcagat 4140
   aaactaatca acccctacct tacttatttc tcactgtttt tatttctacc ttagtagttg 4200
   aagaaacact tttatttatc ttttcgggac ccaaatttga taggatcggg ccattactca 4260
   tgagcgtcag acacatatta gccttatcag attagtgggg taaggttttt ttaattcggt 4320
   aagaagcaac aatcaatgtc ggagaaatta aagaatctgc atgggcgtgg cgtgatgata 4380
   tgtgcatatg gagtcagttg ccgatcatat ataactattt ataaactaca tataaagact 4440
   actaatagat 4450
<210> 93
   <211> 2850
   <212> DNA
   <213> Arabidopsis sp.
<400> 93
   aattaaaatt tgagcggtct aaaccattag accgtttaga gatccctcca acccaaaata   60
   gtcgattttc acgtcttgaa catatattgg gccttaatct gtgtggttag taaagacttt  120
   tattggtcaa agaaaaacaa ccatggccca acatgttgat acttttattt aattatacaa  180
   gtacccctga attctctgaa atatatttga ttgacccaga tattaatttt aattatcatt  240
   tcctgtaaaa gtgaaggagt caccgtgact cgtcgtaatc tgaaaccaat ctgttcatat  300
   gatgaagaag tttctctcgt tctcctccaa cgcgtagaaa attctgacgg cttaacgatg  360
   tggcgaagat ctgttgttta tcgtttctct tcaagaatct ctgtttcttc ttcgttacca  420
   aaccctagac tgattccttg gtcccgcgaa ttatgtgccg ttaatagctt ctcccagcct  480
   ccggtctcga cggaatcaac tgctaagtta gggatcactg gtgttagatc tgatgccaat  540
   cgagtttttg ccactgctac tgccgccgct acagctacag ctaccaccgg tgagatttcg  600
   tctagagttg cggctttggc tggattaggg catcactacg ctcgttgtta ttgggagctt  660
   tctaaagcta aacttaggta tgtgtttact tttcttttct catgaaaaat ctgaaaattt  720
   ccaattgttg gattcttaaa ttctcatttg ttttatggtt gtagtatgct tgtggttgca  780
   acttctggaa ctgggtatat tctgggtacg ggaaatgctg caattagctt cccggggctt  840
   tgttacacat gtgcaggaac catgatgatt gctgcatctg ctaattcctt gaatcaggtc  900
   attgaaatgt tgagaagttc ataaatttcg aatccttgtt gtgtttatgt agttgatctt  960
   gcttgcttat gtttatgtag ttgaaaagtt taaaaatttc taatccttgg tagttgatct 1020
   cgcttgtttg ttttttcatt ttctagattt ttgagataag caatgattct aagatgaaaa 1080
   gaacgatgct aaggccattg ccttcaggac gtattagtgt tccacacgct gttgcatggg 1140
   ctactattgc tggtgcttct ggtgcttgtt tgttggccag caaggtgaat gtttgttttt 1200
   ttatatgtga tttctttgtt ttatgaatgg gtgattgaga gattatggat ctaaactttt 1260
   gcttccacga caaggttatt gcagactaat atgttggctg ctggacttgc atctgccaat 1320
   cttgtacttt atgcgtttgt ttatactccg ttgaagcaac ttcaccctat caatacatgg 1380
   gttggcgctg ttgttggtgc tatcccaccc ttgcttgggt aaatttttgt tccttttctt 1440
   ctttatttta gcagattctg ttttgttgga tactgctttt aattcaaaat gtagtcatgg 1500
   ttcaccaatt ctatgcttat ctattttgtg tgttgtcagg tgggcggcag cgtctggtca 1560
   gatttcatac aattcgatga ttcttccagc tgctctttac ttttggcaga tacctcattt 1620
   tatggccctt gcacatctct gccgcaatga ttatgcagct ggagggtaag accatatggt 1680
   gtcatatgag attagaatgt ctccttccat gtagtgttga tcttgaacta gttcaatttc 1740
   gtggaatgat cagagtgtcc tagatagtgt cacagcagtc gacattttag tggctagata 1800
   atgagttctt tccgttagag ataaacattc gcgaacattg tttccagctt ccgcgaccca 1860
   acttctgatt ttgtttcttg gtaccttgtt ttcagttaca agatgttgtc actctttgat 1920
   ccgtcaggga agagaatagc agcagtggct ctaaggaact gcttttacat gatccctctc 1980
   ggtttcatcg cctatgactg tgagtcttgt agattcatct tttttttgta gtttattgac 2040
   tgcattgctg tatctgattt ttgctgttcc ttccaatttt tgtgacaggg gggttaacct 2100
   caagttggtt ttgcctcgaa tcaacacttc tcacactagc aatcgctgca acagcatttt 2160
   cattctaccg agaccggacc atgcataaag caaggaaaat gttccatgcc agtcttctct 2220
   tccttcctgt tttcatgtct ggtcttcttc tacaccgtgt ctctaatgat aatcagcaac 2280
   aactcgtaga agaagccgga ttaacaaatt ctgtatctgg tgaagtcaaa actcagaggc 2340
   gaaagaaacg tgtggctcaa cctccggtgg cttatgcctc tgctgcaccg tttcctttcc 2400
   tcccagctcc ttccttctac tctccatgat aacctttaag caagctattg aatttttgga 2460
   aacagaaatt aaaaaaaaaa tctgaaaagt tcttaagttt aatctttggt taataatgaa 2520
   gtggagaacg catacaagtt tatgtatttt ttctcatctc cacataattg tattttttct 2580
   ctaagtatgt ttcaaatgat acaaaataca tactttatca attatctgat caaattgatg 2640
   aatttttgag ctttgacgtg ttaggtctat ctaataaacg tagtaacgaa tttggttttg 2700
   gaaatgaaat ccgataaccg atgatggtgt agagttaaac gattaaaccg ggttggttaa 2760
   aggtctcgag tctcgacggc tgcggaaatc ggaaaatcac gattgaggac tttgagctgc 2820
   cacgaagatg gcgatgaggt tgaaatcaat                                  2850
<210> 94
   <211> 3660
   <212> DNA
   <213> Arabidopsis sp.
<400> 94
   tatttgtatt tttattgtta aattttatga tttcacccgg tatatatcat cccatattaa   60
   tattagattt attttttggg ctttatttgg gttttcgatt taaactgggc ccattctgct  120
   tcaatgaaac cctaatgggt tttgtttggg ctttggattt aaaccgggcc cattctgctt  180
   caatgaaggt cctttgtcca acaaaactaa catccgacac aactagtatt gccaagagga  240
   tcgtgccaca tggcagttat tgaatcaaag gccgccaaaa ctgtaacgta gacattactt  300
   atctccggta acggacaacc actcgtttcc cgaaacagca actcacagac tcacaccact  360
   ccagtctccg gcttaactac caccagagac gattctctct tccgtcggtt ctatgacttc  420
   gattctcaac actgtctcca ccatccactc ttccagagtt acctccgtcg atcgagtcgg  480
   agtcctctct cttcggaatt cggattccgt tgagttcact cgccggcgtt ctggtttctc  540
   gacgttgatc tacgaatcac ccggtagtta gcattctgtt ggatagattg atgaatgttt   600
   tcttcgattt tttttttact gatcttgttg tggatctctc gtagggcgga gatttgttgt   660
   gcgtgcggcg gagactgata ctgataaagg tatgattttt tagttgtttt tattttctct   720
   ctcttcaaaa ttctcttttc aaacactgtg gcgtttgaat ttccgacggc agttaaatct   780
   cagacacctg acaaggcacc agccggtggt tcaagcatta accagcttct cggtatcaaa   840
   ggagcatctc aagaaactgt aattttgttc atctcctcag aatcttttaa attatcatat   900
   ttgtggataa tgatgtgtta gtttaggaat tttcctacta aaggtaatct cttttgagga   960
   caagtcttgt ttttagctta gaaatgatgt gaaaatgttg tttgttagct aaaaagagtt  1020
   tgttgttata ttctgtattc agaataaatg gaagattcgt cttcagctta caaaaccagt  1080
   cacttggcct ccactggttt ggggagtcgt ctgtggtgct gctgcttcag gtaatcatac  1140
   gaacctcttt tggatcatgc aatactgtac agaaagtttt ttcattttcc ttccaattgt  1200
   ttcttctggc agggaacttt cattggaccc cagaggatgt tgctaagtcg attctttgca  1260
   tgatgatgtc tggtccttgt cttactggct atacacaggt ctggttttac acaacaaaaa  1320
   gctgacttgt tcttattcta gtgcatttgc ttggtgctac aataacctag acttgtcgat  1380
   ttccagacaa tcaacgactg gtatgataga gatatcgacg caattaatga gccatatcgt  1440
   ccaattccat ctggagcaat atcagagcca gaggtaactg agacagaaca ttgtgagctt  1500
   ttatctcttt tgtgattctg atttctcctt actccttaaa atgcaggtta ttacacaagt  1560
   ctgggtgcta ttattgggag gtcttggtat tgctggaata ttagatgtgt gggtaagttg  1620
   gcccttctga cattaactag tacagttaaa gggcacatca gatttgctaa aatcttccct  1680
   tatcaggcag ggcataccac tcccactgtc ttctatcttg ctttgggagg atcattgcta  1740
   tcttatatat actctgctcc acctcttaag gtaagtttta ttcctaactt ccactctcta  1800
   gtgataagac actccatcca agttttggag ttttgaatat cgatatctga actgatctca  1860
   ttgcagctaa aacaaaatgg atgggttgga aattttgcac ttggagcaag ctatattagt  1920
   ttgccatggt aagatatctc gtgtatcaat aatatatggc gttgttctca tctcattgat  1980
   ttgtttcttg ctcacttgac tgataggtgg gctggccaag cattgtttgg cactcttacg  2040
   ccagatgttg ttgttctaac actcttgtac agcatagctg gggtactctt ttggcaaacc  2100
   ttttatgttg cttttttcgt tatctgttgt aatatgctct tgcttcatgt tgtacctttg  2160
   tgataatgca gttaggaata gccattgtta acgacttcaa aagtgttgaa ggagatagag  2220
   cattaggact tcagtctctc ccagtagctt ttggcaccga aactgcaaaa tggatatgcg  2280
   ttggtgctat agacattact cagctttctg ttgccggtat gtactatcca ctgtttttgt  2340
   gcagctgtgg cttctatttc ttttccttga tcttatcaac tggatattca ccaatggtaa  2400
   agcacaaatt aatgaagctg aatcaacaaa ggcaaaacat aaaagtacat tctaatgaaa  2460
   tgagctaatg aagaggaggc atctactttt atgtttcatt agtgtgattg atggattttc  2520
   atttcatgct tctaaaacaa gtattttcaa cagtgtcatg aaataacaga acttatatct  2580
   tcatttgtac ttttactagt ggatgagtta cacaatcatt gttatagaac caaatcaaag  2640
   gtagagatca tcattagtat atgtctattt tggttgcagg atatctatta gcatctggga  2700
   aaccttatta tgcgttggcg ttggttgctt tgatcattcc tcagattgtg ttccaggtaa  2760
   agacgttaac agtctcacat tataattaat caaattcttg tcactcgtct gattgctaca  2820
   ctcgcttcta taaactgcag tttaaatact ttctcaagga ccctgtcaaa tacgacgtca  2880
   agtaccaggt aagtcaactt agtacacatg tttgtgttct tttgaaatat ctttgagagg  2940
   tctcttaatc agaagttgct tgaaacactc atcttgatta caggcaagcg cgcagccatt  3000
   cttggtgctc ggaatatttg taacggcatt agcatcgcaa cactgaaaaa ggcgtatttt  3060
   gatggggttt tgtcgaaagc agaggtgttg acacatcaaa tgtgggcaag tgatggcatc  3120
   aactagttta aaagattttg taaaatgtat gtaccgttat tactagaaac aactcctgtt  3180
   gtatcaattt agcaaaacgg ctgagaaatt gtaattgatg ttaccgtatt tgcgctccat  3240
   ttttgcattt cctgctcata tcgaggattg gggtttatgt tagttctgtc acttctctgc  3300
   tttcagaatg tttttgtttt ctgtagtgga ttttaactat tttcatcact ttttgtattg  3360
   attctaaaca tgtatccaca taaaaacagt aatatacaaa aatgatactt cctcaaactt  3420
   tttataatct aaatctaaca actagctagt aacccaacta acttcataca attaatttga  3480
   gaaactacaa agactagact atacatatgt tatttaacaa cttgaaactg tgttattact  3540
   acctgatttt tttctattct acagccattt gatatgctgc aatcttaaca tatcaagtct  3600
   cacgttgttg gacacaacat actatcacaa gtaagacacg aagtaaaacc aaccggcaac  3660
<210> 95
   <211> 1236
   <212> DNA
   <213> Glycine sp.
<400> 95
   atggattcac tgcttcttcg atctttccct aatattaata acgcctcttc tctcaccacc   60
   actggtgcaa atttctccag gactaaatct ttcgccaaca tttaccatgc aagttcttat  120
   gtgccaaatg cttcatggca caataggaaa atccaaaaag aatataattt tttgaggttt  180
   cggtggccaa gtttgaacca tcattacaaa ggcattgagg gagcgtgtac atgtaaaaaa  240
   tgtaatataa aatttgttgt gaaagcgacc tctgaaaaat ctcttgagtc tgaacctcaa  300
   gcttttgatc caaaaagcat tttggactct gtcaagaatt ccttggatgc tttctacagg  360
   ttttccaggc ctcacacagt tattggcaca gcattaagca taatttctgt gtctcttctt  420
   gctgttgaga aaatatcaga tatatctcca ttatttttta ctggtgtgtt ggaggctgtg  480
   gttgctgccc tgtttatgaa tatttatatt gttggtttga atcaattgtc tgatgttgaa  540
   atagacaaga taaacaagcc gtatcttcca ttagcatctg gggaatattc ctttgaaact   600
   ggtgtcacta ttgttgcatc tttttcaatt ctgagttttt ggcttggctg ggttgtaggt  660
   tcatggccat tattttgggc cctttttgta agctttgtgc taggaactgc ttattcaatc  720
   aatgtgcctc tgttgagatg gaagaggttt gcagtgcttg cagcgatgtg cattctagct  780
   gttcgggcag taatagttca acttgcattt ttccttcaca tgcagactca tgtgtacaag  840
   aggccacctg tcttttcaag accattgatt tttgctactg cattcatgag cttcttctct  900
   gtagttatag cactgtttaa ggatatacct gacattgaag gagataaagt atttggcatc  960
   caatcttttt cagtgcgttt aggtcagaag ccggtgttct ggacttgtgt tacccttctt 1020
   gaaatagctt atggagtcgc cctcctggtg ggagctgcat ctccttgtct ttggagcaaa 1080
   attttcacgg gtctgggaca cgctgtgctg gcttcaattc tctggtttca tgccaaatct  1140
   gtagatttga aaagcaaagc ttcgataaca tccttctata tgtttatttg gaagctattt 1200
   tatgcagaat acttactcat tccttttgtt agatga                           1236
<210> 96
   <211> 1188
   <212> DNA
   <213> Glycine sp.
<400> 96
<210> 97
   <211> 395
   <212> PRT
   <213> Glycine sp.
<400> 97
   Met Asp Ser Met Leu Leu Arg Ser Phe Pro Asn Ile Asn Asn Ala Ser
   1 5 10 15
   Ser Leu Ala Thr Thr Gly Ser Tyr Leu Pro Asn Ala Ser Trp His Asn
   20 25 30
   Arg Lys Ile Gln Lys Glu Tyr Asn Phe Leu Arg Phe Arg Trp Pro Ser
   35 40 45
   Leu Asn His His Tyr Lys Ser Ile Glu Gly Gly Cys Thr Cys Lys Lys
   50 55 60
   Cys Asn Ile Lys Phe Val Val Lys Ala Thr Ser Glu Lys Ser Phe Glu
   65 70 75 80
   Ser Glu Pro Gln Ala Phe Asp Pro Lys Ser Ile Leu Asp Ser Val Lys
   85 90 95
   Asn Ser Leu Asp Ala Phe Tyr Arg Phe Ser Arg Pro His Thr Val Ile
   100 105 110
   Gly Thr Ala Leu Ser Ile Ile Ser Val Ser Leu Leu Ala Val Glu Lys
   115 120 125
   Ile Ser Asp Ile Ser Pro Leu Phe Phe Thr Gly Val Leu Glu Ala Val
   130 135 140
   Val Ala Ala Leu Phe Met Asn Ile Tyr Ile Val Gly Leu Asn Gln Leu
   145 150 155 160
   Ser Asp Val Glu Ile Asp Lys Ile Asn Lys Pro Tyr Leu Pro Leu Ala
   165 170 175
   Ser Gly Glu Tyr Ser Phe Glu Thr Gly Val Thr Ile Val Ala Ser Phe
   180 185 190
   Ser Ile Leu Ser Phe Trp Leu Gly Trp Val Val Gly Ser Trp Pro Leu
   195 200 205
   Phe Trp Ala Leu Phe Val Ser Phe Val Leu Gly Thr Ala Tyr Ser Ile
   210 215 220
   Asn Val Pro Leu Leu Arg Trp Lys Arg Phe Ala Val Leu Ala Ala Met
   225 230 235 240
   Cys Ile Leu Ala Val Arg Ala Val Ile Val Gln Leu Ala Phe Phe Leu
   245 250 255
   His Ile Gln Thr His Val Tyr Lys Arg Pro Pro Val Phe Ser Arg Ser
   260 265 270
   Leu Ile Phe Ala Thr Ala Phe Met Ser Phe Phe Ser Val Val Ile Ala
   275 280 285
   Leu Phe Lys Asp Ile Pro Asp Ile Glu Gly Asp Lys Val Phe Gly Ile
   290 295 300
   Gln Ser Phe Ser Val Arg Leu Gly Gln Lys Pro Val Phe Trp Thr Cys
   305 310 315 320
   Val Ile Leu Leu Glu Ile Ala Tyr Gly Val Ala Leu Leu Val Gly Ala
   325 330 335
   Ala Ser Pro Cys Leu Trp Ser Lys Ile Val Thr Gly Leu Gly His Ala
   340 345 350
   Val Leu Ala Ser Ile Leu Trp Phe His Ala Lys Ser Val Asp Leu Lys
   355 360 365
   Ser Lys Ala Ser Ile Thr Ser Phe Tyr Met Phe Ile Trp Lys Leu Phe
   370 375 380
   Tyr Ala Glu Tyr Leu Leu Ile Pro Phe Val Arg
   385 390 395
<210> 98
   <211> 411
   <212> PRT
   <213> Glycine sp.
<400> 98
   Met Asp Ser Leu Leu Leu Arg Ser Phe Pro Asn Ile Asn Asn Ala Ser
   1               5                   10                  15
   Ser Leu Thr Thr Thr Gly Ala Asn Phe Ser Arg Thr Lys Ser Phe Ala
                   20                  25                  30
   Asn Ile Tyr His Ala Ser Ser Tyr Val Pro Asn Ala Ser Trp His Asn
               35                  40                  45
   Arg Lys Ile Gln Lys Glu Tyr Asn Phe Leu Arg Phe Arg Trp Pro Ser
           50                  55                  60
   Leu Asn His His Tyr Lys Gly Ile Glu Gly Ala Cys Thr Cys Lys Lys
   65                  70                  75                  80
   Cys Asn Ile Lys Phe Val Val Lys Ala Thr Ser Glu Lys Ser Leu Glu
                   85                  90                  95
   Ser Glu Pro Gln Ala Phe Asp Pro Lys Ser Ile Leu Asp Ser Val Lys
               100                 105                 110
   Asn Ser Leu Asp Ala Phe Tyr Arg Phe Ser Arg Pro His Thr Val Ile
           115                 120                 125
   Gly Thr Ala Leu Ser Ile Ile Ser Val Ser Leu Leu Ala Val Glu Lys
       130                 135                 140
   Ile Ser Asp Ile Ser Pro Leu Phe Phe Thr Gly Val Leu Glu Ala Val
   145                 150                 155                 160
   Val Ala Ala Leu Phe Met Asn Ile Tyr Ile Val Gly Leu Asn Gln Leu
                   165                 170                 175
   Ser Asp Val Glu Ile Asp Lys Ile Asn Lys Pro Tyr Leu Pro Leu Ala
               180                 185                 190
   Ser Gly Glu Tyr Ser Phe Glu Thr Gly Val Thr Ile Val Ala Ser Phe
           195                 200                 205
   Ser Ile Leu Ser Phe Trp Leu Gly Trp Val Val Gly Ser Trp Pro Leu
       210                 215                 220
   Phe Trp Ala Leu Phe Val Ser Phe Val Leu Gly Thr Ala Tyr Ser Ile
   225                 230                 235                 240
   Asn Val Pro Leu Leu Arg Trp Lys Arg Phe Ala Val Leu Ala Ala Met
                   245                 250                 255
   Cys Ile Leu Ala Val Arg Ala Val Ile Val Gln Leu Ala Phe Phe Leu
               260                 265                 270
   His Met Gln Thr His Val Tyr Lys Arg Pro Pro Val Phe Ser Arg Pro
           275                 280                 285
   Leu Ile Phe Ala Thr Ala Phe Met Ser Phe Phe Ser Val Val Ile Ala
       290                 295                 300
   Leu Phe Lys Asp Ile Pro Asp Ile Glu Gly Asp Lys Val Phe Gly Ile
   305                 310                 315                 320
   Gln Ser Phe Ser Val Arg Leu Gly Gln Lys Pro Val Phe Trp Thr Cys
                   325                 330                 335
   Val Thr Leu Leu Glu Ile Ala Tyr Gly Val Ala Leu Leu Val Gly Ala
               340                 345                 350
   Ala Ser Pro Cys Leu Trp Ser Lys Ile Phe Thr Gly Leu Gly His Ala
           355                 360                 365
   Val Leu Ala Ser Ile Leu Trp Phe His Ala Lys Ser Val Asp Leu Lys
   370 375 380
   Ser Lys Ala Ser Ile Thr Ser Phe Tyr Met Phe Ile Trp Lys Leu Phe
   385 390 395 400
   Tyr Ala Glu Tyr Leu Leu Ile Pro Phe Val Arg
   405 410
<210> 99
   <211> 964
   <212> DNA
   <213> Oryza sp.
<400> 99
   gagcagcact gggtcttaca ttccaatgga gctcgcctgt tgctttcatt acatgcttcg   60
   tgactttatt tgctttggtc attgctataa ccaaagatct cccagatgtt gaaggggatc  120
   ggaagtatca aatatcaact ttggcgacaa agctcggtgt cagaaacatt gcatttcttg  180
   gctctggttt attgatagca aattatgttg ctgctattgc tgtagctttt ctcatgcctc  240
   aggctttcag gcgcactgta atggtgcctg tgcatgctgc ccttgccgtt ggtataattt  300
   tccagacatg ggttctggag caagcaaaat atactaagga tgctatttca cagtactacc  360
   ggttcatttg gaatctcttc tatgctgaat acatcttctt cccgttgata tagagaccaa  420
   gcaatctgat atcgtctgca tgttgagtgc ggcaaaaact agaagcccat atgaacagtg  480
   ggagtagggg aacgaacatg ccatccatgg gaagactctg ataactctct ctcgcccggg  540
   ctgtaaaggg taagcactgt tgggcatata tatgaaagga aggtgataaa gcagggatgc  600
   taaattgcta ctgggatcct caaaggctta tagtggtcac cagtggaatg tgccttaata  660
   atttggttac ccagcagagc aagtttttgc aggttattag gtaatatctt tgagggaatg  720
   aacttagatt tcattgtttt aaggtctggt cacacaacgg gtagtagtgc tggagcggca  780
   aaaaacgacc ttgttttaca ctaccaaggg aggttaactc tagttttcat gtgaccactt  840
   accttgagag ttgagaccat ggaatcactt gtcgactcct cggcttgtat atttctagtg  900
   tcagcatttg cattctcctc cccacttgta cttgaaaagt tgaagacaac ttttttgttt  960
   gtgt                                                               964
<210> 100
   <211> 421
   <212> DNA
   <213> Triticum sp.
<400> 100
   cgtccgcgga cgcgtgggtg cttattcagt caatctgccg cactttctat ggaagagatc   60
   tgctgttgtt gcagcactct gcatattagc agtgcgtgcg gtgatagttc aactggcatt  120
   ttttctccac attcagacat ttgttttcag aaggccggca gacttttcaa agccattgat  180
   atttgcaact gccttcatga cattcttctc agttgtaata gcattattca aggatatacc  240
   cgatattgaa ggggaccgca tctttggaat ccaatctttt agtggtagac taggtcaaag  300
   caggggtttc tggacttgcg ttggcctact tgaggttgcc tacggtgttg cgatactgag  360
   gggggtaact tcttccagtt tgtggagcaa atctataact gttgtgggcc atgcaatcct  420
   c                                                                  421
<210> 101
   <211> 705
   <212> DNA
   <213> Allium porrum
<400> 101
   gtttcccccc ctcgaatttt tttttttttt ttttacttca tttttctgtg aataaattct 60
   taaaaaagac aaagaaaacc actggatatc ctaaattcaa cataggctat tgtcattcaa 120
   tgataatctt taacacaaca tacaacatga atataattaa ggagaaatga tctgcaattg 180
   ttgaaagaac tctccgtttt taagatgaca attaaagcgt tgttaattcc agccatttct 240
   gcctccatta tctactcatc ttctcttgcg attcttttcc atgtaggtca taaaccctca 300
   tcttacaaaa ggaatgagca agtactcagc atagaagagc ttccacacga acatataaaa 360
   agatgtaata gtggttttgg tcattggtcc atgagatcta gcacgattcc aaagtaacga 420
   cccaagaatt gcatgaccta tcactgttaa gcatttgctc cataggcatg aggaagtagc 480
   tccaacaacc atgacaacag tgtaggccat ctcaaggaga tatatacata tccaaaacac 540
   cctctcctgg ccaaggcgca cgctgaaaga atggatgcca aatattttgt ctccgtctat 600
   atcaggtata tccttaaata gagcaataac aactgagaag aagctcatga aggcagttgc 660
   aaatatcaat ggccttgtga aacttgctgg tcttttgaaa acaaa 705
<210> 102
   <211> 637
   <212> DNA
   <213> Allium porrum
<220>
   <221> misc_feature
   <222> (1)...(637)
   <223> n = A, T, C, or G
<400> 102
   nattcggcac gagttttgaa gaagttaagc atggactccc tccttaccaa gccagttgta 60
   atacctctgc cttctccagt ttgttcacta ccaatcttgc gaggcagttc tgcaccaggg 120
   cagtattcat gtagaaacta caatccaata agaattcaaa ggtgcctcgt aaattatgaa 180
   catgtgaaac caaggtttac aacatgtagt aggtctcaaa aacttggtca tgtaaaagcc 240
   acatccgagc attctttaga atctggatcc gaaggataca ctcctagaag catatgggaa 300
   gccgtactag cttcactgaa tgttctatac aaattttcac gacctcacac aataatagga 360
   acagcaatgg gcataatgtc agtttctttg cttgttgtcg agagcctatc cgatatttct 420
   cctctgtttt ttgtgggatt attagaggct gtggttgctg cattgtttat gaatgtttac 480
   attgtaggtc tgaatcaatt atttgacata gaaatagaca aggtcaataa acctgatctt 540
   cctcttgcat ctggagaata ctcaccaaga gctggtactg ctattgtcat tgcttcagcc 600
   atcatgagct ttggcattgg atggttagtt ggctctt 637
<210> 103
   <211> 677
   <212> DNA
   <213> Brassica napus
<400> 103
   tttttttttt tttttttcaa aaagaccaat cctttagtat gtacatgaac aaagtgattt 60
   tgtctccaag ctacaaagaa gaagaagaga ggtatacaaa gaaaactaca aatgttcacc 120
   atgaatgcta gaagaagggg aataacagat actctgcgta gaagagattc catataaacc 180
   ggtaatatcc tgctatagct tcctttgtgt agtttgcttt ttctagcacc catgtctgga 240
   aaaccaagca tgaagccaag atcatatgtg caggaatcat caagctacct ctaaaaacct 300
   gaggcatgta gaaagctagt gatatggcag aaatatagtt cactagcaga agtccagaac 360
   cgaggaatgc aatgttcctc actccaagct ttgttgctag tgttgatatt tggaacttgc 420
   gatctccttc aacatcagga agatcttttg taatagcaat gactagtgca aacagtgtca 480
   caaaagacgt gatgaaagcc acaggtgcac tccactgaaa cgaaagtcca agagcagctc 540
   tagtagcatg gtacacacca aaattaagaa gaaaacctcg taccgtggca ataataagaa 600
   acgctgcaac tggaaatctc ttcattctaa atggtggaac agaatagatg gtccccagat 660
   cggacgcgtg ggtcgac 677
<210> 104
   <211> 1431
   <212> DNA
   <213> Zea sp.
<400> 104
   ccacgcgtcc gcccggccaa gggatggacg cgcttcgcct acggccgtcc ctcctccccg    60
   tgcggcccgg cgcggcccgc ccgcgagatc attttctacc accatgttgt tccatacaac   120
   gaaatggtga aggacgaatt tgcttttcta gccaaaggac ccaaggtcct accttgcatc   180
   accatcagaa attcttcgaa tggaaatcct cctattgtag gatatcacat cggtcattaa   240
   atacttctgt taatgcttcg gggcaacagc tgcagtctga acctgaaaca catgattcta   300
   caaccatctg gagggcaata tcatcttctc tagatgcatt ttacagattt tcccggccac   360
   atactgtcat aggaacagca ttaagcatag tctcagtttc ccttctagct gtccagagct   420
   tgtctgatat atcacctttg ttcctcactg gtttgctgga ggcagtggta gctgcccttt   480
   tcatgaatat ctatattgtt ggactgaacc agttattcga cattgagata gacaaggtta   540
   acaagccaac tcttccattg gcatctgggg aatacaccct tgcaactggg gttgcaatag   600
   tttcggtctt tgccgctatg agctttggcc ttggatgggc tgttggatca caacctctgt   660
   tttgggctct tttcataagc tttgttcttg ggactgcata ttcaatcaat ctgccgtacc   720
   ttcgatggaa gagatttgct gttgttgcag cactgtgcat attagcagtt cgtgcagtga   780
   ttgttcagct ggcctttttt ctccacattc agacttttgt tttcaggaga ccggcagtgt   840
   tttctaggcc attattattt gcaactggat ttatgacgtt cttctctgtt gtaatagcac   900
   tattcaagga tatacctgac atcgaagggg accgcatatt cgggatccga tccttcagcg   960
   tccggttagg gcaaaagaag gtcttttgga tctgcgttgg cttgcttgag atggcctaca  1020
   gcgttgcgat actgatggga gctacctctt cctgtttgtg gagcaaaaca gcaaccatcg  1080
   ctggccattc catacttgcc gcgatcctat ggagctgcgc gcgatcggtg gacttgacga  1140
   gcaaagccgc aataacgtcc ttctacatgt tcatctggaa gctgttctac gcggagtacc  1200
   tgctcatccc tctggtgcgg tgagcgcgag gcgaggtggt ggcagacgga tcggcgtcgg  1260
   cggggcggca aacaactcca cgggagaact tgagtgccgg aagtaaactc ccgtttgaaa  1320
   gttgaagcgt gcaccaccgg caccgggcag agagagacac ggtggctgga tggatacgga  1380
   tggccccccc aataaattcc cccgtgcatg gtaaaaaaaa aaaaaaaaaa a           1431
<210> 105
   <211> 1870
   <212> DNA
   <213> Zea sp.
<400> 105
   gccgcgcagc gcgacgagcg ccacctgctt gctgccgcgt gcctgcgtgc gtgtgcgtcc   60
   accactgacc ccgcgcccgc ccgccgcccc tgcccctcca ctccacttgc tcactcgtcg  120
   cggcccgctt cccccccggc caagggatgg acgcgcttcg cctacggccg tccctcctcc  180
   ccgtgcggcc cggcgcggcc cgcccgcgag atcattttct accaccatgt tgttccatac  240
   aacgaaatgg tgaaggacga atttgctttt ctagccaaag gacccaaggt cctaccttgc  300
   atcaccatca gaaattcttc gaatggaaat cctcctattg taggatatca catcggtcat  360
   taaatacttc tgttaatgct tcggggcaac agctgcagtc tgaacctgaa acacatgatt  420
   ctacaaccat ctggagggca atatcatctt ctctagatgc attttacaga ttttcccggc  480
   cacatactgt cataggaaca gcattaagca tagtctcagt ttcccttcta gctgtccaga  540
   gcttgtctga tatatcacct ttgttcctca ctggtttgct ggaggcagtg gtagctgccc  600
   ttttcatgaa tatctatatt gttggactga accagttatt cgacattgag atagacaagg  660
   ttaacaagcc aactcttcca ttggcatctg ggaatacacc ccttgcaact ggggttgcaa  720
   tagtttcggt ctttgccgct atgagctttg gccttggatg ggctgttgga tcacaacctc  780
   tgttttgggc tcttttcata agctttgttc ttgggactgc atattcaatc aatctgccgt  840
   accttcgatg gaagagattt gctgttgttg cagcactgtg catattagca gttcgtgcag  900
   tgattgttca gctggccttt ttctccacaa ttcagacttt tgttttcagg agaccggcag  960
   tgttttctag gccattatta tttgcaactg gatttatgac gttcttctct gttgtaatag 1020
   cactattcaa ggatatacct gacatcgaag gggaccgcat attcgggatc cgatccttca 1080
   gcgtccggtt agggcaaaag aaggtctttt ggatctgcgt tggcttgctt gagatggcct 1140
   acagcgttgc gatactgatg ggagctacct cttcctgttt gtggagcaaa acagcaacca 1200
   tcgctggcca ttccatactt gccgcgatcc tatggagctg cgcgcgatcg gtggacttga 1260
   cgagcaaagc cgcaataacg tccttctaca tgttcatctg gaagctgttc tacgcggagt 1320
   acctgctcat ccctctggtg cggtgagcgc gaggcgaggt ggtggcagac ggatcggcgt 1380
   cggcggggcg gcaaacaact ccacgggaga acttgagtgc cggaagtaaa ctcccgtttg 1440
   aaagttgaag cgtgcaccac cggcaccggg cagagagaga cacggtggct ggatggatac 1500
   ggatggcccc cccaataaat tcccccgtgc atggtacccc acgctgcttg atgatatccc 1560
   atgtgtccgg gtgaccggac ctgatcgtct ctagagagat tggttgcaca acgtccaaca 1620
   tagcccgtag gtattgctac cactgctagt atgatactcc ttcctagtcc ttgccagcac 1680
   cagtgaccca aacttggtcg gctgagctca gcgctcagca gctttacgtg catctgcgcc 1740
   ttgacttgtg cagtgggcgt cgctagcatg aatgatgtat ggtgcgtcac ggcctgacgg 1800
   ttcgtcagtc tgggccgtgt tttgtgtccg aggaagatcg tctgtcagag atctggattg 1860
   cctcgctgct                                                        1870
<210> 106
   <211> 642
   <212> DNA
   <213> Zea sp.
<400> 106
   cggccggact cttctgactt ggcaaccgcc gcgcagcgcg acgagcgcca cctgcttgct   60
   gccgcgtgcc tgcgtgcgtg tgcgtccacc actgaccccg cgcccgcccg ccgcccctgc  120
   ccctccactc cacttgctca ctcgtcggct cgtcgcggcc cgcttccccc ccggccaagg  180
   gatggacgcg cttcgcctac ggccgtccct cctccccgtg cggcccggcg cggcccgccc  240
   gcgaggcagt ggtagctgcc cttttcatga atatctatat tgttggactg aaccagttat  300
   tcgacattga gatagacaag gttaacaagc caactcttcc attggcatct ggggaataca  360
   cccttgcaac tggggttgca atagtttcgg tctttgccgc tatgagcttt ggccttggat  420
   gggctgttgg atcacaacct ctgttttggg ctcttttcat aagctttgtt cttgggactg  480
   catattcaat caatctgccg taccttcgat ggaagagatt tgctgttgtt gcagcactgt  540
   gcatattagc agttcgtgca gtgattgttc agctggcctt ttttctccac attcagactt  600
   ttgttttcag gagaccggca gtgttttcta ggccattatt at                     642
<210> 107
   <211> 362
   <212> DNA
   <213> Gossypium sp.
<400> 107
   cccacgcgtc cgaacattgt ttgcacttgt tattgccata accaaggatc ttccagatgt 60
   agaaggagat cgcaaatttc aaatatcaac attagcaaca aagcttggag ttagaaatat 120
   tgcatttctt ggttccggac ttctactggt gaattatgtt gctgctgtgt tggctgcaat 180
   atacatgcct caggctttca ggcgtagttt aatgatacct gctcatatct ttttggcggt 240
   ctgcttgatt tttcagacat gggtgttgga acaagcaaat tacaaaaagg aagcaatctc 300
   ggggttctat cgtttcatat ggaatctctt ctatgcagag tatgcgattt tccccttcgt 360
   gt 362
<210> 108
   <211> 575
   <212> DNA
   <213> Lycopersicon sp.
<400> 108
   cagatcaatt ccagttcctg ctgagttttc tccactcaaa accagttcac atgcaatagt 60
   acgggttttg aaatgtaaag catggaagag accaaaaaag cactattcct cttcaatgaa 120
   gttgcagcgg cagtatatca cgcaagagca tgttggagga agtgatctaa gcactattgc 180
   tgctgataaa aaacttaaag ggagattttt ggtgcacgca tcatctgaac accctcttga 240
   atctcaacct tctaaaagtc cttgggactc agttaatgat gccgtagatg ctttctacag 300
   gttctcgcgg ccccatacca taataggaac agcattgagc ataatttcag tttctctcct 360
   tgcagttgag aagttctctg atttttctcc attatttttc actggggtgt tagaggccat 420
   tgttgctgcc ctattcatga acatttacat agttggttta aaccagttgt ctgacatcga 480
   aatagacaag gtaaacaagc catatcttcc attggcatca ggggaatact ctgtacaaac 540
   tggagtgatt gttgtgtcgt cttttgccat tttga 575
<210> 109
   <211> 1663
   <212> DNA
   <213> Arabidopsis sp.
<400> 109
   aacaccaaac acacaatttc acattctttt gcatatttct tcttcttctt ccattatgga 60
   gatacggagc ttgattgttt ctatgaaccc taatttatct tcctttgagc tctctcgccc 120
   tgtatctcct ctcactcgct cactagttcc gttccgatcg actaaactag ttccccgctc 180
   catttctagg gggatcccgt cgatctccac cccgaatagt gaaactgaca agatctccgt 240
   taaacctgtt tacgtcccga cgtctcccaa tcgcgaactc cggactcctc acagtggata 300
   ccatttcgat ggaacacctc ggaagttctt cgagggatgg tggatccggg tttccatccc 360
   agagaagagg gagagttttt gttttatgta ttctgtggag aatcctgcat ttcggcagag 420
   tttgtcacca ttggaagtgg ctctatatgg acctagattc actggtgttg gagctcagat 480
   tcttggcgct aatgataaat atttatgcca atacgaacaa gactctcaca atttctgggg 540
   agatcgacat gagctagttt tggggaatac ttttagtgct gtgccaggcg caaaggctcc 600
   aaacaaggag gttccaccag aggaatttaa cagaagagtg tccgaagggt tccaagctac 660
   tccattttgg catcaaggtc acatttgcga tgatggccgt actgactatg cggaaactgt 720
   gaaatctgct cgttgggagt atagtactcg tcccgtttac ggttggggtg atgttggggc 780
   caaacagaag tcaactgcag gctggcctgc agcttttcct gtatttgagc ctcattggca 840
   gatatgcatg gcaggaggcc tttccacagg gtggatagaa tggggcggtg aaaggtttga 900
   gtttcgggat gcaccttctt attcagagaa gaattggggt ggaggcttcc caagaaaatg 960
   gttttgggtc cagtgtaatg tctttgaagg ggcaactgga gaagttgctt taaccgcagg 1020
   tggcgggttg aggcaattgc ctggattgac tgagacctat gaaaatgctg cactggtttg 1080
   tgtacactat gatggaaaaa tgtacgagtt tgttccttgg aatggtgttg ttagatggga 1140
   aatgtctccc tggggttatt ggtatataac tgcagagaac gaaaaccatg tggtggaact 1200
   agaggcaaga acaaatgaag cgggtacacc tctgcgtgct cctaccacag aagttgggct 1260
   agctacggct tgcagagata gttgttacgg tgaattgaag ttgcagatat gggaacggct 1320
   atatgatgga agtaaaggca aggtgatatt agagacaaag agctcaatgg cagcagtgga 1380
   gataggagga ggaccgtggt ttgggacatg gaaaggagat acgagcaaca cgcccgagct 1440
   actaaaacag gctcttcagg tcccattgga tcttgaaagc gccttaggtt tggtcccttt 1500
   cttcaagcca ccgggtctgt aacattgatg agtgttttgt ttgttgatag agacccatgt 1560
   gatgaatgaa gccttagtca tgtcattgct agcttcacta ttatgtatgt atgattttag 1620
   ttcgttcggt ccttgtggta aatgatacgg gccagtgtaa agt                   1663
<210> 110
   <211> 488
   <212> PRT
   <213> Arabidopsis sp.
<400> 110
   Met Glu Ile Arg Ser Leu Ile Val Ser Met Asn Pro Asn Leu Ser Ser
   1               5                   10                  15
   Phe Glu Leu Ser Arg Pro Val Ser Pro Leu Thr Arg Ser Leu Val Pro
               20                  25                  30
   Phe Arg Ser Thr Lys Leu Val Pro Arg Ser Ile Ser Arg Val Ser Ala
           35                  40                  45
   Ser Ile Ser Thr Pro Asn Ser Glu Thr Asp Lys Ile Ser Val Lys Pro
       50                  55                  60
   Val Tyr Val Pro Thr Ser Pro Asn Arg Glu Leu Arg Thr Pro His Ser
   65                  70                  75                  80
   Gly Tyr His Phe Asp Gly Thr Pro Arg Lys Phe Phe Glu Gly Trp Tyr
                   85                  90                  95
   Phe Arg Val Ser Ile Pro Glu Lys Arg Glu Ser Phe Cys Phe Met Tyr
               100                 105                 110
   Ser Val Glu Asn Pro Ala Phe Arg Gln Ser Leu Ser Pro Leu Glu Val
           115                 120                 125
   Ala Leu Tyr Gly Pro Arg Phe Thr Gly Val Gly Ala Gln Ile Leu Gly
       130                 135                 140
   Ala Asn Asp Lys Tyr Leu Cys Gln Tyr Glu Gln Asp Ser His Asn Phe
   145                 150                 155                 160
   Trp Gly Asp Arg His Glu Leu Val Leu Gly Asn Thr Phe Ser Ala Val
                   165                 170                 175
   Pro Gly Ala Lys Ala Pro Asn Lys Glu Val Pro Pro Glu Glu Phe Asn
               180                 185                 190
   Arg Arg Val Ser Glu Gly Phe Gln Ala Thr Pro Phe Trp His Gln Gly
           195                 200                 205
   His Ile Cys Asp Asp Gly Arg Thr Asp Tyr Ala Glu Thr Val Lys Ser
       210                 215                 220
   Ala Arg Trp Glu Tyr Ser Thr Arg Pro Val Tyr Gly Trp Gly Asp Val
   225                 230                 235                 240
   Gly Ala Lys Gln Lys Ser Thr Ala Gly Trp Pro Ala Ala Phe Pro Val
                   245                 250                 255
   Phe Glu Pro His Trp Gln Ile Cys Met Ala Gly Gly Leu Ser Thr Gly
   260 265 270
   Trp Ile Glu Trp Gly Gly Glu Arg Phe Glu Phe Arg Asp Ala Pro Ser
   275 280 285
   Tyr Ser Glu Lys Asn Trp Gly Gly Gly Phe Pro Arg Lys Trp Phe Trp
   290 295 300
   Val Gln Cys Asn Val Phe Glu Gly Ala Thr Gly Glu Val Ala Leu Thr
   305 310 315 320
   Ala Gly Gly Gly Leu Arg Gln Leu Pro Gly Leu Thr Glu Thr Tyr Glu
   325 330 335
   Asn Ala Ala Leu Val Cys Val His Tyr Asp Gly Lys Met Tyr Glu Phe
   340 345 350
   Val Pro Trp Asn Gly Val Val Arg Trp Glu Met Ser Pro Trp Gly Tyr
   355 360 365
   Trp Tyr Ile Thr Ala Glu Asn Glu Asn His Val Val Glu Leu Glu Ala
   370 375 380
   Arg Thr Asn Glu Ala Gly Thr Pro Leu Arg Ala Pro Thr Thr Glu Val
   385 390 395 400
   Gly Leu Ala Thr Ala Cys Arg Asp Ser Cys Tyr Gly Glu Leu Lys Leu
   405 410 415
   Gln Ile Trp Glu Arg Leu Tyr Asp Gly Ser Lys Gly Lys Val Ile Leu
   420 425 430
   Glu Thr Lys Ser Ser Met Ala Ala Val Glu Ile Gly Gly Gly Pro Trp
   435 440 445
   Phe Gly Thr Trp Lys Gly Asp Thr Ser Asn Thr Pro Glu Leu Leu Lys
   450 455 460
   Gln Ala Leu Gln Val Pro Leu Asp Leu Glu Ser Ala Leu Gly Leu Val
   465 470 475 480
   Pro Phe Phe Lys Pro Pro Gly Leu
   485
<210> 111
   <211> 246
   <212> PRT
   <213> Arabidopsis sp.
<400> 111
   Met Ser Ser Ser Asn Ala Cys Ala Ser Pro Ser Pro Phe Pro Ala Val
   1 5 10 15
   Thr Lys Leu His Val Asp Ser Val Thr Phe Val Pro Ser Val Lys Ser
   20 25 30
   Pro Ala Ser Ser Asn Pro Leu Phe Leu Gly Gly Ala Gly Val Arg Gly
   35 40 45
   Leu Asp Ile Gln Gly Lys Phe Val Ile Phe Thr Val Ile Gly Val Tyr
   50 55 60
   Leu Glu Gly Asn Ala Val Pro Ser Leu Ser Val Lys Trp Lys Gly Lys
   65 70 75 80
   Thr Thr Glu Glu Leu Thr Glu Ser Ile Pro Phe Phe Arg Glu Ile Val
   85 90 95
   Thr Gly Ala Phe Glu Lys Phe Ile Lys Val Thr Met Lys Leu Pro Leu
   100 105 110
   Thr Gly Gln Gln Tyr Ser Glu Lys Val Thr Glu Asn Cys Val Ala Ile
   115 120 125
   Trp Lys Gln Leu Gly Leu Tyr Thr Asp Cys Glu Ala Lys Ala Val Glu
   130 135 140
   Lys Phe Leu Glu Ile Phe Lys Glu Glu Thr Phe Pro Pro Gly Ser Ser
   145 150 155 160
   Ile Leu Phe Ala Leu Ser Pro Thr Gly Ser Leu Thr Val Ala Phe Ser
   165 170 175
   Lys Asp Asp Ser Ile Pro Glu Thr Gly Ile Ala Val Ile Glu Asn Lys
   180 185 190
   Leu Leu Ala Glu Ala Val Leu Glu Ser Ile Ile Gly Lys Asn Gly Val
   195 200 205
   Ser Pro Gly Thr Arg Leu Ser Val Ala Glu Arg Leu Ser Gln Leu Met
   210 215 220
   Met Lys Asn Lys Asp Glu Lys Glu Val Ser Asp His Ser Leu Glu Glu
   225 230 235 240
   Lys Leu Ala Lys Glu Asn
   245
<210> 112
   <211> 3115
   <212> DNA
   <213> Arabidopsis sp.
<400> 112
<210> 113
   <211> 536
   <212> DNA
   <213> Arabidopsis sp.
<400> 113
   caccccaaac atcacaattt cacattcttt tgcatatttc ttcttcttct tccattatgg 60
   agatacggag cttgattgtt tctatgaacc ctaatttatc ttcctttgag ctctctcgcc 120
   ctgtatctcc tctcactcgc tcactagttc cgttccgatc gactaaacta gttccccgct 180
   ccatttctag ggtttcggcg tcgatctcca ccccgaatag tgaaactgac aagatctccg 240
   ttaaacctgt ttacgtcccg acgtctccca atcgcgaact ccggactcct cacagtggat 300
   accatttcga tggaacacct cggaagttct tcgagggatg gtatttcagg gtttccatcc 360
   cagagaagag ggagagtttt tgttttatgt attctgtgga gaatcctgca tttcggcaga 420
   gtttgtcacc attggaagtg gctctatatg gacctagatt cactggtgtt ggagctcaga 480
   ttcttggcgc taatgataaa tatttatgcc aatacgaaca agactctcac aatttc 536
<210> 114
   <211> 411
   <212> PRT
   <213> Arabidopsis sp.
<220>
   <223> Peptide PIR: T04448 shown in Figure 31
<400> 114
   Pro Glu Lys Arg Glu Ser Phe Cys Phe Met Tyr Ser Val Glu Asn Pro
   1               5                   10                  15
   Ala Phe Arg Gln Ser Leu Ser Pro Leu Glu Val Ala Leu Tyr Gly Pro
               20                  25                  30
   Arg Phe Thr Gly Val Gly Ala Gln Ile Leu Gly Ala Asn Asp Lys Tyr
           35                  40                  45
   Leu Cys Gln Tyr Glu Gln Asp Ser His Asn Phe Trp Gly Asp Arg His
       50                  55                  60
   Glu Leu Val Leu Gly Asn Thr Phe Ser Ala Val Pro Gly Ala Lys Ala
   65                  70                  75                  80
   Pro Asn Lys Glu Val Pro Pro Glu Glu Phe Asn Arg Arg Val Ser Glu
                   85                  90                  95
   Gly Phe Gln Ala Thr Pro Phe Trp His Gln Gly His Ile Cys Asp Asp
               100                 105                 110
   Gly Arg Thr Asp Tyr Ala Glu Thr Val Lys Ser Ala Arg Trp Glu Tyr
           115                 120                 125
   Ser Thr Arg Pro Val Tyr Gly Trp Gly Asp Val Gly Ala Lys Gln Lys
       130                 135                 140
   Ser Thr Ala Gly Trp Pro Ala Ala Phe Pro Val Phe Glu Pro His Trp
   145                 150                 155                 160
   Gln Ile Cys Met Ala Gly Gly Leu Ser Thr Gly Trp Ile Glu Trp Gly
                   165                 170                 175
   Gly Glu Arg Phe Glu Phe Arg Asp Ala Pro Ser Tyr Ser Glu Lys Asn
               180                 185                 190
   Trp Gly Gly Gly Phe Pro Arg Lys Trp Phe Trp Val Gln Cys Asn Val
           195                 200                 205
   Phe Glu Gly Ala Thr Gly Glu Val Ala Leu Thr Ala Gly Gly Gly Leu
       210                 215                 220
   Arg Gln Leu Pro Gly Leu Thr Glu Thr Tyr Glu Asn Ala Ala Leu Val
   225                 230                 235                 240
   Cys Val His Tyr Asp Gly Lys Met Tyr Glu Phe Val Pro Trp Asn Gly
                   245                 250                 255
   Val Val Arg Trp Glu Met Ser Pro Trp Gly Tyr Trp Tyr Ile Thr Ala
               260                 265                 270
   Glu Asn Glu Asn His Val Val Glu Leu Glu Ala Arg Thr Asn Glu Ala
           275                 280                 285
   Gly Thr Pro Leu Arg Ala Pro Thr Thr Glu Val Gly Leu Ala Thr Ala
       290                 295                 300
   Cys Arg Asp Ser Cys Tyr Gly Glu Leu Lys Leu Gln Ile Trp Glu Arg
   305                 310                 315                 320
   Leu Tyr Asp Gly Ser Lys Gly Lys Leu Lys Val Leu Thr Asn Pro Lys
                   325                 330                 335
   Ala Val Lys Glu Asp Tyr Glu Arg Leu Leu Trp Leu Thr Met Met Gln
               340                 345                 350
   Val Ile Leu Glu Thr Lys Ser Ser Met Ala Ala Val Glu Ile Gly Gly
           355                 360                 365
   Gly Pro Trp Phe Gly Thr Trp Lys Gly Asp Thr Ser Asn Thr Pro Glu
       370                 375                 380
   Leu Leu Lys Gln Ala Leu Gln Val Pro Leu Asp Leu Glu Ser Ala Leu
   385                 390                 395                 400
   Gly Leu Val Pro Phe Phe Lys Pro Pro Gly Leu
                   405                 410

### SEQUENCE LISTING

<110> Subramaniam, Sai
   Slater, Steven
   Karberg, Katherine
   Chen, Ridong
   Valentin, Henry
   Huang Wong, Yun-Hua
<120> Nucleic Acid Sequences Involved in
   Tocopherol Synthesis
<130> MOCO.008.00WO
<150> 09/549,848
   <151> 2000-04-15
<150> 09/688,069
   <151> 2000-10-15
<160> 94
<170> FastSEQ for Windows Version 4.0
<210> 1
   <211> 1182
   <212> DNA
   <213> Arabidopsis sp
<400> 1
   atggagtctc tgctctctag ttcttctctt gtttccgctg ctggtgggtt ttgttggaag      60
   aagcagaatc taaagctcca ctctttatca gaaatccgag ttctgcgttg tgattcgagt     120
   aaagttgtcg caaaaccgaa gtttaggaac aatcttgtta ggcctgatgg tcaaggatct     180
   tcattgttgt tgtatccaaa acataagtcg agatttcggg ttaatgccac tgcgggtcag     240
   cctgaggctt tcgactcgaa tagcaaacag aagtctttta gagactcgtt agatgcgttt     300
   tacaggtttt ctaggcctca tacagttatt ggcacagtgc ttagcatttt atctgtatct     360
   ttcttagcag tagagaaggt ttctgatata tctcctttac ttttcactgg catcttggag     420
   gctgttgttg cagctctcat gatgaacatt tacatagttg ggctaaatca gttgtctgat     480
   gttgaaatag ataaggttaa caagccctat cttccattgg catcaggaga atattctgtt     540
   aacaccggca ttgcaatagt agcttccttc tccatcatga gtttctggct tgggtggatt     600
   gttggttcat ggccattgtt ctgggctctt tttgtgagtt tcatgctcgg tactgcatac     660
   tctatcaatt tgccactttt acggtggaaa agatttgcat tggttgcagc aatgtgtatc     720
   ctcgctgtcc gagctattat tgttcaaatc gccttttatc tacatattca gacacatgtg     780
   tttggaagac caatcttgtt cactaggcct cttattttcg ccactgcgtt tatgagcttt     840
   ttctctgtcg ttattgcatt gtttaaggat atacctgata tcgaagggga taagatattc     900
   ggaatccgat cattctctgt aactctgggt cagaaacggg tgttttggac atgtgttaca     960
   ctacttcaaa tggcttacgc tgttgcaatt ctagttggag ccacatctcc attcatatgg    1020
   agcaaagtca tctcggttgt gggtcatgtt atactcgcaa caactttgtg ggctcgagct    1080
   aagtccgttg atctgagtag caaaaccgaa ataacttcat gttatatgtt catatggaag    1140
   ctcttttatg cagagtactt gctgttacct tttttgaagt ga                       1182
<210> 2
   <211> 393
   <212> PRT
   <213> Arabidopsis sp
<400> 2
   Met Glu Ser Leu Leu Ser Ser Ser Ser Leu Val Ser Ala Ala Gly Gly
   1 5 10 15
   Phe Cys Trp Lys Lys Gln Asn Leu Lys Leu His Ser Leu Ser Glu Ile
   20 25 30
   Arg Val Leu Arg Cys Asp Ser Ser Lys Val Val Ala Lys Pro Lys Phe
   35 40 45
   Arg Asn Asn Leu Val Arg Pro Asp Gly Gln Gly Ser Ser Leu Leu Leu
   50 55 60
   Tyr Pro Lys His Lys Ser Arg Phe Arg Val Asn Ala Thr Ala Gly Gln
   65 70 75 80
   Pro Glu Ala Phe Asp Ser Asn Ser Lys Gln Lys Ser Phe Arg Asp Ser
   85 90 95
   Leu Asp Ala Phe Tyr Arg Phe Ser Arg Pro His Thr Val Ile Gly Thr
   100 105 110
   Val Leu Ser Ile Leu Ser Val Ser Phe Leu Ala Val Glu Lys Val Ser
   115 120 125
   Asp Ile Ser Pro Leu Leu Phe Thr Gly Ile Leu Glu Ala Val Val Ala
   130 135 140
   Ala Leu Met Met Asn Ile Tyr Ile Val Gly Leu Asn Gln Leu Ser Asp
   145 150 155 160
   Val Glu Ile Asp Lys Val Asn Lys Pro Tyr Leu Pro Leu Ala Ser Gly
   165 170 175
   Glu Tyr Ser Val Asn Thr Gly Ile Ala Ile Val Ala Ser Phe Ser Ile
   180 185 190
   Met Ser Phe Trp Leu Gly Trp Ile Val Gly Ser Trp Pro Leu Phe Trp
   195 200 205
   Ala Leu Phe Val Ser Phe Met Leu Gly Thr Ala Tyr Ser Ile Asn Leu
   210 215 220
   Pro Leu Leu Arg Trp Lys Arg Phe Ala Leu Val Ala Ala Met Cys Ile
   225 230 235 240
   Leu Ala Val Arg Ala Ile Ile Val Gln Ile Ala Phe Tyr Leu His Ile
   245 250 255
   Gln Thr His Val Phe Gly Arg Pro Ile Leu Phe Thr Arg Pro Leu Ile
   260 265 270
   Phe Ala Thr Ala Phe Met Ser Phe Phe Ser Val Val Ile Ala Leu Phe
   275 280 285
   Lys Asp Ile Pro Asp Ile Glu Gly Asp Lys Ile Phe Gly Ile Arg Ser
   290 295 300
   Phe Ser Val Thr Leu Gly Gln Lys Arg Val Phe Trp Thr Cys Val Thr
   305 310 315 320
   Leu Leu Gln Met Ala Tyr Ala Val Ala Ile Leu Val Gly Ala Thr Ser
   325 330 335
   Pro Phe Ile Trp Ser Lys Val Ile Ser Val Val Gly His Val Ile Leu
   340 345 350
   Ala Thr Thr Leu Trp Ala Arg Ala Lys Ser Val Asp Leu Ser Ser Lys
   355 360 365
   Thr Glu Ile Thr Ser Cys Tyr Met Phe Ile Trp Lys Leu Phe Tyr Ala
   370 375 380
   Glu Tyr Leu Leu Leu Pro Phe Leu Lys
   385 390
<210> 3
   <211> 1224
   <212> DNA
   <213> Arabidopsis sp
<400> 3
   atggcgtttt ttgggctctc ccgtgtttca agacggttgt tgaaatcttc cgtctccgta 60
   actccatctt cttcctctgc tcttttgcaa tcacaacata aatccttgtc caatcctgtg     120
   actacccatt acacaaatcc tttcactaag tgttatcctt catggaatga taattaccaa     180
   gtatggagta aaggaagaga attgcatcag gagaagtttt ttggtgttgg ttggaattac     240
   agattaattt gtggaatgtc gtcgtcttct tcggttttgg agggaaagcc gaagaaagat     300
   gataaggaga agagtgatgg tgttgttgtt aagaaagctt cttggataga tttgtattta     360
   ccagaagaag ttagaggtta tgctaagctt gctcgattgg ataaacccat tggaacttgg     420
   ttgcttgcgt ggccttgtat gtggtcgatt gcgttggctg ctgatcctgg aagccttcca     480
   agttttaaat atatggcttt atttggttgc ggagcattac ttcttagagg tgctggttgt     540
   actataaatg atctgcttga tcaggacata gatacaaagg ttgatcgtac aaaactaaga     600
   cctatcgcca gtggtctttt gacaccattt caagggattg gatttctcgg gctgcagttg     660
   cttttaggct tagggattct tctccaactt aacaattaca gccgtgtttt aggggcttca     720
   tctttgttac ttgtcttttc ctacccactt atgaagaggt ttacattttg gcctcaagcc     780
   tttttaggtt tgaccataaa ctggggagca ttgttaggat ggactgcagt taaaggaagc     840
   atagcaccat ctattgtact ccctctctat ctctccggag tctgctggac ccttgtttat     900
   gatactattt atgcacatca ggacaaagaa gatgatgtaa aagttggtgt taagtcaaca     960
   gcccttagat tcggtgataa tacaaagctt tggttaactg gatttggcac agcatccata    1020
   ggttttcttg cactttctgg attcagtgca gatctcgggt ggcaatatta cgcatcactg    1080
   gccgctgcat caggacagtt aggatggcaa atagggacag ctgacttatc atctggtgct    1140
   gactgcagta gaaaatttgt gtcgaacaag tggtttggtg ctattatatt tagtggagtt    1200
   gtacttggaa gaagttttca ataa                                           1224
<210> 4
   <211> 407
   <212> PRT
   <213> Arabidopsis sp
<400> 4
   Met Ala Phe Phe Gly Leu Ser Arg Val Ser Arg Arg Leu Leu Lys Ser
   1               5                   10                  15
   Ser Val Ser Val Thr Pro Ser Ser Ser Ser Ala Leu Leu Gln Ser Gln
               20                  25                  30
   His Lys Ser Leu Ser Asn Pro Val Thr Thr His Tyr Thr Asn Pro Phe
           35                  40                  45
   Thr Lys Cys Tyr Pro Ser Trp Asn Asp Asn Tyr Gln Val Trp Ser Lys
       50                  55                  60
   Gly Arg Glu Leu His Gln Glu Lys Phe Phe Gly Val Gly Trp Asn Tyr
   65                  70                  75                  80
   Arg Leu Ile Cys Gly Met Ser Ser Ser Ser Ser Val Leu Glu Gly Lys
                   85                  90                  95
   Pro Lys Lys Asp Asp Lys Glu Lys Ser Asp Gly Val Val Val Lys Lys
   100 105 110
   Ala Ser Trp Ile Asp Leu Tyr Leu Pro Glu Glu Val Arg Gly Tyr Ala
   115 120 125
   Lys Leu Ala Arg Leu Asp Lys Pro Ile Gly Thr Trp Leu Leu Ala Trp
   130 135 140
   Pro Cys Met Trp Ser Ile Ala Leu Ala Ala Asp Pro Gly Ser Leu Pro
   145 150 155 160
   Ser Phe Lys Tyr Met Ala Leu Phe Gly Cys Gly Ala Leu Leu Leu Arg
   165 170 175
   Gly Ala Gly Cys Thr Ile Asn Asp Leu Leu Asp Gln Asp Ile Asp Thr
   180 185 190
   Lys Val Asp Arg Thr Lys Leu Arg Pro Ile Ala Ser Gly Leu Leu Thr
   195 200 205
   Pro Phe Gln Gly Ile Gly Phe Leu Gly Leu Gln Leu Leu Leu Gly Leu
   210 215 220
   Gly Ile Leu Leu Gln Leu Asn Asn Tyr Ser Arg Val Leu Gly Ala Ser
   225 230 235 240
   Ser Leu Leu Leu Val Phe Ser Tyr Pro Leu Met Lys Arg Phe Thr Phe
   245 250 255
   Trp Pro Gln Ala Phe Leu Gly Leu Thr Ile Asn Trp Gly Ala Leu Leu
   260 265 270
   Gly Trp Thr Ala Val Lys Gly Ser Ile Ala Pro Ser Ile Val Leu Pro
   275 280 285
   Leu Tyr Leu Ser Gly Val Cys Trp Thr Leu Val Tyr Asp Thr Ile Tyr
   290 295 300
   Ala His Gln Asp Lys Glu Asp Asp Val Lys Val Gly Val Lys Ser Thr
   305 310 315 320
   Ala Leu Arg Phe Gly Asp Asn Thr Lys Leu Trp Leu Thr Gly Phe Gly
   325 330 335
   Thr Ala Ser Ile Gly Phe Leu Ala Leu Ser Gly Phe Ser Ala Asp Leu
   340 345 350
   Gly Trp Gln Tyr Tyr Ala Ser Leu Ala Ala Ala Ser Gly Gln Leu Gly
   355 360 365
   Trp Gln Ile Gly Thr Ala Asp Leu Ser Ser Gly Ala Asp Cys Ser Arg
   370 375 380
   Lys Phe Val Ser Asn Lys Trp Phe Gly Ala Ile Ile Phe Ser Gly Val
   385 390 395 400
   Val Leu Gly Arg Ser Phe Gln
<210> 5
   <211> 1296
   <212> DNA
   <213> Arabidopsis sp
<400> 5
   atgtggcgaa gatctgttgt ttctcgttta tcttcaagaa tctctgtttc ttcttcgtta      60
   ccaaacccta gactgattcc ttggtcccgc gaattatgtg ccgttaatag cttctcccag     120
   cctccggtct cgacggaatc aactgctaag ttagggatca ctggtgttag atctgatgcc     180
   aatcgagttt ttgccactgc tactgccgcc gctacagcta cagctaccac cggtgagatt     240
   tcgtctagag ttgcggcttt ggctggatta gggcatcact acgctcgttg ttattgggag     300
   ctttctaaag ctaaacttag tatgcttgtg gttgcaactt ctggaactgg gtatattctg     360
   ggtacgggaa atgctgcaat tagcttcccg gggctttgtt acacatgtgc aggaaccatg      420
   atgattgctg catctgctaa ttccttgaat cagatttttg agataagcaa tgattctaag     480
   atgaaaagaa cgatgctaag gccattgcct tcaggacgta ttagtgttcc acacgctgtt     540
   gcatgggcta ctattgctgg tgcttctggt gcttgtttgt tggccagcaa gactaatatg     600
   ttggctgctg gacttgcatc tgccaatctt gtactttatg cgtttgttta tactccgttg     660
   aagcaacttc accctatcaa tacatgggtt ggcgctgttg ttggtgctat cccacccttg     720
   cttgggtggg cggcagcgtc tggtcagatt tcatacaatt cgatgattct tccagctgct     780
   ctttactttt ggcagatacc tcattttatg gcccttgcac atctctgccg caatgattat     840
   gcagctggag gttacaagat gttgtcactc tttgatccgt cagggaagag aatagcagca     900
   gtggctctaa ggaactgctt ttacatgatc cctctcggtt tcatcgccta tgactggggg     960
   ttaacctcaa gttggttttg cctcgaatca acacttctca cactagcaat cgctgcaaca    1020
   gcattttcat tctaccgaga ccggaccatg cataaagcaa ggaaaatgtt ccatgccagt    1080
   cttctcttcc ttcctgtttt catgtctggt cttcttctac accgtgtctc taatgataat    1140
   cagcaacaac tcgtagaaga agccggatta acaaattctg tatctggtga agtcaaaact    1200
   cagaggcgaa agaaacgtgt ggctcaacct ccggtggctt atgcctctgc tgcaccgttt    1260
   cctttcctcc cagctccttc cttctactct ccatga                              1296
<210> 6
   <211> 431
   <212> PRT
   <213> Arabidopsis sp
<400> 6
   Met Trp Arg Arg Ser Val Val Tyr Arg Phe Ser Ser Arg Ile Ser Val
   1               5                   10                  15
   Ser Ser Ser Leu Pro Asn Pro Arg Leu Ile Pro Trp Ser Arg Glu Leu
   20 25 30
   Cys Ala Val Asn Ser Phe Ser Gln Pro Pro Val Ser Thr Glu Ser Thr
   35 40 45
   Ala Lys Leu Gly Ile Thr Gly Val Arg Ser Asp Ala Asn Arg Val Phe
   50 55 60
   Ala Thr Ala Thr Ala Ala Ala Thr Ala Thr Ala Thr Thr Gly Glu Ile
   65 70 75 80
   Ser Ser Arg Val Ala Ala Leu Ala Gly Leu Gly His His Tyr Ala Arg
   85 90 95
   Cys Tyr Trp Glu Leu Ser Lys Ala Lys Leu Ser Met Leu Val Val Ala
   100 105 110
   Thr Ser Gly Thr Gly Tyr Ile Leu Gly Thr Gly Asn Ala Ala Ile Ser
   115 120 125
   Phe Pro Gly Leu Cys Tyr Thr Cys Ala Gly Thr Met Met Ile Ala Ala
   130 135 140
   Ser Ala Asn Ser Leu Asn Gln Ile Phe Glu Ile Ser Asn Asp Ser Lys
   145 150 155 160
   Met Lys Arg Thr Met Leu Arg Pro Leu Pro Ser Gly Arg Ile Ser Val
   165 170 175
   Pro His Ala Val Ala Trp Ala Thr Ile Ala Gly Ala Ser Gly Ala Cys
   180 185 190
   Leu Leu Ala Ser Lys Thr Asn Met Leu Ala Ala Gly Leu Ala Ser Ala
   195 200 205
   Asn Leu Val Leu Tyr Ala Phe Val Tyr Thr Pro Leu Lys Gln Leu His
   210 215 220
   Pro Ile Asn Thr Trp Val Gly Ala Val Val Gly Ala Ile Pro Pro Leu
   225 230 235 240
   Leu Gly Trp Ala Ala Ala Ser Gly Gln Ile Ser Tyr Asn Ser Met Ile
   245 250 255
   Leu Pro Ala Ala Leu Tyr Phe Trp Gln Ile Pro His Phe Met Ala Leu
   260 265 270
   Ala His Leu Cys Arg Asn Asp Tyr Ala Ala Gly Gly Tyr Lys Met Leu
   275 280 285
   Ser Leu Phe Asp Pro Ser Gly Lys Arg Ile Ala Ala Val Ala Leu Arg
   290 295 300
   Asn Cys Phe Tyr Met Ile Pro Leu Gly Phe Ile Ala Tyr Asp Trp Gly
   305 310 315 320
   Leu Thr Ser Ser Trp Phe Cys Leu Glu Ser Thr Leu Leu Thr Leu Ala
   325 330 335
   Ile Ala Ala Thr Ala Phe Ser Phe Tyr Arg Asp Arg Thr Met His Lys
   340 345 350
   Ala Arg Lys Met Phe His Ala Ser Leu Leu Phe Leu Pro Val Phe Met
   355 360 365
   Ser Gly Leu Leu Leu His Arg Val Ser Asn Asp Asn Gln Gln Gln Leu
   370 375 380
   Val Glu Glu Ala Gly Leu Thr Asn Ser Val Ser Gly Glu Val Lys Thr
   385 390 395 400
   Gln Arg Arg Lys Lys Arg Val Ala Gln Pro Pro Val Ala Tyr Ala Ser
   405 410 415
   Ala Ala Pro Phe Pro Phe Leu Pro Ala Pro Ser Phe Tyr Ser Pro
   420 425 430
<210> 7
   <211> 479
   <212> DNA
   <213> Arabidopsis sp
<400> 7
   ggaaactccc ggagcacctg tttgcaggta ccgctaacct taatcgataa tttatttctc 60
   ttgtcaggaa ttatgtaagt ctggtggaag gctcgcatac catttttgca ttgcctttcg 120
   ctatgatcgg gtttactttg ggtgtgatga gaccaggcgt ggctttatgg tatggcgaaa 180
   acccattttt atccaatgct gcattccctc ccgatgattc gttctttcat tcctatacag 240
   gtatcatgct gataaaactg ttactggtac tggtttgtat ggtatcagca agaagcgcgg 300
   cgatggcgtt taaccggtat ctcgacaggc attttgacgc gaagaacccg cgtactgcca 360
   tccgtgaaat acctgcgggc gtcatatctg ccaacagtgc gctggtgttt acgataggct 420
   gctgcgtggt attctgggtg gcctgttatt tcattaacac gatctgtttt tacctggcg 479
<210> 8
   <211> 551
   <212> DNA
   <213> Arabidopsis sp
<220>
   <221> misc_feature
   <222> (1)...(551)
   <223> n = A,T,C or G
<210> 9
   <211> 297
   <212> PRT
   <213> Arabidopsis sp
<400> 9
   Met Val Leu Ala Glu Val Pro Lys Leu Ala Ser Ala Ala Glu Tyr Phe
   1               5                   10                  15
   Phe Lys Arg Gly Val Gln Gly Lys Gln Phe Arg Ser Thr Ile Leu Leu
               20                  25                  30
   Leu Met Ala Thr Ala Leu Asn Val Arg Val Pro Glu Ala Leu Ile Gly
           35                  40                  45
   Glu Ser Thr Asp Ile Val Thr Ser Glu Leu Arg Val Arg Gln Arg Gly
       50                  55                  60
   Ile Ala Glu Ile Thr Glu Met Ile His Val Ala Ser Leu Leu His Asp
   65                  70                  75                  80
   Asp Val Leu Asp Asp Ala Asp Thr Arg Arg Gly Val Gly Ser Leu Asn
                   85                  90                  95
   Val Val Met Gly Asn Lys Val Val Ala Leu Leu Ala Thr Ala Val Glu
               100                 105                 110
   His Leu Val Thr Gly Glu Thr Met Glu Ile Thr Ser Ser Thr Glu Gln
           115                 120                 125
   Arg Tyr Ser Met Asp Tyr Tyr Met Gln Lys Thr Tyr Tyr Lys Thr Ala
       130                 135                 140
   Ser Leu Ile Ser Asn Ser Cys Lys Ala Val Ala Val Leu Thr Gly Gln
   145                 150                 155                 160
   Thr Ala Glu Val Ala Val Leu Ala Phe Glu Tyr Gly Arg Asn Leu Gly
   165 170 175
   Leu Ala Phe Gln Leu Ile Asp Asp Ile Leu Asp Phe Thr Gly Thr Ser
   180 185 190
   Ala Ser Leu Gly Lys Gly Ser Leu Ser Asp Ile Arg His Gly Val Ile
   195 200 205
   Thr Ala Pro Ile Leu Phe Ala Met Glu Glu Phe Pro Gln Leu Arg Glu
   210 215 220
   Val Val Asp Gln Val Glu Lys Asp Pro Arg Asn Val Asp Ile Ala Leu
   225 230 235 240
   Glu Tyr Leu Gly Lys Ser Lys Gly Ile Gln Arg Ala Arg Glu Leu Ala
   245 250 255
   Met Glu His Ala Asn Leu Ala Ala Ala Ala Ile Gly Ser Leu Pro Glu
   260 265 270
   Thr Asp Asn Glu Asp Val Lys Arg Ser Arg Arg Ala Leu Ile Asp Leu
   275 280 285
   Thr His Arg Val Ile Thr Arg Asn Lys
   290 295
<210> 10
   <211> 561
   <212> DNA
   <213> Arabidopsis sp
<400> 10
   aagcgcatcc gtcctcttct acgattgccg ccagccgcat gtatggctgc ataaccgacc      60
   gcccctatcc gctcgcggcc gcggtcgaat tcattcacac cgcgacgctg ctgcatgacg     120
   acgtcgtcga tgaaagcgat ttgcgccgcg gccgcgaaag cgcgcataag gttttcggca     180
   atcaggcgag cgtgctcgtc ggcgatttcc ttttctcccg cgccttccag ctgatggtgg     240
   aagacggctc gctcgacgcg ctgcgcattc tctcggatgc ctccgccgtg atcgcgcagg     300
   gcgaagtgat gcagctcggc accgcgcgca atcttgaaac caatatgagc cagtatctcg     360
   atgtgatcag cgcgaagacc gccgcgctct ttgccgccgc ctgcgaaatc ggcccggtga     420
   tggcgaacgc gaaggcggaa gatgctgccg cgatgtgcga atacggcatg aatctcggta     480
   tcgccttcca gatcatcgac gaccttctcg attacggcac cggcggccac gccgagcttg     540
   gcaagaacac gggcgacgat t                                               561
<210> 11
   <211> 966
   <212> DNA
   <213> Arabidopsis sp
<400> 11
   atggtacttg ccgaggttcc aaagcttgcc tctgctgctg agtacttctt caaaaggggt 60
   gtgcaaggaa aacagtttcg ttcaactatt ttgctgctga tggcgacagc tctgaatgta 120
   cgcgttccag aagcattgat tggggaatca acagatatag tcacatcaga attacgcgta 180
   aggcaacggg gtattgctga aatcactgaa atgatacacg tcgcaagtct actgcacgat 240
   gatgtcttgg atgatgccga tacaaggcgt ggtgttggtt ccttaaatgt tgtaatgggt 300
   aacaagatgt cggtattagc aggagacttc ttgctctccc gggcttgtgg ggctctcgct 360
   gctttaaaga acacagaggt tgtagcatta cttgcaactg ctgtagaaca tcttgttacc 420
   ggtgaaacca tggaaataac tagttcaacc gagcagcgtt atagtatgga ctactacatg 480
   cagaagacat attataagac agcatcgcta atctctaaca gctgcaaagc tgttgccgtt 540
   ctcactggac aaacagcaga agttgccgtg ttagcttttg agtatgggag gaatctgggt 600
   ttagcattcc aattaataga cgacattctt gatttcacgg gcacatctgc ctctctcgga 660
   aagggatcgt tgtcagatat tcgccatgga gtcataacag ccccaatcct ctttgccatg 720
   gaagagtttc ctcaactacg cgaagttgtt gatcaagttg aaaaagatcc taggaatgtt 780
   gacattgctt tagagtatct tgggaagagc aagggaatac agagggcaag agaattagcc 840
   atggaacatg cgaatctagc agcagctgca atcgggtctc tacctgaaac agacaatgaa 900
   gatgtcaaaa gatcgaggcg ggcacttatt gacttgaccc atagagtcat caccagaaac 960
   aagtga 966
<210> 12
   <211> 321
   <212> PRT
   <213> Arabidopsis sp
<400> 12
   Met Val Leu Ala Glu Val Pro Lys Leu Ala Ser Ala Ala Glu Tyr Phe
   1               5                   10                  15
   Phe Lys Arg Gly Val Gln Gly Lys Gln Phe Arg Ser Thr Ile Leu Leu
               20                  25                  30
   Leu Met Ala Thr Ala Leu Asn Val Arg Val Pro Glu Ala Leu Ile Gly
           35                  40                  45
   Glu Ser Thr Asp Ile Val Thr Ser Glu Leu Arg Val Arg Gln Arg Gly
       50                  55                  60
   Ile Ala Glu Ile Thr Glu Met Ile His Val Ala Ser Leu Leu His Asp
   65                  70                  75                  80
   Asp Val Leu Asp Asp Ala Asp Thr Arg Arg Gly Val Gly Ser Leu Asn
                   85                  90                  95
   Val Val Met Gly Asn Lys Met Ser Val Leu Ala Gly Asp Phe Leu Leu
   100 105 110
   Ser Arg Ala Cys Gly Ala Leu Ala Ala Leu Lys Asn Thr Glu Val Val
   115 120 125
   Ala Leu Leu Ala Thr Ala Val Glu His Leu Val Thr Gly Glu Thr Met
   130 135 140
   Glu Ile Thr Ser Ser Thr Glu Gln Arg Tyr Ser Met Asp Tyr Tyr Met
   145 150 155 160
   Gln Lys Thr Tyr Tyr Lys Thr Ala Ser Leu Ile Ser Asn Ser Cys Lys
   165 170 175
   Ala Val Ala Val Leu Thr Gly Gln Thr Ala Glu Val Ala Val Leu Ala
   180 185 190
   Phe Glu Tyr Gly Arg Asn Leu Gly Leu Ala Phe Gln Leu Ile Asp Asp
   195 200 205
   Ile Leu Asp Phe Thr Gly Thr Ser Ala Ser Leu Gly Lys Gly Ser Leu
   210 215 220
   Ser Asp Ile Arg His Gly Val Ile Thr Ala Pro Ile Leu Phe Ala Met
   225 230 235 240
   Glu Glu Phe Pro Gln Leu Arg Glu Val Val Asp Gln Val Glu Lys Asp
   245 250 255
   Pro Arg Asn Val Asp Ile Ala Leu Glu Tyr Leu Gly Lys Ser Lys Gly
   260 265 270
   Ile Gln Arg Ala Arg Glu Leu Ala Met Glu His Ala Asn Leu Ala Ala
   275 280 285
   Ala Ala Ile Gly Ser Leu Pro Glu Thr Asp Asn Glu Asp Val Lys Arg
   290 295 300
   Ser Arg Arg Ala Leu Ile Asp Leu Thr His Arg Val Ile Thr Arg Asn
   305 310 315 320
   Lys
<210> 13
   <211> 621
   <212> DNA
   <213> Arabidopsis sp
<400> 13
   gctttctcct ttgctaattc ttgagctttc ttgatcccac cgcgatttct aactatttca 60
   atcgcttctt caagcgatcc aggctcacaa aactcagact caatgatctc tcttagcctt 120
   ggctcattct ctagcgcgaa gatcactggc gccgttatgt tacctttggc taagtcatta 180
   gctgcaggct tacctaactg ctctgtggac tgagtgaagt ccagaatgtc atcaactact 240
   tgaaaagata aaccgagatt cttcccgaac tgatacattt gctctgcgac cttgctttcg 300
   actttactga aaattgctgc tcctttggtg cttgcagcta ctaatgaagc tgtcttgtag 360
   taactcttta gcatgtagtc atcaagcttg acatcacaat cgaataaact cgatgcttgc 420
   tttatctcac cgcttgcaaa atctttgatc acctgcaaaa agataaatca agattcagac 480
   caaatgttct ttgtattgag tagcttcatc taatctcaga aaggaatatt acctgactta 540
   tgagcttaat gacttcaagg ttttcgagat ttgtaagtac catgatgctt gagcaacatg 600
   aaatccccag ctaatacagc t 621
<210> 14
   <211> 741
   <212> DNA
   <213> Arabidopsis sp
<400> 14
   ggtgagtttt gttaatagtt atgagattca tctatttttg tcataaaatt gtttggtttg 60
   gtttaaactc tgtgtataat tgcaggaaag gaaacagttc atgagctttt cggcacaaga 120
   gtagcggtgc tagctggaga tttcatgttt gctcaagcgt catggtactt agcaaatctc 180
   gagaatcttg aagttattaa gctcatcagt caggtactta gttactctta cattgttttt 240
   ctatgaggtt gagctatgaa tctcatttcg ttgaataatg ctgtgcctca aacttttttt 300
   catgttttca ggtgatcaaa gactttgcaa gcggagagat aaagcaggcg tccagcttat 360
   ttgactgcga caccaagctc gacgagtact tactcaaaag tttctacaag acagcctctt 420
   tagtggctgc gagcaccaaa ggagctgcca ttttcagcag agttgagcct gatgtgacag 480
   aacaaatgta cgagtttggg aagaatctcg gtctctcttt ccagatagtt gatgatattt 540
   tggatttcac tcagtcgaca gagcagctcg ggaagccagc agggagtgat ttggctaaag 600
   gtaacttaac agcacctgtg attttcgctc tggagaggga gccaaggcta agagagatca 660
   ttgagtcaaa gttctgtgag gcgggttctc tggaagaagc gattgaagcg gtgacaaaag 720
   gtggggggat taagagagca c 741
<210> 15
   <211> 1087
   <212> DNA
   <213> Arabidopsis sp
<400> 15
   cctcttcagc caatccagag gaagaagaga caacttttta tctttcgtca agagtctccg 60
   aaaacgcacg gttttatgct ctctcttctg ccctcacctc acaagacgca gggcacatga 120
   ttcaaccaga gggaaaaagc aacgataaca actctgcttt tgatttcaag ctgtatatga 180
   tccgcaaagc cgagtctgta aatgcggctc tcgacgtttc cgtaccgctt ctgaaacccc 240
   ttacgatcca agaagcggtc aggtactctt tgctagccgg cggaaaacgt gtgaggcctc 300
   tgctctgcat tgccgcttgt gagcttgtgg ggggcgacga ggctactgcc atgtcagccg 360
   cttgcgcggt cgagatgatc cacacaagct ctctcattca tgacgatctt ccgtgcatgg 420
   acaatgccga cctccgtaga ggcaagccca ccaatcacaa ggtatgttgt ttaattatat 480
   gaaggctcag agataatgct gaactagtgt tgaaccaatt tttgctcaaa caaggtatat 540
   ggagaagaca tggcggtttt ggcaggtgat gcactccttg cattggcgtt tgagcacatg 600
   acggttgtgt cgagtgggtt ggtcgctccc gagaagatga ttcgcgccgt ggttgagctg 660
   gccagggcca tagggactac agggctagtt gctggacaaa tgatagacct agccagcgaa 720
   agactgaatc cagacaaggt tggattggag catctagagt tcatccatct ccacaaaacg 780
   gcggcattgt tggaggcagc ggcagtttta ggggttataa tgggaggtgg aacagaggaa 840
   gaaatcgaaa agcttagaaa gtatgctagg tgtattggac tactgtttca ggttgttgat 900
   gacattctcg acgtaacaaa atctactgag gaattgggta agacagccgg aaaagacgta 960
   atggccggaa agctgacgta tccaaggctg ataggtttgg agggatccag ggaagttgca 1020
   gagcacctga ggagagaagc agaggaaaag cttaaagggt ttgatccaag tcaggcggcg 1080
   cctctgg 1087
<210> 16
   <211> 1164
   <212> DNA
   <213> Arabidopsis sp
<400> 16
   atgacttcga ttctcaacac tgtctccacc atccactctt ccagagttac ctccgtcgat 60
   cgagtcggag tcctctctct tcggaattcg gattccgttg agttcactcg ccggcgttct 120
   ggtttctcga cgttgatcta cgaatcaccc gggcggagat ttgttgtgcg tgcggcggag 180
   actgatactg ataaagttaa atctcagaca cctgacaagg caccagccgg tggttcaagc 240
   attaaccagc ttctcggtat caaaggagca tctcaagaaa ctaataaatg gaagattcgt 300
   cttcagctta caaaaccagt cacttggcct ccactggttt ggggagtcgt ctgtggtgct 360
   gctgcttcag ggaactttca ttggacccca gaggatgttg ctaagtcgat tctttgcatg 420
   atgatgtctg gtccttgtct tactggctat acacagacaa tcaacgactg gtatgataga 480
   gatatcgacg caattaatga gccatatcgt ccaattccat ctggagcaat atcagagcca 540
   gaggttatta cacaagtctg ggtgctatta ttgggaggtc ttggtattgc tggaatatta 600
   gatgtgtggg cagggcatac cactcccact gtcttctatc ttgctttggg aggatcattg 660
   ctatcttata tatactctgc tccacctctt aagctaaaac aaaatggatg ggttggaaat 720
   tttgcacttg gagcaagcta tattagtttg ccatggtggg ctggccaagc attgtttggc 780
   actcttacgc cagatgttgt tgttctaaca ctcttgtaca gcatagctgg gttaggaata 840
   gccattgtta acgacttcaa aagtgttgaa ggagatagag cattaggact tcagtctctc 900
   ccagtagctt ttggcaccga aactgcaaaa tggatatgcg ttggtgctat agacattact 960
   cagctttctg ttgccggata tctattagca tctgggaaac cttattatgc gttggcgttg 1020
   gttgctttga tcattcctca gattgtgttc cagtttaaat actttctcaa ggaccctgtc 1080
   aaatacgacg tcaagtacca ggcaagcgcg cagccattct tggtgctcgg aatatttgta 1140
   acggcattag catcgcaaca ctga 1164
<210> 17
   <211> 387
   <212> PRT
   <213> Arabidopsis sp
<400> 17
   Met Thr Ser Ile Leu Asn Thr Val Ser Thr Ile His Ser Ser Arg Val
   1 5 10 15
   Thr Ser Val Asp Arg Val Gly Val Leu Ser Leu Arg Asn Ser Asp Ser
   20 25 30
   Val Glu Phe Thr Arg Arg Arg Ser Gly Phe Ser Thr Leu Ile Tyr Glu
   35 40 45
   Ser Pro Gly Arg Arg Phe Val Val Arg Ala Ala Glu Thr Asp Thr Asp
   50 55 60
   Lys Val Lys Ser Gln Thr Pro Asp Lys Ala Pro Ala Gly Gly Ser Ser
   65 70 75 80
   Ile Asn Gln Leu Leu Gly Ile Lys Gly Ala Ser Gln Glu Thr Asn Lys
   85 90 95
   Trp Lys Ile Arg Leu Gln Leu Thr Lys Pro Val Thr Trp Pro Pro Leu
   100 105 110
   Val Trp Gly Val Val Cys Gly Ala Ala Ala Ser Gly Asn Phe His Trp
   115 120 125
   Thr Pro Glu Asp Val Ala Lys Ser Ile Leu Cys Met Met Met Ser Gly
   130 135 140
   Pro Cys Leu Thr Gly Tyr Thr Gln Thr Ile Asn Asp Trp Tyr Asp Arg
   145 150 155 160
   Asp Ile Asp Ala Ile Asn Glu Pro Tyr Arg Pro Ile Pro Ser Gly Ala
   165 170 175
   Ile Ser Glu Pro Glu Val Ile Thr Gln Val Trp Val Leu Leu Leu Gly
   180 185 190
   Gly Leu Gly Ile Ala Gly Ile Leu Asp Val Trp Ala Gly His Thr Thr
   195 200 205
   Pro Thr Val Phe Tyr Leu Ala Leu Gly Gly Ser Leu Leu Ser Tyr Ile
   210 215 220
   Tyr Ser Ala Pro Pro Leu Lys Leu Lys Gln Asn Gly Trp Val Gly Asn
   225 230 235 240
   Phe Ala Leu Gly Ala Ser Tyr Ile Ser Leu Pro Trp Trp Ala Gly Gln
   245 250 255
   Ala Leu Phe Gly Thr Leu Thr Pro Asp Val Val Val Leu Thr Leu Leu
   260 265 270
   Tyr Ser Ile Ala Gly Leu Gly Ile Ala Ile Val Asn Asp Phe Lys Ser
   275 280 285
   Val Glu Gly Asp Arg Ala Leu Gly Leu Gln Ser Leu Pro Val Ala Phe
   290 295 300
   Gly Thr Glu Thr Ala Lys Trp Ile Cys Val Gly Ala Ile Asp Ile Thr
   305 310 315 320
   Gln Leu Ser Val Ala Gly Tyr Leu Leu Ala Ser Gly Lys Pro Tyr Tyr
   325 330 335
   Ala Leu Ala Leu Val Ala Leu Ile Ile Pro Gln Ile Val Phe Gln Phe
   340 345 350
   Lys Tyr Phe Leu Lys Asp Pro Val Lys Tyr Asp Val Lys Tyr Gln Ala
   355 360 365
   Ser Ala Gln Pro Phe Leu Val Leu Gly Ile Phe Val Thr Ala Leu Ala
   370 375 380
   Ser Gln His
   385
<210> 18
   <211> 981
   <212> DNA
   <213> Arabidopsis sp
<400> 18
   atgttgttta gtggttcagc gatcccatta agcagcttct gctctcttcc ggagaaaccc 60
   cacactcttc ctatgaaact ctctcccgct gcaatccgat cttcatcctc atctgccccg 120
   gggtcgttga acttcgatct gaggacgtat tggacgactc tgatcaccga gatcaaccag 180
   aagctggatg aggccatacc ggtcaagcac cctgcgggga tctacgaggc tatgagatac 240
   tctgtactcg cacaaggcgc caagcgtgcc cctcctgtga tgtgtgtggc ggcctgcgag 300
   ctcttcggtg gcgatcgcct cgccgctttc cccaccgcct gtgccctaga aatggtgcac 360
   gcggcttcgt tgatacacga cgacctcccc tgtatggacg acgatcctgt gcgcagagga 420
   aagccatcta accacactgt ctacggctct ggcatggcca ttctcgccgg tgacgccctc 480
   ttcccactcg ccttccagca cattgtctcc cacacgcctc ctgaccttgt tccccgagcc 540
   accatcctca gactcatcac tgagattgcc cgcactgtcg gctccactgg tatggctgca 600
   ggccagtacg tcgaccttga aggaggtccc tttcctcttt cctttgttca ggagaagaaa 660
   ttcggagcca tgggtgaatg ctctgccgtg tgcggtggcc tattgggcgg tgccactgag 720
   gatgagctcc agagtctccg aaggtacggg agagccgtcg ggatgctgta tcaggtggtc 780
   gatgacatca ccgaggacaa gaagaagagc tatgatggtg gagcagagaa gggaatgatg 840
   gaaatggcgg aagagctcaa ggagaaggcg aagaaggagc ttcaagtgtt tgacaacaag 900
   tatggaggag gagacacact tgttcctctc tacaccttcg ttgactacgc tgctcatcga 960
   cattttcttc ttcccctctg a 981
<210> 19
   <211> 245
   <212> DNA
   <213> GLycine sp
<400> 19
   gcaacatctg ggactgggtt tgtcttgggg agtggtagtg ctgttgatct ttcggcactt 60
   tcttgcactt gcttgggtac catgatggtt gctgcatctg ctaactcttt gaatcaggtg 120
   tttgagatca ataatgatgc taaaatgaag agaacaagtc gcaggccact accctcagga 180
   cgcatcacaa tacctcatgc agttggctgg gcatcctctg ttggattagc tggtacggct 240
   ctact 245
<210> 20
   <211> 253
   <212> DNA
   <213> Glycine sp
<400> 20
   attggctttc caagatcatt gggttttctt gttgcattca tgaccttcta ctccttgggt 60
   ttggcattgt ccaaggatat acctgacgtt gaaggagata aagagcacgg cattgattct 120
   tttgcagtac gtctaggtca gaaacgggca ttttggattt gcgtttcctt ttttgaaatg 180
   gctttcggag ttggtatcct ggccggagca tcatgctcac acttttggac taaaattttc 240
   acgggtatgg gaa 253
<210> 21
   <211> 275
   <212> DNA
   <213> Glycine sp
<400> 21
   tgatcttcta ctctctgggt atggcattgt ccaaggatat atctgacgtt aaaggagata 60
   aagcatacgg catcgatact ttagcgatac gtttgggtca aaaatgggta ttttggattt 120
   gcattatcct ttttgaaatg gcttttggag ttgccctctt ggcaggagca acatcttctt 180
   acctttggat taaaattgtc acgggtctgg gacatgctat tcttgcttca attctcttgt 240
   accaagccaa atctatatac ttgagcaaca aagtt 275
<210> 22
   <211> 299
   <212> DNA
   <213> Glycine sp
<220>
   <221> misc_feature
   <222> (1)...(299)
   <223> n = A,T,C or G
<400> 22
   ccanaatang tncatcttng aaagacaatt ggcctcttca acacacaagt ctgcatgtga 60
   agaagaggcc aattgtcttt ccaagatcac ttatngtggc tattgtaatc atgaacttct 120
   tctttgtggg tatggcattg gcaaaggata tacctanctg ttgaaggaga taaaatatat 180
   ggcattgata cttttgcaat acgtataggt caaaaacaag tattttggat ttgtattttc 240
   ctttttgaaa ggctttcgga gtttccctag tggcaggagc aacatcttct agccttggt 299
<210> 23
   <211> 767
   <212> DNA
   <213> Glycine sp
<400> 23
   gtggaggctg tggttgctgc cctgtttatg aatatttata ttgttggttt gaatcaattg 60
   tctgatgttg aaatagacaa gataaacaag ccgtatcttc cattagcatc tggggaatat 120
   tcctttgaaa ctggtgtcac tattgttgca tctttttcaa ttctgagttt ttggcttggc 180
   tgggttgtag gttcatggcc attattttgg gccctttttg taagctttgt gctaggaact 240
   gcttattcaa tcaatgtgcc tctgttgaga tggaagaggt ttgcagtgct tgcagcgatg 300
   tgcattctag ctgttcgggc agtaatagtt caacttgcat ttttccttca catgcagact 360
   catgtgtaca agaggccacc tgtcttttca agaccattga tttttgctac tgcattcatg 420
   agcttcttct ctgtagttat agcactgttt aaggatatac ctgacattga aggagataaa 480
   gtatttggca tccaatcttt ttcagtgtgt ttaggtcaga agccggtgtt ctggacttgt 540
   gttacccttc ttgaaatagc ttatggagtc gccctcctgg tgggagctgc atctccttgt 600
   ctttggagca aaattttcac gggtctggga cacgctgtgc tggcttcaat tctctggttt 660
   catgccaaat ctgtagattt gaaaagcaaa gcttcgataa catccttcta tatgtttatt 720
   tggaagctat tttatgcaga atacttactc attccttttg ttagatg 767
<210> 24
   <211> 255
   <212> PRT
   <213> Glycine sp
<400> 24
   Val Glu Ala Val Val Ala Ala Leu Phe Met Asn Ile Tyr Ile Val Gly
   1               5                   10                  15
   Leu Asn Gln Leu Ser Asp Val Glu Ile Asp Lys Ile Asn Lys Pro Tyr
               20                  25                  30
   Leu Pro Leu Ala Ser Gly Glu Tyr Ser Phe Glu Thr Gly Val Thr Ile
           35                  40                  45
   Val Ala Ser Phe Ser Ile Leu Ser Phe Trp Leu Gly Trp Val Val Gly
       50                  55                  60
   Ser Trp Pro Leu Phe Trp Ala Leu Phe Val Ser Phe Val Leu Gly Thr
   65                  70                  75                  80
   Ala Tyr Ser Ile Asn Val Pro Leu Leu Arg Trp Lys Arg Phe Ala Val
                   85                  90                  95
   Leu Ala Ala Met Cys Ile Leu Ala Val Arg Ala Val Ile Val Gln Leu
               100                 105                 110
   Ala Phe Phe Leu His Met Gln Thr His Val Tyr Lys Arg Pro Pro Val
           115                 120                 125
   Phe Ser Arg Pro Leu Ile Phe Ala Thr Ala Phe Met Ser Phe Phe Ser
       130                 135                 140
   Val Val Ile Ala Leu Phe Lys Asp Ile Pro Asp Ile Glu Gly Asp Lys
   145                 150                 155                 160
   Val Phe Gly Ile Gln Ser Phe Ser Val Cys Leu Gly Gln Lys Pro Val
                   165                 170                 175
   Phe Trp Thr Cys Val Thr Leu Leu Glu Ile Ala Tyr Gly Val Ala Leu
               180                 185                 190
   Leu Val Gly Ala Ala Ser Pro Cys Leu Trp Ser Lys Ile Phe Thr Gly
           195                 200                 205
   Leu Gly His Ala Val Leu Ala Ser Ile Leu Trp Phe His Ala Lys Ser
       210                 215                 220
   Val Asp Leu Lys Ser Lys Ala Ser Ile Thr Ser Phe Tyr Met Phe Ile
   225                 230                 235                 240
   Trp Lys Leu Phe Tyr Ala Glu Tyr Leu Leu Ile Pro Phe Val Arg
   245 250 255
<210> 25
   <211> 360
   <212> DNA
   <213> Zea sp
<220>
   <221> misc_feature
   <222> (1)...(360)
   <223> n = A,T,C or G
<400> 25
   ggcgtcttca cttgttctgg tcttctcgta tcccctgatg aagaggttca cattttggcc     60
   tcaggcttat cttggcctga cattcaactg gggagcttta ctagggtggg ctgctattaa   120
   ggaaagcata gaccctgcaa atcatccttc cattgtatac agctggtatt tgttggacgc   180
   tggtgtatga tactatatat gcgcatcagg tgtttcgcta tccctacttt catattaatc   240
   cttgatgaag tggccatttc atgttgtcgc ggtggtctta tacttgcata tctccatgca   300
   tctcaggaca aagangatga cctgaaagta ggagtccaag tccacagctt aagatttggg   360
<210> 26
   <211> 299
   <212> DNA
   <213> Zea sp
<220>
   <221> misc_feature
   <222> (1)...(299)
   <223> n = A,T,C or G
<400> 26
   gatggttgca gcatctgcaa ataccctcaa ccaggtgttt gngataaaaa atgatgctaa    60
   aatgaaaagg acaatgcgtg ccccctgcca tctggtcgca ttagtcctgc acatgctgcg   120
   atgtgggcta caagtgttgg agttgcagga acagctttgt tggcctggaa ggctaatggc   180
   ttggcagctg ggcttgcagc ttctaatctt gttctgtatg catttgtgta tacgccgttg   240
   aagcaaatac accctgttaa tacatgggtt ggggcagtcg ttggtgccat cccaccact    299
<210> 27
   <211> 255
   <212> DNA
   <213> Zea sp
<220>
   <221> misc_feature
   <222> (1)...(255)
   <223> n = A,T,C or G
<400> 27
   anacttgcat atctccatgc ntctcaggac aaagangatg acctgaaagt aggtgtcaag 60
   tccacagcat taagatttgg agatttgacc nnatactgna tcagtggctt tggcgcggca 120
   tgcttcggca gcttagcact cagtggttac aatgctgacc ttggttggtg tttagtgtga 180
   tgcttgagcg aagaatggta tngtttttac ttgatattga ctccagacct gaaatcatgt 240
   tggacagggt ggccc 255
<210> 28
   <211> 257
   <212> DNA
   <213> Zea sp
<400> 28
   attgaagggg ataggactct ggggcttcag tcacttcctg ttgcttttgg gatggaaact 60
   gcaaaatgga tttgtgttgg agcaattgat atcactcaat tatctgttgc aggttaccta 120
   ttgagcaccg gtaagctgta ttatgccctg gtgttgcttg ggctaacaat tcctcaggtg 180
   ttctttcagt tccagtactt cctgaaggac cctgtgaagt atgatgtcaa atatcaggca 240
   agcgcacaac cattctt 257
<210> 29
   <211> 368
   <212> DNA
   <213> Zea sp
<400> 29
   atccagttgc aaataataat ggcgttcttc tctgttgtaa tagcactatt caaggatata 60
   cctgacatcg aaggggaccg catattcggg atccgatcct tcagcgtccg gttagggcaa 120
   aagaaggtct tttggatctg cgttggcttg cttgagatgg cctacagcgt tgcgatactg 180
   atgggagcta cctcttcctg tttgtggagc aaaacagcaa ccatcgctgg ccattccata 240
   cttgccgcga tcctatggag ctgcgcgcga tcggtggact tgacgagcaa agccgcaata 300
   acgtccttct acatgttcat ctggaagctg ttctacgcgg agtacctgct catccctctg 360
   gtgcggtg 368
<210> 30
   <211> 122
   <212> PRT
   <213> Zea sp
<400> 30
   Ile Gln Leu Gln Ile Ile Met Ala Phe Phe Ser Val Val Ile Ala Leu
   1 5 10 15
   Phe Lys Asp Ile Pro Asp Ile Glu Gly Asp Arg Ile Phe Gly Ile Arg
   20 25 30
   Ser Phe Ser Val Arg Leu Gly Gln Lys Lys Val Phe Trp Ile Cys Val
   35 40 45
   Gly Leu Leu Glu Met Ala Tyr Ser Val Ala Ile Leu Met Gly Ala Thr
   50 55 60
   Ser Ser Cys Leu Trp Ser Lys Thr Ala Thr Ile Ala Gly His Ser Ile
   65 70 75 80
   Leu Ala Ala Ile Leu Trp Ser Cys Ala Arg Ser Val Asp Leu Thr Ser
   85 90 95
   Lys Ala Ala Ile Thr Ser Phe Tyr Met Phe Ile Trp Lys Leu Phe Tyr
   100 105 110
   Ala Glu Tyr Leu Leu Ile Pro Leu Val Arg
   115 120
<210> 31
   <211> 278
   <212> DNA
   <213> Zea sp
<400> 31
   tattcagcac cacctctcaa gctcaagcag aatggatgga ttgggaactt cgctctgggt 60
   gcgagttaca tcagcttgcc ctggtgggct ggccaggcgt tatttggaac tcttacacca 120
   gatatcattg tcttgactac tttgtacagc atagctgggc tagggattgc tattgtaaat 180
   gatttcaaga gtattgaagg ggataggact ctggggcttc agtcacttcc tgttgctttt 240
   gggatggaaa ctgcaaaatg gatttgtgtt ggagcaat 278
<210> 32
   <211> 292
   <212> PRT
   <213> Synechocystis sp
<400> 32
   Met Val Ala Gln Thr Pro Ser Ser Pro Pro Leu Trp Leu Thr Ile Ile
   1 5 10 15
   Tyr Leu Leu Arg Trp His Lys Pro Ala Gly Arg Leu Ile Leu Met Ile
   20 25 30
   Pro Ala Leu Trp Ala Val Cys Leu Ala Ala Gln Gly Leu Pro Pro Leu
   35 40 45
   Pro Leu Leu Gly Thr Ile Ala Leu Gly Thr Leu Ala Thr Ser Gly Leu
   50 55 60
   Gly Cys Val Val Asn Asp Leu Trp Asp Arg Asp Ile Asp Pro Gln Val
   65 70 75 80
   Glu Arg Thr Lys Gln Arg Pro Leu Ala Ala Arg Ala Leu Ser Val Gln
   85 90 95
   Val Gly Ile Gly Val Ala Leu Val Ala Leu Leu Cys Ala Ala Gly Leu
   100 105 110
   Ala Phe Tyr Leu Thr Pro Leu Ser Phe Trp Leu Cys Val Ala Ala Val
   115 120 125
   Pro Val Ile Val Ala Tyr Pro Gly Ala Lys Arg Val Phe Pro Val Pro
   130 135 140
   Gln Leu Val Leu Ser Ile Ala Trp Gly Phe Ala Val Leu Ile Ser Trp
   145 150 155 160
   Ser Ala Val Thr Gly Asp Leu Thr Asp Ala Thr Trp Val Leu Trp Gly
   165 170 175
   Ala Thr Val Phe Trp Thr Leu Gly Phe Asp Thr Val Tyr Ala Met Ala
   180 185 190
   Asp Arg Glu Asp Asp Arg Arg Ile Gly Val Asn Ser Ser Ala Leu Phe
   195 200 205
   Phe Gly Gln Tyr Val Gly Glu Ala Val Gly Ile Phe Phe Ala Leu Thr
   210 215 220
   Ile Gly Cys Leu Phe Tyr Leu Gly Met Ile Leu Met Leu Asn Pro Leu
   225 230 235 240
   Tyr Trp Leu Ser Leu Ala Ile Ala Ile Val Gly Trp Val Ile Gln Tyr
   245 250 255
   Ile Gln Leu Ser Ala Pro Thr Pro Glu Pro Lys Leu Tyr Gly Gln Ile
   260 265 270
   Phe Gly Gln Asn Val Ile Ile Gly Phe Val Leu Leu Ala Gly Met Leu
   275 280 285
   Leu Gly Trp Leu
   290
<210> 33
   <211> 316
   <212> PRT
   <213> Synechocystis sp
<400> 33
   Met Val Thr Ser Thr Lys Ile His Arg Gln His Asp Ser Met Gly Ala
   1 5 10 15
   Val Cys Lys Ser Tyr Tyr Gln Leu Thr Lys Pro Arg Ile Ile Pro Leu
   20 25 30
   Leu Leu Ile Thr Thr Ala Ala Ser Met Trp Ile Ala Ser Glu Gly Arg
   35 40 45
   Val Asp Leu Pro Lys Leu Leu Ile Thr Leu Leu Gly Gly Thr Leu Ala
   50 55 60
   Ala Ala Ser Ala Gln Thr Leu Asn Cys Ile Tyr Asp Gln Asp Ile Asp
   65 70 75 80
   Tyr Glu Met Leu Arg Thr Arg Ala Arg Pro Ile Pro Ala Gly Lys Val
   85 90 95
   Gln Pro Arg His Ala Leu Ile Phe Ala Leu Ala Leu Gly Val Leu Ser
   100 105 110
   Phe Ala Leu Leu Ala Thr Phe Val Asn Val Leu Ser Gly Cys Leu Ala
   115 120 125
   Leu Ser Gly Ile Val Phe Tyr Met Leu Val Tyr Thr His Trp Leu Lys
   130 135 140
   Arg His Thr Ala Gln Asn Ile Val Ile Gly Gly Ala Ala Gly Ser Ile
   145 150 155 160
   Pro Pro Leu Val Gly Trp Ala Ala Val Thr Gly Asp Leu Ser Trp Thr
   165 170 175
   Pro Trp Val Leu Phe Ala Leu Ile Phe Leu Trp Thr Pro Pro His Phe
   180 185 190
   Trp Ala Leu Ala Leu Met Ile Lys Asp Asp Tyr Ala Gln Val Asn Val
   195 200 205
   Pro Met Leu Pro Val Ile Ala Gly Glu Glu Lys Thr Val Ser Gln Ile
   210 215 220
   Trp Tyr Tyr Ser Leu Leu Val Val Pro Phe Ser Leu Leu Leu Val Tyr
   225 230 235 240
   Pro Leu His Gln Leu Gly Ile Leu Tyr Leu Ala Ile Ala Ile Ile Leu
   245 250 255
   Gly Gly Gln Phe Leu Val Lys Ala Trp Gln Leu Lys Gln Ala Pro Gly
   260 265 270
   Asp Arg Asp Leu Ala Arg Gly Leu Phe Lys Phe Ser Ile Phe Tyr Leu
   275 280 285
   Met Leu Leu Cys Leu Ala Met Val Ile Asp Ser Leu Pro Val Thr His
   290 295 300
   Gln Leu Val Ala Gln Met Gly Thr Leu Leu Leu Gly
   305 310 315
<210> 34
   <211> 324
   <212> PRT
   <213> Synechocystis sp
<400> 34
   Met Ser Asp Thr Gln Asn Thr Gly Gln Asn Gln Ala Lys Ala Arg Gln
   1 5 10 15
   Leu Leu Gly Met Lys Gly Ala Ala Pro Gly Glu Ser Ser Ile Trp Lys
   20 25 30
   Ile Arg Leu Gln Leu Met Lys Pro Ile Thr Trp Ile Pro Leu Ile Trp
   35 40 45
   Gly Val Val Cys Gly Ala Ala Ser Ser Gly Gly Tyr Ile Trp Ser Val
   50 55 60
   Glu Asp Phe Leu Lys Ala Leu Thr Cys Met Leu Leu Ser Gly Pro Leu
   65 70 75 80
   Met Thr Gly Tyr Thr Gln Thr Leu Asn Asp Phe Tyr Asp Arg Asp Ile
   85 90 95
   Asp Ala Ile Asn Glu Pro Tyr Arg Pro Ile Pro Ser Gly Ala Ile Ser
   100 105 110
   Val Pro Gln Val Val Thr Gln Ile Leu Ile Leu Leu Val Ala Gly Ile
   115 120 125
   Gly Val Ala Tyr Gly Leu Asp Val Trp Ala Gln His Asp Phe Pro Ile
   130 135 140
   Met Met Val Leu Thr Leu Gly Gly Ala Phe Val Ala Tyr Ile Tyr Ser
   145 150 155 160
   Ala Pro Pro Leu Lys Leu Lys Gln Asn Gly Trp Leu Gly Asn Tyr Ala
   165 170 175
   Leu Gly Ala Ser Tyr Ile Ala Leu Pro Trp Trp Ala Gly His Ala Leu
   180 185 190
   Phe Gly Thr Leu Asn Pro Thr Ile Met Val Leu Thr Leu Ile Tyr Ser
   195 200 205
   Leu Ala Gly Leu Gly Ile Ala Val Val Asn Asp Phe Lys Ser Val Glu
   210 215 220
   Gly Asp Arg Gln Leu Gly Leu Lys Ser Leu Pro Val Met Phe Gly Ile
   225 230 235 240
   Gly Thr Ala Ala Trp Ile Cys Val Ile Met Ile Asp Val Phe Gln Ala
   245 250 255
   Gly Ile Ala Gly Tyr Leu Ile Tyr Val His Gln Gln Leu Tyr Ala Thr
   260 265 270
   Ile Val Leu Leu Leu Leu Ile Pro Gln Ile Thr Phe Gln Asp Met Tyr
   275 280 285
   Phe Leu Arg Asn Pro Leu Glu Asn Asp Val Lys Tyr Gln Ala Ser Ala
   290 295 300
   Gln Pro Phe Leu Val Phe Gly Met Leu Ala Thr Gly Leu Ala Leu Gly
   305 310 315 320
   His Ala Gly Ile
<210> 35
   <211> 307
   <212> PRT
   <213> Synechocystis sp
<400> 35
   Met Thr Glu Ser Ser Pro Leu Ala Pro Ser Thr Ala Pro Ala Thr Arg
   1 5 10 15
   Lys Leu Trp Leu Ala Ala Ile Lys Pro Pro Met Tyr Thr Val Ala Val
   20 25 30
   Val Pro Ile Thr Val Gly Ser Ala Val Ala Tyr Gly Leu Thr Gly Gln
   35 40 45
   Trp His Gly Asp Val Phe Thr Ile Phe Leu Leu Ser Ala Ile Ala Ile
   50 55 60
   Ile Ala Trp Ile Asn Leu Ser Asn Asp Val Phe Asp Ser Asp Thr Gly
   65 70 75 80
   Ile Asp Val Arg Lys Ala His Ser Val Val Asn Leu Thr Gly Asn Arg
   85 90 95
   Asn Leu Val Phe Leu Ile Ser Asn Phe Phe Leu Leu Ala Gly Val Leu
   100 105 110
   Gly Leu Met Ser Met Ser Trp Arg Ala Gln Asp Trp Thr Val Leu Glu
   115 120 125
   Leu Ile Gly Val Ala Ile Phe Leu Gly Tyr Thr Tyr Gln Gly Pro Pro
   130 135 140
   Phe Arg Leu Gly Tyr Leu Gly Leu Gly Glu Leu Ile Cys Leu Ile Thr
   145 150 155 160
   Phe Gly Pro Leu Ala Ile Ala Ala Ala Tyr Tyr Ser Gln Ser Gln Ser
   165 170 175
   Phe Ser Trp Asn Leu Leu Thr Pro Ser Val Phe Val Gly Ile Ser Thr
   180 185 190
   Ala Ile Ile Leu Phe Cys Ser His Phe His Gln Val Glu Asp Asp Leu
   195 200 205
   Ala Ala Gly Lys Lys Ser Pro Ile Val Arg Leu Gly Thr Lys Leu Gly
   210 215 220
   Ser Gln Val Leu Thr Leu Ser Val Val Ser Leu Tyr Leu Ile Thr Ala
   225 230 235 240
   Ile Gly Val Leu Cys His Gln Ala Pro Trp Gln Thr Leu Leu Ile Ile
   245 250 255
   Ala Ser Leu Pro Trp Ala Val Gln Leu Ile Arg His Val Gly Gln Tyr
   260 265 270
   His Asp Gln Pro Glu Gln Val Ser Asn Cys Lys Phe Ile Ala Val Asn
   275 280 285
   Leu His Phe Phe Ser Gly Met Leu Met Ala Ala Gly Tyr Gly Trp Ala
   290 295 300
   Gly Leu Gly
   305
<210> 36
   <211> 927
   <212> DNA
   <213> Synechocystis sp
<400> 36
   atggcaacta tccaagcttt ttggcgcttc tcccgccccc ataccatcat tggtacaact 60
   ctgagcgtct gggctgtgta tctgttaact attctcgggg atggaaactc agttaactcc 120
   cctgcttccc tggatttagt gttcggcgct tggctggcct gcctgttggg taatgtgtac 180
   attgtcggcc tcaaccaatt gtgggatgtg gacattgacc gcatcaataa gccgaatttg      240
   cccctagcta acggagattt ttctatcgcc cagggccgtt ggattgtggg actttgtggc      300
   gttgcttcct tggcgatcgc ctggggatta gggctatggc tggggctaac ggtgggcatt      360
   agtttgatta ttggcacggc ctattcggtg ccgccagtga ggttaaagcg cttttccctg      420
   ctggcggccc tgtgtattct gacggtgcgg ggaattgtgg ttaacttggg cttattttta      480
   ttttttagaa ttggtttagg ttatcccccc actttaataa cccccatctg ggttttgact      540
   ttatttatct tagttttcac cgtggcgatc gccattttta aagatgtgcc agatatggaa      600
   ggcgatcggc aatttaagat tcaaacttta actttgcaaa tcggcaaaca aaacgttttt      660
   cggggaacct taattttact cactggttgt tatttagcca tggcaatctg gggcttatgg      720
   gcggctatgc ctttaaatac tgctttcttg attgtttccc atttgtgctt attagcctta      780
   ctctggtggc ggagtcgaga tgtacactta gaaagcaaaa ccgaaattgc tagtttttat      840
   cagtttattt ggaagctatt tttcttagag tacttgctgt atcccttggc tctgtggtta      900
   cctaattttt ctaatactat tttttag                                          927
<210> 37
   <211> 308
   <212> PRT
   <213> Synechocystis sp
<400> 37
   Met Ala Thr Ile Gln Ala Phe Trp Arg Phe Ser Arg Pro His Thr Ile
   1               5                   10                  15
   Ile Gly Thr Thr Leu Ser Val Trp Ala Val Tyr Leu Leu Thr Ile Leu
               20                  25                  30
   Gly Asp Gly Asn Ser Val Asn Ser Pro Ala Ser Leu Asp Leu Val Phe
           35                  40                  45
   Gly Ala Trp Leu Ala Cys Leu Leu Gly Asn Val Tyr Ile Val Gly Leu
       50                  55                  60
   Asn Gln Leu Trp Asp Val Asp Ile Asp Arg Ile Asn Lys Pro Asn Leu
   65                  70                  75                  80
   Pro Leu Ala Asn Gly Asp Phe Ser Ile Ala Gln Gly Arg Trp Ile Val
                   85                  90                  95
   Gly Leu Cys Gly Val Ala Ser Leu Ala Ile Ala Trp Gly Leu Gly Leu
               100                 105                 110
   Trp Leu Gly Leu Thr Val Gly Ile Ser Leu Ile Ile Gly Thr Ala Tyr
           115                 120                 125
   Ser Val Pro Pro Val Arg Leu Lys Arg Phe Ser Leu Leu Ala Ala Leu
       130                 135                 140
   Cys Ile Leu Thr Val Arg Gly Ile Val Val Asn Leu Gly Leu Phe Leu
   145 150 155 160
   Phe Phe Arg Ile Gly Leu Gly Tyr Pro Pro Thr Leu Ile Thr Pro Ile
   165 170 175
   Trp Val Leu Thr Leu Phe Ile Leu Val Phe Thr Val Ala Ile Ala Ile
   180 185 190
   Phe Lys Asp Val Pro Asp Met Glu Gly Asp Arg Gln Phe Lys Ile Gln
   195 200 205
   Thr Leu Thr Leu Gln Ile Gly Lys Gln Asn Val Phe Arg Gly Thr Leu
   210 215 220
   Ile Leu Leu Thr Gly Cys Tyr Leu Ala Met Ala Ile Trp Gly Leu Trp
   225 230 235 240
   Ala Ala Met Pro Leu Asn Thr Ala Phe Leu Ile Val Ser His Leu Cys
   245 250 255
   Leu Leu Ala Leu Leu Trp Trp Arg Ser Arg Asp Val His Leu Glu Ser
   260 265 270
   Lys Thr Glu Ile Ala Ser Phe Tyr Gln Phe Ile Trp Lys Leu Phe Phe
   275 280 285
   Leu Glu Tyr Leu Leu Tyr Pro Leu Ala Leu Trp Leu Pro Asn Phe Ser
   290 295 300
   Asn Thr Ile Phe
   305
<210> 38
   <211> 1092
   <212> DNA
   <213> Synechocystis sp
<400> 38
   atgaaatttc cgccccacag tggttaccat tggcaaggtc aatcaccttt ctttgaaggt 60
   tggtacgtgc gcctgctttt gccccaatcc ggggaaagtt ttgcttttat gtactccatc 120
   gaaaatcctg ctagcgatca tcattacggc ggcggtgctg tgcaaatttt agggccggct 180
   acgaaaaaac aagaaaatca ggaagaccaa cttgtttggc ggacatttcc ctcggtaaaa 240
   aaattttggg ccagtcctcg ccagtttgcc ctagggcatt ggggaaaatg tagggataac 300
   aggcaggcga aacccctact ctccgaagaa ttttttgcca cggtcaagga aggttatcaa 360
   atccatcaaa atcagcacca aggacaaatc attcatggcg atcgccattg tcgttggcag 420
   ttcaccgtag aaccggaagt aacttggggg agtcctaacc gatttcctcg ggctacagcg 480
   ggttggcttt cctttttacc cttgtttgat cccggttggc aaattctttt agcccaaggt 540
   agagcgcacg gctggctgaa atggcagagg gaacagtatg aatttgacca cgccctagtt 600
   tatgccgaaa aaaattgggg tcactccttt ccctcccgct ggttttggct ccaagcaaat 660
   tattttcctg accatccagg actgagcgtc actgccgctg gcggggaacg gattgttctt    720
   ggtcgccccg aagaggtagc tttaattggc ttacatcacc aaggtaattt ttacgaattt    780
   ggcccgggcc atggcacagt cacttggcaa gtagctccct ggggccgttg gcaattaaaa    840
   gccagcaatg ataggtattg ggtcaagttg tccggaaaaa cagataaaaa aggcagttta    900
   gtccacactc ccaccgccca gggcttacaa ctcaactgcc gagataccac taggggctat    960
   ttgtatttgc aattgggatc tgtgggtcac ggcctgatag tgcaagggga aacggacacc   1020
   gcggggctag aagttggagg tgattggggt ttaacagagg aaaatttgag caaaaaaaca   1080
   gtgccattct ga                                                       1092
<210> 39
   <211> 363
   <212> PRT
   <213> Synechocystis sp
<400> 39
   Met Lys Phe Pro Pro His Ser Gly Tyr His Trp Gln Gly Gln Ser Pro
   1               5                   10                  15
   Phe Phe Glu Gly Trp Tyr Val Arg Leu Leu Leu Pro Gln Ser Gly Glu
               20                  25                  30
   Ser Phe Ala Phe Met Tyr Ser Ile Glu Asn Pro Ala Ser Asp His His
           35                  40                  45
   Tyr Gly Gly Gly Ala Val Gln Ile Leu Gly Pro Ala Thr Lys Lys Gln
       50                  55                  60
   Glu Asn Gln Glu Asp Gln Leu Val Trp Arg Thr Phe Pro Ser Val Lys
   65                  70                  75                  80
   Lys Phe Trp Ala Ser Pro Arg Gln Phe Ala Leu Gly His Trp Gly Lys
                   85                  90                  95
   Cys Arg Asp Asn Arg Gln Ala Lys Pro Leu Leu Ser Glu Glu Phe Phe
               100                 105                 110
   Ala Thr Val Lys Glu Gly Tyr Gln Ile His Gln Asn Gln His Gln Gly
           115                 120                 125
   Gln Ile Ile His Gly Asp Arg His Cys Arg Trp Gln Phe Thr Val Glu
       130                 135                 140
   Pro Glu Val Thr Trp Gly Ser Pro Asn Arg Phe Pro Arg Ala Thr Ala
   145                 150                 155                 160
   Gly Trp Leu Ser Phe Leu Pro Leu Phe Asp Pro Gly Trp Gln Ile Leu
                   165                 170                 175
   Leu Ala Gln Gly Arg Ala His Gly Trp Leu Lys Trp Gln Arg Glu Gln
               180                 185                 190
   Tyr Glu Phe Asp His Ala Leu Val Tyr Ala Glu Lys Asn Trp Gly His
   195 200 205
   Ser Phe Pro Ser Arg Trp Phe Trp Leu Gln Ala Asn Tyr Phe Pro Asp
   210 215 220
   His Pro Gly Leu Ser Val Thr Ala Ala Gly Gly Glu Arg Ile Val Leu
   225 230 235 240
   Gly Arg Pro Glu Glu Val Ala Leu Ile Gly Leu His His Gln Gly Asn
   245 250 255
   Phe Tyr Glu Phe Gly Pro Gly His Gly Thr Val Thr Trp Gln Val Ala
   260 265 270
   Pro Trp Gly Arg Trp Gln Leu Lys Ala Ser Asn Asp Arg Tyr Trp Val
   275 280 285
   Lys Leu Ser Gly Lys Thr Asp Lys Lys Gly Ser Leu Val His Thr Pro
   290 295 300
   Thr Ala Gln Gly Leu Gln Leu Asn Cys Arg Asp Thr Thr Arg Gly Tyr
   305 310 315 320
   Leu Tyr Leu Gln Leu Gly Ser Val Gly His Gly Leu Ile Val Gln Gly
   325 330 335
   Glu Thr Asp Thr Ala Gly Leu Glu Val Gly Gly Asp Trp Gly Leu Thr
   340 345 350
   Glu Glu Asn Leu Ser Lys Lys Thr Val Pro Phe
   355 360
<210> 40
   <211> 56
   <212> DNA
   <213> Artificial Sequence
<400> 40
   cgcgatttaa atggcgcgcc ctgcaggcgg ccgcctgcag ggcgcgccat ttaaat 56
<210> 41
   <211> 32
   <212> DNA
   <213> Artificial Sequence
<400> 41
   tcgaggatcc gcggccgcaa gcttcctgca gg 32
<210> 42
   <211> 32
   <212> DNA
   <213> Artificial Sequence
<400> 42
   tcgacctgca ggaagcttgc ggccgcggat cc 32
<210> 43
   <211> 32
   <212> DNA
   <213> Artificial Sequence
<400> 43
   tcgacctgca ggaagcttgc ggccgcggat cc 32
<210> 44
   <211> 32
   <212> DNA
   <213> Artificial Sequence
<400> 44
   tcgaggatcc gcggccgcaa gcttcctgca gg 32
<210> 45
   <211> 36
   <212> DNA
   <213> Artificial Sequence
<400> 45
   tcgaggatcc gcggccgcaa gcttcctgca ggagct 36
<210> 46
   <211> 28
   <212> DNA
   <213> Artificial Sequence
<400> 46
   cctgcaggaa gcttgcggcc gcggatcc 28
<210> 47
   <211> 36
   <212> DNA
   <213> Artificial Sequence
<400> 47
   tcgacctgca ggaagcttgc ggccgcggat ccagct 36
<210> 48
   <211> 28
   <212> DNA
   <213> Artificial Sequence
<400> 48
   ggatccgcgg ccgcaagctt cctgcagg 28
<210> 49
   <211> 39
   <212> DNA
   <213> Artificial Sequence
<400> 49
   gatcacctgc aggaagcttg cggccgcgga tccaatgca 39
<210> 50
   <211> 31
   <212> DNA
   <213> Artificial Sequence
<400> 50
   ttggatccgc ggccgcaagc ttcctgcagg t 31
<210> 51
   <211> 41
   <212> DNA
   <213> Artificial Sequence
<400> 51
   ggatccgcgg ccgcacaatg gagtctctgc tctctagttc t 41
<210> 52
   <211> 38
   <212> DNA
   <213> Artificial Sequence
<400> 52
   ggatcctgca ggtcacttca aaaaaggtaa cagcaagt 38
<210> 53
   <211> 45
   <212> DNA
   <213> Artificial Sequence
<400> 53
   ggatccgcgg ccgcacaatg gcgttttttg ggctctcccg tgttt 45
<210> 54
   <211> 40
   <212> DNA
   <213> Artificial Sequence
<400> 54
   ggatcctgca ggttattgaa aacttcttcc aagtacaact 40
<210> 55
   <211> 38
   <212> DNA
   <213> Artificial Sequence
<400> 55
   ggatccgcgg ccgcacaatg tggcgaagat ctgttgtt 38
<210> 56
   <211> 37
   <212> DNA
   <213> Artificial Sequence
<400> 56
   ggatcctgca ggtcatggag agtagaagga aggagct 37
<210> 57
   <211> 50
   <212> DNA
   <213> Artificial Sequence
<400> 57
   ggatccgcgg ccgcacaatg gtacttgccg aggttccaaa gcttgcctct 50
<210> 58
   <211> 38
   <212> DNA
   <213> Artificial Sequence
<400> 58
   ggatcctgca ggtcacttgt ttctggtgat gactctat 38
<210> 59
   <211> 38
   <212> DNA
   <213> Artificial Sequence
<400> 59
   ggatccgcgg ccgcacaatg acttcgattc tcaacact 38
<210> 60
   <211> 36
   <212> DNA
   <213> Artificial Sequence
<400> 60
   ggatcctgca ggtcagtgtt gcgatgctaa tgccgt 36
<210> 61
   <211> 22
   <212> DNA
   <213> Artificial Sequence
<400> 61
   taatgtgtac attgtcggcc tc 22
<210> 62
   <211> 60
   <212> DNA
   <213> Artificial Sequence
<400> 62
   gcaatgtaac atcagagatt ttgagacaca acgtggcttt ccacaattcc ccgcaccgtc 60
<210> 63
   <211> 22
   <212> DNA
   <213> Artificial Sequence
<400> 63
   aggctaataa gcacaaatgg ga 22
<210> 64
   <211> 63
   <212> DNA
   <213> Artificial Sequence
<400> 64
   ggtatgagtc agcaacacct tcttcacgag gcagacctca gcggaattgg tttaggttat 60
   ccc 63
<210> 65
   <211> 26
   <212> DNA
   <213> Artificial Sequence
<400> 65
   ggatccatgg ttgcccaaac cccatc 26
<210> 66
   <211> 61
   <212> DNA
   <213> Artificial Sequence
<400> 66
   gcaatgtaac atcagagatt ttgagacaca acgtggcttt gggtaagcaa caatgaccgg 60
   c 61
<210> 67
   <211> 25
   <212> DNA
   <213> Artificial Sequence
<400> 67
   gaattctcaa agccagccca gtaac 25
<210> 68
   <211> 63
   <212> DNA
   <213> Artificial Sequence
<400> 68
   ggtatgagtc agcaacacct tcttcacgag gcagacctca gcgggtgcga aaagggtttt 60
   ccc 63
<210> 69
   <211> 23
   <212> DNA
   <213> Artificial Sequence
<400> 69
   ccagtggttt aggctgtgtg gtc 23
<210> 70
   <211> 21
   <212> DNA
   <213> Artificial Sequence
<400> 70
   ctgagttgga tgtattggat c 21
<210> 71
   <211> 28
   <212> DNA
   <213> Artificial Sequence
<400> 71
   ggatccatgg ttacttcgac aaaaatcc 28
<210> 72
   <211> 60
   <212> DNA
   <213> Artificial Sequence
<400> 72
   gcaatgtaac atcagagatt ttgagacaca acgtggcttt gctaggcaac cgcttagtac 60
<210> 73
   <211> 28
   <212> DNA
   <213> Artificial Sequence
<400> 73
   gaattcttaa cccaacagta aagttccc 28
<210> 74
   <211> 63
   <212> DNA
   <213> Artificial Sequence
<400> 74
   ggtatgagtc agcaacacct tcttcacgag gcagacctca gcgccggcat tgtcttttac 60
   atg 63
<210> 75
   <211> 20
   <212> DNA
   <213> Artificial Sequence
<400> 75
   ggaacccttg cagccgcttc 20
<210> 76
   <211> 22
   <212> DNA
   <213> Artificial Sequence
<400> 76
   gtatgcccaa ctggtgcaga gg 22
<210> 77
   <211> 28
   <212> DNA
   <213> Artificial Sequence
<400> 77
   ggatccatgt ctgacacaca aaataccg 28
<210> 78
   <211> 62
   <212> DNA
   <213> Artificial Sequence
<400> 78
   gcaatgtaac atcagagatt ttgagacaca acgtggcttt cgccaatacc agccaccaac 60
   ag 62
<210> 79
   <211> 27
   <212> DNA
   <213> Artificial Sequence
<400> 79
   gaattctcaa atccccgcat ggcctag 27
<210> 80
   <211> 65
   <212> DNA
   <213> Artificial Sequence
<400> 80
   ggtatgagtc agcaacacct tcttcacgag gcagacctca gcggcctacg gcttggacgt 60
   gtggg 65
<210> 81
   <211> 21
   <212> DNA
   <213> Artificial Sequence
<400> 81
   cacttggatt cccctgatct g 21
<210> 82
   <211> 21
   <212> DNA
   <213> Artificial Sequence
<400> 82
   gcaatacccg cttggaaaac g 21
<210> 83
   <211> 29
   <212> DNA
   <213> Artificial Sequence
<400> 83
   ggatccatga ccgaatcttc gcccctagc 29
<210> 84
   <211> 61
   <212> DNA
   <213> Artificial Sequence
<400> 84
   gcaatgtaac atcagagatt ttgagacaca acgtggcttt caatcctagg tagccgaggc 60
   g 61
<210> 85
   <211> 27
   <212> DNA
   <213> Artificial Sequence
<400> 85
   gaattcttag cccaggccag cccagcc 27
<210> 86
   <211> 66
   <212> DNA
   <213> Artificial Sequence
<400> 86
   ggtatgagtc agcaacacct tcttcacgag gcagacctca gcggggaatt gatttgttta 60
   attacc 66
<210> 87
   <211> 21
   <212> DNA
   <213> Artificial Sequence
<400> 87
   gcgatcgcca ttatcgcttg g 21
<210> 88
   <211> 24
   <212> DNA
   <213> Artificial Sequence
<400> 88
   gcagactggc aattatcagt aacg 24
<210> 89
   <211> 25
   <212> DNA
   <213> Artificial Sequence
<400> 89
   ccatggattc gagtaaagtt gtcgc 25
<210> 90
   <211> 0
   <213> Artificial Sequence
<400> 90
   gaattcactt caaaaaaggt aacag
<210> 91
   <211> 4550
   <212> DNA
   <213> Arabidopsis sp
<400> 91
   attttacacc aatttgatca cttaactaaa ttaattaaat tagatgatta tcccaccata 60
   tttttgagca ttaaaccata aaaccatagt tataagtaac tgttttaatc gaatatgact 120
   cgattaagat taggaaaaat ttataaccgg taattaagaa aacattaacc gtagtaaccg 180
   taaatgccga ttcctccctt gtctaaaaga cagaaaacat atattttatt ttgccccata 240
   tgtttcactc tatttaattt caggcacaat acttttggtt ggtaacaaaa ctaaaaagga 300
   caacacgtga tacttttcct cgtccgtcag tcagattttt tttaaactag aaacaagtgg 360
   caaatctaca ccacattttt tgcttaatct attaacttgt aagttttaaa ttcctaaaaa 420
   agtctaacta attcttctaa tataagtaca ttccctaaat ttcccaaaaa gtcaaattaa 480
   taattttcaa aatctaatct aaatatctaa taattcaaaa tcattaaaaa gacacgcaac 540
   aatgacacca attaatcatc ctcgacccac acaattctac agttctcatg ctaaaccata 600
   ttttttgctc tctgttcctt caaaatcatt tctttctctt ctttgattcc caaagatcac 660
   ttctttgtct ttgatttttg attttttttc tctctggcgt gaaggaagaa gctttatttc 720
   atggagtctc tgctctctag ttcttctctt gtttccgctg gtaaatctcg tccttttctg 780
   gtttcaggtt ttatttgttg tttaggtttc gtttttgtga ttcagaacca tacaaaaagt 840
   ttgaactttt ctgaatataa aataaggaaa aagtttcgat ttttataatg aattgtttac 900
   tagatcgaag taggtgacaa aggttattgt gtggagaagc ataatttctg ggcttgactt 960
   tgaattttgt ttctcatgca tgcaacttat caatcagctg gtgggttttg ttggaagaag 1020
   cagaatctaa agctccactc tttatcaggt tcgttagggt tttatgggtt tttgaaatta 1080
   aatactcaat catcttagtc tcattattct attggttgaa tcacattttc taatttggaa 1140
   tttatgagac aatgtatgtt ggacttagtt gaagttcttc tctttggtta tagttgaagt 1200
   gttactgatg ttgtttagct ctttacacca atatatacac ccaattttgc agaaatccga 1260
   gttctgcgtt gtgattcgag taaagttgtc gcaaaaccga agtttaggaa caatcttgtt 1320
   aggcctgatg gtcaaggatc ttcattgttg ttgtatccaa aacataagtc gagatttcgg 1380
   gttaatgcca ctgcgggtca gcctgaggct ttcgactcga atagcaaaca gaagtctttt 1440
   agagactcgt tagatgcgtt ttacaggttt tctaggcctc atacagttat tggcacagtt 1500
   aagtttctct ttaaaaatgt aactctttta aaacgcaatc tttcagggtt ttcaaggaga 1560
   taacattagc tctgtgattg gatttgcagg tgcttagcat tttatctgta tctttcttag 1620
   cagtagagaa ggtttctgat atatctcctt tacttttcac tggcatcttg gaggtaatga 1680
   atatataaca cataatgacc gatgaagaag atacattttt ttcgtctctc tgtttaaaca 1740
   attgggtttt gttttcaggc tgttgttgca gctctcatga tgaacattta catagttggg 1800
   ctaaatcagt tgtctgatgt tgaaatagat aaggtaacat gcaaattttc ttcatatgag 1860
   ttcgagagac tgatgagatt aatagcagct agtgcctaga tcatctctat gtgggttttt 1920
   gcaggttaac aagccctatc ttccattggc atcaggagaa tattctgtta acaccggcat 1980
   tgcaatagta gcttccttct ccatcatggt atggtgccat tttcacaaaa tttcaacttt 2040
   tagaattcta taagttactg aaatagtttg ttataaatcg ttatagagtt tctggcttgg 2100
   gtggattgtt ggttcatggc cattgttctg ggctcttttt gtgagtttca tgctcggtac 2160
   tgcatactct atcaatgtaa gtaagtttct caatactaga atttggctca aatcaaaatc 2220
   tgcagtttct agttttaggt taatgaggtt ttaataactt acttctacta caaacagttg 2280
   ccacttttac ggtggaaaag atttgcattg gttgcagcaa tgtgtatcct cgctgtccga 2340
   gctattattg ttcaaatcgc cttttatcta catattcagg tactaaacca ttttccttat 2400
   gttttgtagt tgttttcatc aaaatcactt ttatattact aaagctgtga aactttgttg 2460
   cagacacatg tgtttggaag accaatcttg ttcactaggc ctcttatttt cgccactgcg 2520
   tttatgagct ttttctctgt cgttattgca ttgtttaagg taaacaaaga tggaaaaaga 2580
   ttaaatctat gtatacttaa agtaaagcat tctactgtta ttgatgagaa gttttctttt 2640
   ttggttggat gcaggatata cctgatatcg aaggggataa gatattcgga atccgatcat 2700
   tctctgtaac tctgggtcag aaacgggtac gatatctaaa ctaaagaaat tgttttgact 2760
   caagtgttgg attaagatta cagaagaaag aaaactgttt ttgtttcttg caaaattcag 2820
   gtgttttgga catgtgttac actacttcaa atggcttacg ctgttgcaat tctagttgga 2880
   gccacatctc cattcatatg gagcaaagtc atctcggtaa caatctttct ttacccatcg 2940
   aaaactcgct aattcatcgt ttgagtggta ctggtttcat tttgttccgt tctgttgatt 3000
   ttttttcagg ttgtgggtca tgttatactc gcaacaactt tgtgggctcg agctaagtcc 3060
   gttgatctga gtagcaaaac cgaaataact tcatgttata tgttcatatg gaaggttaga 3120
   ttcgtttata aatagagtct ttactgcctt tttatgcgct ccaatttgga attaaaatag 3180
   cctttcagtt tcatcgaatc accattatac tgataaattc tcatttctgc atcagctctt 3240
   ttatgcagag tacttgctgt tacctttttt gaagtgactg acattagaag agaagaagat 3300
   ggagataaaa gaataagtca tcactatgct tctgttttta ttacaagttc atgaaattag 3360
   gtagtgaact agtgaattag agttttattc tgaaacatgg cagactgcaa aaatatgtca 3420
   aagatatgaa tttctgttgg gtaaagaagt ctctgcttgg gcaaaatctt aaggttcggt 3480
   gtgttgatat aatgctaagc gaagaaatcg attctatgta gaaatttccg aaactatgtg 3540
   taaacatgtc agaacatctc cattctatat cttcttctgc aagaaagctc tgtttttatc 3600
   acctaaactc tttatctctg tgtagttaag atatgtatat gtacgtgact acattttttt 3660
   gttgatgtaa tttgcagaac gtatggattt ttgttagaaa gcatgagttc gaaagtatat 3720
   gtttatatat atggataatt cagacctaac gtcgaagctc acaagcataa attcactact 3780
   atagtttgct ctgtaataga tagttccatt gatgtcttga aactgtacgt aactgcctgg 3840
   gcgttttgtg gttgatactg actactgagt gttctttgtg agtgttgtaa gtatacaaga 3900
   agaagaatat aggctcacgg gaacgactgt ggtggaagat gaaatggaga tcatcacgta 3960
   gcggctttgc caaagaccga gtcacgatcg agtctatgaa gtctttacag ctgctgatta 4020
   tgattgacca ttgcttagag acgcattgga atcttactag ggacttgcct gggagtttct 4080
   tcaagtacgt gtcagatcat acgatgtagg agatttcacg gctttgatgt gtttgtttgg 4140
   agtcacaatg cttaatgggc ttattggccc aataatagct agctcttttg ctttagccgt 4200
   ttcgtttgtc ccctggtggt gagtattatt agggtatggt gtgaccaaag tcaccagacc 4260
   tagagtgaat ctagtagagt cctagaccat ggtccatggc ttttatttgt aatttgaaaa 4320
   atgaacaatt ctttttgtaa ggaaaacttt tatatagtag acgtttacta tatagaaact 4380
   agttgaacta acttcgtgca attgcataat aatggtgtga aatagagggt gcaaaactca 4440
   ataaacattt cgacgtacca agagttcgaa acaataagca aaatagattt ttttgcttca 4500
   gactaatttg tacaatgaat ggttaataaa ccattgaagc ttttattaat 4550
<210> 92
   <211> 4450
   <212> DNA
   <213> Arabidopsis sp
<400> 92
   tttaggttac aaaatcaatg atattgcgta tgtcaactat aaaagccaaa agtaaagcct 60
   cttgtttgac cagaaggtca tgatcattgt atacatacag ccaaactacc tcctggaaga 120
   aaagacatgg atcccaaaca acaacaatag cttcttttac aagaaccagt agtaactagt 180
   cactaatcta aaagagttaa gtttcagctt ttctggcaat ggctccttga tcatttcaat 240
   cctgaaggag acccactttg tagcaagacc atgtcctctg tttcacttac agtgtgtctc 300
   aaaagtctac ttcaattctt catatatagg ttcctcacac tacagcttca tcctcattcg 360
   ttgacagaga gagagtcttt attgaaaact tcttccaagt acaactccac taaatataat 420
   agcaccaaac cacttgttcg acacaaatct gtacagatat aaaaacacta ttaggttttc 480
   caaggcaaat cacataattg gattgtgaaa gagtacaaaa gataaaccca aattttcata 540
   ctttctactg cagtcagcac cagatgataa gtcagctgtc cctatttgcc atcctaactg 600
   tcctgatgca gcggccagtg atgcgtaata ttgccaccct taatcattag agcgagaaac 660
   aaaaagaatc aaaagacagt aaatggaatt aggaatcaca aatgagtcct tgtaaagttt 720
   attgagtacc gagatctgca ctgaatccag aaagtgcaag aaaacctatg gatgctgtgc 780
   caaatccagt taaccaaagc tttgtattat caccgaatct aagggctgtt gacttaacac 840
   caacttttac atcatcttct ttgtcctgga gacacaatat attagacatt agtccatgga 900
   aaaaaaatga tttaacctag aatatctcaa aattacttgc ataaaaactg aacttgagct 960
   gaaattttgg gttcgtagct tgtggcatat actatttcat tttcaatggg ccacaaaggt 1020
   aactttcttt tctcacttct gttgcaaacg ggaagacttt tatggggcta actcttcact 1080
   taaagtatag aaatcagatg gaaaaggtgg gagatcaggg taattttctt ctttatgatt 1140
   gacaaaagtc gaacatcgaa atggatgcat ttgcatgaga catgaaacaa aagctgaaaa 1200
   agaaatctgt ggtggtgaag ctagaaaaag aaaacaaagc aagcaatatg cacacattga 1260
   gattaactac tttgctactg gtcataatca aatagatttt gaagctaaaa aataaaaagt 1320
   gaatatacct gatgtgcata aatagtatca taaacaaggg tccagcagac tccggagaga 1380
   tagagaggga gtacaataga tggtgctatg cttcctttaa ctgcagtcca tcctaacaat 1440
   gctccccagt ttatggtcaa acctaaaaag gcttgaggct gcaattataa aaacgaatca 1500
   atcataagaa aatcagaaaa tatataatgt ctaactttga gaagccagaa tagatttaaa 1560
   ttacccaaaa tgtaaacctc ttcataagtg ggtaggaaaa gacaagtaac aaagatgaag 1620
   cccctaaaac acggctgcag aatatacata ctgaaatgag ctcaagtaga aaagaatttg 1680
   atcacaaaac taaagacaag acctgagaac atatcttcag aatttgggcc aactacataa 1740
   gggtgaacca tatgtgtatg tgaattttta aacaaacact tgcaaatacg cgactttagg 1800
   gcaagtaaaa aatccaaaca aacctgtaat tgttaagttg gagaagaatc cctaagccta 1860
   aaagcaactg cagcccgaga aatccaatcc cttgaaatgg tgtcaaaaga ccactggcga 1920
   taggtcttag ttttgtacga tcaacctgga tataaaagaa atttgtaaga caacataatc 1980
   taaaacaaaa caaccataca aaatcttgag ctttacatac aagcaaccca tctttgttta 2040
   tggaagaatg aatccagtta catgaatgct gtgtatctac cctaactact aaacacatat 2100
   ttcaatcgaa aaacatattc caccttcacc atatctaaca cctgaagtct ttcacttttt 2160
   gaacgaagtc atcagaacat gcagataagc tattacccaa aacagagata tgactggaaa 2220
   tgttgtcgta aattgatcca acatagaaaa atcaagacca gttccagatg tcaaagcaat 2280
   aacactttcc caccatggtt acagaaacca tagttacaca aaacatgttt cctaaaccaa 2340
   catactaaag ggatatataa atttgacatc actttatcac cataccataa gatagcttaa 2400
   aaacaaactg acctttgtat ctatgtcctg atcaagcaga tcatttatag tacaaccagc 2460
   acctctaaga agtaatgctc cgcaaccaaa taaagccata tatttaaaac ttggaaggct 2520
   tccaggatca gcagccaacg caatcgacct atacaacaat gatggagatt cagagtatcg 2580
   atctatttac atagctctgg aactagatcc atgacgaaac atggaacatc gttataatat 2640
   ctaaagactt ccaaacagat tcctgagtaa gaaacccagt ggaactatag tactgtaaca 2700
   tatataaaat caaagaaaac tcaggtttat agcattatcc aatcctgatt tctgccaatc 2760
   cttaaccact ctcccatgct atcaaaaacc tcagctcaag atcatactac ctaattgcct 2820
   atgagctctt gggaagatca ttatggattt gataactgaa aaaagtaaca gagaaatagc 2880
   agactgcaag aactactcca aacttctcca ctgatatgta tgtagtctaa caataataaa 2940
   cagacataaa ttcttttatc aagcttcaag agcaagttag tcagaaaaca tcacagccaa 3000
   accaaccagg aaaacacata actttatcac ataaaactaa atttaatgta atctgactta 3060
   acataaacca tcctttggga cgaaaggaaa ctatataaac atgcagtctt tctttccctc 3120
   agctattctt tcggatggat tataatgaat ctcaaaagtg aaatgtcttg attctcagct 3180
   acattactca aaggcgaaga taaacttacc acatacaagg ccacgcaagc aaccaagttc 3240
   caatgggttt atccaatcga gcaagcttag cataacctct aacttcttct ggtaaataca 3300
   aatctatcca agaagcttcc ttaacaacaa caccatcact cttctcctta tcatctttct 3360
   tcggctttcc ctccaaaacc gaagaagacg acgacattcc acaaattaat ctgtaattcc 3420
   aaccaacacc aaaaaacttc tcctgatgca attctcttcc tttactccat acttggtaat 3480
   tatcattcca tgaaggataa cacttagtga aaggatttgt gtaatgggta gtcacaggat 3540
   tggacaagga tttatgttgt gattgcaaaa gagcagagga agaagatgga gttacggaga 3600
   cggaagattt caacaaccgt cttgaaacac gggagagccc aaaaaacgcc atctttgaga 3660
   gaaattgttg cctggaagaa acaaagactt gagatttcaa acgtaagtga attcttacga 3720
   acgaaagcta acttctcaag agaatcagat tagtgattcc tcaaaaacaa acaaaactat 3780
   ctaatttcag tttcgagtga tgaagcctta agaatctaga acctccatgg cgtttctaat 3840
   ctctcagaga taatcgaatt ccttaaacaa tcaaagctta gaaagagaag aacaacaaca 3900
   acaacaaaaa aaatcagatt aacaaccgac cagagagcaa cgacgacgcc ggcgagaaag 3960
   agcacgtcgt ctcggagcaa gacttcttct ccagtaaccc ggatggatcg ttaatgggcc 4020
   tgtagattat tatatttggg ccgaaacaat tgggtcagca aaaacttggg ggataatgaa 4080
   gaaacacgta cagtatgcat ttaggctcca aattaattgg ccatataatt cgaatcagat    4140
   aaactaatca acccctacct tacttatttc tcactgtttt tatttctacc ttagtagttg    4200
   aagaaacact tttatttatc ttttcgggac ccaaatttga taggatcggg ccattactca 4260
   tgagcgtcag acacatatta gccttatcag attagtgggg taaggttttt ttaattcggt 4320
   aagaagcaac aatcaatgtc ggagaaatta aagaatctgc atgggcgtgg cgtgatgata 4380
   tgtgcatatg gagtcagttg ccgatcatat ataactattt ataaactaca tataaagact 4440
   actaatagat 4450
<210> 93
   <211> 2850
   <212> DNA
   <213> Arabidopsis sp
<400> 93
   aattaaaatt tgagcggtct aaaccattag accgtttaga gatccctcca acccaaaata 60
   gtcgattttc acgtcttgaa catatattgg gccttaatct gtgtggttag taaagacttt 120
   tattggtcaa agaaaaacaa ccatggccca acatgttgat acttttattt aattatacaa 180
   gtacccctga attctctgaa atatatttga ttgacccaga tattaatttt aattatcatt 240
   tcctgtaaaa gtgaaggagt caccgtgact cgtcgtaatc tgaaaccaat ctgttcatat 300
   gatgaagaag tttctctcgt tctcctccaa cgcgtagaaa attctgacgg cttaacgatg 360
   tggcgaagat ctgttgttta tcgtttctct tcaagaatct ctgtttcttc ttcgttacca 420
   aaccctagac tgattccttg gtcccgcgaa ttatgtgccg ttaatagctt ctcccagcct 480
   ccggtctcga cggaatcaac tgctaagtta gggatcactg gtgttagatc tgatgccaat 540
   cgagtttttg ccactgctac tgccgccgct acagctacag ctaccaccgg tgagatttcg 600
   tctagagttg cggctttggc tggattaggg catcactacg ctcgttgtta ttgggagctt 660
   tctaaagcta aacttaggta tgtgtttact tttcttttct catgaaaaat ctgaaaattt 720
   ccaattgttg gattcttaaa ttctcatttg ttttatggtt gtagtatgct tgtggttgca 780
   acttctggaa ctgggtatat tctgggtacg ggaaatgctg caattagctt cccggggctt 840
   tgttacacat gtgcaggaac catgatgatt gctgcatctg ctaattcctt gaatcaggtc 900
   attgaaatgt tgagaagttc ataaatttcg aatccttgtt gtgtttatgt agttgatctt 960
   gcttgcttat gtttatgtag ttgaaaagtt taaaaatttc taatccttgg tagttgatct 1020
   cgcttgtttg ttttttcatt ttctagattt ttgagataag caatgattct aagatgaaaa 1080
   gaacgatgct aaggccattg ccttcaggac gtattagtgt tccacacgct gttgcatggg 1140
   ctactattgc tggtgcttct ggtgcttgtt tgttggccag caaggtgaat gtttgttttt 1200
   ttatatgtga tttctttgtt ttatgaatgg gtgattgaga gattatggat ctaaactttt 1260
   gcttccacga caaggttatt gcagactaat atgttggctg ctggacttgc atctgccaat 1320
   cttgtacttt atgcgtttgt ttatactccg ttgaagcaac ttcaccctat caatacatgg 1380
   gttggcgctg ttgttggtgc tatcccaccc ttgcttgggt aaatttttgt tccttttctt 1440
   ctttatttta gcagattctg ttttgttgga tactgctttt aattcaaaat gtagtcatgg 1500
   ttcaccaatt ctatgcttat ctattttgtg tgttgtcagg tgggcggcag cgtctggtca 1560
   gatttcatac aattcgatga ttcttccagc tgctctttac ttttggcaga tacctcattt 1620
   tatggccctt gcacatctct gccgcaatga ttatgcagct ggagggtaag accatatggt 1680
   gtcatatgag attagaatgt ctccttccat gtagtgttga tcttgaacta gttcaatttc 1740
   gtggaatgat cagagtgtcc tagatagtgt cacagcagtc gacattttag tggctagata 1800
   atgagttctt tccgttagag ataaacattc gcgaacattg tttccagctt ccgcgaccca 1860
   acttctgatt ttgtttcttg gtaccttgtt ttcagttaca agatgttgtc actctttgat 1920
   ccgtcaggga agagaatagc agcagtggct ctaaggaact gcttttacat gatccctctc 1980
   ggtttcatcg cctatgactg tgagtcttgt agattcatct tttttttgta gtttattgac 2040
   tgcattgctg tatctgattt ttgctgttcc ttccaatttt tgtgacaggg gggttaacct 2100
   caagttggtt ttgcctcgaa tcaacacttc tcacactagc aatcgctgca acagcatttt 2160
   cattctaccg agaccggacc atgcataaag caaggaaaat gttccatgcc agtcttctct 2220
   tccttcctgt tttcatgtct ggtcttcttc tacaccgtgt ctctaatgat aatcagcaac 2280
   aactcgtaga agaagccgga ttaacaaatt ctgtatctgg tgaagtcaaa actcagaggc 2340
   gaaagaaacg tgtggctcaa cctccggtgg cttatgcctc tgctgcaccg tttcctttcc 2400
   tcccagctcc ttccttctac tctccatgat aacctttaag caagctattg aatttttgga 2460
   aacagaaatt aaaaaaaaaa tctgaaaagt tcttaagttt aatctttggt taataatgaa 2520
   gtggagaacg catacaagtt tatgtatttt ttctcatctc cacataattg tattttttct 2580
   ctaagtatgt ttcaaatgat acaaaataca tactttatca attatctgat caaattgatg 2640
   aatttttgag ctttgacgtg ttaggtctat ctaataaacg tagtaacgaa tttggttttg 2700
   gaaatgaaat ccgataaccg atgatggtgt agagttaaac gattaaaccg ggttggttaa 2760
   aggtctcgag tctcgacggc tgcggaaatc ggaaaatcac gattgaggac tttgagctgc 2820
   cacgaagatg gcgatgaggt tgaaatcaat 2850
<210> 94
   <211> 3660
   <212> DNA
   <213> Arabidopsis sp
<400> 94
<210> 95
   <211> 1236
   <212> DNA
   <213> SOY
<400> 95
   atggattcac tgcttcttcg atctttccct aatattaata acgcctcttc tctcaccacc 60
   actggtgcaa atttctccag gactaaatct ttcgccaaca tttaccatgc aagttcttat 120
   gtgccaaatg cttcatggca caataggaaa atccaaaaag aatataattt tttgaggttt 180
   cggtggccaa gtttgaacca tcattacaaa ggcattgagg gagcgtgtac atgtaaaaaa 240
   tgtaatataa aatttgttgt gaaagcgacc tctgaaaaat ctcttgagtc tgaacctcaa 300
   gcttttgatc caaaaagcat tttggactct gtcaagaatt ccttggatgc tttctacagg 360
   ttttccaggc ctcacacagt tattggcaca gcattaagca taatttctgt gtctcttctt 420
   gctgttgaga aaatatcaga tatatctcca ttatttttta ctggtgtgtt ggaggctgtg 480
   gttgctgccc tgtttatgaa tatttatatt gttggtttga atcaattgtc tgatgttgaa 540
   atagacaaga taaacaagcc gtatcttcca ttagcatctg gggaatattc ctttgaaact 600
   ggtgtcacta ttgttgcatc tttttcaatt ctgagttttt ggcttggctg ggttgtaggt 660
   tcatggccat tattttgggc cctttttgta agctttgtgc taggaactgc ttattcaatc 720
   aatgtgcctc tgttgagatg gaagaggttt gcagtgcttg cagcgatgtg cattctagct 780
   gttcgggcag taatagttca acttgcattt ttccttcaca tgcagactca tgtgtacaag 840
   aggccacctg tcttttcaag accattgatt tttgctactg cattcatgag cttcttctct 900
   gtagttatag cactgtttaa ggatatacct gacattgaag gagataaagt atttggcatc 960
   caatcttttt cagtgcgttt aggtcagaag ccggtgttct ggacttgtgt tacccttctt 1020
   gaaatagctt atggagtcgc cctcctggtg ggagctgcat ctccttgtct ttggagcaaa 1080
   attttcacgg gtctgggaca cgctgtgctg gcttcaattc tctggtttca tgccaaatct 1140
   gtagatttga aaagcaaagc ttcgataaca tccttctata tgtttatttg gaagctattt 1200
   tatgcagaat acttactcat tccttttgtt agatga 1236
<210> 96
   <211> 1188
   <212> DNA
   <213> SOY
<400> 96
   atggattcga tgcttcttcg atcttttcct aatattaaca acgcttcttc tctcgccacc 60
   actggttctt atttgccaaa tgcttcatgg cacaatagga aaatccaaaa agaatataat 120
   tttttgaggt ttcggtggcc aagtttgaac caccattaca aaagcattga aggagggtgt 180
   acatgtaaaa aatgtaatat aaaatttgtt gtgaaagcga cctctgaaaa atcttttgag 240
   tctgaacccc aagcttttga tccaaaaagc attttggact ctgtcaagaa ttccttggat 300
   gctttctaca ggttttccag acctcacaca gttattggca cagcattaag cataatttct 360
   gtgtccctcc ttgctgttga gaaaatatca gatatatctc cattattttt tactggtgtg 420
   ttggaggctg tggttgctgc cctgtttatg aatatttata ttgttggttt gaatcaattg 480
   tctgatgttg aaatagacaa gataaacaag ccgtatcttc cattagcatc tggggaatat 540
   tcctttgaaa ctggtgtcac tattgttgca tctttttcaa ttctgagttt ttggcttggc 600
   tgggttgtag gttcatggcc attattttgg gccctttttg taagctttgt gctaggaact 660
   gcttattcaa tcaatgtgcc tctgttgaga tggaagaggt ttgcagtgct tgcagcgatg 720
   tgcattctag ctgttcgggc agtaatagtt caacttgcat ttttccttca catccagact 780
   catgtataca agaggccacc tgtcttttca agatcattga tttttgctac tgcattcatg 840
   agcttcttct ctgtagttat agcactgttt aaggatatac ctgacattga aggagataaa 900
   gtatttggca tccaatcttt ttcagtgcgt ttaggtcaga agccggtatt ctggacttgt 960
   gttatccttc ttgaaatagc ttatggagtc gccctcctgg tgggagctgc atctccttgt 1020
   ctttggagca aaattgtcac gggtctggga cacgctgttc tggcttcaat tctctggttt 1080
   catgccaaat ctgtagattt gaaaagcaaa gcttcgataa catccttcta tatgtttatt 1140
   tggaagctat tttatgcaga atacttactc attccttttg ttagatga 1188
<210> 97
   <211> 395
   <212> PRT
   <213> SOY
<400> 97
   Met Asp Ser Met Leu Leu Arg Ser Phe Pro Asn Ile Asn Asn Ala Ser
   1 5 10 15
   Ser Leu Ala Thr Thr Gly Ser Tyr Leu Pro Asn Ala Ser Trp His Asn
   20 25 30
   Arg Lys Ile Gln Lys Glu Tyr Asn Phe Leu Arg Phe Arg Trp Pro Ser
   35 40 45
   Leu Asn His His Tyr Lys Ser Ile Glu Gly Gly Cys Thr Cys Lys Lys
   50 55 60
   Cys Asn Ile Lys Phe Val Val Lys Ala Thr Ser Glu Lys Ser Phe Glu
   65 70 75 80
   Ser Glu Pro Gln Ala Phe Asp Pro Lys Ser Ile Leu Asp Ser Val Lys
   85 90 95
   Asn Ser Leu Asp Ala Phe Tyr Arg Phe Ser Arg Pro His Thr Val Ile
   100 105 110
   Gly Thr Ala Leu Ser Ile Ile Ser Val Ser Leu Leu Ala Val Glu Lys
   115 120 125
   Ile Ser Asp Ile Ser Pro Leu Phe Phe Thr Gly Val Leu Glu Ala Val
   130 135 140
   Val Ala Ala Leu Phe Met Asn Ile Tyr Ile Val Gly Leu Asn Gln Leu
   145 150 155 160
   Ser Asp Val Glu Ile Asp Lys Ile Asn Lys Pro Tyr Leu Pro Leu Ala
   165 170 175
   Ser Gly Glu Tyr Ser Phe Glu Thr Gly Val Thr Ile Val Ala Ser Phe
   180 185 190
   Ser Ile Leu Ser Phe Trp Leu Gly Trp Val Val Gly Ser Trp Pro Leu
   195 200 205
   Phe Trp Ala Leu Phe Val Ser Phe Val Leu Gly Thr Ala Tyr Ser Ile
   210 215 220
   Asn Val Pro Leu Leu Arg Trp Lys Arg Phe Ala Val Leu Ala Ala Met
   225 230 235 240
   Cys Ile Leu Ala Val Arg Ala Val Ile Val Gln Leu Ala Phe Phe Leu
   245 250 255
   His Ile Gln Thr His Val Tyr Lys Arg Pro Pro Val Phe Ser Arg Ser
   260 265 270
   Leu Ile Phe Ala Thr Ala Phe Met Ser Phe Phe Ser Val Val Ile Ala
   275 280 285
   Leu Phe Lys Asp Ile Pro Asp Ile Glu Gly Asp Lys Val Phe Gly Ile
   290 295 300
   Gln Ser Phe Ser Val Arg Leu Gly Gln Lys Pro Val Phe Trp Thr Cys
   305 310 315 320
   Val Ile Leu Leu Glu Ile Ala Tyr Gly Val Ala Leu Leu Val Gly Ala
   325 330 335
   Ala Ser Pro Cys Leu Trp Ser Lys Ile Val Thr Gly Leu Gly His Ala
   340 345 350
   Val Leu Ala Ser Ile Leu Trp Phe His Ala Lys Ser Val Asp Leu Lys
   355 360 365
   Ser Lys Ala Ser Ile Thr Ser Phe Tyr Met Phe Ile Trp Lys Leu Phe
   370 375 380
   Tyr Ala Glu Tyr Leu Leu Ile Pro Phe Val Arg
   385 390 395
<210> 98
   <211> 411
   <212> PRT
   <213> SOY
<400> 98
   Met Asp Ser Leu Leu Leu Arg Ser Phe Pro Asn Ile Asn Asn Ala Ser
   1               5                   10                  15
   Ser Leu Thr Thr Thr Gly Ala Asn Phe Ser Arg Thr Lys Ser Phe Ala
               20                  25                  30
   Asn Ile Tyr His Ala Ser Ser Tyr Val Pro Asn Ala Ser Trp His Asn
           35                  40                  45
   Arg Lys Ile Gln Lys Glu Tyr Asn Phe Leu Arg Phe Arg Trp Pro Ser
       50                  55                  60
   Leu Asn His His Tyr Lys Gly Ile Glu Gly Ala Cys Thr Cys Lys Lys
   65                  70                  75                  80
   Cys Asn Ile Lys Phe Val Val Lys Ala Thr Ser Glu Lys Ser Leu Glu
                   85                  90                  95
   Ser Glu Pro Gln Ala Phe Asp Pro Lys Ser Ile Leu Asp Ser Val Lys
               100                 105                 110
   Asn Ser Leu Asp Ala Phe Tyr Arg Phe Ser Arg Pro His Thr Val Ile
           115                 120                 125
   Gly Thr Ala Leu Ser Ile Ile Ser Val Ser Leu Leu Ala Val Glu Lys
       130                 135                 140
   Ile Ser Asp Ile Ser Pro Leu Phe Phe Thr Gly Val Leu Glu Ala Val
   145                 150                 155                 160
   Val Ala Ala Leu Phe Met Asn Ile Tyr Ile Val Gly Leu Asn Gln Leu
                   165                 170                 175
   Ser Asp Val Glu Ile Asp Lys Ile Asn Lys Pro Tyr Leu Pro Leu Ala
               180                 185                 190
   Ser Gly Glu Tyr Ser Phe Glu Thr Gly Val Thr Ile Val Ala Ser Phe
           195                 200                 205
   Ser Ile Leu Ser Phe Trp Leu Gly Trp Val Val Gly Ser Trp Pro Leu
       210                 215                 220
   Phe Trp Ala Leu Phe Val Ser Phe Val Leu Gly Thr Ala Tyr Ser Ile
   225                 230                 235                 240
   Asn Val Pro Leu Leu Arg Trp Lys Arg Phe Ala Val Leu Ala Ala Met
                   245                 250                 255
   Cys Ile Leu Ala Val Arg Ala Val Ile Val Gln Leu Ala Phe Phe Leu
               260                 265                 270
   His Met Gln Thr His Val Tyr Lys Arg Pro Pro Val Phe Ser Arg Pro
           275                 280                 285
   Leu Ile Phe Ala Thr Ala Phe Met Ser Phe Phe Ser Val Val Ile Ala
   290 295 300
   Leu Phe Lys Asp Ile Pro Asp Ile Glu Gly Asp Lys Val Phe Gly Ile
   305 310 315 320
   Gln Ser Phe Ser Val Arg Leu Gly Gln Lys Pro Val Phe Trp Thr Cys
   325 330 335
   Val Thr Leu Leu Glu Ile Ala Tyr Gly Val Ala Leu Leu Val Gly Ala
   340 345 350
   Ala Ser Pro Cys Leu Trp Ser Lys Ile Phe Thr Gly Leu Gly His Ala
   355 360 365
   Val Leu Ala Ser Ile Leu Trp Phe His Ala Lys Ser Val Asp Leu Lys
   370 375 380
   Ser Lys Ala Ser Ile Thr Ser Phe Tyr Met Phe Ile Trp Lys Leu Phe
   385 390 395 400
   Tyr Ala Glu Tyr Leu Leu Ile Pro Phe Val Arg
   405 410
<210> 99
   <211> 964
   <212> DNA
   <213> RICE
<400> 99
   gagcagcact gggtcttaca ttccaatgga gctcgcctgt tgctttcatt acatgcttcg 60
   tgactttatt tgctttggtc attgctataa ccaaagatct cccagatgtt gaaggggatc 120
   ggaagtatca aatatcaact ttggcgacaa agctcggtgt cagaaacatt gcatttcttg 180
   gctctggttt attgatagca aattatgttg ctgctattgc tgtagctttt ctcatgcctc 240
   aggctttcag gcgcactgta atggtgcctg tgcatgctgc ccttgccgtt ggtataattt 300
   tccagacatg ggttctggag caagcaaaat atactaagga tgctatttca cagtactacc 360
   ggttcatttg gaatctcttc tatgctgaat acatcttctt cccgttgata tagagaccaa 420
   gcaatctgat atggtctgca tgttgagtgc ggcaaaaact agaagcccat atgaacagtg 480
   ggagtagggg aacgaacatg ccatccatgg gaagactctg ataactctct ctcgcccggg 540
   ctgtaaaggg taagcactgt tgggcatata tatgaaagga aggtgataaa gcagggatgc 600
   taaattgcta ctgggatcct caaaggctta tagtggtcac cagtggaatg tgccttaata 660
   atttggttac ccagcagagc aagtttttgc aggttattag gtaatatctt tgagggaatg 720
   aacttagatt tcattgtttt aaggtctggt cacacaacgg gtagtagtgc tggagcggca 780
   aaaaacgacc ttgttttaca ctaccaaggg aggttaactc tagttttcat gtgaccactt 840
   accttgagag ttgagaccat ggaatcactt gtcgactcct cggcttgtat atttctagtg 900
   tcagcatttg cattctcctc cccacttgta cttgaaaagt tgaagacaac ttttttgttt 960
   gtgt 964
<210> 100
   <211> 421
   <212> DNA
   <213> WHEAT
<400> 100
   cgtccgcgga cgcgtgggtg cttattcagt caatctgccg cactttctat ggaagagatc 60
   tgctgttgtt gcagcactct gcatattagc agtgcgtgcg gtgatagttc aactggcatt 120
   ttttctccac attcagacat ttgttttcag aaggccggca gacttttcaa agccattgat 180
   atttgcaact gccttcatga cattcttctc agttgtaata gcattattca aggatatacc 240
   cgatattgaa ggggaccgca tctttggaat ccaatctttt agtggtagac taggtcaaag 300
   caggggtttc tggacttgcg ttggcctact tgaggttgcc tacggtgttg cgatactgag 360
   gggggtaact tcttccagtt tgtggagcaa atctataact gttgtgggcc atgcaatcct 420
   c 421
<210> 101
   <211> 705
   <212> DNA
   <213> LEEK
<400> 101
   gtttcccccc ctcgaatttt tttttttttt ttttacttca tttttctgtg aataaattct 60
   taaaaaagac aaagaaaacc actggatatc ctaaattcaa cataggctat tgtcattcaa 120
   tgataatctt taacacaaca tacaacatga atataattaa ggagaaatga tctgcaattg 180
   ttgaaagaac tctccgtttt taagatgaca attaaagcgt tgttaattcc agccatttct 240
   gcctccatta tctactcatc ttctcttgcg attcttttcc atgtaggtca taaaccctca 300
   tcttacaaaa ggaatgagca agtactcagc atagaagagc ttccacacga acatataaaa 360
   agatgtaata gtggttttgg tcattggtcc atgagatcta gcacgattcc aaagtaacga 420
   cccaagaatt gcatgaccta tcactgttaa gcatttgctc cataggcatg aggaagtagc 480
   tccaacaacc atgacaacag tgtaggccat ctcaaggaga tatatacata tccaaaacac 540
   cctctcctgg ccaaggcgca cgctgaaaga atggatgcca aatattttgt ctccgtctat 600
   atcaggtata tccttaaata gagcaataac aactgagaag aagctcatga aggcagttgc 660
   aaatatcaat ggccttgtga aacttgctgg tcttttgaaa acaaa 705
<210> 102
   <211> 637
   <212> DNA
   <213> LEEK
<220>
   <221> misc_feature
   <222> (1)..(637)
   <223> n = g, a, t or c
<400> 102
   nattcggcac gagttttgaa gaagttaagc atggactccc tccttaccaa gccagttgta 60
   atacctctgc cttctccagt ttgttcacta ccaatcttgc gaggcagttc tgcaccaggg 120
   cagtattcat gtagaaacta caatccaata agaattcaaa ggtgcctcgt aaattatgaa 180
   catgtgaaac caaggtttac aacatgtagt aggtctcaaa aacttggtca tgtaaaagcc 240
   acatccgagc attctttaga atctggatcc gaaggataca ctcctagaag catatgggaa 300
   gccgtactag cttcactgaa tgttctatac aaattttcac gacctcacac aataatagga 360
   acagcaatgg gcataatgtc agtttctttg cttgttgtcg agagcctatc cgatatttct 420
   cctctgtttt ttgtgggatt attagaggct gtggttgctg cattgtttat gaatgtttac 480
   attgtaggtc tgaatcaatt atttgacata gaaatagaca aggtcaataa acctgatctt 540
   cctcttgcat ctggagaata ctcaccaaga gctggtactg ctattgtcat tgcttcagcc 600
   atcatgagct ttggcattgg atggttagtt ggctctt 637
<210> 103
   <211> 677
   <212> DNA
   <213> CANOLA
<400> 103
   tttttttttt tttttttcaa aaagaccaat cctttagtat gtacatgaac aaagtgattt 60
   tgtctccaag ctacaaagaa gaagaagaga ggtatacaaa gaaaactaca aatgttcacc 120
   atgaatgcta gaagaagggg aataacagat actctgcgta gaagagattc catataaacc 180
   ggtaatatcc tgctatagct tcctttgtgt agtttgcttt ttctagcacc catgtctgga 240
   aaaccaagca tgaagccaag atcatatgtg caggaatcat caagctacct ctaaaaacct 300
   gaggcatgta gaaagctagt gatatggcag aaatatagtt cactagcaga agtccagaac 360
   cgaggaatgc aatgttcctc actccaagct ttgttgctag tgttgatatt tggaacttgc 420
   gatctccttc aacatcagga agatcttttg taatagcaat gactagtgca aacagtgtca 480
   caaaagacgt gatgaaagcc acaggtgcac tccactgaaa cgaaagtcca agagcagctc 540
   tagtagcatg gtacacacca aaattaagaa gaaaacctcg taccgtggca ataataagaa 600
   acgctgcaac tggaaatctc ttcattctaa atggtggaac agaatagatg gtccccagat 660
   cggacgcgtg ggtcgac 677
<210> 104
   <211> 1431
   <212> DNA
   <213> CORN
<400> 104
   ccacgcgtcc gcccggccaa gggatggacg cgcttcgcct acggccgtcc ctcctccccg 60
   tgcggcccgg cgcggcccgc ccgcgagatc attttctacc accatgttgt tccatacaac 120
   gaaatggtga aggacgaatt tgcttttcta gccaaaggac ccaaggtcct accttgcatc 180
   accatcagaa attcttcgaa tggaaatcct cctattgtag gatatcacat cggtcattaa 240
   atacttctgt taatgcttcg gggcaacagc tgcagtctga acctgaaaca catgattcta 300
   caaccatctg gagggcaata tcatcttctc tagatgcatt ttacagattt tcccggccac 360
   atactgtcat aggaacagca ttaagcatag tctcagtttc ccttctagct gtccagagct 420
   tgtctgatat atcacctttg ttcctcactg gtttgctgga ggcagtggta gctgcccttt 480
   tcatgaatat ctatattgtt ggactgaacc agttattcga cattgagata gacaaggtta 540
   acaagccaac tcttccattg gcatctgggg aatacaccct tgcaactggg gttgcaatag 600
   tttcggtctt tgccgctatg agctttggcc ttggatgggc tgttggatca caacctctgt 660
   tttgggctct tttcataagc tttgttcttg ggactgcata ttcaatcaat ctgccgtacc 720
   ttcgatggaa gagatttgct gttgttgcag cactgtgcat attagcagtt cgtgcagtga 780
   ttgttcagct ggcctttttt ctccacattc agacttttgt tttcaggaga ccggcagtgt 840
   tttctaggcc attattattt gcaactggat ttatgacgtt cttctctgtt gtaatagcac 900
   tattcaagga tatacctgac atcgaagggg accgcatatt cgggatccga tccttcagcg 960
   tccggttagg gcaaaagaag gtcttttgga tctgcgttgg cttgcttgag atggcctaca 1020
   gcgttgcgat actgatggga gctacctctt cctgtttgtg gagcaaaaca gcaaccatcg 1080
   ctggccattc catacttgcc gcgatcctat ggagctgcgc gcgatcggtg gacttgacga 1140
   gcaaagccgc aataacgtcc ttctacatgt tcatctggaa gctgttctac gcggagtacc 1200
   tgctcatccc tctggtgcgg tgagcgcgag gcgaggtggt ggcagacgga tcggcgtcgg 1260
   cggggcggca aacaactcca cgggagaact tgagtgccgg aagtaaactc ccgtttgaaa 1320
   gttgaagcgt gcaccaccgg caccgggcag agagagacac ggtggctgga tggatacgga 1380
   tggccccccc aataaattcc cccgtgcatg gtaaaaaaaa aaaaaaaaaa a 1431
<210> 105
   <211> 1870
   <212> DNA
   <213> CORN
<400> 105
   gccgcgcagc gcgacgagcg ccacctgctt gctgccgcgt gcctgcgtgc gtgtgcgtcc 60
   accactgacc ccgcgcccgc ccgccgcccc tgcccctcca ctccacttgc tcactcgtcg 120
   cggcccgctt cccccccggc caagggatgg acgcgcttcg cctacggccg tccctcctcc 180
   ccgtgcggcc cggcgcggcc cgcccgcgag atcattttct accaccatgt tgttccatac 240
   aacgaaatgg tgaaggacga atttgctttt ctagccaaag gacccaaggt cctaccttgc 300
   atcaccatca gaaattcttc gaatggaaat cctcctattg taggatatca catcggtcat 360
   taaatacttc tgttaatgct tcggggcaac agctgcagtc tgaacctgaa acacatgatt 420
   ctacaaccat ctggagggca atatcatctt ctctagatgc attttacaga ttttcccggc 480
   cacatactgt cataggaaca gcattaagca tagtctcagt ttcccttcta gctgtccaga 540
   gcttgtctga tatatcacct ttgttcctca ctggtttgct ggaggcagtg gtagctgccc 600
   ttttcatgaa tatctatatt gttggactga accagttatt cgacattgag atagacaagg 660
   ttaacaagcc aactcttcca ttggcatctg gggaatacac ccttgcaact ggggttgcaa 720
   tagtttcggt ctttgccgct atgagctttg gccttggatg ggctgttgga tcacaacctc 780
   tgttttgggc tcttttcata agctttgttc ttgggactgc atattcaatc aatctgccgt 840
   accttcgatg gaagagattt gctgttgttg cagcactgtg catattagca gttcgtgcag 900
   tgattgttca gctggccttt tttctccaca ttcagacttt tgttttcagg agaccggcag 960
   tgttttctag gccattatta tttgcaactg gatttatgac gttcttctct gttgtaatag 1020
   cactattcaa ggatatacct gacatcgaag gggaccgcat attcgggatc cgatccttca 1080
   gcgtccggtt agggcaaaag aaggtctttt ggatctgcgt tggcttgctt gagatggcct 1140
   acagcgttgc gatactgatg ggagctacct cttcctgttt gtggagcaaa acagcaacca 1200
   tcgctggcca ttccatactt gccgcgatcc tatggagctg cgcgcgatcg gtggacttga 1260
   cgagcaaagc cgcaataacg tccttctaca tgttcatctg gaagctgttc tacgcggagt 1320
   acctgctcat ccctctggtg cggtgagcgc gaggcgaggt ggtggcagac ggatcggcgt 1380
   cggcggggcg gcaaacaact ccacgggaga acttgagtgc cggaagtaaa ctcccgtttg 1440
   aaagttgaag cgtgcaccac cggcaccggg cagagagaga cacggtggct ggatggatac 1500
   ggatggcccc cccaataaat tcccccgtgc atggtacccc acgctgcttg atgatatccc 1560
   atgtgtccgg gtgaccggac ctgatcgtct ctagagagat tggttgcaca acgtccaaca 1620
   tagcccgtag gtattgctac cactgctagt atgatactcc ttcctagtcc ttgccagcac 1680
   cagtgaccca aacttggtcg gctgagctca gcgctcagca gctttacgtg catctgcgcc 1740
   ttgacttgtg cagtgggcgt cgctagcatg aatgatgtat ggtgcgtcac ggcctgacgg 1800
   ttcgtcagtc tgggccgtgt tttgtgtccg aggaagatcg tctgtcagag atctggattg 1860
   cctcgctgct 1870
<210> 106
   <211> 642
   <212> DNA
   <213> CORN
<400> 106
   cggccggact cttctgactt ggcaaccgcc gcgcagcgcg acgagcgcca cctgcttgct 60
   gccgcgtgcc tgcgtgcgtg tgcgtccacc actgaccccg cgcccgcccg ccgcccctgc 120
   ccctccactc cacttgctca ctcgtcggct cgtcgcggcc cgcttccccc ccggccaagg 180
   gatggacgcg cttcgcctac ggccgtccct cctccccgtg cggcccggcg cggcccgccc 240
   gcgaggcagt ggtagctgcc cttttcatga atatctatat tgttggactg aaccagttat 300
   tcgacattga gatagacaag gttaacaagc caactcttcc attggcatct ggggaataca 360
   cccttgcaac tggggttgca atagtttcgg tctttgccgc tatgagcttt ggccttggat 420
   gggctgttgg atcacaacct ctgttttggg ctcttttcat aagctttgtt cttgggactg 480
   catattcaat caatctgccg taccttcgat ggaagagatt tgctgttgtt gcagcactgt 540
   gcatattagc agttcgtgca gtgattgttc agctggcctt ttttctccac attcagactt 600
   ttgttttcag gagaccggca gtgttttcta ggccattatt at 642
<210> 107
   <211> 362
   <212> DNA
   <213> COTTON
<400> 107
   cccacgcgtc cgaacattgt ttgcacttgt tattgccata accaaggatc ttccagatgt 60
   agaaggagat cgcaaatttc aaatatcaac attagcaaca aagcttggag ttagaaatat 120
   tgcatttctt ggttccggac ttctactggt gaattatgtt gctgctgtgt tggctgcaat 180
   atacatgcct caggctttca ggcgtagttt aatgatacct gctcatatct ttttggcggt 240
   ctgcttgatt tttcagacat gggtgttgga acaagcaaat tacaaaaagg aagcaatctc 300
   ggggttctat cgtttcatat ggaatctctt ctatgcagag tatgcgattt tccccttcgt 360
   gt 362
<210> 108
   <211> 575
   <212> DNA
   <213> TOMATO
<400> 108
   cagatcaatt ccagttcctg ctgagttttc tccactcaaa accagttcac atgcaatagt 60
   acgggttttg aaatgtaaag catggaagag accaaaaaag cactattcct cttcaatgaa 120
   gttgcagcgg cagtatatca cgcaagagca tgttggagga agtgatctaa gcactattgc 180
   tgctgataaa aaacttaaag ggagattttt ggtgcacgca tcatctgaac accctcttga 240
   atctcaacct tctaaaagtc cttgggactc agttaatgat gccgtagatg ctttctacag 300
   gttctcgcgg ccccatacca taataggaac agcattgagc ataatttcag tttctctcct 360
   tgcagttgag aagttctctg atttttctcc attatttttc actggggtgt tagaggccat 420
   tgttgctgcc ctattcatga acatttacat agttggttta aaccagttgt ctgacatcga 480
   aatagacaag gtaaacaagc catatcttcc attggcatca ggggaatact ctgtacaaac 540
   tggagtgatt gttgtgtcgt cttttgccat tttga 575
<210> 109
   <211> 1663
   <212> DNA
   <213> ARABIDOPSIS
<400> 109
   aacaccaaac acacaatttc acattctttt gcatatttct tcttcttctt ccattatgga 60
   gatacggagc ttgattgttt ctatgaaccc taatttatct tcctttgagc tctctcgccc 120
   tgtatctcct ctcactcgct cactagttcc gttccgatcg actaaactag ttccccgctc 180
   catttctagg gggatcccgt cgatctccac cccgaatagt gaaactgaca agatctccgt 240
   taaacctgtt tacgtcccga cgtctcccaa tcgcgaactc cggactcctc acagtggata 300
   ccatttcgat ggaacacctc ggaagttctt cgagggatgg tggatccggg tttccatccc 360
   agagaagagg gagagttttt gttttatgta ttctgtggag aatcctgcat ttcggcagag 420
   tttgtcacca ttggaagtgg ctctatatgg acctagattc actggtgttg gagctcagat 480
   tcttggcgct aatgataaat atttatgcca atacgaacaa gactctcaca atttctgggg 540
   agatcgacat gagctagttt tggggaatac ttttagtgct gtgccaggcg caaaggctcc 600
   aaacaaggag gttccaccag aggaatttaa cagaagagtg tccgaagggt tccaagctac 660
   tccattttgg catcaaggtc acatttgcga tgatggccgt actgactatg cggaaactgt 720
   gaaatctgct cgttgggagt atagtactcg tcccgtttac ggttggggtg atgttggggc 780
   caaacagaag tcaactgcag gctggcctgc agcttttcct gtatttgagc ctcattggca 840
   gatatgcatg gcaggaggcc tttccacagg gtggatagaa tggggcggtg aaaggtttga 900
   gtttcgggat gcaccttctt attcagagaa gaattggggt ggaggcttcc caagaaaatg 960
   gttttgggtc cagtgtaatg tctttgaagg ggcaactgga gaagttgctt taaccgcagg 1020
   tggcgggttg aggcaattgc ctggattgac tgagacctat gaaaatgctg cactggtttg 1080
   tgtacactat gatggaaaaa tgtacgagtt tgttccttgg aatggtgttg ttagatggga 1140
   aatgtctccc tggggttatt ggtatataac tgcagagaac gaaaaccatg tggtggaact 1200
   agaggcaaga acaaatgaag cgggtacacc tctgcgtgct cctaccacag aagttgggct 1260
   agctacggct tgcagagata gttgttacgg tgaattgaag ttgcagatat gggaacggct 1320
   atatgatgga agtaaaggca aggtgatatt agagacaaag agctcaatgg cagcagtgga 1380
   gataggagga ggaccgtggt ttgggacatg gaaaggagat acgagcaaca cgcccgagct 1440
   actaaaacag gctcttcagg tcccattgga tcttgaaagc gccttaggtt tggtcccttt 1500
   cttcaagcca ccgggtctgt aacattgatg agtgttttgt ttgttgatag agacccatgt 1560
   gatgaatgaa gccttagtca tgtcattgct agcttcacta ttatgtatgt atgattttag 1620
   ttcgttcggt ccttgtggta aatgatacgg gccagtgtaa agt 1663
<210> 110
   <211> 488
   <212> PRT
   <213> ARABIDOPSIS
<400> 110
   Met Glu Ile Arg Ser Leu Ile Val Ser Met Asn Pro Asn Leu Ser Ser
   1 5 10 15
   Phe Glu Leu Ser Arg Pro Val Ser Pro Leu Thr Arg Ser Leu Val Pro
   20 25 30
   Phe Arg Ser Thr Lys Leu Val Pro Arg Ser Ile Ser Arg Val Ser Ala
   35 40 45
   Ser Ile Ser Thr Pro Asn Ser Glu Thr Asp Lys Ile Ser Val Lys Pro
   50 55 60
   Val Tyr Val Pro Thr Ser Pro Asn Arg Glu Leu Arg Thr Pro His Ser
   65 70 75 80
   Gly Tyr His Phe Asp Gly Thr Pro Arg Lys Phe Phe Glu Gly Trp Tyr
   85 90 95
   Phe Arg Val Ser Ile Pro Glu Lys Arg Glu Ser Phe Cys Phe Met Tyr
   100 105 110
   Ser Val Glu Asn Pro Ala Phe Arg Gln Ser Leu Ser Pro Leu Glu Val
   115 120 125
   Ala Leu Tyr Gly Pro Arg Phe Thr Gly Val Gly Ala Gln Ile Leu Gly
   130 135 140
   Ala Asn Asp Lys Tyr Leu Cys Gln Tyr Glu Gln Asp Ser His Asn Phe
   145 150 155 160
   Trp Gly Asp Arg His Glu Leu Val Leu Gly Asn Thr Phe Ser Ala Val
   165 170 175
   Pro Gly Ala Lys Ala Pro Asn Lys Glu Val Pro Pro Glu Glu Phe Asn
   180 185 190
   Arg Arg Val Ser Glu Gly Phe Gln Ala Thr Pro Phe Trp His Gln Gly
   195 200 205
   His Ile Cys Asp Asp Gly Arg Thr Asp Tyr Ala Glu Thr Val Lys Ser
   210 215 220
   Ala Arg Trp Glu Tyr Ser Thr Arg Pro Val Tyr Gly Trp Gly Asp Val
   225 230 235 240
   Gly Ala Lys Gln Lys Ser Thr Ala Gly Trp Pro Ala Ala Phe Pro Val
   245 250 255
   Phe Glu Pro His Trp Gln Ile Cys Met Ala Gly Gly Leu Ser Thr Gly
   260 265 270
   Trp Ile Glu Trp Gly Gly Glu Arg Phe Glu Phe Arg Asp Ala Pro Ser
   275 280 285
   Tyr Ser Glu Lys Asn Trp Gly Gly Gly Phe Pro Arg Lys Trp Phe Trp
   290 295 300
   Val Gln Cys Asn Val Phe Glu Gly Ala Thr Gly Glu Val Ala Leu Thr
   305 310 315 320
   Ala Gly Gly Gly Leu Arg Gln Leu Pro Gly Leu Thr Glu Thr Tyr Glu
   325 330 335
   Asn Ala Ala Leu Val Cys Val His Tyr Asp Gly Lys Met Tyr Glu Phe
   340 345 350
   Val Pro Trp Asn Gly Val Val Arg Trp Glu Met Ser Pro Trp Gly Tyr
   355 360 365
   Trp Tyr Ile Thr Ala Glu Asn Glu Asn His Val Val Glu Leu Glu Ala
   370 375 380
   Arg Thr Asn Glu Ala Gly Thr Pro Leu Arg Ala Pro Thr Thr Glu Val
   385 390 395 400
   Gly Leu Ala Thr Ala Cys Arg Asp Ser Cys Tyr Gly Glu Leu Lys Leu
   405 410 415
   Gln Ile Trp Glu Arg Leu Tyr Asp Gly Ser Lys Gly Lys Val Ile Leu
   420 425 430
   Glu Thr Lys Ser Ser Met Ala Ala Val Glu Ile Gly Gly Gly Pro Trp
   435 440 445
   Phe Gly Thr Trp Lys Gly Asp Thr Ser Asn Thr Pro Glu Leu Leu Lys
   450 455 460
   Gln Ala Leu Gln Val Pro Leu Asp Leu Glu Ser Ala Leu Gly Leu Val
   465 470 475 480
   Pro Phe Phe Lys Pro Pro Gly Leu
   485

## Description

### INTRODUCTION

### TECHNICAL FIELD

The present invention is directed to nucleic acid and amino acid sequences and constructs, and methods related thereto.

### BACKGROUND

Isoprenoids are ubiquitous compounds found in all living organisms. Plants synthesize a diverse array of greater than 22,000 isoprenoids (Connolly and Hill (1992) Dictionary of Terpenoids, Chapman and Hall, New York, NY). In plants, isoprenoids play essential roles in particular cell functions such as production of sterols, contributing to eukaryotic membrane architecture, acyclic polyprenoids found in the side chain of ubiquinone and plastoquinone, growth regulators like abscisic acid, gibberellins, brassinosteroids or the photosynthetic pigments chlorophylls and carotenoids. Although the physiological role of other plant isoprenoids is less evident, like that of the vast array of secondary metabolites, some are known to play key roles mediating the adaptative responses to different environmental challenges. In spite of the remarkable diversity of structure and function, all isoprenoids originate from a single metabolic precursor, isopentenyl diphosphate (IPP) (Wright, (1961) Annu. Rev. Biochem. 20:525-548; and Spurgeon and Porter, (1981) in Biosynthesis of Isoprenoid Compounds., Porter and Spurgeon eds (John Wiley, New York) Vol. 1, pp1-46).

A number of unique and interconnected biochemical pathways derived from the isoprenoid pathway leading to secondary metabolites, including tocopherols, exist in chloroplasts of higher plants. Tocopherols not only perform vital functions in plants, but are also important from mammalian nutritional perspectives. In plastids, tocopherols account for up to 40% of the total quinone pool.

Tocopherols and tocotrienols (unsaturated tocopherol derivatives) are well known antioxidants, and play an important role in protecting cells from free radical damage, and in the prevention of many diseases, including cardiac disease, cancer, cataracts, retinopathy, Alzheimer's disease, and neurodegeneration, and have been shown to have beneficial effects on symptoms of arthritis, and in anti-aging. Vitamin E is used in chicken feed for improving the shelf life, appearance, flavor, and oxidative stability of meat, and to transfer tocols from feed to eggs. Vitamin E has been shown to be essential for normal reproduction, improves overall performance, and enhances immunocompetence in livestock animals. Vitamin E supplement in animal feed also imparts oxidative stability to milk products.

The demand for natural tocopherols as supplements has been steadily growing at a rate of 10-20% for the past three years. At present, the demand exceeds the supply for natural tocopherols, which are known to be more biopotent than racemic mixtures of synthetically produced tocopherols. Naturally occurring tocopherols are all *d*-stereomers, whereas synthetic α-tocopherol is a mixture of eight *d,l*-α-tocopherol isomers, only one of which (12.5%) is identical to the natural *d*-α-tocopherol. Natural *d*-α-tocopherol has the highest vitamin E activity (1.49 IU/mg) when compared to other natural tocopherols or tocotrienols. The synthetic α-tocopherol has a vitamin E activity of 1.1 IU/mg. In 1995, the worldwide market for raw refined tocopherols was $1020 million; synthetic materials comprised 85-88% of the market, the remaining 12-15% being natural materials. The best sources of natural tocopherols and tocotrienols are vegetable oils and grain products. Currently, most of the natural Vitamin E is produced from γ-tocopherol derived from soy oil processing, which is subsequently converted to α-tocopherol by chemical modification (α-tocopherol exhibits the greatest biological activity).

Methods of enhancing the levels of tocopherols and tocotrienols in plants, especially levels of the more desirable compounds that can be used directly, without chemical modification, would be useful to the art as such molecules exhibit better functionality and biovailability.

In addition, methods for the increased production of other isoprenoid derived compounds in a host plant cell is desirable. Furthermore, methods for the production of particular isoprenoid compounds in a host plant cell is also needed.

WO 99/04522 and WO 00/10380 both disclose the gene sequences encoding gamma-tocopherol methyltransferases.

EMBL Sequence Database Accession number D90909; XP002193030; and TREMBL database Accession number =73727, XP002193031 disclose nucleotide and deduced amino acid sequences of a gene from Cyanobacterium Synchocystis sp. However, these publications neither disclose the function of the gene nor of the protein encoded.

### SUMMARY OF THE INVENTION

The present invention is directed to sequences to proteins involved in tocopherol synthesis. Specifically, the present invention relates to a nucleic acid encoding a tocopherol cyclase comprising an amino acid sequence having at least 90% identity to the amino acid sequence of SEQ ID NO: 39 or SEQ ID NO: 110, comprising, as operably linked components, a transcriptional initiation region functional in a host plant cell, a nucleic acid sequence encoding the tocopherol cyclase, and a transcriptional termination region.

The present invention further relates to plant cells comprising respective constructs, which plants may be selected from the group of *Acacia,* alfalfa, aneth, apple, apricot, artichoke, aragula, asparagus, avocado, banana, barley, beans, beet, blackberry, blueberry, broccoli, brussels sprouts, cabbage, canola, cantaloupe, carrot, cassava, cauliflower, celery, cherry, chicory, cilantro, citrus, clementines, coffee, corn, cotton, cucumber, Douglas fir, eggplant, endive, escarole, eucalyptus, fennel, figs, garlic, gourd, grape, grapefruit, honey dew, jicama, kiwifruit, lettuce, leeks, lemon, lime, Loblolly pine, mango, melon, mushroom, nectarine, nut, oat, oil palm, oil seed rape, okra, onion, orange, an ornamental plant, papaya, parsley, pea, peach, peanut, pear, pepper, persimmon, pine, pineapple, plantain, plum, pomegranate, poplar, potato, pumpkin, quince, radiata pine, radicchio, radish, raspberry, rice, rye, sorghum, Southern pine, soybean, spinach, squash, strawberry, sugarbeet, sugarcane, sunflower, sweet potato, sweetgum, tangerine, tea, tobacco, tomato, triticale, turf, turnip, a vine, watermelon, wheat, yams and zucchini, safflower, coconut palm.

In a further embodiment the invention relates to plants and seeds comprising these cells as well as a feed composition comprising respective plants

In an alternative embodiment the invention relates to a method of producing oil using these seed.

In related aspects the present invention provides:
- methods for the alteration of the isoprenoid content in a host cell, wherein these methods comprise transforming host cell with the above constructs, wherein said isoprenoid compound is selected from the group of tocopherols and tocotrienols;
- methods for producing an isoprenoid compound of interest in a host cell, which methods comprise obtaining a transformed host cell into which the above construct has been introduced, wherein the host cell has the construct in its genome and expresses it; and wherein said isoprenoid compound is selected from the group consisting of tocopherols and tocotrienols;
- methods for increasing the biosynthetic flux in a host cell toward production of an isoprenoid compound, wherein the methods comprise transforming a host cell with the above construct, wherein said isoprenoid compound is selected from the group of tocopherols and tocotrienols.

### BRIEF DESCRIPTION OF THE DRAWINGS

Figure 1 provides an amino acid sequence alignment between ATPT2, ATPT3, ATPT4, ATPT8, and ATPTI2 are performed using ClustalW.
Figure 2 provides a schematic picture of the expression construct pCGN10800.
Figure 3 provides a schematic picture of the expression construct pCGN 10801.
Figure 4 provides a schematic picture of the expression construct pCGN10803.
Figure 5 provides a schematic picture of the construct pCGN10806.
Figure 6 provides a schematic picture of the construct pCGN10807.
Figure 7 provides a schematic picture of the construct pCGN10808.
Figure 8 provides a schematic picture of the expression construct pCGN 10809.
Figure 9 provides a schematic picture of the expression construct pCGN10810.
Figure 10 provides a schematic picture of the expression construct pCGN10811.
Figure 11 provides a schematic picture of the expression construct pCGN10812.
Figure 12 provides a schematic picture of the expression construct pCGN10813.
Figure 13 provides a schematic picture of the expression construct pCGN10814.
Figure 14 provides a schematic picture of the expression construct pCGN 10815.
Figure 15 provides a schematic picture of the expression construct pCGN10816.

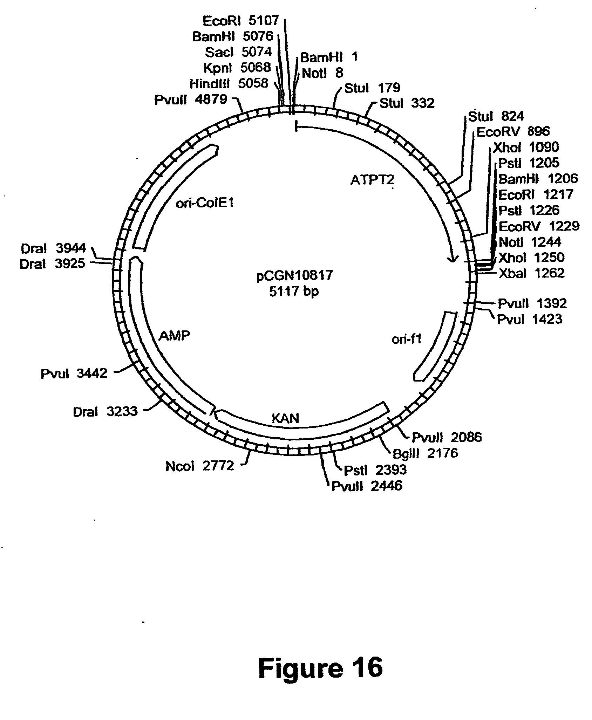

Figure 16 provides a schematic picture of the expression construct pCGN10817.
Figure 17 provides a schematic picture of the expression construct pCGN10819.
Figure 18 provides a schematic picture of the expression construct PCGN10824.
Figure 19 provides a schematic picture of the expression construct pCGN10825.
Figure 20 provides a schematic picture of the expression construct pCGN10826.
Figure 21 provides an amino acid sequence alignment using ClustalW between the *Synechocystis* prenyltransferase sequences.
Figure 22 provides an amino acid sequence of the ATPT2, ATPT3, ATPT4, ATPT8, and ATPT12 protein sequences from *Arabidopsis* and the slr1736, slr0926, sll1899, slr0056, and the slr1518 amino acid sequences from *Synechocystis*.
Figure 23 provides the results of the enzymatic assay from preparations of wild type *Synechocystis* strain 6803, and *Synechocystis* slr1736 knockout.
Figure 24 provides bar graphs of HPLC data obtained from seed extracts of transgenic *Arabidopsis* containing pCGN10822, which provides of the expression of the ATPT2 sequence, in the sense orientation, from the napin promoter. Provided are graphs for alpha, gamma, and delta tocopherols, as well as total tocopherol for 22 transformed lines, as well as a nontransformed (wildtype) control.
Figure 25 provides a bar graph of HPLC analysis of seed extracts from *Arabidopsis* plants transformed with pCGN10803 (35S-ATPT2, in the antisense orientation), pCGN10822 (line 1625, napin ATPT2 in the sense orientation), pCGN10809 (line 1627, 35S-ATPT3 in the sense orientation), a nontransformed (wt) control, and an empty vector transformed control.
Figure 26 shows total tocopherol levels measured in T# *Arabidopsis* seed of line.
Figure 27 shows total tocopherol levels measured in T# *Arabidopsis* seed of line.
Figure 28 shows total tocopherol levels measured in developing canola seed of line 10822-1.
Figure 29: shows results of phytyl prenyltransferase activity assay using *Synechocystis* wild type and slr1737 knockout mutant membrane preparations.
Figure 30 is the chromatograph from an HPLC analysis of *Synechocystis* extracts.
Figure 31 is a sequence alignment of the *Arabidopsis* homologue with the sequence of the public database.
Figure 32 shows the results of hydropathic analysis of slr1737
Figure 33 shows the results of hydropathic analysis of the *Arabidopsis* homologue of slr1 737.
Figure 34 shows the catalytic mechanism of various cyclase enzymes
Figure 35 is a sequence alignment of slr1737, slr1737 *Arabidopsis* homologue and the *Arabidopsis* chalcone isomerase.

### DETAILED DESCRIPTION OF THE INVENTION

The present invention provides, *inter alia,* compositions and methods for altering (for example, increasing and decreasing) the tocopherol levels and/or modulating their ratios in host cells. In particular, the present invention provides polynucleotides, polypeptides, and methods of use thereof for the modulation of tocopherol content in host plant cells.

The biosynthesis of α-tocopherol in higher plants involves condensation of homogentisic acid and phytylpyrophosphate to form 2-methyl-6 phytylbenzoquinol that can, by cyclization and subsequent methylations (Fiedler et al., 1982, Planta, 155: 511-515, Soll et al., 1980, Arch. Biochem. Biophys. 204: 544-550, Marshall et al., 1985 Phytochem., 24: 1705-1711), form various tocopherols.

The *Arabidopsis pds2* mutant identified and characterized by Norris *et al.* (1995), is deficient in tocopherol and plastiquinone-9 accumulation. Further genetic and biochemical analysis suggested that the protein encoded by *PDS2* may be responsible for the prenylation of homogentisic acid The *PDS2* locus identified by Norris *et al.* (1995) has been hypothesized to possibly encode the tocopherol phytyl-prenyltransferase, as the *pds2* mutant fails to accumulate tocopherols.

Norris *et al.* (1995) determined that in *Arabidopsis pds2* lies at the top of chromosome 3, approximately 7 centimorgans above long hypocotyl2, based on the genetic map. ATPT2 is located on chromosome 2 between 36 and 41 centimorgans, lying on BAC F19F24, indicating that ATPT2 does not correspond to *PDS2.* Thus, it is an aspect of the present invention to provide novel polynucleotides and polypeptides involved in the prenylation of homogentisic acid. This reaction may be a rate limiting step in tocopherol biosynthesis, and this gene has yet to be isolated.

U.S. Patent No. 5,432,069 describes the partial purification and characterization of tocopherol cyclase from *Chlorella protothecoides, Dunaliella salina* and wheat. The cyclase described as being glycine rich, water soluble and with a predicted MW of 48-50kDa. However, only limited peptide fragment sequences were available.

In another aspect, the invention provides polynucleotide and polypeptide sequences as defined above to tocopherol cyclization enzymes. The 2,3-dimethyl-5-phytylplastoquinol cyclase (tocopherol cyclase) is responsible for the cyclization of 2,3-dimethyl-5-phytylplastoquinol to tocopherol.

### Isolated Polynucleotides, Proteins, and Polypeptides

The present invention relates to isolated tocopherol cyclase polynucleotides as defined above. The polynucleotide sequences of the present invention include isolated polynucleotides that encode the polypeptides of the invention having a deduced amino acid sequence selected from the group of sequences set forth in the Sequence Listing and to other polynucleotide sequences closely related to such sequences and variants thereof as defined above.

Typically, a tocopherol cyclase sequence obtainable from the use of nucleic acid probes will show 90% sequence identity between the target prenyltransferase or tocopherol cyclase sequence and the encoding sequence used as a probe.

"Identity", as is well understood in the art, is a relationship between two or more polypeptide sequences or two or more polynucleotide sequences, as determined by comparing the sequences. In the art, "identity" also means the degree of sequence relatedness between polypeptide or polynucleotide sequences, as determined by the match between strings of such sequences. "Identity" can be readily calculated by known methods including, but not limited to, those described in Computational Molecular Biology, Lesk, A.M., ed., Oxford University Press, New York (1988); Biocomputing: Informatics and Genome Projects, Smith, D.W., ed., Academic Press, New York, 1993; Computer Analysis of Sequence Data, Part I, Griffin, A.M. and Griffin, H.G., eds., Humana Press, New Jersey (1994); Sequence Analysis in Molecular Biology, von Heinje, G., Academic Press (1987); Sequence Analysis Primer, Gribskov, M. and Devereux, J., eds., Stockton Press, New York (1991); and Carillo, H., and Lipman, D., SIAM J Applied Math, 48:1073 (1988). Methods to determine identity are designed to give the largest match between the sequences tested. Moreover, methods to determine identity are codified in publicly available programs. Computer programs which can be used to determine identity between two sequences include, but are not limited to, GCG (Devereux, J., et al., Nucleic Acids Research 12(1):387 (1984); suite of five BLAST programs, three designed for nucleotide sequences queries (BLASTN, BLASTX, and TBLASTX) and two designed for protein sequence queries (BLASTP and TBLASTN) (Coulson, Trends in Biotechnology, 12: 76-80 (1994); Birren, et al., Genome Analysis, 1: 543-559 (1997)). The BLAST X program is publicly available from NCBI and other sources (BLAST Manual, Altschul, S., et al., NCBI NLM NIH, Bethesda, MD 20894; Altschul, S., et al., J Mol. Biol., 215:403-410 (1990)). The well known Smith Waterman algorithm can also be used to determine identity.

Parameters for polypeptide sequence comparison typically include the following:
Algorithm: Needleman and Wunsch, J Mol. Biol. 45:443-453 (1970)
Comparison matrix: BLOSSUM62 from Hentikoff and Hentikoff, Proc. Natl. Acad Sci USA 89:10915-10919 (1992)
Gap Penalty: 12
Gap Length Penalty: 4

A program which can be used with these parameters is publicly available as the "gap" program from Genetics Computer Group, Madison Wisconsin. The above parameters along with no penalty for end gap are the default parameters for peptide comparisons.

Parameters for polynucleotide sequence comparison include the following:
Algorithm: Needleman and Wunsch, J. Mol. Biol. 48:443-453 (1970)
Comparison matrix: matches = +10; mismatches = 0
Gap Penalty: 50
Gap Length Penalty: 3

A program which can be used with these parameters is publicly available as the "gap" program from Genetics Computer Group, Madison Wisconsin. The above parameters are the default parameters for nucleic acid comparisons.

The polynucleotides of the invention can be used, for example, in the transformation of host cells, such as plant host cells, as further discussed herein.

The invention also provides polynucleotides that encode a polypeptide that is a mature protein plus additional amino or carboxyl-terminal amino acids, or amino acids within the mature polypeptide (for example, when the mature form of the protein has more than one polypeptide chain). Such sequences can, for example, play a role in the processing of a protein from a precursor to a mature form, allow protein transport, shorten or lengthen protein half-life, or facilitate manipulation of the protein in assays or production. It is contemplated that cellular enzymes can be used to remove any additional amino acids from the mature protein.

A precursor protein, having the mature form of the polypeptide fused to one or more prosequences may be an inactive form of the polypeptide. The inactive precursors generally are activated when the prosequences are removed. Some or all of the prosequences may be removed prior to activation. Such precursor protein are generally called proproteins.

### Plant Constructs and Methods of Use

Of particular interest is the use of the nucleotide sequences as defined above in recombinant DNA constructs to direct the transcription or transcription and translation (expression) of the tocopherol cyclase sequences of the present invention in a host plant cell. The expression constructs comprise a promoter functional in a host plant cell operably linked to a nucleic acid sequence encoding a tocopherol cyclase of the present invention and a transcriptional termination region functional in a host plant cell.

A first nucleic acid sequence is "operably linked" or "operably associated" with a second nucleic acid sequence when the sequences are so arranged that the first nucleic acid sequence affects the function of the second nucleic-acid sequence. Preferably, the two sequences are part of a single contiguous nucleic acid molecule and more preferably are adjacent. For example, a promoter is operably linked to a gene if the promoter regulates or mediates transcription of the gene in a cell.

Those skilled in the art will recognize that there are a number of promoters which are functional in plant cells, and have been described in the literature. Chloroplast and plastid specific promoters, chloroplast or plastid functional promoters, and chloroplast or plastid operable promoters are also envisioned.

One set of plant functional promoters are constitutive promoters such as the CaMV35S or FMV35S promoters that yield high levels of expression in most plant organs. Enhanced or duplicated versions of the CaMV35S and FMV35S promoters are useful in the practice of this invention (Odell, et al. (1985) Nature 313:810-812; Rogers, U.S. Patent Number 5,378, 619). In addition, it may also be preferred to bring about expression of the tocopherol cyclase gene in specific tissues of the plant, such as leaf, stem, root, tuber, seed, fruit, etc., and the promoter chosen should have the desired tissue and developmental specificity.

Of particular interest is the expression of the nucleic acid sequences of the present invention from transcription initiation regions which are preferentially expressed in a plant seed tissue. Examples of such seed preferential transcription initiation sequences include those sequences derived from sequences encoding plant storage protein genes or from genes involved in fatty acid biosynthesis in oilseeds. Examples of such promoters include the 5' regulatory regions from such genes as napin (Kridl et al., Seed Sci. Res. 1:209:219 (1991)), phaseolin, zein, soybean trypsin inhibitor, ACP, stearoyl-ACP desaturase, soybean α' subunit of β-conglycinin (soy 7s, (Chen et al., Proc. Natl. Acad. Sci., 83:8560-8564 (1986))) and oleosin.

It may be advantageous to direct the localization of proteins conferring tocopherol cyclase to a particular subcellular compartment, for example, to the mitochondrion, endoplasmic reticulum, vacuoles, chloroplast or other plastidic compartment. For example, where the genes of interest of the present invention will be targeted to plastids, such as chloroplasts, for expression, the constructs will also employ the use of sequences to direct the gene to the plastid. Such sequences are referred to herein as chloroplast transit peptides (CTP) or plastid transit peptides (PTP). In this manner, where the gene of interest is not directly inserted into the plastid, the expression construct will additionally contain a gene encoding a transit peptide to direct the gene of interest to the plastid. The chloroplast transit peptides may be derived from the gene of interest, or may be derived from a heterologous sequence having a CTP. Such transit peptides are known in the art. See, for example, Von Heijne et al. (1991) Plant Mol. Biol. Rep. 9:104-126; Clark et al. (1989) J. Biol. Chem. 264:17544-17550; della-Cioppa et al. (1987) Plant Physiol. 84:965-968; Romer et al. (1993) Biochem. Biophys. Res Commun. 196:1414-1421; and, Shah et al. (1986) Science 233:478-481.

Depending upon the intended use, the constructs may contain the nucleic acid sequence which encodes the entire tocopherol cyclase protein, or a portion thereof. For example, where antisense inhibition of a given prenyltransferase or tocopherol cyclase protein is desired, the entire tocopherol cyclase sequence is not required. Furthermore, where tocopherol cyclase sequences used in constructs are intended for use as probes, it may be advantageous to prepare constructs containing only a particular portion of a tocopherol cyclase encoding sequence, for example a sequence which is discovered to encode a highly conserved tocopherol cyclase region.

The skilled artisan will recognize that there are various methods for the inhibition of expression of endogenous sequences in a host cell. Such methods include, but are not limited to, antisense suppression (Smith, et al. (1988) Nature 334:724-726), co-suppression (Napoli, et al. (1989) Plant Cell 2:279-289), ribozymes (PCT Publication WO 97/10328), and combinations of sense and antisense Waterhouse, et al. (1998) Proc. Natl. Acad. Sci. USA 95:13959-13964. Methods for the suppression of endogenous sequences in a host cell typically employ the transcription or transcription and translation of at least a portion of the sequence to be suppressed. Such sequences may be homologous to coding as well as non-coding regions of the endogenous sequence.

Regulatory transcript termination regions may be provided in plant expression constructs of this invention as well. Transcript termination regions may be provided by the DNA sequence encoding the tocopherol cyclase or a convenient transcription termination region derived from a different gene source, for example, the transcript termination region which is naturally associated with the transcript initiation region. The skilled artisan will recognize that any convenient transcript termination region which is capable of terminating transcription in a plant cell may be employed in the constructs of the present invention.

Alternatively, constructs may be prepared to direct the expression of the tocopherol cyclase sequences directly from the host plant cell plastid. Such constructs and methods are known in the art and are generally described, for example, in Svab, et al. (1990) Proc. Natl. Acad Sci. USA 87:8526-8530 and Svab and Maliga (1993) Proc. Natl. Acad. Sci. USA 90:913-917 and in U.S. Patent Number 5,693,507.

The tocopherol cyclase constructs of the present invention can be used in transformation methods with additional constructs providing for the expression of other nucleic acid sequences encoding proteins involved in the production of tocopherols, or tocopherol precursors such as homogentisic acid and/or phytylpyrophosphate. Nucleic acid sequences encoding proteins involved in the production of homogentisic acid are known in the art, and include but not are limited to, 4-hydroxyphenylpyruvate dioxygenase (HPPD, EC 1.13.11.27) described for example, by Garcia, et al. ((1999) Plant Physiol. 119(4):1507-1516), mono or bifunctional tyrA (described for example by Xia, et al. (1992) J. Gen Microbiol. 138:1309-1316, and Hudson, et al. (1984) J. Mol. Biol. 180:1023-1051), Oxygenase, 4-hydroxyphenylpyruvate di- (9CI), 4-Hydroxyphenylpyruvate dioxygenase; p-Hydroxyphenylpyruvate dioxygenase; p-Hydroxyphenylpyruvate hydroxylase; p-Hydroxyphenylpyruvate oxidase; p-Hydroxyphenylpyruvic acid hydroxylase; p-Hydroxyphenylpyruvic hydroxylase; p-Hydroxyphenylpyruvic oxidase), 4-hydroxyphenylacetate, NAD(P)H:oxygen oxidoreductase (1-hydroxylating); 4-hydroxyphenylacetate 1-monooxygenase, and the like. In addition, constructs for the expression of nucleic acid sequences encoding proteins involved in the production of phytylpyrophosphate can also be employed with the tocopherol cyclase constructs of the present invention. Nucleic acid sequences encoding proteins involved in the production of phytylpyrophosphate are known in the art, and include, but are not limited to geranylgeranylpyrophosphate synthase (GGPPS), geranylgeranylpyrophosphate reductase (GGH), 1-deox-yxylulose-5-phosphate synthase, 1- deoxy-D-xylolose-5-phosphate reductoisomerase, 4-diphosphocytidyl-2-C-methylerythritol synthase, isopentyl pyrophosphate isomerase.

The tocopherol cyclase sequences of the present invention find use in the preparation of transformation constructs having a second expression cassette for the expression of additional sequences involved in tocopherol biosynthesis. Additional tocopherol biosynthesis sequences of interest in the present invention include, but are not limited to gamma-tocpherol methyltransferase (Shintani, et al. (1998) Science 282(5396):2098-2100), tocopherol cyclase, and tocopherol methyltransferase.

A plant cell, tissue, organ, or plant into which the recombinant DNA constructs containing the expression constructs have been introduced.is considered transformed, transfected, or transgenic. A transgenic or transformed cell or plant also includes progeny of the cell or plant and progeny produced from a breeding program employing such a transgenic plant as a parent in a cross and exhibiting an altered phenotype resulting from the presence of a tocopherol cyclase nucleic acid sequence.

Plant expression or transcription constructs having a tocopherol cyclase as the DNA sequence of interest for increased or decreased expression thereof may be employed with a wide variety of plant life, particularly, plant life involved in the production of vegetable oils for edible and industrial uses. Particularly preferred plants for use in the methods of the present invention include, but are not limited to: *Acacia,* alfalfa, aneth, apple, apricot, artichoke, arugula, asparagus, avocado, banana, barley, beans, beet, blackberry, blueberry, broccoli, brussels sprouts, cabbage, canola, cantaloupe, carrot, cassava, cauliflower, celery, cherry, chicory, cilantro, citrus, clementines, coffee, corn, cotton, cucumber, Douglas fir, eggplant, endive, escarole, eucalyptus, fennel, figs, garlic, gourd, grape, grapefruit, honey dew, jicama, kiwifruit, lettuce, leeks, lemon, lime, Loblolly pine, mango, melon, mushroom, nectarine, nut, oat, oil palm, oil seed rape, okra, onion, orange, an ornamental plant, papaya, parsley, pea, peach, peanut, pear, pepper, persimmon, pine, pineapple, plantain, plum, pomegranate, poplar, potato, pumpkin, quince, radiata pine, radicchio, radish, raspberry, rice, rye, sorghum, Southern pine, soybean, spinach, squash, strawberry, sugarbeet, sugarcane, sunflower, sweet potato, sweetgum, tangerine, tea, tobacco, tomato, triticale, turf, turnip, a vine, watermelon, wheat, yams, and zucchini.

Most especially preferred are temperate oilseed crops. Temperate oilseed crops of interest include, but are not limited to, rapeseed (Canola and High Erucic Acid varieties), sunflower, safflower, cotton, soybean, peanut, coconut and oil palms, and corn. Depending on the method for introducing the recombinant constructs into the host cell, other DNA sequences may be required. Importantly, this invention is applicable to dicotyledyons and monocotyledons species alike and will be readily applicable to new and/or improved transformation and regulation techniques.

Of particular interest, is the use tocopherol cyclase constructs in plants to produce plants or plant parts, including, but not limited to leaves, stems, roots, reproductive, and seed, with a modified content of tocopherols in plant parts having transformed plant cells.

For immunological screening, antibodies to the protein can be prepared by injecting rabbits or mice with the purified protein or portion thereof, such methods of preparing antibodies being well known to those in the art. Either monoclonal or polyclonal antibodies can be produced, although typically polyclonal antibodies are more useful for gene isolation. Western analysis may be conducted to determine that a related protein is present in a crude extract of the desired plant species, as determined by cross-reaction with the antibodies to the encoded proteins. When cross-reactivity is observed, genes encoding the related proteins are isolated by screening expression libraries representing the desired plant species. Expression libraries can be constructed in a variety of commercially available vectors, including lambda gt11, as described in Sambrook, et al. (Molecular Cloning: A Laboratory Manual, Second Edition (1989) Cold Spring Harbor Laboratory, Cold Spring Harbor, New York).

To confirm the activity and specificity of the proteins encoded by the identified nucleic acid sequences as tocopherol cyclase enzymes, *in vitro* assays are performed in insect cell cultures using baculovirus expression systems. Such baculovirus expression systems are known in the art and are described by Lee, et al. U.S. Patent Number 5,348,886, the entirety of which is herein incorporated by reference.

In addition, other expression constructs may be prepared to assay for protein activity utilizing different expression systems. Such expression constructs are transformed into yeast or prokaryotic host and assayed for tocopherol cyclase activity. Such expression systems are known in the art and are readily available through commercial sources.

In addition to the sequences described in the present invention, DNA coding sequences useful in the present invention can be derived from algae, fungi, bacteria, mammalian sources, plants, etc. Homology searches in existing databases using signature sequences corresponding to conserved nucleotide and amino acid sequences tocopherol cyclase can be employed to isolate equivalent, related genes from other sources such as plants and microorganisms. Searches in EST databases can also be employed. Furthermore, the use of DNA sequences encoding enzymes functionally enzymatically equivalent to those disclosed herein, wherein such DNA sequences are degenerate equivalents of the nucleic acid sequences disclosed herein in accordance with the degeneracy of the genetic code, is also encompassed by the present invention. Demonstration of the functionality of coding sequences identified by any of these methods can be carried out by complementation of mutants of appropriate organisms, such as *Synechocystis, Shewanella, yeast, Pseudomonas, Rhodobacteria,* etc., that lack specific biochemical reactions, or that have been mutated. The sequences of the DNA coding regions can be optimized by gene resynthesis, based on codon usage, for maximum expression in particular hosts.

For the alteration of tocopherol production in a host cell, a second expression construct can be used in accordance with the present invention. For example, the prenyltransferase or tocopherol cyclase expression construct can be introduced into a host cell in conjunction with a second expression construct having a nucleotide sequence for a protein involved in tocopherol biosynthesis.

The method of transformation in obtaining such transgenic plants is not critical to the instant invention, and various methods of plant transformation are currently available. Furthermore, as newer methods become available to transform crops, they may also be directly applied hereunder. For example, many plant species naturally susceptible to *Agrobacterium* infection may be successfully transformed via tripartite or binary vector methods of *Agrobacterium* mediated transformation. In many instances, it will be desirable to have the construct bordered on one or both sides by T-DNA, particularly having the left and right borders, more particularly the right border. This is particularly useful when the construct uses *A. tumefaciens* or *A. rhizogenes* as a mode for transformation, although the T-DNA borders may find use with other modes of transformation. In addition, techniques of microinjection, DNA particle bombardment, and electroporation have been developed which allow for the transformation of various monocot and dicot plant species.

Normally, included with the DNA construct will be a structural gene having the necessary regulatory regions for expression in a host and providing for selection of transformant cells. The gene may provide for resistance to a cytotoxic agent, e.g. antibiotic, heavy metal, toxin, etc., complementation providing prototrophy to an auxotrophic host, viral immunity or the like. Depending upon the number of different host species the expression construct or components thereof are introduced, one or more markers may be employed, where different conditions for selection are used for the different hosts.

Where *Agrobacterium* is used for plant cell transformation, a vector may be used which may be introduced into the *Agrobacterium* host for homologous recombination with T-DNA or the Ti- or Ri-plasmid present in the *Agrobacterium* host. The Ti- or Ri-plasmid containing the T-DNA for recombination may be armed (capable of causing gall formation) or disarmed (incapable of causing gall formation), the latter being permissible, so long as the *vir* genes are present in the transformed *Agrobacterium* host. The armed plasmid can give a mixture of normal plant cells and gall.

In some instances where *Agrobacterium* is used as the vehicle for transforming host plant cells, the expression or transcription construct bordered by the T-DNA border region(s) will be inserted into a broad host range vector capable of replication in *E. coli* and *Agrobacterium,* there being broad host range vectors described in the literature. Commonly used is pRK2 or derivatives thereof. See, for example, Ditta, et al., (Proc. Nat. Acad. Sci., U.SA. (1980) 77:7347-7351) and EPA 0 120 515, which are incorporated herein by reference. Alternatively, one may insert the sequences to be expressed in plant cells into a vector containing separate replication sequences, one of which stabilizes the vector in *E. coli,* and the other in *Agrobacterium.* See, for example, McBride, et al. (Plant Mol. BioL (1990) 14:269-276), wherein the pRiHRI (Jouanin, et al., Mol. Gen. Genet. (1985) 201:370-374) origin of replication is utilized and provides for added stability of the plant expression vectors in host *Agrobacterium* cells.

Included with the expression construct and the T-DNA will be one or more markers, which allow for selection of transformed Agrobacterium and transformed plant cells. A number of markers have been developed for use with plant cells, such as resistance to chloramphenicol, kanamycin, the aminoglycoside G418, hygromycin, or the like. The particular marker employed is not essential to this invention, one or another marker being preferred depending on the particular host and the manner of construction.

For transformation of plant cells using *Agrobacterium,* explants may be combined and incubated with the transformed *Agrobacterium* for sufficient time for transformation, the bacteria killed, and the plant cells cultured in an appropriate selective medium. Once callus forms, shoot formation can be encouraged by employing the appropriate plant hormones in accordance with known methods and the shoots transferred to rooting medium for regeneration of plants. The plants may then be grown to seed and the seed used to establish repetitive generations and for isolation of vegetable oils.

There are several possible ways to obtain the plant cells of this invention which contain multiple expression constructs. Any means for producing a plant comprising a construct having a DNA sequence encoding the expression construct of the present invention, and at least one other construct having another DNA sequence encoding an enzyme are encompassed by the present invention. For example, the expression construct can be used to transform a plant at the same time as the second construct either by inclusion of both expression constructs in a single transformation vector or by using separate vectors, each of which express desired genes. The second construct can be introduced into a plant which has already been transformed with the prenyltransferase or tocopherol cyclase expression construct, or alternatively, transformed plants, one expressing the prenyltransferase or tocopherol cyclase construct and one expressing the second construct, can be crossed to bring the constructs together in the same plant.

Transgenic plants of the present invention may be produced from tissue culture, and subsequent generations grown from seed. Alternatively, transgenic plants may be grown using apomixis. Apomixis is a genetically controlled method of reproduction in plants where the embryo is formed without union of an egg and a sperm. There are three basic types of apomictic reproduction: 1) apospory where the embryo develops from a chromosomally unreduced egg in an embryo sac derived from the nucleus, 2) diplospory where the embryo develops from an unreduced egg in an embryo sac derived from the megaspore mother cell, and 3) adventitious embryony where the embryo develops directly from a somatic cell. In most forms of apomixis, pseudogamy or fertilization of the polar nuclei to produce endosperm is necessary for seed viability. In apospory, a nurse cultivar can be used as a pollen source for endosperm formation in seeds. The nurse cultivar does not affect the genetics of the aposporous apomictic cultivar since the unreduced egg of the cultivar develops parthenogenetically, but makes possible endosperm production. Apomixis is economically important, especially in transgenic plants, because it causes any genotype, no matter how heterozygous, to breed true. Thus, with apomictic reproduction, heterozygous transgenic plants can maintain their genetic fidelity throughout repeated life cycles. Methods for the production of apomictic plants are known in the art. See, U.S. Patent No.5,811,636.

Host cells of the present invention preferably include monocotyledenous and dicotyledenous plant cells.

In general, the skilled artisan is familiar with the standard resource materials which describe specific conditions and procedures for the construction, manipulation and isolation of macromolecules (e.g., DNA molecules, plasmids, etc.), generation of recombinant organisms and the screening and isolating of clones, (see for example, Sambrook et al., Molecular Cloning: A Laboratory Manual, Cold Spring Harbor Press (1989); Maliga et al., Methods in Plant Molecular Biology, Cold Spring Harbor Press (1995), Birren et al., Genome Analysis: Analyzing DNA, 1, Cold Spring Harbor, New York).

The invention also includes plants and plant parts, such as seed, oil and meal derived from seed, and feed and food products processed from plants, which are enriched in tocopherols. Of particular interest is seed oil obtained from transgenic plants where the tocopherol level has been increased as compared to seed oil of a non-transgenic plant.

The harvested plant material may be subjected to additional processing to further enrich the tocopherol content. The skilled artisan will recognize that there are many such processes or methods for refining, bleaching and degumming oil. United States Patent Number 5,932,261, issued August 3, 1999, discloses on such process, for the production of a natural carotene rich refined and deodorised oil by subjecting the oil to a pressure of less than 0.060 mbar and to a temperature of less than 200 degree. C. Oil distilled by this process has reduced free fatty acids, yielding a refined, deodorised oil where Vitamin E contained in the feed oil is substantially retained in the processed oil. The teachings of this patent are incorporated herein by reference.

The invention now being generally described, it will be more readily understood by reference to the following examples which are included for purposes of illustration only and are not intended to limit the present invention.

### EXAMPLES

### Example 1: Identification of Prenyltransferase or tocopherol cyclase Sequences

PSI-BLAST (Altschul, et al. (1997) Nuc Acid Res 25:3389-3402) profiles were generated for both the straight chain and aromatic classes of prenyltransferases. To generate the straight chain profile, a prenyl- transferase from *Porphyra purpurea* (Genbank accession 1709766) was used as a query against the NCBI non-redundant protein database. The *E. coli* enzyme involved in the formation of ubiquinone, ubiA (genbank accession 1790473) was used as a starting sequence to generate the aromatic prenyltransferase profile. These profiles were used to search public and proprietary DNA and protein data bases. In *Arabidopsis* six putative prenyltransferases of the straight-chain class were identified, ATPT1, (SEQ ID NO:9), ATPT7 (SEQ ID NO: 10), ATPT8 (SEQ ID NO:11), ATPT9 (SEQ ID NO:13), ATPT10 (SEQ ID NO:14), and ATPT11 (SEQ ID NO:15), and six were identified of the aromatic class, ATPT2 (SEQ ID NO:1), ATPT3 (SEQ ID NO:3), ATPT4 (SEQ ID NO:5), ATPT5 (SEQ ID NO:7), ATPT6 (SEQ ID NO:8), and ATPT12 (SEQ ID NO:16). Additional prenyltransferase sequences from other plants related to the aromatic class of prenyltransferases, such as soy (SEQ ID NOs: 19-23, the deduced amino acid sequence of SEQ ID NO:23 is provided in SEQ ID NO:24) and maize (SEQ ID NOs:25-29, and 31) are also identified. The deduced amino acid sequence of ZMPT5 (SEQ ID NO:29) is provided in SEQ ID NO:30.

Searches are performed on a Silicon Graphics Unix computer using additional Bioaccellerator hardware and GenWeb software supplied by Compugen Ltd. This software and hardware enables the use of the Smith-Waterman algorithm in searching DNA and protein databases using profiles as queries. The program used to query protein databases is profilesearch. This is a search where the query is not a single sequence but a profile based on a multiple alignment of amino acid or nucleic acid sequences. The profile is used to query a sequence data set, i.e., a sequence database. The profile contains all the pertinent information for scoring each position in a sequence, in effect replacing the "scoring matrix" used for the standard query searches. The program used to query nucleotide databases with a protein profile is tprofilesearch. Tprofilesearch searches nucleic acid databases using an amino acid profile query. As the search is running, sequences in the database are translated to amino acid sequences in six reading frames. The output file for tprofilesearch is identical to the output file for profilesearch except for an additional column that indicates the frame in which the best alignment occurred.

The Smith-Waterman algorithm, (Smith and Waterman (1981) *supra*), is used to search for similarities between one sequence from the query and a group of sequences contained in the database. E score values as well as other sequence information, such as conserved peptide sequences are used to identify related sequences.

To obtain the entire coding region corresponding to the *Arabidopsis* prenyltransferase sequences, synthetic oligo-nucleotide primers are designed to amplify the 5' and 3' ends of partial cDNA clones containing prenyltransferase sequences. Primers are designed according to the respective *Arabidopsis* prenyltransferase sequences and used in Rapid Amplification of cDNA Ends (RACE) reactions (Frohman et al. (1988) Proc. Natl. Acad. Sci. USA 85:8998-9002) using the Marathon cDNA amplification kit (Clontech Laboratories Inc, Palo Alto, CA).

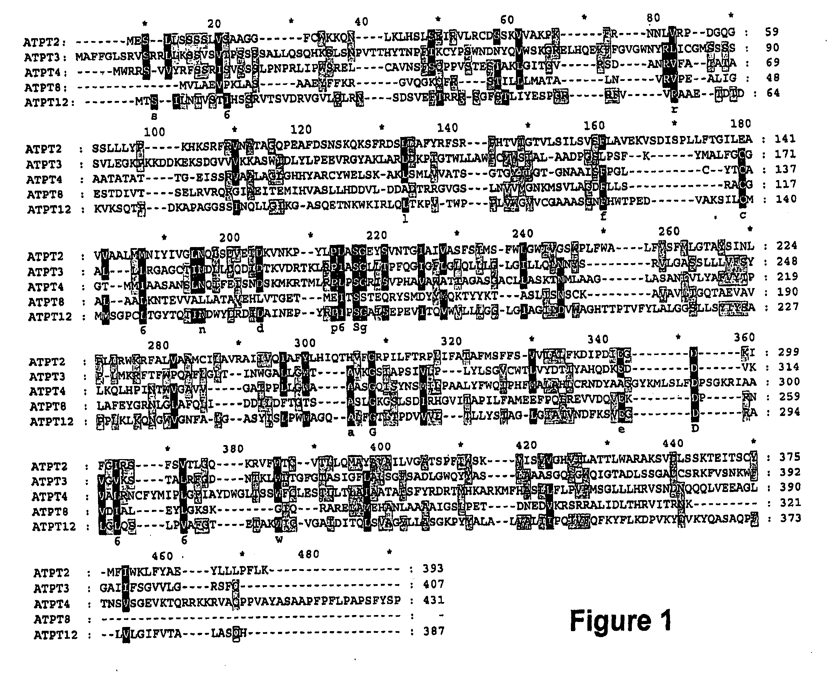

Amino acid sequence alignments between ATPT2 (SEQ ID NO:2), ATPT3 (SEQ ID NO:4), ATPT4 (SEQ ID NO:6), ATPT8 (SEQ ID NO:12), and ATPT12 (SEQ ID NO:17) are performed using ClustalW (Figure 1), and the percent identity and similarities are provided in Table 1 below.

**Table 1:**

| | | ATPT2 | ATPT3 | ATPT4 | ATPT8 | ATPT12 |
|---|---|---|---|---|---|---|
| ATPT2 | % Identity | | 12 | 13 | 11 | 15 |
| | % similar | | 25 | 25 | 22 | 32 |
| | % Gap | | 17 | 20 | 20 | 9 |
| ATPT3 | % Identity | | | 12 | 6 | 22 |
| % similar | | | | 29 | 16 | 38 |
| % Gap | | | | 20 | 24 | 14 |
| ATPT4 | % Identity | | | | 9 | 14 |
| | % similar | | | | 18 | 29 |
| | % Gap | | | | 26 | 19 |
| ATPT8 | % Identity | | | | | 7 |
| | % similar | | | | | 19 |
| | % Gap | | | | | 20 |
| ATPT12 | % Identity | | | | | |
| | % similar | | | | | |
| | % Gap | | | | | |

### Comparative Example 2: Preparation of Prenyl Transferase Expression Constructs

A plasmid containing the napin cassette derived from pCGN3223 (described in USPN 5,639,790, the entirety of which is incorporated herein by reference) was modified to make it more useful for cloning large DNA fragments containing multiple restriction sites, and to allow the cloning of multiple napin fusion genes into plant binary transformation vectors. An adapter comprised of the self annealed oligonucleotide of sequence CGCGATTTAAATGGCGCGCCCTGCAGGCGGCCGCCTGCAGGGCGCGCCAT TTAAAT (SEQ ID NO:40) was ligated into the cloning vector pBC SK+ (Stratagene) after digestion with the restriction endonuclease BssHII to construct vector pCGN7765. Plamids pCGN3223 and pCGN7765 were digested with NotI and ligated together. The resultant vector, pCGN7770, contains the pCGN7765 backbone with the napin seed specific expression cassette from pCGN3223.

The cloning cassette, pCGN7787, essentially the same regulatory elements as pCGN7770, with the exception of the napin regulatory regions of pCGN7770 have been replaced with the double CAMV 35S promoter and the tml polyadenylation and transcriptional termination region.

A binary vector for plant transformation, pCGN5139, was constructed from pCGN1558 (McBride and Summerfelt, (1990) Plant Molecular Biology, 14:269-276). The polylinker of pCGN1558 was replaced as a HindIII/Asp718 fragment with a polylinker containing unique restriction endonuclease sites, AscI, PacI, XbaI, SwaI, BamHI, and NotI. The Asp718 and HindIII restriction endonuclease sites are retained in pCGN5139.

A series of turbo binary vectors are constructed to allow for the rapid cloning of DNA sequences into binary vectors containing transcriptional initiation regions (promoters) and transcriptional termination regions.

The plasmid pCGN8618 was constructed by ligating oligonucleotides 5'-TCGAGGATCCGCGGCCGCAAGCTTCCTGCAGG-3' (SEQ ID NO:41) and 5'-TCGACCTGCAGGAAGCTTGCGGCCGCGGATCC-3' (SEQ ID NO:42) into SalI/XhoI-digested pCGN7770. A fragment containing the napin promoter, polylinker and napin 3' region was excised from pCGN8618 by digestion with Asp718I; the fragment was blunt-ended by filling in the 5' overhangs with Klenow fragment then ligated into pCGN5139 that had been digested with Asp718I and HindIII and blunt-ended by filling in the 5' overhangs with Klenow fragment. A plasmid containing the insert oriented so that the napin promoter was closest to the blunted Asp718I site of pCGN5139 and the napin 3' was closest to the blunted HindIII site was subjected to sequence analysis to confirm both the insert orientation and the integrity of cloning junctions. The resulting plasmid was designated pCGN8622.

The plasmid pCGN8619 was constructed by ligating oligonucleotides 5'-TCGACCTGCAGGAAGCTTGCGGCCGCGGATCC -3' (SEQ ID NO:43) and 5'-TCGAGGATCCGCGGCCGCAAGCTTCCTGCAGG-3' (SEQ ID NO:44) into SalI/XhoI-digested pCGN7770. A fragment containing the napin promoter, polylinker and napin 3' region was removed from pCGN8619 by digestion with Asp718I; the fragment was blunt-ended by filling in the 5' overhangs with Klenow fragment then ligated into pCGN5139 that had been digested with Asp718I and HindIII and blunt-ended by filling in the 5' overhangs with Klenow fragment. A plasmid containing the insert oriented so that the napin promoter was closest to the blunted Asp718I site of pCGN5139 and the napin 3' was closest to the blunted HindIII site was subjected to sequence analysis to confirm both the insert orientation and the integrity of cloning junctions. The resulting plasmid was designated pCGN8623.

The plasmid pCGN8620 was constructed by ligating oligonucleotides 5'-TCGAGGATCCGCGGCCGCAAGCTTCCTGCAGGAGCT -3' (SEQ ID NO:45) and 5'-CCTGCAGGAAGCTTGCGGCCGCGGATCC-3' (SEQ ID NO:46) into SalI/SacI-digested pCGN7787. A fragment containing the d35S promoter, polylinker and tml 3' region was removed from pCGN8620 by complete digestion with Asp718I and partial digestion with NotI. The fragment was blunt-ended by filling in the 5' overhangs with Klenow fragment then ligated into pCGN5139 that had been digested with Asp71·8I and HindIII and blunt-ended by filling in the 5' overhangs with Klenow fragment. A plasmid containing the insert oriented so that the d35S promoter was closest to the blunted Asp718I site of pCGN5139 and the tml 3' was closest to the blunted HindIII site was subjected to sequence analysis to confirm both the insert orientation and the integrity of cloning junctions. The resulting plasmid was designated pCGN8624.

The plasmid pCGN8621 was constructed by ligating oligonucleotides 5'-TCGACCTGCAGGAAGCTTGCGGCCGCGGATCCAGCT -3' (SEQ ID NO:47) and 5'-GGATCCGCGGCCGCAAGCTTCCTGCAGG-3' (SEQ ID NO:48) into SaII/SacI-digested pCGN7787. A fragment containing the d35S promoter, polylinker and tml 3' region was removed from pCGN8621 by complete digestion with Asp718I and partial digestion with NotI. The fragment was blunt-ended by filling in the 5' overhangs with Klenow fragment then ligated into pCGN5139 that had been digested with Asp7181 and HindIII and blunt-ended by filling in the 5' overhangs with Klenow fragment A plasmid containing the insert oriented so that the d35S promoter was closest to the blunted Asp7181 site of pCGN5139 and the tml 3' was closest to the blunted HindIII site was subjected to sequence analysis to confirm both the insert orientation and the integrity of cloning junctions. The resulting plasmid was designated pCGN8625.

The plasmid construct pCGN8640 is a modification of pCGN8624 described above. A 938bp PstI fragment isolated from transposon Tn7 which encodes bacterial spectinomycin and streptomycin resistance (Fling et al. (1985), Nucleic Acids Research 13(19):7095-7106), a determinant for E. coli and Agrobacterium selection, was blunt ended with Pfu polymerase. The blunt ended fragment was ligated into pCGN8624 that had been digested with Spel and blunt ended with Pfu polymerase. The region containing the PstI fragment was sequenced to confirm both the insert orientation and the integrity of cloning junctions.

The spectinomycin resistance marker was introduced into pCGN8622 and pCGN8623 as follows. A 7.7 Kbp AvrII-SnaBI fragment from pCGN8640 was ligated to a 10.9 Kbp AvrII-SnaBI fragment from pCGN8623 or pCGN8622, described above. The resulting plasmids were pCGN8641 and pCGN8643, respectively.

The plasmid pCGN8644 was constructed by ligating oligonucleotides 5'-GATCACCTGCAGGAAGCTTGCGGCCGCGGATCCAATGCA-3' (SEQ ID NO:49) and 5'- TTGGATCCGCGGCCGCAAGCTTCCTGCAGGT-3' (SEQ ID NO:50) into BamHI-PstI digested pCGN8640.

Synthetic oligonulceotides were designed for use in Polymerase Chain Reactions (PCR) to amplify the coding sequences of ATPT2, ATPT3, ATPT4, ATPT8, and ATPT12 for the preparation of expression constructs and are provided in Table 2 below.

**Table 2:**

| Name | Restriction Site | Sequence | SEQ ID NO: |
|---|---|---|---|
| ATPT2 | 5' NotI | GGATCCGCGGCCGCACAATGGAGTCTCTGCTCTCTAGTTCT | 51 |
| ATPT2 | 3' SseI | GGATCCTGCAGGTCACTTCAAAAAAGGTAACAGCAAGT | 52 |
| ATPT3 | 5' NotI | GGATCCGCGGCCGCACAATGGCGTTTTTTGGGCTCTCCCGTGTTT | 53 |
| ATPT3 | 3' SseI | GGATCCTGCAGGTTATTGAAAACTTCTTCCAAGTACAACT | 54 |
| ATPT4 | 5' NotI | GGATCCGCGGCCGCACAATGTGGCGAAGATCTGTTGTT | 55 |
| ATPT4 | 3' SseI | GGATCCTGCAGGTCATGGAGAGTAGAAGGAAGGAGCT | 56 |
| ATPT8 | 5' NotI | GGATCCGCGGCCGCACAATGGTACTTGCCGAGGTTCCAAAGCTTGCCTCT | 57 |
| ATPT8 | 3' SseI | GGATCCTGCAGGTCACTTGTTTCTGGTGATGACTCTAT | 58 |
| ATPT12 | 5' NotI | GGATCCGCGGCCGCACAATGACTTCGATTCTCAACACT | 59 |
| ATPT12 | 3' SseI | GGATCCTGCAGGTCAGTGTTGCGATGCTAATGCCGT | 60 |

The coding sequences of ATPT2, ATPT3, ATPT4, ATPT8, and ATPT12 were all amplified using the respective PCR primers shown in Table 2 above and cloned into the TopoTA vector (Invitrogen). Constructs containing the respective prenyltransferase sequences were digested with NotI and Sse8387I and cloned into the turbobinary vectors described above.

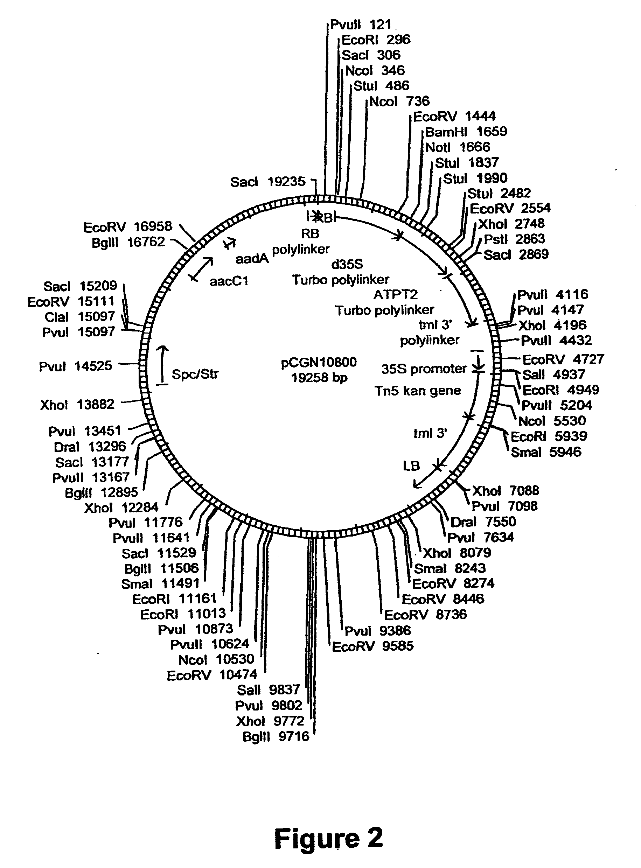

The sequence encoding ATPT2 prenyltransferase was cloned in the sense orientation into pCGN8640 to produce the plant transformation construct pCGN10800 (Figure 2). The ATPT2 sequence is under control of the 35S promoter.

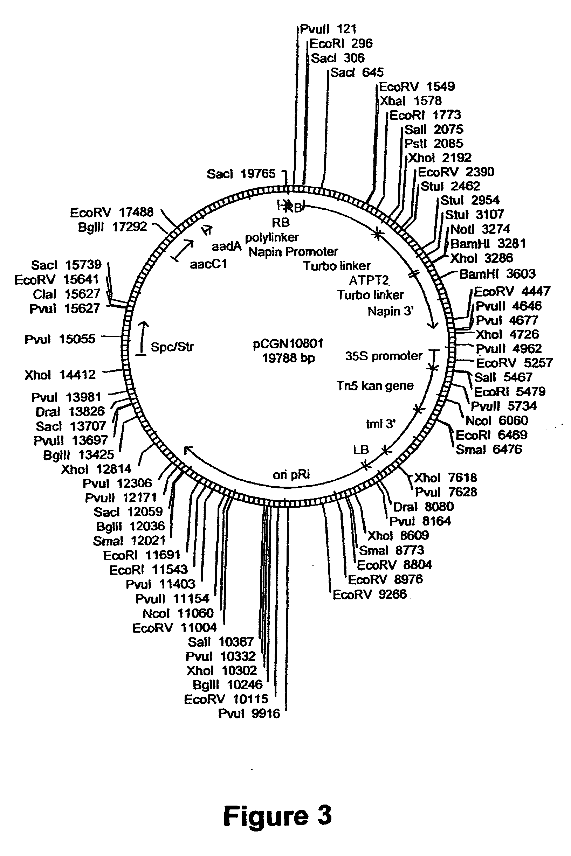

The ATPT2 sequence was also cloned in the antisense orientation into the construct pCGN8641 to create pCGN10801 (Figure 3). This construct provides for the antisense expression of the ATPT2 sequence from the napin promoter.

The ATPT2 coding sequence was also cloned in the sense orientation into the vector pCGN8643 to create the plant transformation construct pCGN10822

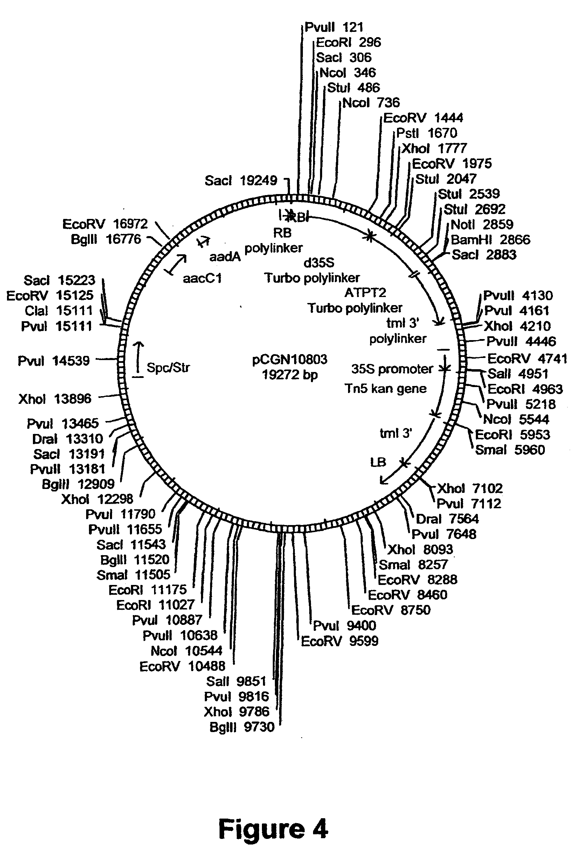

The ATPT2 coding sequence was also cloned in the antisense orientation into the vector pCGN8644 to create the plant transformation construct pCGN10803 (Figure 4).

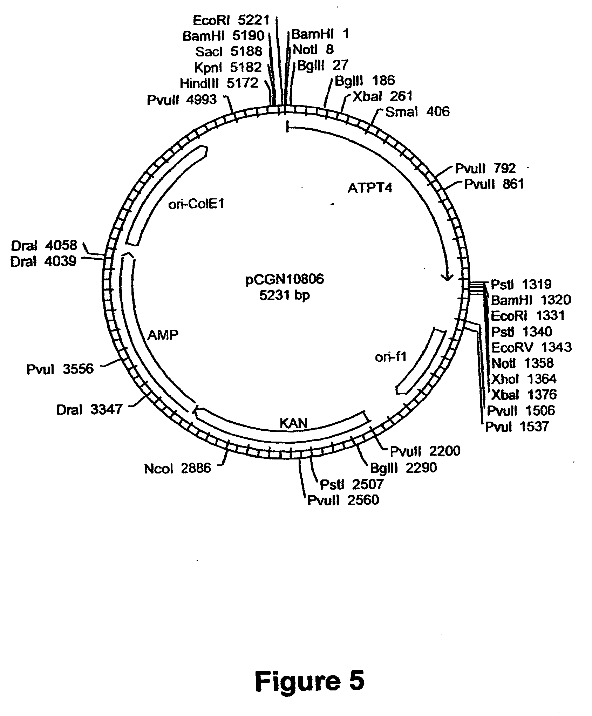

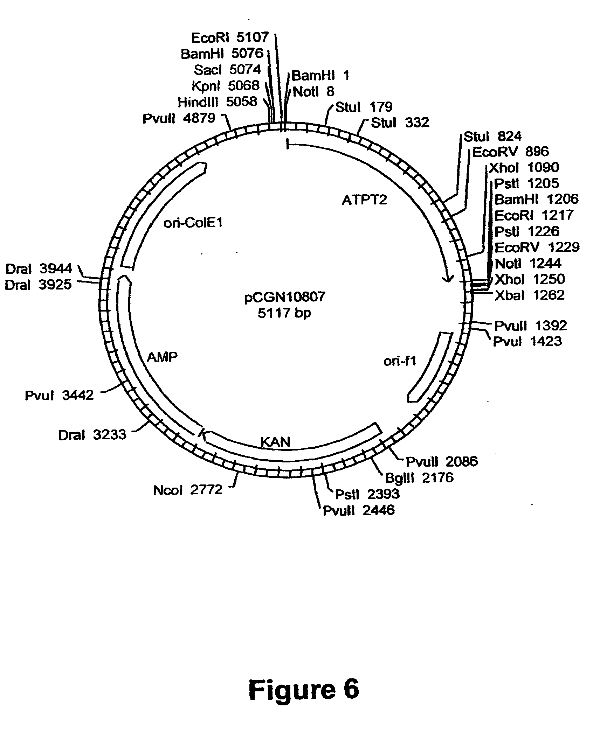

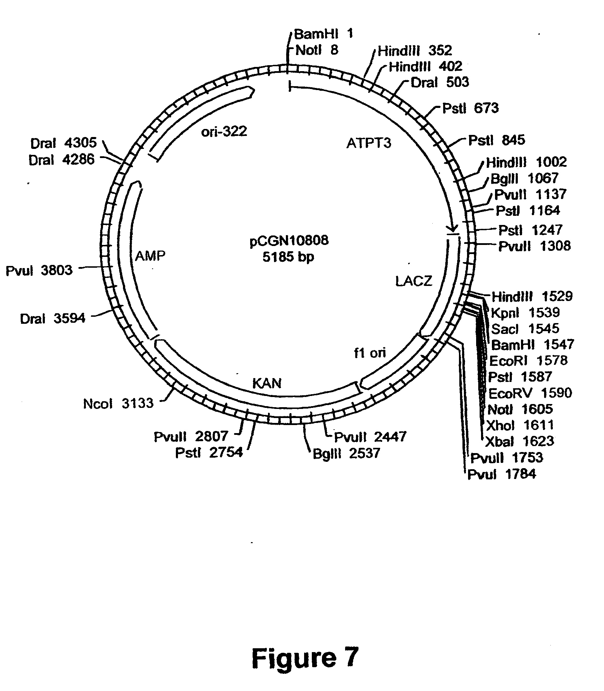

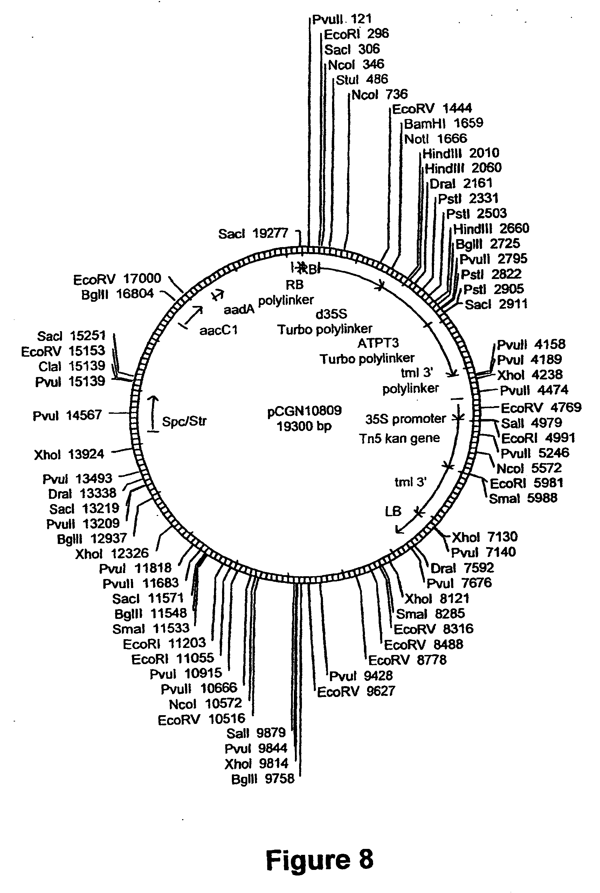

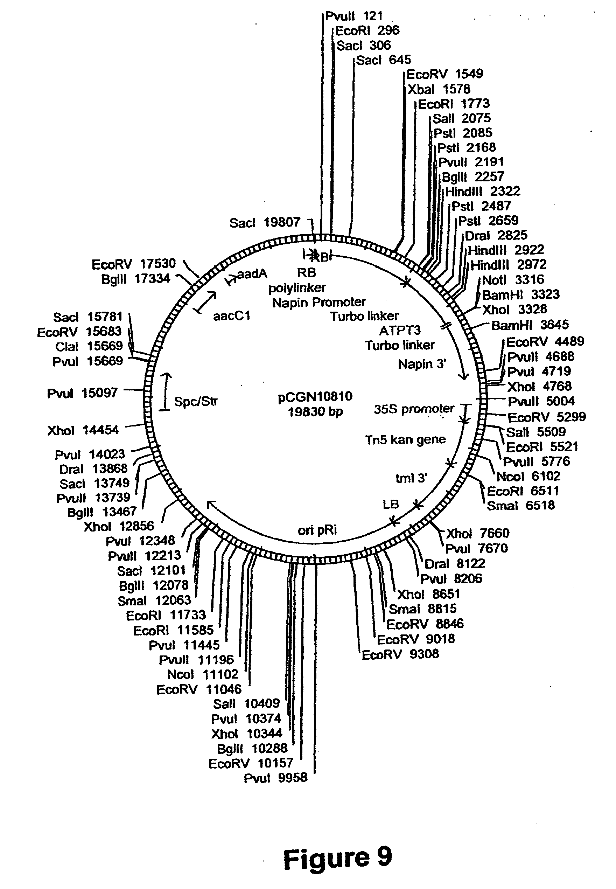

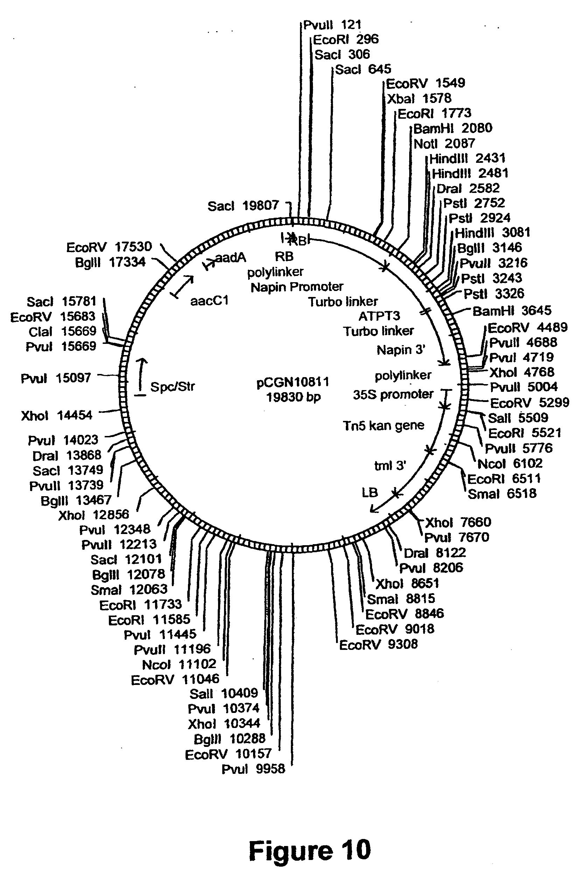

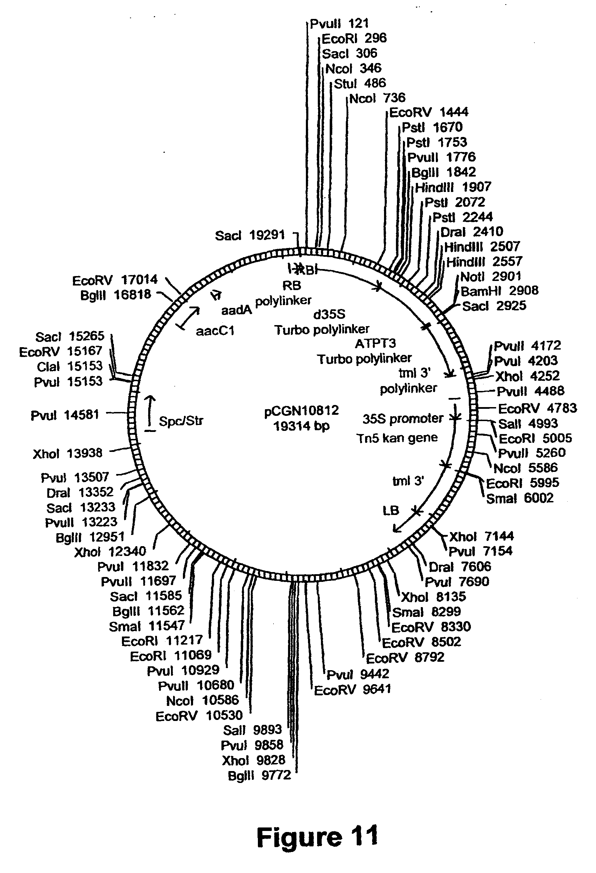

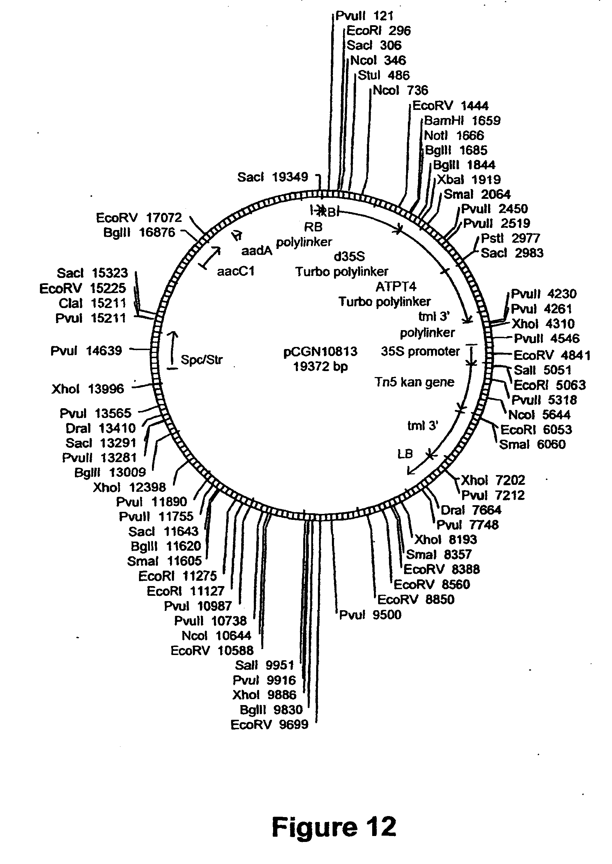

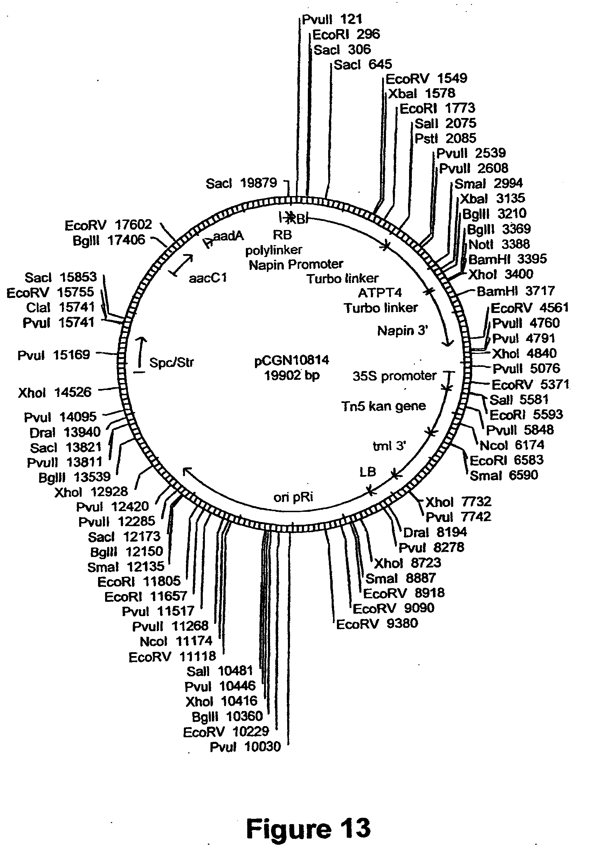

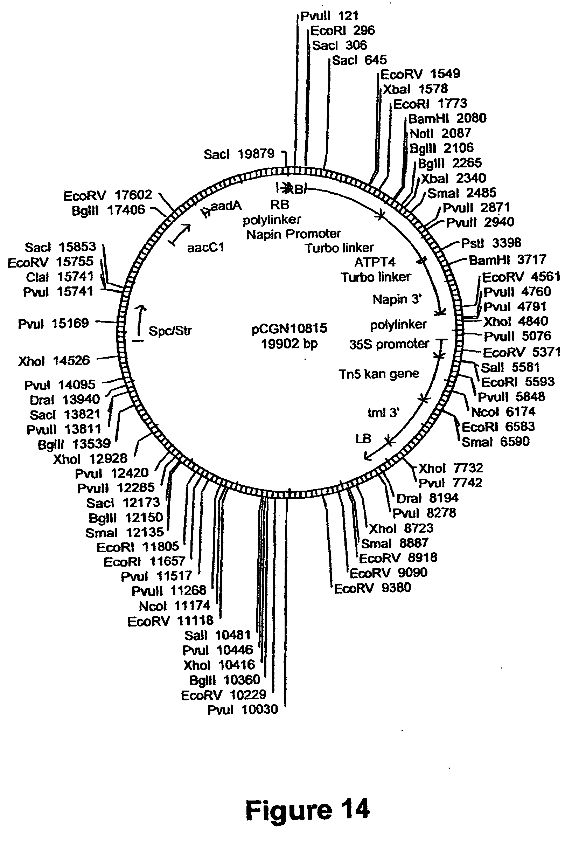

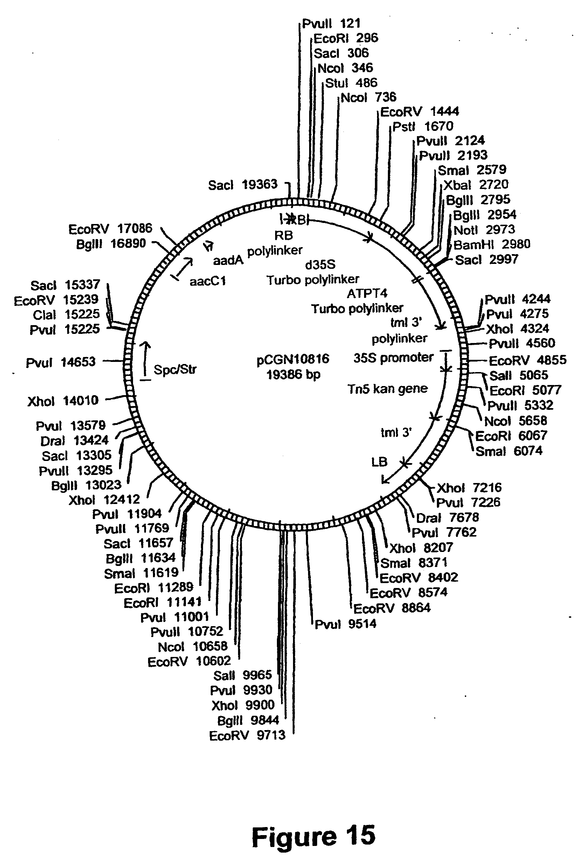

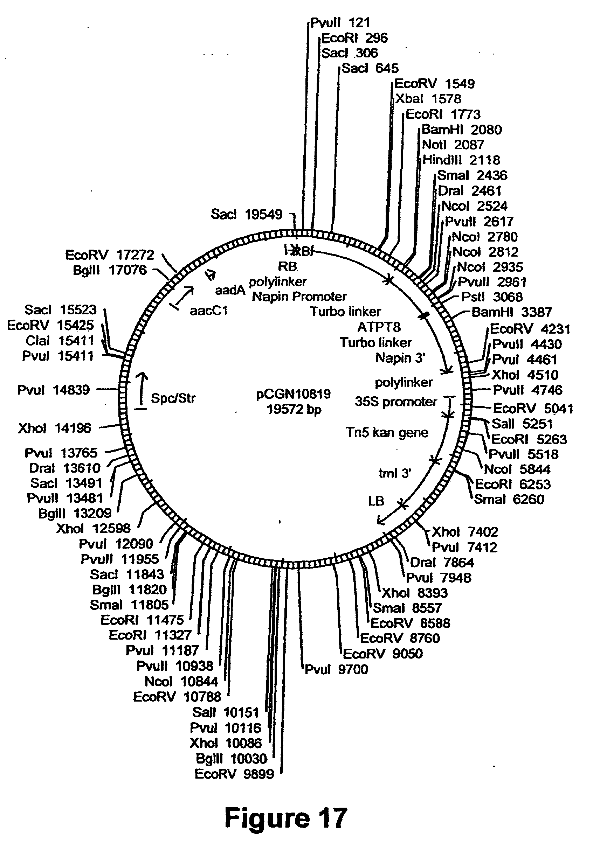

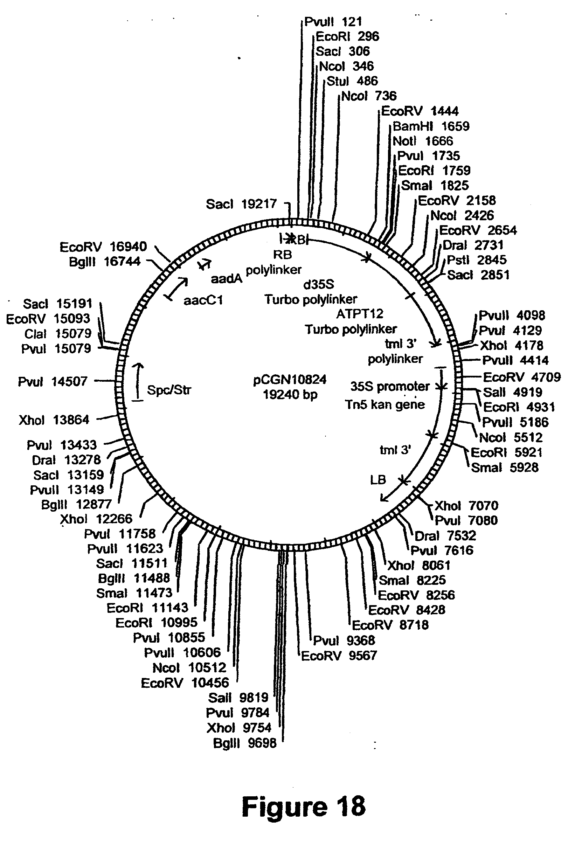

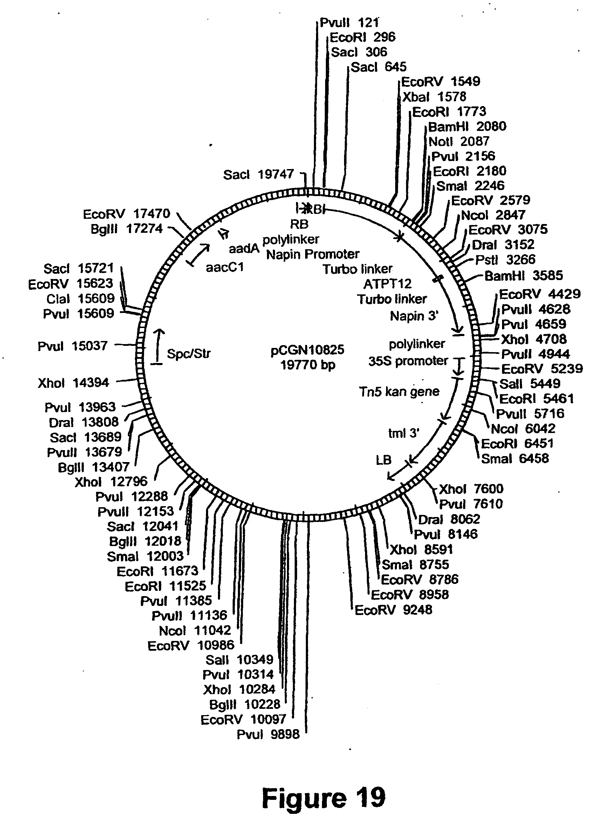

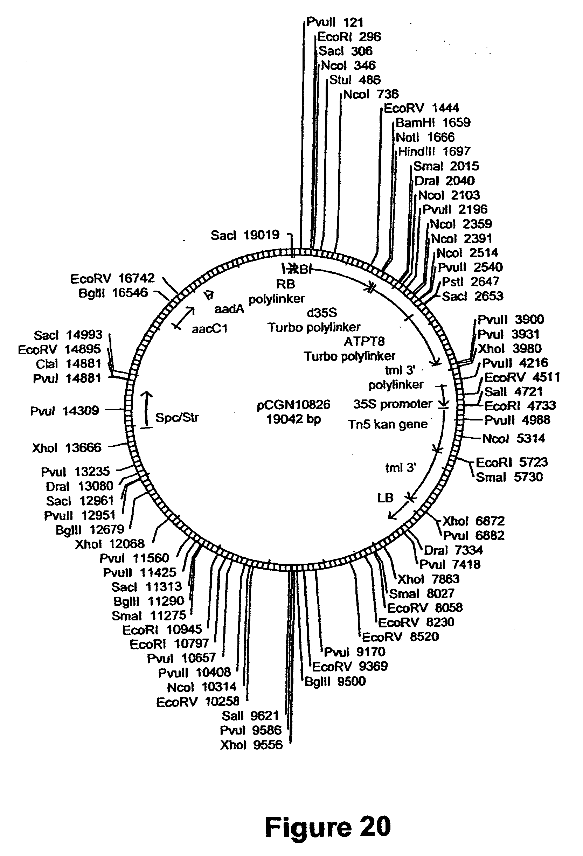

The ATPT4 coding sequence was cloned into the vector pCGN864 to create the plant transformation construct pCGN10806 (Figure 5). The ATPT2 coding sequence was cloned into the vector TopoTA^{™} vector from Invitrogen, to create the plant transformation construct pCGN10807(Figure 6). The ATPT3 coding sequence was cloned into the TopoTA vector to create the plant transformation construct pCGN10808 (Figure 7). The ATPT3 coding sequence was cloned in the sense orientation into the vector pCGN8640 to create the plant transformation construct pCGN10809 (Figure 8). The ATPT3 coding sequence was cloned in the antisense orientation into the vector pCGN8641 to create the plant transformation construct pCGN10810 (Figure 9). The ATPT3 coding sequence was cloned into the vector pCGN8643 to create the plant transformation construct pCGN10811 (Figure 10). The ATPT3 coding sequence was cloned into the vector pCGN8644 to create the plant transformation construct pCGN10812 (Figure 11). The ATPT4 coding sequence was cloned into the vector pCGN8640 to create the plant transformation construct pCGN10813 (Figure 12). The ATPT4 coding sequence was cloned into the vector pCGN8641 to create the plant transformation construct pCGN10814 (Figure 13). The ATPT4 coding sequence was cloned into the vector pCGN8643 to create the plant transformation construct pCGN10815 (Figure 14). The ATPT4 coding sequence was cloned in the antisense orientation into the vector pCGN8644 to create the plant transformation construct pCGN 10816 (Figure 15). The ATPT8 coding sequence was cloned in the sense orientation into the vector pCGN8643 to create the plant transformation construct pCGN10819 (Figure 17). The ATPT12 coding sequence was cloned into the vector pCGN8640 to create the plant transformation construct pCGN10824 (Figure 18). The ATPT12 coding sequence was cloned into the vector pCGN8643 to create the plant transformation construct pCGN10825 (Figure 19). The ATPT8 coding sequence was cloned into the vector pCGN8640 to create the plant transformation construct pCGN10826 (Figure 20).

### Comparative Example 3: Plant Transformation with Prenyl Transferase Constructs

Transgenic *Brassica* plants are obtained by *Agrobacterium*-mediated transformation as described by Radke et al. (Theor. Appl. Genet. (1988) 75:685-694; Plant. Cell Reports (1992) 11:499-505). Transgenic *Arabidopsis thaliana* plants may be obtained by *Agrobacterium*-mediated transformation as described by Valverkens et al., (Proc. Nat. Acad. Sci. (1988) 85:5536-5540), or as described by Bent et al. ((1994), Science 265:1856-1860), or Bechtold et al. ((1993), C.R.Acad.Sci, Life Sciences 316:1194-1199). Other plant species may be similarly transformed using related techniques.

Alternatively, microprojectile bombardment methods, such as described by Klein et al. (Bio/Technology 10:286-291) may also be used to obtain nuclear transformed plants.

### Comparative Example 4: Identification of Additional Prenyltransferases

Additional BLAST searches were performed using the ATPT2 sequence, a sequence in the class of aromatic prenyltransferases. ESTs, and in some case, full-length coding regions, were identified in proprietary DNA libraries.

Soy full-length homologs to ATPT2 were identified by a combination of BLAST (using ATPT2 protein sequence) and 5' RACE. Two homologs resulted (SEQ ID NO:95 and SEQ ID NO:96). Translated amino acid sequences are provided by SEQ ID NO:97 and SEQ ID NO:98.

A rice est ATPT2 homolog is shown in SEQ ID NO:99 (obtained from BLAST using the wheat ATPT2 homolog).

Other homolog sequences were obtained using ATPT2 and PSI-BLAST, including est sequences from wheat (SEQ ID NO: 100), leek (SEQ ID NOs:101 and 102), canola (SEQ ID NO:103), corn (SEQ ID NOs:104, 105 and 106), cotton (SEQ ID NO:107) and tomato (SEQ ID NO:108).

A PSI-Blast profile generated using the *E. coli* ubiA (genbank accession 1790473) sequence was used to analyze the *Synechocystis* genome. This analysis identified 5 open reading frames (ORFs) in the *Synechocystis* genome that were potentially prenyltransferases; slr0926 (annotated as ubiA (4-hydroxybenzoate-octaprenyltransferase, SEQ ID NO:32), sll1899 (annotated as ctaB (cytocrome c oxidase folding protein, SEQ ID NO:33), slr0056 (annotated as g4 (chlorophyll synthase 33 kd subunit, SEQ ID NO:34), slr1518 (annotated as menA (menaquinone biosynthesis protein, SEQ ID NO:35), and slr1736 (annotated as a hypothetical protein of unknown function (SEQ ID NO:36).

### 4A. Synechocystis Knock-outs

To determine the functionality of these ORFs and their involvement, if any, in the biosynthesis of tocopherols, knockouts constructs were made to disrupt the ORF identified in *Synechocystis.*

Synthetic oligos were designed to amplify regions from the 5' (5'-TAATGTGTACATTGTCGGCCTC (17365') (SEQ ID NO:61) and 5'-GCAATGTAACATCAGAGATTTTGAGACACAACGTGGCTTTCCACAATTCC CCGCACCGTC (1736kanpr1)) (SEQ ID NO:62) and 3' (5'-AGGCTAATAAGCACAAATGGGA (17363') (SEQ ID NO:63) and 5'-GGTATGAGTCAGCAACACCTTCTTCACGAGGCAGACCTCAGC GGAATTGGTTTAGGTTATCCC (1736kanpr2)) (SEQ ID NO:64) ends of the slr1736 ORF. The 1736kanpr1 and 1736kanpr2 oligos contained 20 bp of homology to the slr1736 ORF with an additional 40 bp of sequence homology to the ends of the kanamycin resistance cassette. Separate PCR steps were completed with these oligos and the products were gel purified and combined with the kanamycin resistance gene from puc4K (Pharmacia) that had been digested with *Hinc*II and gel purified away from the vector backbone. The combined fragments were allowed to assemble without oligos under the following conditions: 94°C for 1 min, 55°C for 1 min, 72°C for 1 min plus 5 seconds per cycle for 40 cycles using pfu polymerase in 100ul reaction volume (Zhao, H and Arnold (1997) Nucleic Acids Res. 25(6):1307-1308). One microliter or five microliters of this assembly reaction was then amplified using 5' and 3' oligos nested within the ends of the ORF fragment, so that the resulting product contained 100-200 bp of the 5' end of the *Synechocystis* gene to be knocked out, the kanamycin resistance cassette, and 100-200 bp of the 3' end of the gene to be knocked out. This PCR product was then cloned into the vector pGemT easy (Promega) to create the construct pMON21681 and used for *Synechocystis* transformation.

Primers were also synthesized for the preparation of *Synechocystis* knockout constructs for the other sequences using the same method as described above, with the following primers. The ubiA 5' sequence was amplified using the primers 5'-GGATCCATGGTT GCCCAAACCCCATC (SEQ ID NO:65) and 5'-GCAATGTAACATCAGAGA TTTTGAGACACAACG TGGCTTTGGGTAAGCAACAATGACCGGC (SEQ ID NO:66). The 3' region was amplified using the synthetic oligonucleotide primers 5'-GAATTCTCAAAGCCAGCCCAGTAAC (SEQ ID NO:67) and 5'-GGTATGAGTC AGCAACACCTTCTTCACGAGGCAGACCTCAGCGGGTGCGAAAAGGGTTTT CCC (SEQ ID NO:68). The amplification products were combined with the kanamycin resistance gene from puc4K (Pharmacia) that had been digested with *Hinc*II and gel purified away from the vector backbone. The annealed fragment was amplified using 5' and 3' oligos nested within the ends of the ORF fragment (5'-CCAGTGGTTTAGGCTGTGTGGTC (SEQ ID NO:69) and 5'-CTGAGTTGOATGTAITGGATC (SEQ ID NO:70)), so that the resulting product contained 100-200 bp of the 5' end of the *Synechocystis* gene to be knocked out, the kanamycin resistance cassette, and 100-200 bp of the 3' end of the gene to be knocked out. This PCR product was then cloned into the vector pGemT easy (Promega) to create the construct pMON21682 and used for *Synechocystis* transformation.

Primers were also synthesized for the preparation of *Synechocystis* knockout constructs for the other sequences using the same method as described above, with the following primers. The sl11899 5' sequence was amplified using the primers 5'-GGATCCATGGTTACTT CGACAAAAATCC (SEQ ID NO:71) and 5'-GCAATGTAACATCAGAG ATTTTGAGACACAACGTGGCTTTGCTAGGCAACCGCTTAGTAC (SEQ ID NO:72). The 3' region was amplified using the synthetic oligonucleotide primers 5'-GAATTCTTAACCCAACAGTAAAGTTCCC (SEQ ID NO:73) and 5'-GGTATGAGTCAGC AACACCTTCTTCACGAGGCAGACCTCAGCGCCGGCATTGTCTTTTACATG (SEQ ID NO:74). The amplification products were combined with the kanamycin resistance gene from puc4K (Pharmacia) that had been digested with *Hinc*II and gel purified away from the vector backbone. The annealed fragment was amplified using 5' and 3' oligos nested within the ends of the ORF fragment (5'-GGAACCCTTGCAGCCGCTTC (SEQ ID NO:75) and 5'- GTATGCCCAACTGGTGCAGAGG (SEQ ID NO:76)), so that the resulting product contained 100-200 bp of the 5' end of the *Synechocystis* gene to be knocked out, the kanamycin resistance cassette, and 100-200 bp of the 3' end of the gene to be knocked out. This PCR product was then cloned into the vector pGemT easy (Promega) to create the construct pMON21679 and used for *Symechocystis* transformation.

Primers were also synthesized for the preparation of *Synechocystis* knockout constructs for the other sequences using the same method as described above, with the following primers. The slr0056 5' sequence was amplified using the primers 5'-GGATCCATGTCTGACACACAAAATACCG (SEQ ID NO:77) and 5'-GCAATGTAACATCAGAGATTTTGAGACACAACGTGGCTTTCGCCAATACC AGCCACCAACAG (SEQ ID NO:78). The 3' region was amplified using the synthetic oligonucleotide primers 5'- GAATTCTCAAAT CCCCGCATGGCCTAG (SEQ ID NO:79) and 5'-GGTATGAGTCAGCAACACCTTCTTCACGAGGCAGACCTCAGCGGCCTACG GCTTGGACGTGTGGG (SEQ ID NO:80). The amplification products were combined with the kanamycin resistance gene from puc4K (Pharmacia) that had been digested with *Hinc*II and gel purified away from the vector backbone. The annealed fragment was amplified using 5' and 3' oligos nested within the ends of the ORF fragment (5'- CACTTGGATTCCCCTGATCTG (SEQ ID NO:81) and 5'-GCAATACCCGCTTGGAAAACG (SEQ ID NO:82)), so that the resulting product contained 100-200 bp of the 5' end of the *Synechocystis* gene to be knocked out, the kanamycin resistance cassette, and 100-200 bp of the 3' end of the gene to be knocked out. This PCR product was then cloned into the vector pGemT easy (Promega) to create the construct pMON21677 and used for *Synechocystis* transformation.

Primers were also synthesized for the preparation of *Synechocystis* knockout constructs for the other sequences using the same method as described above, with the following primers. The slr1518 5' sequence was amplified using the primers 5'-GGATCCATGACCGAAT CTTCGCCCCTAGC (SEQ ID NO:83) and 5'-GCAATGTAACATCAGAGATTTTGA GACACAACGTGGC TTTCAATCCTAGGTAGCCGAGGCG (SEQ ID NO:84). The 3' region was amplified using the synthetic oligonucleotide primers 5'-GAATTCTTAGCCCAGGCC AGCCCAGCC (SEQ ID NO:85)and 5'-GGTATGAGTCAGCAACACCTTCTTCACGA GGCAGACCTCAGCGGGGAATTGATTTGTTTAATTACC (SEQ ID NO:86). The amplification products were combined with the kanamycin resistance gene from puc4K (Pharmacia) that had been digested with *Hinc*II and gel purified away from the vector backbone. The annealed fragment was amplified using 5' and 3' oligos nested within the ends of the ORF fragment (5'- GCGATCGCCATTATCGCTTGG (SEQ ID NO:87) and 5'- GCAGACTGGCAATTATCAGTAACG (SEQ ID NO:88)), so that the resulting product contained 100-200 bp of the 5' end of the *Synechocystis* gene to be knocked out, the kanamycin resistance cassette, and 100-200 bp of the 3' end of the gene to be knocked out. This PCR product was then cloned into the vector pGemT easy (Promega) to create the construct pMON21680 and used for *Synechocystis* transformation.

### 4B. Transformation of Synechocystis

Cells of *Synechocystis* 6803 were grown to a density of approximately 2x10⁸ cells per ml and harvested by centrifugation. The cell pellet was re-snspended in fresh BG-11 medium (ATCC Medium 616) at a density of 1x10⁹ cells per ml and used immediately for transformation. One-hundred microliters of these cells were mixed with 5 ul of mini prep DNA and incubated with light at 30C for 4 hours. This mixture was then plated onto nylon filters resting on BG-11 agar supplemented with TES pH8 and allowed to grow for 12-18 hours. The filters were then transferred to BG-11 agar + TES + 5ug/ml kanamycin and allowed to grow until colonies appeared within 7-10 days (Packer and Glazer, 1988). Colonies were then picked into BG-11 liquid media containing 5 ug/ml kanamycin and allowed to grow for 5 days. These cells were then transferred to Bg-1I media containing 10ug/ml kanamycin and allowed to grow for 5 days and then transferred to Bg-11 + kanamycin at 25ug/ml and allowed to grow for 5 days. Cells were then harvested for PCR analysis to determine the presence of a disrupted ORF and also for HPLC analysis to determine if the disruption had any effect on tocopherol levels.

PCR analysis of the *Synechocystis* isolates for slr1736 and sll1899 showed complete segregation of the mutant genome, meaning no copies of the wild type genome could be detected in these strains. This suggests that function of the native gene is not essential for cell function. HPLC analysis of these same isolates showed that the sll1899 strain had no detectable reduction in tocopherol levels. However, the strain carrying the knockout for slr1736 produced no detectable levels of tocopherol.

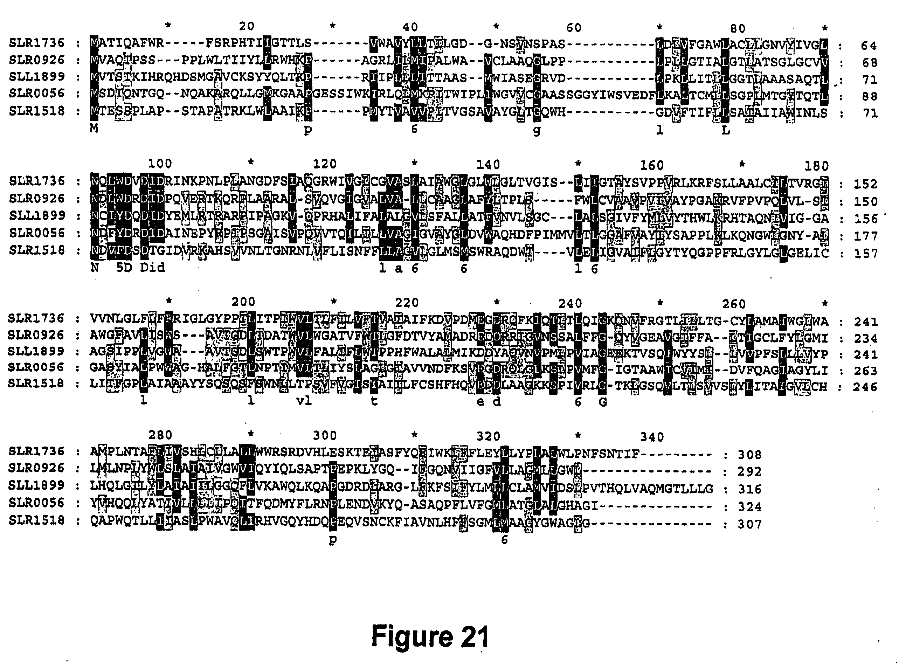

The amino acid sequences for the *Synechocystis* knockouts are compared using ClustalW, and are provided in Table 3 below. Provided are the percent identities, percent similarity, and the percent gap. The alignment of the sequences is provided in Figure 21.

**Table 3:**

| | | Slr1736 | slr0926 | sll1899 | slr0056 | slr1518 |
|---|---|---|---|---|---|---|
| slr1736 | %identity | | 14 | 12 | 18 | 11 |
| | %similar | | 29 | 30 | 34 | 26 |
| | %gap | | 8 | 7 | 10 | 5 |
| slr0926 | %identity | | | 20 | 19 | 14 |
| | %similar | | | 39 | 32 | 28 |
| | %gap | | | 7 | 9 | 4 |
| sll1899 | %identity | | | | 17 | 13 |
| | %similar | | | | 29 | 29 |
| | %gap | | | | 12 | 9 |
| slr0056 | %identity | | | | | 15 |
| | %similar | | | | | 31 |
| | %gap | | | | | 8 |
| slr1518 | %identity | | | | | |
| | %similar | | | | | |
| | %gap | | | | | |

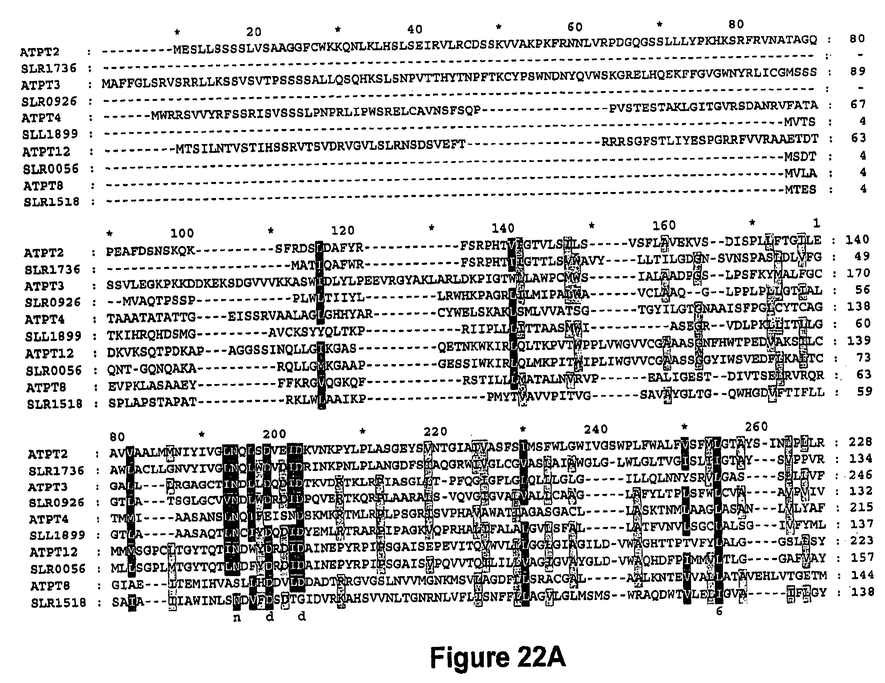

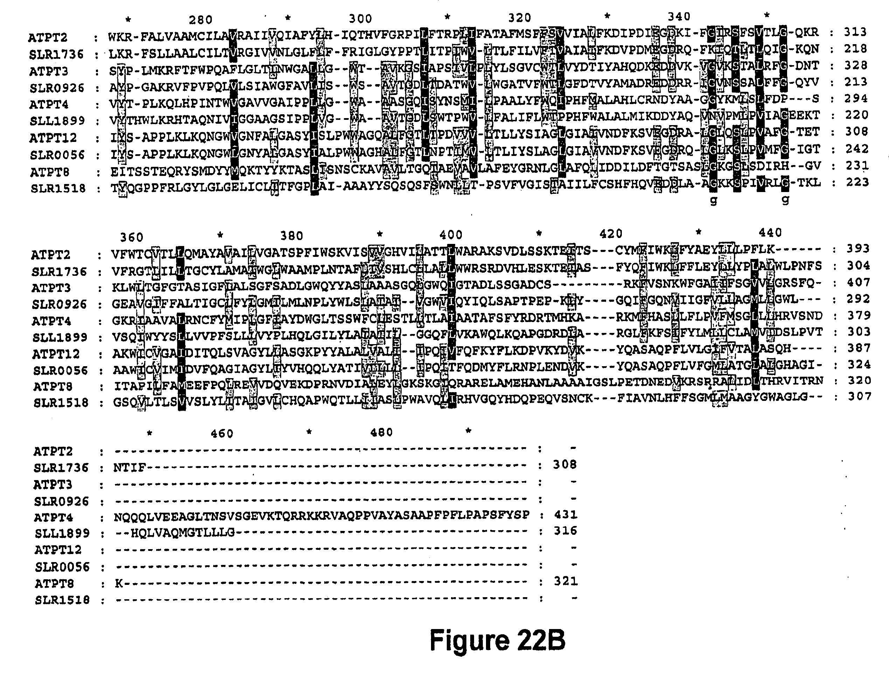

Amino acid sequence comparisons are performed using various *Arabidopsis* prenyltransferase sequences and the *Synechocystis* sequences. The comparisons are presented in Table 4 below. Provided are the percent identities, percent similarity, and the percent gap. The alignment of the sequences is provided in Figure 22.

**Table 4:**

| | ATPT2 | slr1736 | ATPT3 | slr0926 | ATPT4 | sll1899 | ATPT12 | slr0056 | ATPT8 | slr1518 |
|---|---|---|---|---|---|---|---|---|---|---|
| ATPT2 | | 29 | 9 | 9 | 8 | 8 | 12 | 9 | 7 | 9 |
| | | 46 | 23 | 21 | 20 | 20 | 28 | 23 | 21 | 20 |
| | | 27 | 13 | 28 | 23 | 29 | 11 | 24 | 25 | 24 |
| slr1736 | | | 9 | 13 | 8 | 12 | 13 | 15 | 8 | 10 |
| | | | 19 | 28 | 19 | 28 | 26 | 33 | 21 | 26 |
| | | | 34 | 12 | 34 | 15 | 26 | 10 | 12 | 10 |
| ATPT3 | | | | 23 | 11 | 14 | 13 | 10 | 5 | 11 |
| | | | | 36 | 26 | 26 | 26 | 21 | 14 | 22 |
| | | | | 29 | 21 | 31 | 16 | 30 | 30 | 30 |
| | | | | | 12 | 20 | 17 | 20 | 11 | 14 |
| slr0926 | | | | | 24 | 37 | 28 | 33 | 24 | 29 |
| | | | | | 33 | 12 | 25 | 10 | 11 | 9 |
| | | | | | | 18 | 11 | 8 | 6 | 7 |
| ATPT4 | | | | | | 33 | 23 | 18 | 16 | 19 |
| | | | | | | 28 | 19 | 32 | 32 | 33 |
| | | | | | | | 13 | 17 | 10 | 12 |
| sll1899 | | | | | | | 24 | 30 | 23 | 26 |
| | | | | | | | 27 | 13 | 10 | 11 |
| | | | | | | | | 52 | 8 | 11 |
| ATPT1 | | | | | | | | 66 | 19 | 26 |
| | | | | | | | | 18 | 25 | 23 |
| | | | | | | | | | 9 | 13 |
| slr0056 | | | | | | | | | 23 | 32 |
| | | | | | | | | | 10 | 8 |
| | | | | | | | | | | 7 |
| ATPT8 | | | | | | | | | | 23 |
| | | | | | | | | | | 7 |
| slr1518 | | | | | | | | | | |

### 4C. Phytyl Prenyltransferase Enzyme Assays

[³H] Homogentisic acid in 0.1% H₃PO₄ (specific radioactivity 40 Ci/mmol). Phytyl pyrophosphate was synthesized as described by Joo, et al. (1973) Can J. Biochem. 51:1527. 2-methyl-6-phytylquinol and 2,3-dimethyl-5-phytylquinol were synthesized as described by Soll, et al. (1980) Phytochemistry 19:215. Homogentisic acid, α, β, δ, and γ-tocopherol, and tocol, were purchased commercially.

The wild-type strain of *Synechocystis* sp. PCC 6803 was grown in BG11 medium with bubbling air at 30°C under 50 µE.m⁻².s⁻¹ fluorescent light, and 70% relative humidity. The growth medium of slr1736 knock-out (potential PPT) strain of this organism was supplemented with 25 µg mL⁻¹ kanamycin. Cells were collected from 0.25 to 1 liter culture by centrifugation at 5000 g for 10 min and stored at -80°C.

Total membranes were isolated according to Zak's procedures with some modifications (Zak, et al. (1999) Eur J. Biochem 261:311). Cells were broken on a French press. Before the French press treatment, the cells were incubated for 1 hour with lysozyme (0.5%, w/v) at 30 °C in a medium containing 7 mM EDTA, 5 mM NaCl and 10 mM Hepes-NaOH, pH 7.4. The spheroplasts were collected by centrifugation at 5000 g for 10 min and resuspended at 0.1 - 0.5 mg chlorophyll·mL⁻¹ in 20 mM potassium phosphate buffer, pH 7.8. Proper amount of protease inhibitor cocktail and DNAase I from Boehringer Mannheim were added to the solution. French press treatments were performed two to three times at 100 MPa. After breakage, the cell suspension was centrifuged for 10 min at 5000g to pellet unbroken cells, and this was followed by centrifugation at 100 000 *g* for 1 hour to collect total membranes. The final pellet was resuspended in a buffer containing 50 mM Tris-HCL and 4 mM MgCl₂.

Chloroplast pellets were isolated from 250 g of spinach leaves obtained from local markets. Devined leaf sections were cut into grinding buffer (21/250 g leaves) containing 2 mM EDTA, 1 mM MgCl₂, 1 mM MnCl₂, 0.33 M sorbitol, 0.1% ascorbic acid, and 50 mM Hepes at pH 7.5. The leaves were homogenized for 3 sec three times in a 1-L blendor, and filtered through 4 layers of mirocloth. The supernatant was then centrifuged at 5000g for 6 min. The chloroplast pellets were resuspended in small amount of grinding buffer (Douce,et al Methods in Chloroplast Molecular Biology, 239 (1982)

Chloroplasts in pellets can be broken in three ways. Chloroplast pellets were first aliquoted in 1 mg of chlorophyll per tube, centrifuged at 6000 rpm for 2 min in microcentrifuge, and grinding buffer was removed. Two hundred microliters of Triton X-100 buffer (0. 1 % Triton X-100, 50 mM Tris-HCl pH 7.6 and 4 mM MgCl₂) or swelling buffer (10 mM Tris pH 7.6 and 4 mM MgCl₂) was added to each tube and incubated for ½ hour at 4°C. Then the broken chloroplast pellets were used for the assay immediately. In addition, broken chloroplasts can also be obtained by freezing in liquid nitrogen and stored at -80°C for ½ hour, then used for the assay.

In some cases chloroplast pellets were further purified with 40%/ 80% percoll gradient to obtain intact chloroplasts. The intact chloroplasts were broken with swelling buffer, then either used for assay or further purified for envelope membranes with 20.5%/ 31.8% sucrose density gradient (Sol, *et al* (1980) *supra*)*.* The membrane fractions were centrifuged at 100 000g for 40 min and resuspended in 50 mM Tris-HCl pH 7.6, 4 mM MgCl₂.

Various amounts of [³H]HGA, 40 to 60 µM unlabelled HGA with specific activity in the range of 0.16 to 4 Ci/mmole were mixed with a proper amount of 1M Tris-NaOH pH 10 to adjust pH to 7.6. HGA was reduced for 4 min with a trace amount of solid NaBH₄. In addition to HGA, standard incubation mixture (final vol 1 mL) contained 50 mM Tris-HCl, pH 7.6, 3-5 mM MgCl₂, and 100 µM phytyl pyrophosphate. The reaction was initiated by addition of *Synechocystis* total membranes, spinach chloroplast pellets, spinach broken chloroplasts, or spinach envelope membranes. The enzyme reaction was carried out for 2 hour at 23°C or 30°C in the dark or light. The reaction is stopped by freezing with liquid nitrogen, and stored at -80°C or directly by extraction.

A constant amount of tocol was added to each assay mixture and reaction products were extracted with a 2 mL mixture of chloroform/methanol (1:2, v/v) to give a monophasic solution. NaCl solution (2 mL; 0.9%) was added with vigorous shaking. This extraction procedure was repeated three times. The organic layer containing the prenylquinones was filtered through a 20 mµ filter, evaporated under N₂, and then resuspended in 100 µL of ethanol.

The samples were mainly analyzed by Normal-Phase HPLC method (Isocratic 90% Hexane and 10% Methyl-t-butyl ether), and use a Zorbax silica column, 4.6 x 250 mm. The samples were also analyzed by Reversed-Phase HPLC method (Isocratic 0.1% H₃PO₄ in MeOH), and use a Vydac 201HS54 C18 column; 4.6 x 250 mm coupled with an All-tech C18 guard column. The amount of products were calculated based on the substrate specific radioactivity, and adjusted according to the % recovery based on the amount of internal standard.

The amount of chlorophyll was determined as described in Arnon (1949) Plant Physiol. 24:1. Amount of protein was determined by the Bradford method using gamma globulin as a standard (Bradford, (1976) Anal. Biochem. 72:248)

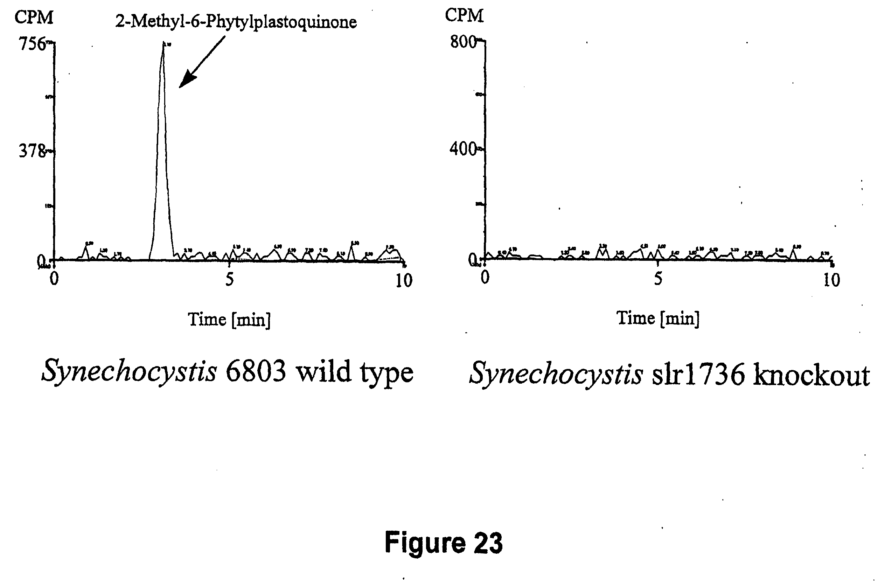

Results of the assay demonstrate that 2-Methyl-6-Phytylplastoquinone is not produced in the *Synechocystis* slr1736 knockout preparations. The results of the phytyl prenyltransferase enzyme activity assay for the slr1736 knock out are presented in Figure 23.

### 4D. Complementation of the slr1736 knockout with ATPT2

In order to determine whether ATPT2 could complement the knockout of slr1 736 in *Synechocystis 6803,* a plasmid was constructed to express the ATPT2 sequence from the TAC promoter. A vector, plasmid psl1211, was obtained from the lab of Dr. Himadri Pakrasi of Washington University, and is based on the plasmid RSF1010 which is a broad host range plasmid (Ng W.-O., Zentella R., Wang, Y., Taylor J-S. A., Pakrasi, H.B. 2000. *phrA,* the major photoreactivating factor in the cyanobacterium *Synechocystis* sp. strain PCC 6803 codes for a cyclobutane pyrimidine dimer specific DNA photolyase. *Arch. Microbiol.* (in press)). The ATPT2 gene was isolated from the vector pCGN10817 by PCR using the following primers. ATPT2nco.pr 5'-CCATGGATTCGAGTAAAGTTGTCGC (SEQ ID NO:89); ATPT2ri.pr- 5'-GAATTCACTTCAAAAAAGGTAACAG (SEQ ID NO:90). These primers will remove approximately 112 BP from the 5' end of the ATPT2 sequence, which is thought to be the chloroplast transit peptide. These primers will also add an NcoI site at the 5' end and an EcoRI site at the 3' end which can be used for subcloning into subsequent vectors. The PCR product from using these primers and pCGN10817 was ligated into pGEM T easy and the resulting vector pMON21689 was confirmed by sequencing using the m13forward and m13reverse primers. The NcoI/EcoRI fragment from pMON21689 was then ligated with the EagI/EcoRI and EagI/NcoI fragments from psl1211 resulting in pMON21690. The plasmid pMON21690 was introduced into the slr1736 *Synechocystis 6803* KO strain via conjugation. Cells of s1906 (a helper strain) and DH10B cells containing pMON21690 were grown to log phase (O.D. 600= 0.4) and 1 ml was harvested by centrifugation. The cell pellets were washed twice with a sterile BG-11 solution and resuspended in 200 ul of BG-11. The following was mixed in a sterile eppendorf tube: 50 ul SL906,50 ul DH10B cells containing pMON21690, and 100 ul of a fresh culture of the slr1736 *Synechocystis 6803* KO strain (O.D. 730 = 0.2-0.4). The cell mixture was immediately transferred to a nitrocellulose filter resting on BG-11 and incubated for 24 hours at 30C and 2500 LUX(50 ue) of light. The filter was then transferred to BG-11 supplemented with 10ug/ml Gentamycin and incubated as above for ~5 days. When colonies appeared, they were picked and grown up in liquid BG-11 + Gentamycin 10 ug/ml. (Elhai, J. and Wolk, P. 1988. Conjugal transfer of DNA to Cyanobacteria. Methods in Enzymology 167, 747-54) The liquid cultures were then assayed for tocopherols by harvesting 1ml of culture by centrifugation, extracting with ethanol/pyrogallol, and HPLC separation. The slr1736 *Synechoeystis 6803* KO strain, did not contain any detectable tocopherols, while the sir 1736 *Synechocystis 6803* KO strain transformed with pmon21690 contained detectable alpha tocopherol. A *Synechocystis 6803* strain transformed with psl1211(vector control) produced alpha tocopherol as well.

### 4E: Additional Evidence of Prenyltransferase Activity

To test the hypothesis that slr1736 or ATPT2 are sufficient as single genes to obtain phytyl prenyltransferase activity, both genes were expressed in SF9 cells and in yeast. When either slr1736 or ATPT2 were expressed in insect cells (Table 5) or in yeast, phytyl prenyltransferase activity was detectable in membrane preparations, whereas membrane preparations of the yeast vector control, or membrane preparations of insect cells did not exhibit phytyl prenyltransferase activity.

**Table 5: Phytyl prenyltransferase activity**

| **Enzyme source** | **Enzyme activity [pmol/mg x h]** |
|---|---|
| slr1736 expressed in SF9 cells | 20 |
| ATPT2 expressed in SF9 cells | 6 |
| SF9 cell control | < 0.05 |
| *Synechocystis* 6803 | 0.25 |
| Spinach chloroplasts | 0.20 |

### Comparative Example 5: Transgenic Plant Analysis

### 5A. Arabidopsis

*Arabidopsis* plants transformed with constructs for the sense or antisense expression of the ATPT proteins were analyzed by High Pressure Liquid Chromatography (HPLC) for altered levels of total tocopherols, as well as altered levels of specific tocopherols (alpha, beta, gamma, and delta tocopherol).

Extracts of leaves and seeds were prepared for HPLC as follows. For seed extracts, 10 mg of seed was added to 1 g of microbeads (Biospec) in a sterile microfuge tube to which 500 ul 1% pyrogallol (Sigma Chem)/ethanol was added-The mixture was shaken for 3 minutes in a mini Beadbeater (Biospec) on "fast" speed. The extract was filtered through a 0.2 um filter into an autosampler tube. The filtered extracts were then used in HPLC analysis described below.

Leaf extracts were prepared by mixing 30-50 mg of leaf tissue with 1 g microbeads and freezing in liquid nitrogen until extraction. For extraction, 500 ul 1% pyrogallol in ethanol was added to the leaf/bead mixture and shaken for I minute on a Beadbeater (Biospec) on "fast" speed. The resulting mixture was centrifuged for 4 minutes at 14,000 rpm and filtered as described above prior to HPLC analysis.

HPLC was performed on a Zorbax silica HPLC column (4.6 mm X 250 mm) with a fluorescent detection, an excitation at 290 nm, an emission at 336 nm, and bandpass and slits. Solvent A was hexane and solvent B was methyl-t-butyl ether. The injection volume was 20 ul, the flow rate was 1.5 ml/min, the run time was 12 min (40°C) using the gradient (Table 6):

**Table 6:**

| Time | Solvent A | Solvent B |
|---|---|---|
| 0 min. | 90% | 10% |
| 10 min. | 90% | 10% |
| 11 min. | 25% | 75% |
| 12 min. | 90% | 10% |

Tocopherol standards in 1% pyrogallol/ ethanol were also run for comparison (alpha tocopherol, gamma tocopherol, beta tocopherol, delta tocopherol, and tocopherol (tocol) (all from Matreya).

Standard curves for alpha, beta, delta, and gamma tocopherol were calculated using Chemstation software. The absolute amount of component x is: Absolute amount of x= Responseₓ x RFₓ x dilution factor where Responseₓ is the area of peak x, RFₓ is the response factor for component x (Amountₓ/Responseₓ) and the dilution factor is 500 ul. The ng/mg tissue is found by: total ng component/mg plant tissue.

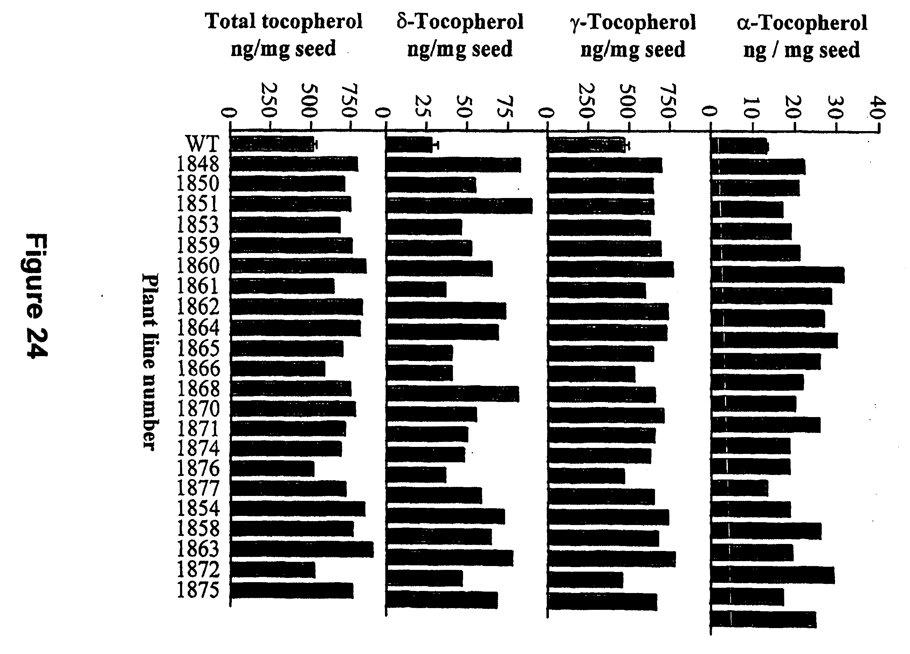

Results of the HPLC analysis of seed extracts of transgenic *Arabidopsis* lines containing pMON10822 for the expression of ATPT2 from the napin promoter are provided in Figure 24.

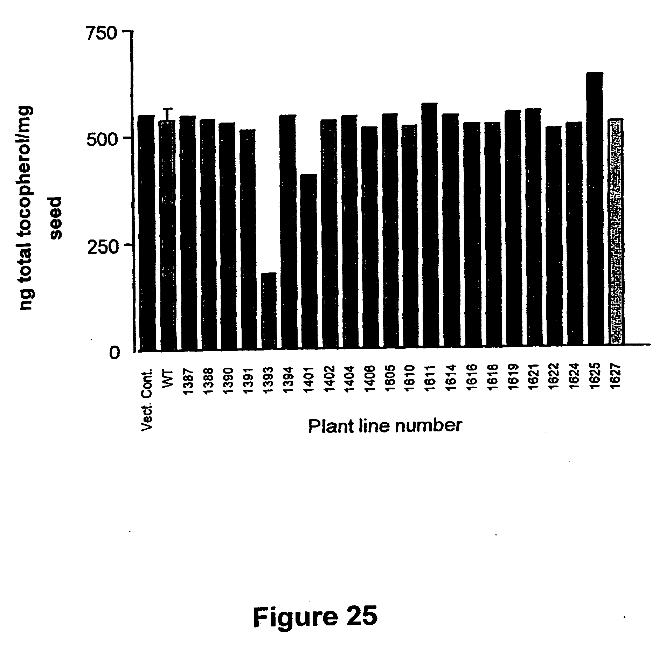

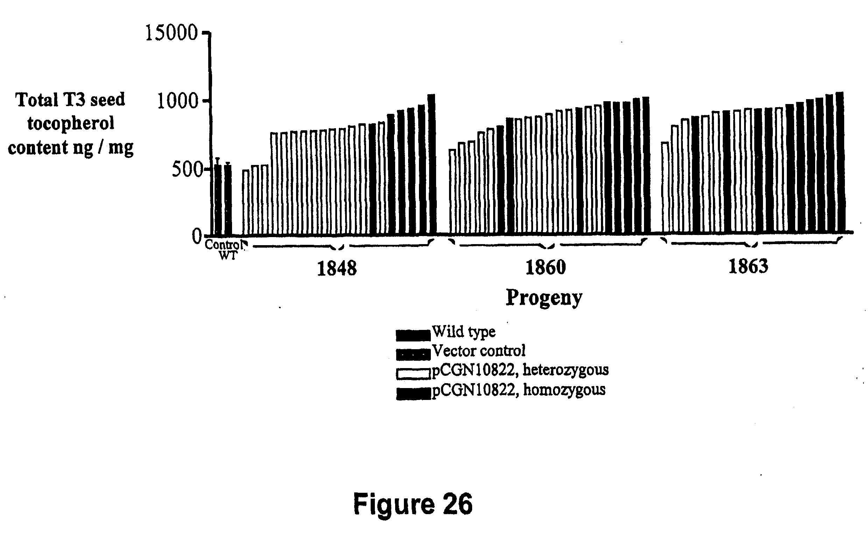

HPLC analysis results of segregating T2 *Arabidopsis* seed tissue expressing the ATPT2 sequence from the napin promoter (pCGN10822) demonstrates an increased level of tocopherols in the seed. Total tocopherol levels are increased as much as 50% over the total tocopherol levels of non-transformed (wild-type) *Arabidopsis* plants (Figure 25). Homozygous progeny from the top 3 lines (T3 seed) have up to a two-fold (100%) increase in total tocopherol levels over control *Arabidopsis* seed (Figure 26.)

Furthermore, increases of particular tocopherols are also increased in transgenic *Arabidopsis* plants expressing the ATPT2 nucleic acid sequence from the napin promoter. Levels of delta tocopherol in these lines are increased greater than 3 fold over the delta tocopherol levels obtained from the seeds of wild type *Arabidopsis* lines. Levels of gamma tocopherol in transgenic *Arabidopsis* lines expressing the ATPT2 nucleic acid sequence are increased as much as about 60% over the levels obtained in the seeds of non-transgenic control lines. Furthermore, levels of alpha tocopherol are increased as much as 3 fold over those obtained from non-transgenic control lines.

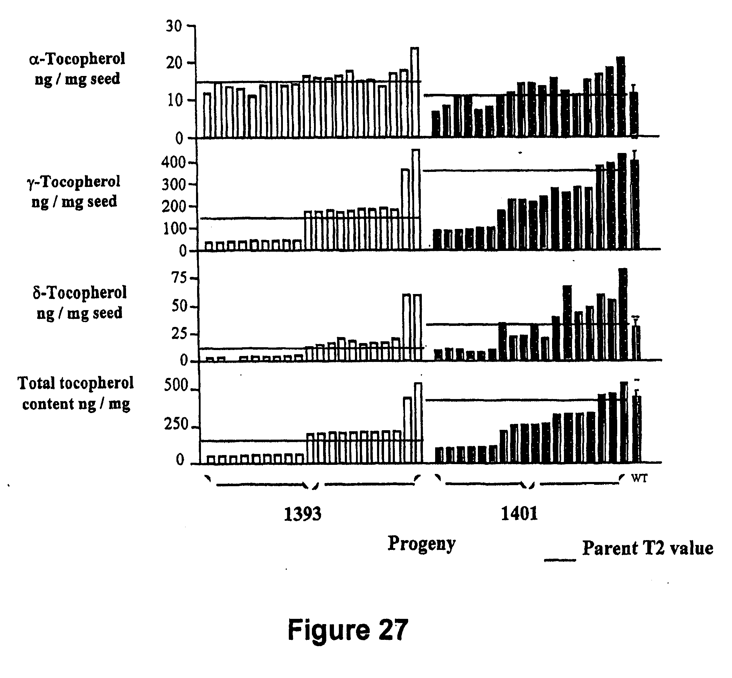

Results of the HPLC analysis of seed extracts of transgenic *Arabidopsis* lines containing pCGN10803 for the expression of ATPT2 from the enhanced 35S promoter (antisense orientation) are provided in Figure 25. Two lines were identified that have reduced total tocopherols, up to a ten-fold decrease observed in T3 seed compared to control *Arabidopsis* (Figure 27.)

### 5B. Canola

Brassica napus, variety SP30021, was transformed with pCGN10822 (napin-ATPT2-napin 3', sense orientation) using *Agrobacterium tumefaciens-*mediated transformation. Flowers of the R0 plants were tagged upon pollination and developing seed was collected at 35 and 45 days after pollination (DAP).

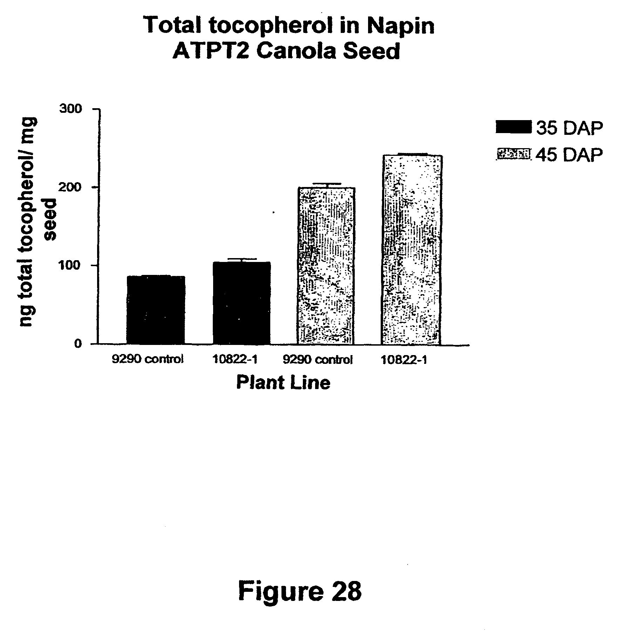

Developing seed was assayed for tocopherol levels, as described above for *Arabidopsis.* Line 10822-1 shows a 20% increase of total tocopherols, compared to the wild-type control, at 45 DAP. Figure 28 shows total tocopherol levels measured in developing canola seed.

### Example 6: Sequences to Tocopherol Cyclase

### 6A. Preparation of the slr1737 Knockout

The *Synechocystis sp. 6803* sir 1737 knockout was constructed by the following method. The GPS^{™}-1 Genome Priming System (New England Biolabs) was used to insert, by a Tn7 Transposase system, a Kanamycin resistance cassette into *slr1737.* A plasmid from a *Synechocystis* genomic library clone containing 652 base pairs of the targeted orf (*Synechcocystis* genome base pairs 1324051 - 1324703; the predicted orf base pairs 1323672 - 1324763, as annotated by Cyanobase) was used as target DNA. The reaction was performed according to the manufacturers protocol. The reaction mixture was then transformed into *E. coli* DH10B electrocompetant cells and plated. Colonies from this transformation were then screened for transposon insertions into the target sequence by amplifying with M13 Forward and Reverse Universal primers, yielding a product of 652 base pairs plus ~1700 base pairs, the size of the transposon kanamycin cassette, for a total fragment size of ~2300 base pairs. After this determination, it was then necessary to determine the approximate location of the insertion within the targeted orf, as 100 base pairs of orf sequence was estimated as necessary for efficient homologous recombination in *Synechocystis.* This was accomplished through amplification reactions using either of the primers to the ends of the transposon, Primer S (5' end) or N (3' end), in combination with either a M13 Forward or Reverse primer. That is, four different primer combinations were used to map each potential knockout construct: Primer S - M 13 Forward, Primer S - M13 Reverse, Primer N - M13 Forward, Primer N - M13 Reverse. The construct used to transform *Synechocystis* and knockout slr1737 was determined to consist of a approximately 150 base pairs of slr1737 sequence on the 5' side of the transposon insertion and approximately 500 base pairs on the 3' side, with the transcription of the orf and kanamycin cassette in the same direction. The nucleic acid sequence of slr1737 is provided in SEQ ID NO:38 the deduced amino acid sequence is provided in SEQ ID NO:39.

Cells of *Synechocystis 6803* were gown to a density of ~ 2x10⁸ cells per ml and harvested by centrifugation. The cell pellet was re-suspended in fresh BG-11 medium at a density of 1x10⁹ cells per ml and used immediately for transformation. 100 ul of these cells were mixed with 5 ul of mini prep DNA and incubated with light at 30C for 4 hours. This mixture was then plated onto nylon filters resting on BG-11 agar supplemented with TES ph8 and allowed to grow for 12-18 hours. The filters were then transferred to BG-11 agar + TES + 5ug/ml kanamycin and allowed to grow until colonies appeared within 7-10 days (Packer and Glazer, 1988). Colonies were then picked into BG-11 liquid media containing 5 ug/ml kanamycin and allowed to grow for 5 days. These cells were then transferred to Bg-11 media containing 10ug/ml kanamycin and allowed to grow for 5 days and then transferred to Bg-11 + kanamycin at 25ug/ml and allowed to grow for 5 days. Cells were then harvested for PCR analysis to determine the presence of a disrupted ORF and also for HPLC analysis to determine if the disruption had any effect on tocopherol levels.

PCR analysis of the *Synechocystis* isolates, using primers to the ends of the *slr1737* orf , showed complete segregation of the mutant genome, meaning no copies of the wild type genome could be detected in these strains. This suggests that function of the native gene is not essential for cell function. HPLC analysis of the strain carrying the knockout for *slr1737* produced no detectable levels of tocopherol.

### 6B. The relation of slr1737 and slr1736

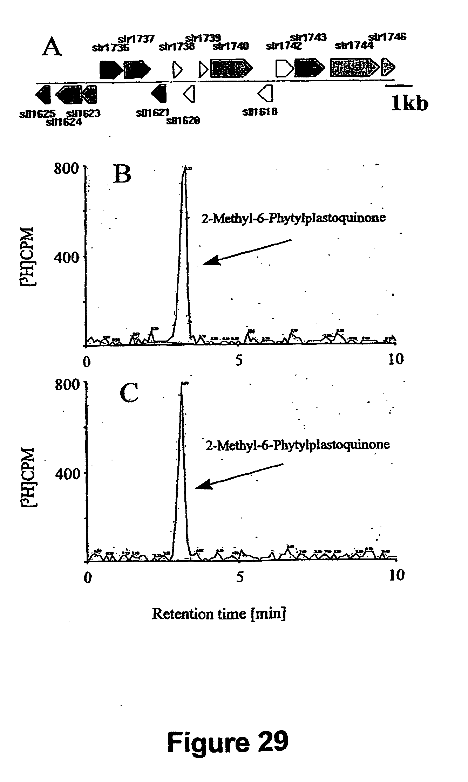

The slr1737 gene occurs in *Synechocystis* downstream and in the same orientation as slr1736, the phytyl prenyltransferase. In bacteria this proximity often indicates an operon structure and therefore an expression pattern that is linked in all genes belonging to this operon. Occasionally such operons contain several genes that are required to constitute one enzyme. To confirm that slr1737 is not required for phytyl prenyltransferase activity, phytyl prenyltransferase was measured in extracts from the *Synechocystis* slr1737 knockout mutant. Figure 29 shows that extracts from the *Synechocystis* slr1737 knockout mutant still contain phytyl prenyltransferase activity. The molecular organization of genes in *Synechocystis* 6803 is shown in A. Figures B and C show HPLC traces (normal phase HPLC) of reaction products obtained with membrane preparations from *Synechocystis* wild type and slr1737⁻ membrane preparations, respectively.

The fact that slr1737 is not required for the PPT activity provides additional data that ATPT2 and slr1736 encode phytyl prenyltransferases.

### 6C Synechocystis Knockouts

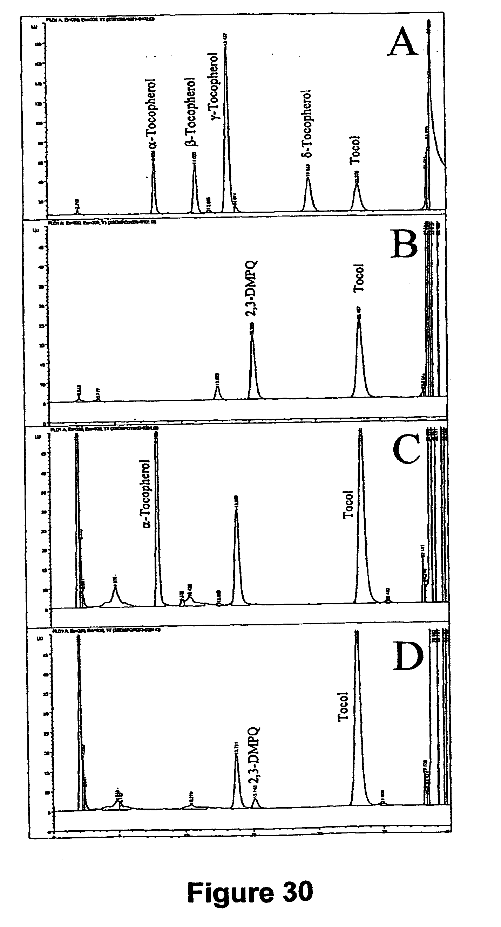

*Synechocystis* 6803 wild type and *Syrrechocystis* slr1737 knockout mutant were grown photoautotrophically. Cells from a 20 ml culture of the late logarithmic growth phase were harvested and extracted with ethanol. Extracts were separated by isocratic normal-phase HPLC using a Hexane/Methyl-t-butyl ether (95/5) and a Zorbax silica column, 4.6 x 250 mm. Tocopherols and tocopherol intermediates were detected by fluorescence (excitement 290 nm, emission 336 nm) (Figure 30).

Extracts of *Synechocystis* 6803 contained a clear signal of alpha-tocopherol. 2,3-Dimethyl-5-phytylplastoquinol was below the limit of detection in extracts from the *Synechocystis* wild type (C). In contrast, extracts from the *Synechocystis* slr1737 knockout mutant did not contain alpha-tocopherol, but contained 2,3-dimethyl-5-phytylplastoquinol (D), indicating that the interruption of slr1737 has resulted in a block of the 2,3-dimethyl-5-phytylplastoquinol cyclase reaction.

Chromatograms of standard compounds alpha, beta, gamma, delta-tocopherol and 2,3-dimethyl-5-phytylplastoquinol are shown in A and B. Chromatograms of extracts form *Synechocystis* wild type and the *Synechocystis* slr1737 knockout mutant are shown in C and D, respectively. Abbreviations: 2,3-DMPQ, 2,3-dimethyl-5-phytylplastoquinol.

### 6D. Incubation with Lysozyme treated Synechocystis

*Synechocystis* 6803 wild type and slr1737 knockout mutant cells from the late logarithmic growth phase (approximately 1 g wet cells per experiment in a total volume of 3 ml) were treated with Lysozyme and sulisequently incubated with S-adenosylmethionine, and phytylpyrophosphate, plus radiolabelled homogentisic acid. After 17h incubation in the dark at room temperature the samples were extracted with 6 ml chloroform / methanol (1/2 v/v). Phase separation was obtained by the addition of 6 ml 0.9% NaCl solution. This procedure was repeated three times. Under these conditions 2,3-dimethyl-5-phytylplastoquinol is oxidized to form 2,3-dimethyl-5-phytylplastoquinone.

The extracts were analyzed by normal phase and reverse phase HPLC. Using extracts from wild type *Synechocystis* cells radiolabelled gamma-tocopherol and traces of radiolabelled 2,3-dimethyl-5-phytylplastoquinone were detected. When extracts from the slr1737 knockout mutant were analyzed, only radiolabelled 2,3-dimethyl-5-phytylplastoquinone was detectable. The amount of 2,3-dimethyl-5-phytylplastoquinone was significantly increased compared to wild type extracts. Heat treated samples of the wild type and the slr1737 knockout mutant did not produce radiolabelled 2,3-dimethyl-5-phytylplastoquinone, nor radiolabelled tocopherols. These results further support the role of the slr1737 expression product in the cyclization of 2,3-dimethyl-5-phytylplastoquinol.

### 6E. Arabidopsis Homologue to slr1737

An *Arabidopsis* homologue to slr1737 was identified from a BLASTALL search using *Synechocystis* sp 6803 gene slr1737 as the query, in both public and proprietary databases. SEQ ID NO:109 and SEQ ID NO:110 are the DNA and translated amino acid sequences, respectively, of the *Arabidopsis* homologue to slr1737. The start if found at the ATG at base 56 in SEQ ID NO:109.

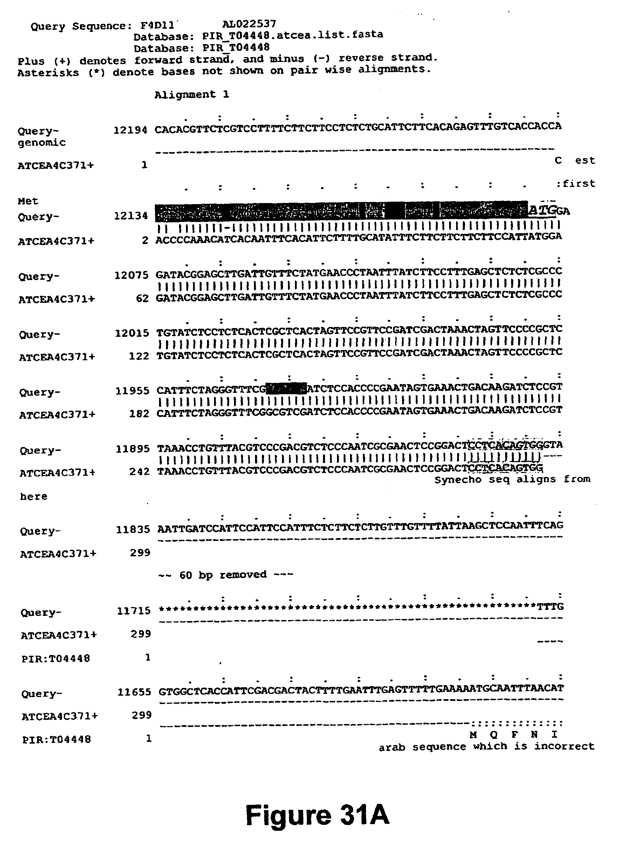

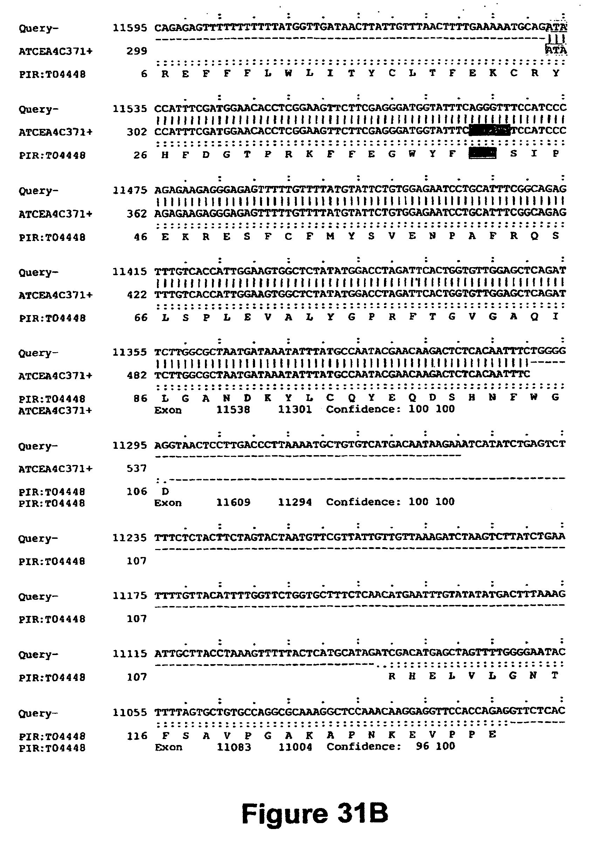

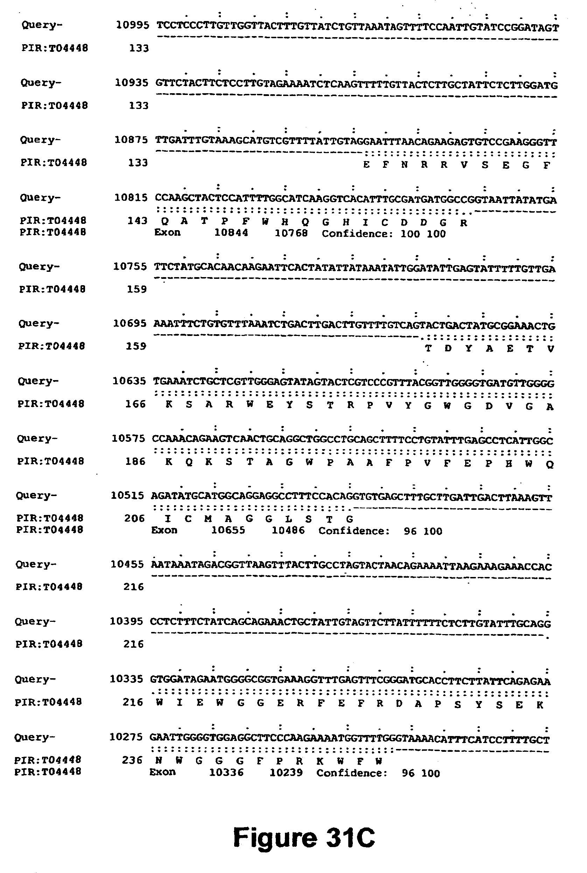

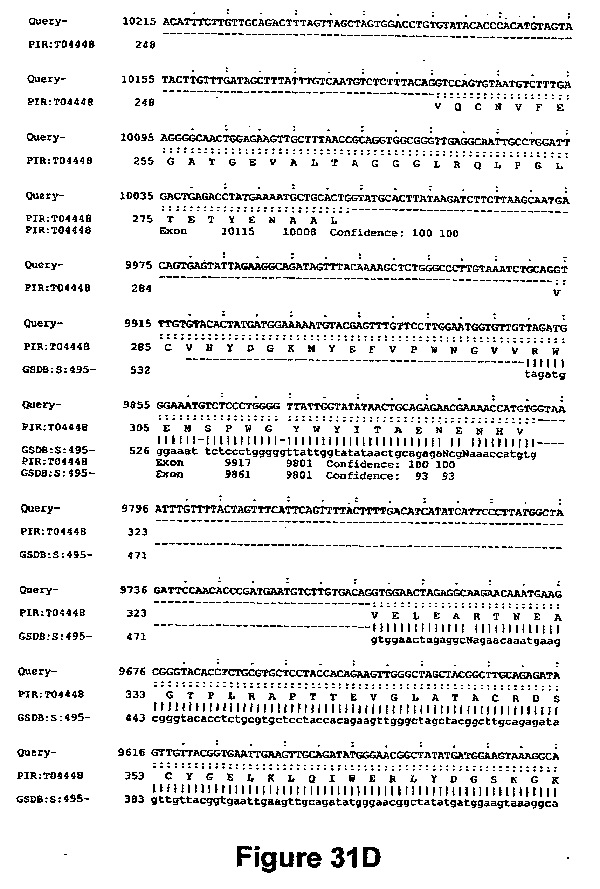

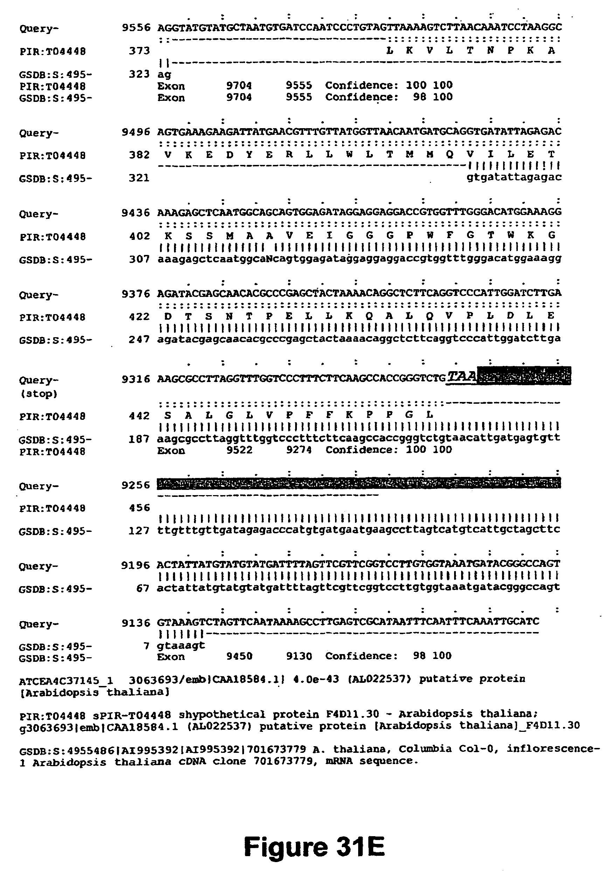

The sequences obtained for the homologue from the proprietary database differs from the public database (F4D11.30, BAC AL022537), in having a start site 471 base pairs upstream of the start identified in the public sequence. A comparison of the public and proprietary sequences is provided in Figure 31. The correct start correlates within the public database sequence is at 12080, while the public sequence start is given as being at 11609.

Attempts to amplify a slr1737 homologue were unsuccessful using primers designed from the public database, while amplification of the gene was accomplished with primers obtained from SEQ ID NO:109.

Analysis of the protein sequence to identify transit peptide sequence predicted two potential cleavage sites, one between amino acids 48 and 49, and the other between amino acids 98 and 99.

### 6F. slr1737 Protein Information

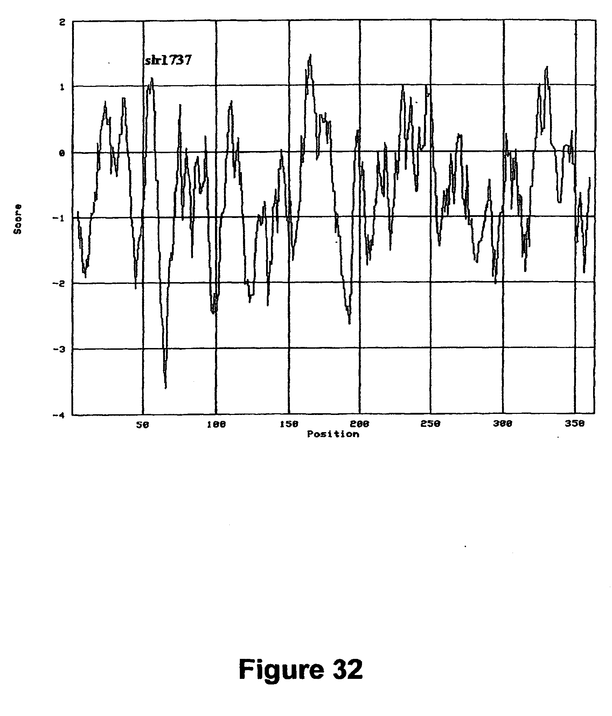

The slr1737 orf comprises 363 amino acid residues and has a predicted MW of 41kDa (SEQ ID NO: 39). Hydropathic analysis indicates the protein is hydrophillic (Figure 32).

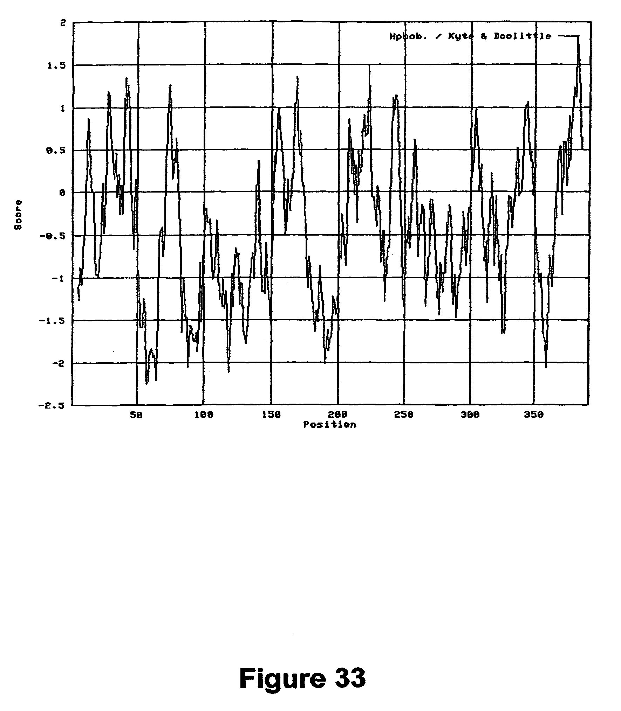

The *Arabidopsis* homologue to slr1737 (SEQ ID xx) comprises 488 amino acid residues, has a predicted MW of 55kDa, and a has a putative transit peptide sequence comprising the first 98 amino acids. The predicted MW of the mature form of the *Arabidopsis* homologue is 44kDa The hydropathic plot for the *Arabidopsis* homologue also reveals that it is hydrophillic (Figure 33). Further blast analysis of the *Arabidopsis* homologue reveals limited sequence identity (25 % sequence identity) with the beta-subunit of respiratory nitrate reductase. Based on the sequence identity to nitrate reductase, it suggests the slr1737 orf is an enzyme that likely involves general acid catalysis mechanism.

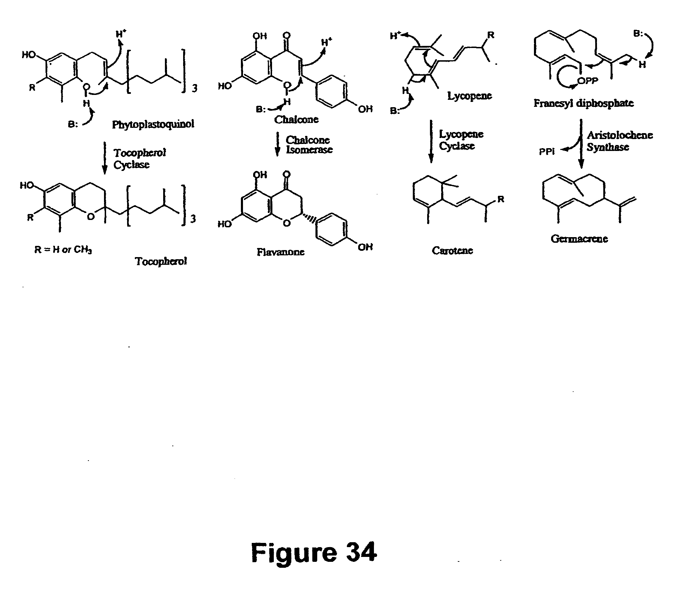

Investigation of known enzymes involved in tocopherol metabolism indicated that the best candidate corresponding to the general acid mechanism is the tocopherol cyclase. There are many known examples of cyclases including, tocopherol cyclase, chalcone isomerase, lycopene cyclase, and aristolochene synthase. By further examination of the microscopic catalytic mechanism of phytoplastoquinol cyclization, as an example, chalcone isomerase has a catalytic mechanism most similar to tocopherol cyclase. (Figure 34).

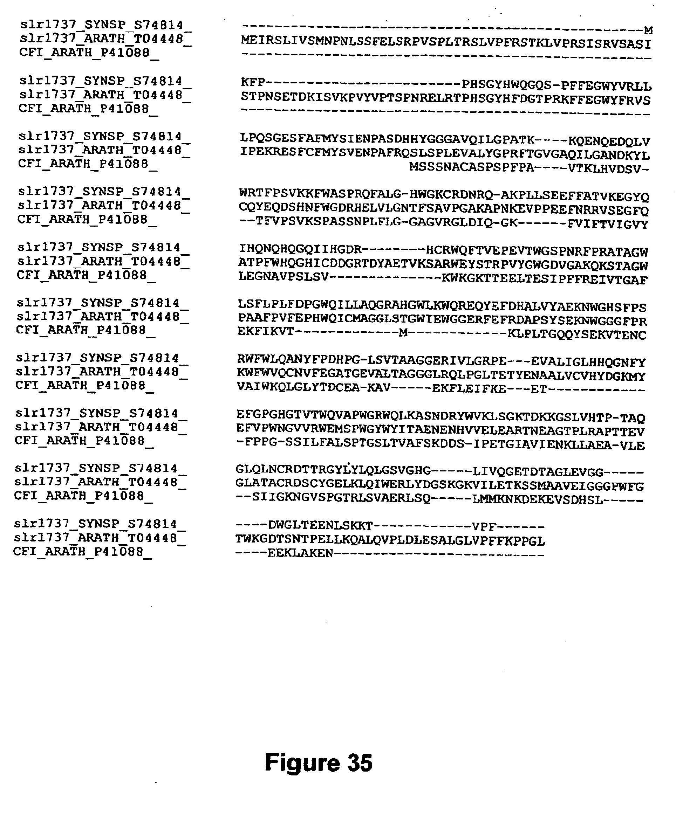

Multiple sequence alignment was performed between slr1737, slr1737 *Arabidopsis* homologue and the *Arabidopsis* chalcone isomerase (Genbank:P41088) (Figure 35). 65% of the conserved residues among the three enzymes are strictly conserved within the known chalcone isomerases. The crystal structure of alfalfa chalcone isomerase has been solved (Jez, Joseph M., Bowman, Marianne E., Dixon, Richard A., and Noel, Joseph P. (2000) "Structure and mechanism of the evolutionarily unique plant enzyme chalcone isomerase". Nature Structural Biology 7: 786-791.) It has been demonstrated tyrosine (Y) 106 of the alfalfa chalcone isomerase serves as the general acid during cyclization reaction (Genbank: P28012). The equivalent residue in sir1737 and the slr1737 *Arabidopsis* homolog is lysine (K), which is an excellent catalytic residue as general acid.

The information available from partial purification of tocopherol cyclase from *Chlorella protothecoides* (U.S. Patent No. 5,432,069), *i.e.,* described as being glycine rich, water soluble and with a predicted MW of 49-50kDa, is consistent with the protein informatics information obtained for the slr1737 and the Arabidopsis slr1737 homologue.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claim.

## Claims

1. A nucleic acid encoding a tocopherol cyclase comprising an amino acid sequence having at least 90% identity to the amino acid sequence of SEQ ID NO: 39 or SEQ ID NO: 110, comprising, as operably linked components, a transcriptional initiation region functional in a host plant cell, a nucleic acid sequence encoding the tocopherol cyclase, and a transcriptional termination region.

2. A nucleic acid according to Claim 1, wherein said tocopherol cyclase is active in the cyclization of 2,3-dimethyl-5-phytylplastoquinol to tocopherol.

3. A nucleic acid according to Claim 1, wherein said tocopherol cyclase is active in the cyclization of 2,3-dimethyl-5-geranylgeranylplastoquinol to tocotrienol.

4. The nucleic acid of Claim 1, comprising a sequence of SEQ ID NO: 38 or SEQ ID NO:109.

5. A plant cell comprising the construct of Claim 1, wherein the plant is selected from the group of *Acacia*, alfalfa, aneth, apple, apricot, artichoke, aragula, asparagus, avocado, banana, barley, beans, beet, blackberry, blueberry, broccoli, brussels sprouts, cabbage, canola, cantaloupe, carrot, cassava, cauliflower, celery, cherry, chicory, cilantro, citrus, clementines, coffee, corn, cotton, cucumber, Douglas fir, eggplant, endive, escarole, eucalyptus, fennel, figs, garlic, gourd, grape, grapefruit, honey dew, jicama, kiwifruit, lettuce, leeks, lemon, lime, Loblolly pine, mango, melon, mushroom, nectarine, nut, oat, oil palm, oil seed rape, okra, onion, orange, an ornamental plant, papaya, parsley, pea, peach, peanut, pear, pepper, persimmon, pine, pineapple, plantain, plum, pomegranate, poplar, potato, pumpkin, quince, radiata pine, radicchio, radish, raspberry, rice, rye, sorghum, Southern pine, soybean, spinach, squash, strawberry, sugarbeet, sugarcane, sunflower, sweet potato, sweetgum, tangerine, tea, tobacco, tomato, triticale, turf, turnip, a vine, watermelon, wheat, yams and zucchini, safflower, coconut palm.

6. A plant cell comprising the construct of Claim 1, wherein the plant is selected from rapeseed (Canola and High Erucic Acid varieties), sunflower, safflower, cotton, soybean, peanut, coconut, oil palms and corn.

7. A plant comprising a cell of Claims 5 or 6.

8. A feed composition comprising a plant according to Claim 7.

9. A seed comprising a cell of Claims 5 or 6.

10. A method of producing oil using a seed of Claim 9.

11. The method of Claim 10, wherein the oil is a natural tocopherol rich refined and deodorised oil which has been produced by a method of treating an oil by distilling under a pressure of less than 0.060 mbar and a temperature of less than 200°C, wherein said refined oil has reduced free fatty acids and a substantial percentage of tocopherol present in the pretreated oil.

12. The method of Claim 11, wherein the pretreated oil is crude or pretreated soybean oil.

13. The method of Claim 11, wherein the refined oil is degummed and bleached.

14. A method for the alteration of the isoprenoid content in a host cell, said method comprising; transforming said host cell with the construct of claim 1, wherein said isoprenoid compound is selected from the group of tocopherols and tocotrienols.

15. A method for producing an isoprenoid compound of interest in a host cell, said method comprising obtaining a transformed host cell into which the construct of Claim 1 has been introduced, said host cell having and expressing in its genome: said construct;
wherein said isoprenoid compound is selected from the group consisting of tocopherols and tocotrienols.

16. A method for increasing the biosynthetic flux in a host cell toward production of an isoprenoid compound, said method comprising; transforming said host cell with the construct of Claim 1, wherein said isoprenoid compound is selected from the group of tocopherols and tocotrienols.

17. The method according to Claim 15, wherein said host cell is a plant cell.

18. The method according to Claim 17, wherein said plant cell is obtained from a plant selected from the group consisting of *Arabidopsis,* soybean, corn, rice, wheat, leek canola, leek, cotton, and tomato.

19. The method according to Claim 17, wherein said transcriptional initiation region is a seed-specific promoter and the method is for increasing the biosynthetic flux in a host cell toward production of an isoprenoid compound.

## Patentansprüche

1. Nukleinsäure, die für eine Tocopherol-Cyclase kodiert, welche eine Aminosäuresequenz mit mindestens 90 % Identität zu der Aminosäuresequenz von SEQ ID NO: 39 oder SEQ ID NO: 110 umfasst, und die als in funktionsfähiger Weise verknüpfte Bestandteile eine in pflanzlichen Wirtszellen funktionstüchtige Transkriptionsinitiationsregion, eine für eine Tocopherol-Cyclase kodierende Nukleinsäuresequenz und eine Transkriptionsterminationsregion umfasst.

2. Nukleinsäure gemäß Anspruch 1, bei der die Tocopherol-Cyclase bei der Zyklisierung von 2,3-Dimethyl-5-phytylplastochinol zu Tocopherol aktiv ist.

3. Nukleinsäure gemäß Anspruch 1, bei der die Tocopherol-Cyclase aktiv bei der Zyklisierung von 2,3-Dimethyl-5-geranylgeranylplastochinol zu Tocotrienol ist.

4. Nukleinsäure gemäß Anspruch 1, die eine Sequenz nach SEQ ID NO: 38 oder SEQ ID NO: 109 umfasst.

5. Pflanzenzelle, die das Konstrukt von Anspruch 1 umfasst, wobei die Pflanze ausgewählt ist aus der Gruppe von Acacia, Alfalfa, Dill, Apfel, Aprikose, Artischocke, Rukola, Spargel, Avocado, Banane, Gerste, Bohnen, Rübe, Brombeere, Blaubeere, Broccoli, Rosenkohl, Kohl, Canola, Melone, Karotte, Cassava, Blumenkohl, Sellerie, Kirsche, Chicoree, Koriander, Zitrus, Clementinen, Kaffee, Mais, Baumwolle, Gurke, Douglasie, Aubergine, Endivie, Eskariol, Eukalyptus, Fenchel, Feigen, Knoblauch, Kürbis, Traube, Grapefruit, Honigtau, Yambohne, Kiwi, Salat, Lauch, Zitrone, Limette, Weihrauch-Kiefer (punis staeda), Mango, Melone, Champignon, Nektarine, Nuss, Hafer, Ölpalme, Ölsamenraps, Eibisch, Zwiebel, Orange, eine Zierpflanze, Papaya, Petersilie, Erbse, Pfirsich, Erdnuss, Birne, Pfeffer, Persimone, Kiefer, Ananas, Kochbanane, Pflaume, Granatapfel, Pappel, Kartoffel, Kürbis, Quitte, Radiatakiefer, Radicchio, Rettich, Himbeere, Reis, Roggen, Sorghum, Südkiefer, Sojabohne, Spinat, Squash-Kürbis, Erdbeere, Zuckerrübe, Zukkerrohr, Sonnenblume, Süßkartoffel, Amberbaum, Mandarine, Tee, Tabak, Tomate, Triticale, Rasen, Weißrübe, ein Wein, Wassermelone, Weizen, Yamswurzeln und Zucchini, Distel, Kokosnuss-Palme.

6. Pflanzliche Zelle, die das Konstrukt nach Anspruch 1 umfasst, wobei die Pflanze ausgewählt ist aus Raps (Canola und Sorten mit einem hohen Anteil von Erucasäure), Sonnenblume, Distel, Baumwolle, Sojabohne, Erdnuss, Kokosnuß, Ölpalmen und Mais.

7. Pflanze, die eine Zelle nach den Ansprüchen 5 oder 6 umfasst.

8. Nahrungszusammensetzung, die eine Pflanze nach Anspruch 7 umfasst.

9. Samen, der eine Zelle nach den Ansprüchen 5 oder 6 umfasst.

10. Verfahren zur Herstellung von Öl unter Verwendung eines Samens nach Anspruch 9.

11. Verfahren nach Anspruch 10, wobei das Öl ein natürliches Tocopherol-reiches, raffiniertes oder deodorierendes Öl ist, das durch ein Verfahren hergestellt worden ist, bei dem ein Öl durch Destillieren unter einem Druck von weniger als 0,060 mbar und bei einer Temperatur von weniger als 200 °C behandelt worden ist, wobei das raffinierte Öl reduzierte freie Fettsäuren und einen wesentlichen prozentualen Anteil des in dem vorbehandelten Öl vorhandenen Tocopherols aufweist.

12. Verfahren nach Anspruch 11, bei dem das vorbehandelte Öl ungereinigtes Sojabohnenöl oder vorbehandeltes Sojabohnenöl ist.

13. Verfahren nach Anspruch 11, bei dem das raffinierte Öl degummiert und gebleicht ist.

14. Verfahren zur Veränderung des Isoprenoid-Gehalts in einer Wirtszelle, bei dem man die Wirtszelle mit einem Konstrukt nach Anspruch 1 transformiert, wobei die Isoprenoid-Verbindung ausgewählt ist aus der Gruppe von Tocopherolen und Tocotrienolen.

15. Verfahren zur Herstellung einer gewünschten Isoprenoid-Verbindung in einer Wirtszelle, bei dem man eine transformierte Wirtszelle erhält, in die das Konstrukt nach Anspruch 1 eingebracht worden ist, wobei die Wirtszelle dieses Konstrukt in ihrem Genom enthält und dieses exprimiert, wobei die Isoprenoid-Verbindung ausgewählt ist aus der Gruppe bestehend aus Tocopherolen und Tocotrienolen.

16. Verfahren zur Erhöhung des biosynthetischen Flusses in einer Wirtszelle in Richtung der Herstellung einer Isoprenoid-Verbindung, bei dem man die Wirtszelle mit einem Konstrukt nach Anspruch 1 transformiert, wobei die Isoprenoid-Verbindung ausgewählt ist aus der Gruppe von Tocopherolen und Tocotrienolen.

17. Verfahren nach Anspruch 15, bei dem die Wirtszelle eine Pflanzenzelle ist.

18. Verfahren nach Anspruch 17, bei die Pflanzenzelle aus einer Pflanze erhalten wird, die ausgewählt ist aus der Gruppe bestehend aus *Arabidopsis,* Sojabohne, Mais, Reis, Weizen, Lauch, Canola, Lauch, Baumwolle und Tomate.

19. Verfahren nach Anspruch 17, bei dem die Transkriptionsinitiationsregion ein samenspezifischer Promotor ist und das Verfahren der Erhöhung des biosynthetischen Flusses in Richtung der Herstellung einer Isoprenoid-Verbindung in einer Wirtszelle dient.

## Revendications

1. Acide nucléique codant une tocophérol cyclase comprenant une séquence d'acide aminé ayant au moins 90 % d'identité avec la séquence d'acide aminé de SEQ ID n° 39 ou de SEQ ID n° 110, comprenant, comme composants liés opérationnellement, une région d'initiation de la transcription fonctionnelle dans une cellule végétale hôte, une séquence d'acide nucléique codant la tocophérol cyclase, et une région de terminaison de la transcription.

2. Acide nucléique selon la revendication 1, dans lequel ladite tocophérol cyclase est active dans la cyclisation du 2, 3-diméthyl-5-phytylplastoquinol en tocophérol.

3. Acide nucléique selon la revendication 1, dans laquel ladite tocophérol cyclase est active dans la cyclisation du 2, 3-diméthyl-5-géranylgéranylplastoquinol en tocotriénol.

4. Acide nucléique selon la revendication 1, comprenant une séquence de SEQ ID n° 38 ou SEQ ID n° 109.

5. Cellule végétale comprenant la construction selon la revendication 1, où la plante est choisie dans le groupe des *Acacia,* de la luzerne, de l'aneth, de la pomme, dé l'abricot, de l'artichaut, de la roquette, de l'asperge, de l'avocat, de la banane, de l'orge, des haricots, de la betterave, de la mûre, de la myrtille, des brocoli, des choux de Bruxelles, du chou, de la canola, du cantaloup, de la carotte, du manioc, du chou-fleur, du céleri, de la cerise, de la chicorée, de la coriandre fraîche, du citron, des clémentines, du café, du maïs, du coton, du concombre, du sapin de Douglas, de l'aubergine, de l'endive, de l'escarole, de l'eucalyptus, du fenouil, des figues, de l'ail, de la gourde, du raisin, du pamplemousse, de la miellée, du jicama, du kiwi, de la laitue, des poireaux, du citron, du lime, du pin à encens, de la mangue, du melon, du champignon, du brugnon, de la noisette, de l'avoine, du palmier à huile, de la graine d'huile de colza, du gombo, de l'oignon, de l'orange d'une plante ornementale, de la papaye, du persil, du pois, de la pêche, de la cacahuète, de la poire, du poivre, du kaki, du pin, de l'ananas, du plantain, de la prune, de la grenade, du peuplier, de la pomme de terre, du potiron, du coing, du pin radiata, de la trévise, du radis, de la framboise, du riz, du seigle, du sorgho, du pin des marais, du soja, de l'épinard, de la courge, de la fraise, de la betterave à sucre, de la canne à sucre, du tournesol, de la patate douce, du noyer satiné, de la mandarine, du thé, du tabac, de la tomate, du triticale, du gazon, du navet, d'une vigne, de la pastèque, du blé, de l'igname et de la courgette, de la carthame, du cocotier.

6. Plante comprenant la construction selon la revendication 1, où la plante est choisie parmi le colza (variétés de canola et riches en acide érucique), le tournesol, la carthame, le coton, le soja, la cacahuète, la noix de coco, les palmiers à huile et le maïs.

7. Plante comprenant une cellule selon les revendications 5 ou 6.

8. Composition alimentaire comprenant une plante selon la revendication 7.

9. Graine comprenant une cellule selon les revendications 5 ou 6.

10. Procédé de production d'une huile en utilisant une graine selon la revendication 9.

11. Procédé selon la revendication 10, dans lequel l'huile est une huile raffinée et désodorisée riche en tocophérol naturel qui a été produite par un procédé de traitement d'une huile par distillation sous une pression de moins de 0,060 mbar et à une température de moins de 200°C, dans lequel ladite huile raffinée a moins d'acides gras libres et un pourcentage important de tocophérol présent dans l'huile pré-traitée.

12. Procédé selon la revendication 11, dans lequel l'huile pré-traitée est une huile de soja brute ou pré-traitée.

13. Procédé selon la revendication 11, dans lequel l'huile raffinée est mi-cuite et décolorée.

14. Procédé pour la modification de la teneur en isoprénoïdes dans une cellule hôte, ledit procédé comprenant : transformer ladite cellule hôte avec la construction selon la revendication 1, dans lequel ledit composé isoprénoïde est choisi dans le groupe des tocophérols et des tocotriénols.

15. Procédé de production d'un composé isoprénoïde d'intérêt dans une cellule hôte, ledit procédé comprenant l'obtention d'une cellule hôte transformée dans laquelle la construction selon la revendication 1 a été introduite, ladite cellule hôte ayant et exprimant dans son génome ladite construction ;
dans lequel ledit composé isoprénoïde est choisi dans le groupe constitué des tocophérols et des tocotriénols.

16. Procédé d'augmentation du flux biosynthétique dans une cellule hôte vers la production d'un composé isoprénoïde, ledit procédé comprenant de : transformer ladite cellule hôte avec la construction selon la revendication 1, dans lequel ledit composé isoprénoïde est choisi dans le groupe des tocophérols et de tocotriénols.

17. Procédé selon la revendication 15, dans lequel la cellule hôte est une cellule végétale.

18. Procédé selon la revendication 17, dans lequel ladite cellule végétale est obtenue à partir d'une plante choisie dans le groupe constitué par *Arabidopsis,* le soja, le maïs, le riz, le blé, la canola de poireau, le poireau, le coton et la tomate.

19. Procédé selon la revendication 17, dans lequel ladite région d'initiation de la transcription est un promoteur spécifique de la graine et le procédé est destiné à augmenter le flux biosynthétique dans une cellule hôte vers la production d'un composé isoprénoïde.
